# EUROPEAN PATENT SPECIFICATION

(11) **EP 3 126 394 B1**
(45) Date of publication and mention of the grant of the patent: **30.10.2019**
(21) Application number: 15716335.3
(22) Date of filing: 30.03.2015
(51) Int. Cl.: C07K 16/28

(54) **ANTI-OX40 ANTIBODIES AND METHODS OF USE**
ANTI-OX40-ANTIKÖRPER UND VERFAHREN ZUR VERWENDUNG
ANTICORPS ANTI-OX40 ET PROCÉDÉS D'UTILISATION CORRESPONDANTS

(30) Priority: 31.03.2014 US 201461973193 P; 06.05.2014 US 201461989448 P; 31.10.2014 US 201462073873 P; 14.11.2014 US 201462080171 P
(43) Date of publication of application: 08.02.2017
(60) Divisional application: 19183548.7
(73) Proprietor: F. Hoffmann-La Roche AG, 4070 Basel (CH)
(72) Inventor: DU, Changchun, South San Francisco, CA 94080 (US); KIM, Jeong, South San Francisco, CA 94080 (US); ZHU, Jing, South San Francisco, CA 94080 (US); BEVERS, Jack III, South San Francisco, CA 94080 (US); WALSH, Kevin, South San Francisco, CA 94080 (US); DE ALMEIDA, Patricia, South San Francisco, CA 94080 (US); ANDYA, James, South San Francisco, CA 94080 (US); SHEN, Ye, South San Francisco, CA 94080 (US)
(74) Representative: Klostermeyer-Rauber, Dörte
(86) International application number: PCT/US2015/023432
(87) International publication number: WO 2015/153513

(56) References cited:
- WO-A1-2009/079335
- WO-A2-03/106498
- XIE F ET AL: "Characterization and application of two novel monoclonal antibodies against human OX40: costimulation of T cells and expression on tumor as well as normal gland tissues", TISSUE ANTIGENS, MUNKSGAARD, COPENHAGEN, DK, vol. 67, no. 4, 1 April 2006 (2006-04-01), pages 307-317, XP002525127, ISSN: 0001-2815, DOI: 10.1111/J.1399-0039.2006.00584.X
- K. S. VOO ET AL: "Antibodies Targeting Human OX40 Expand Effector T Cells and Block Inducible and Natural Regulatory T Cell Function", THE JOURNAL OF IMMUNOLOGY, vol. 191, no. 7, 6 September 2013 (2013-09-06), pages 3641-3650, XP055194259, ISSN: 0022-1767, DOI: 10.4049/jimmunol.1202752
- B. D. CURTI ET AL: "OX40 Is a Potent Immune-Stimulating Target in Late-Stage Cancer Patients", CANCER RESEARCH, vol. 73, no. 24, 31 October 2013 (2013-10-31), pages 7189-7198, XP055194254, ISSN: 0008-5472, DOI: 10.1158/0008-5472.CAN-12-4174
- ZHIQIANG GUO ET AL: "PD-1 Blockade and OX40 Triggering Synergistically Protects against Tumor Growth in a Murine Model of Ovarian Cancer", PLOS ONE, vol. 9, no. 2, 27 February 2014 (2014-02-27), page e89350, XP055132840, ISSN: 1932-6203, DOI: 10.1371/journal.pone.0089350

### SEQUENCE LISTING

<110> Genentech, Inc. F. HOFFMANN-LA ROCHE AG DU, Changchun KIM, Jeong ZHU, Jing BEVERS III, Jack WALSH, Kevin DE ALMEIDA, Patricia ANDYA, James SHEN, Ye
<120> ANTI-OX40 ANTIBODIES AND METHODS OF USE
<130> 146392029140
<140> Not Yet Assigned
   <141> Concurrently Herewith
<150> US 61/973,193
   <151> 2014-03-31
<150> US 61/989,448
   <151> 2014-05-06
<150> US 62/073,873
   <151> 2014-10-31
<150> US 62/080,171
   <151> 2014-11-14
<160> 209
<170> FastSEQ for Windows Version 4.0
<210> 1
   <211> 249
   <212> PRT
   <213> Homo sapiens
<400> 1
   Leu His Cys Val Gly Asp Thr Tyr Pro Ser Asn Asp Arg Cys Cys His
    1               5                  10                  15
   Glu Cys Arg Pro Gly Asn Gly Met Val Ser Arg Cys Ser Arg Ser Gln
               20                  25                  30
   Asn Thr Val Cys Arg Pro Cys Gly Pro Gly Phe Tyr Asn Asp Val Val
           35                  40                  45
   Ser Ser Lys Pro Cys Lys Pro Cys Thr Trp Cys Asn Leu Arg Ser Gly
       50                  55                  60
   Ser Glu Arg Lys Gln Leu Cys Thr Ala Thr Gln Asp Thr Val Cys Arg
   65                  70                  75                  80
   Cys Arg Ala Gly Thr Gln Pro Leu Asp Ser Tyr Lys Pro Gly Val Asp
                   85                  90                  95
   Cys Ala Pro Cys Pro Pro Gly His Phe Ser Pro Gly Asp Asn Gln Ala
               100                 105                 110
   Cys Lys Pro Trp Thr Asn Cys Thr Leu Ala Gly Lys His Thr Leu Gln
           115                 120                 125
   Pro Ala Ser Asn Ser Ser Asp Ala Ile Cys Glu Asp Arg Asp Pro Pro
       130                 135                 140
   Ala Thr Gln Pro Gln Glu Thr Gln Gly Pro Pro Ala Arg Pro Ile Thr
   145                 150                 155                 160
   Val Gln Pro Thr Glu Ala Trp Pro Arg Thr Ser Gln Gly Pro Ser Thr
                   165                 170                 175
   Arg Pro Val Glu Val Pro Gly Gly Arg Ala Val Ala Ala Ile Leu Gly
               180                 185                 190
   Leu Gly Leu Val Leu Gly Leu Leu Gly Pro Leu Ala Ile Leu Leu Ala
           195                 200                 205
   Leu Tyr Leu Leu Arg Arg Asp Gln Arg Leu Pro Pro Asp Ala His Lys
       210                 215                 220
   Pro Pro Gly Gly Gly Ser Phe Arg Thr Pro Ile Gln Glu Glu Gln Ala
   225                 230                 235                 240
   Asp Ala His Ser Thr Leu Ala Lys Ile
                   245
<210> 2
   <211> 5
   <212> PRT
   <213> Artificial Sequence
<220>
   <223> Synthetic Construct
<400> 2
   Asp Ser Tyr Met Ser
    1               5
<210> 3
   <211> 17
   <212> PRT
   <213> Artificial Sequence
<220>
   <223> Synthetic Construct
<400> 3
   Asp Met Tyr Pro Asp Asn Gly Asp Ser Ser Tyr Asn Gln Lys Phe Arg
   1 5 10 15
   Glu
<210> 4
   <211> 8
   <212> PRT
   <213> Artificial Sequence
<220>
   <223> Synthetic Construct
<400> 4
   Ala Pro Arg Trp Tyr Phe Ser Val
   1 5
<210> 5
   <211> 11
   <212> PRT
   <213> Artificial Sequence
<220>
   <223> Synthetic Construct
<400> 5
   Arg Ala Ser Gln Asp Ile Ser Asn Tyr Leu Asn
   1 5 10
<210> 6
   <211> 7
   <212> PRT
   <213> Artificial Sequence
<220>
   <223> Synthetic Construct
<400> 6
   Tyr Thr Ser Arg Leu Arg Ser
   1 5
<210> 7
   <211> 9
   <212> PRT
   <213> Artificial Sequence
<220>
   <223> Synthetic Construct
<400> 7
   Gln Gln Gly His Thr Leu Pro Pro Thr
   1 5
<210> 8
   <211> 5
   <212> PRT
   <213> Artificial Sequence
<220>
   <223> Synthetic Construct
<400> 8
   Asp Ala Tyr Met Ser
   1 5
<210> 9
   <211> 5
   <212> PRT
   <213> Artificial Sequence
<220>
   <223> Synthetic Construct
<400> 9
   Glu Ser Tyr Met Ser
   1 5
<210> 10
   <211> 17
   <212> PRT
   <213> Artificial Sequence
<220>
   <223> Synthetic Construct
<400> 10
   Asp Met Tyr Pro Asp Asn Ala Asp Ser Ser Tyr Asn Gln Lys Phe Arg
   1 5 10 15
   Glu
<210> 11
   <211> 17
   <212> PRT
   <213> Artificial Sequence
<220>
   <223> Synthetic Construct
<400> 11
   Asp Met Tyr Pro Asp Asn Ala Asp Ala Ser Tyr Asn Gln Lys Phe Arg
   1 5 10 15
   Glu
<210> 12
   <211> 17
   <212> PRT
   <213> Artificial Sequence
<220>
   <223> Synthetic Construct
<400> 12
   Asp Met Tyr Pro Asp Asn Gly Asp Ala Ser Tyr Asn Gln Lys Phe Arg
    1              5                   10                  15
   Glu
<210> 13
   <211> 17
   <212> PRT
   <213> Artificial Sequence
<220>
   <223> Synthetic Construct
<400> 13
   Asp Met Tyr Pro Asp Ser Gly Asp Ser Ser Tyr Asn Gln Lys Phe Arg
    1              5                   10                  15
   Glu
<210> 14
   <211> 17
   <212> PRT
   <213> Artificial Sequence
<220>
   <223> Synthetic Construct
<400> 14
   Asp Met Tyr Pro Asp Asn Gly Ser Ser Ser Tyr Asn Gln Lys Phe Arg
    1              5                   10                  15
   Glu
<210> 15
   <211> 8
   <212> PRT
   <213> Artificial Sequence
<220>
   <223> Synthetic Construct
<400> 15
   Ala Pro Arg Trp Tyr Phe Ser Ala
    1              5
<210> 16
   <211> 8
   <212> PRT
   <213> Artificial Sequence
<220>
   <223> Synthetic Construct
<400> 16
   Ala Pro Arg Trp Tyr Ala Ser Val
   1 5
<210> 17
   <211> 8
   <212> PRT
   <213> Artificial Sequence
<220>
   <223> Synthetic Construct
<400> 17
   Ala Pro Arg Trp Ala Phe Ser Val
   1 5
<210> 18
   <211> 8
   <212> PRT
   <213> Artificial Sequence
<220>
   <223> Synthetic Construct
<400> 18
   Ala Pro Ala Trp Tyr Phe Ser Val
   1 5
<210> 19
   <211> 8
   <212> PRT
   <213> Artificial Sequence
<220>
   <223> Synthetic Construct
<400> 19
   Ala Pro Arg Trp Tyr Phe Ala Val
   1 5
<210> 20
   <211> 8
   <212> PRT
   <213> Artificial Sequence
<220>
   <223> Synthetic Construct
<400> 20
   Ala Pro Arg Ala Tyr Phe Ser Val
   1 5
<210> 21
   <211> 8
   <212> PRT
   <213> Artificial Sequence
<220>
   <223> Synthetic Construct
<400> 21
   Ala Ala Arg Trp Tyr Phe Ser Val
   1 5
<210> 22
   <211> 9
   <212> PRT
   <213> Artificial Sequence
<220>
   <223> Synthetic Construct
<400> 22
   Gln Gln Gly His Thr Leu Pro Ala Thr
   1 5
<210> 23
   <211> 9
   <212> PRT
   <213> Artificial Sequence
<220>
   <223> Synthetic Construct
<400> 23
   Gln Gln Gly His Thr Ala Pro Pro Thr
   1 5
<210> 24
   <211> 9
   <212> PRT
   <213> Artificial Sequence
<220>
   <223> Synthetic Construct
<400> 24
   Gln Gln Gly Ala Thr Leu Pro Pro Thr
   1 5
<210> 25
   <211> 9
   <212> PRT
   <213> Artificial Sequence
<220>
   <223> Synthetic Construct
<400> 25
   Gln Gln Gly His Ala Leu Pro Pro Thr
   1 5
<210> 26
   <211> 9
   <212> PRT
   <213> Artificial Sequence
<220>
   <223> Synthetic Construct
<400> 26
   Gln Gln Ala His Thr Leu Pro Pro Thr
   1 5
<210> 27
   <211> 9
   <212> PRT
   <213> Artificial Sequence
<220>
   <223> Synthetic Construct
<400> 27
   Gln Gln Gly His Thr Leu Ala Pro Thr
   1 5
<210> 28
   <211> 9
   <212> PRT
   <213> Artificial Sequence
<220>
   <223> Synthetic Construct
<400> 28
   Gln Ala Gly His Thr Leu Pro Pro Thr
   1 5
<210> 29
   <211> 5
   <212> PRT
   <213> Artificial Sequence
<220>
   <223> Synthetic Construct
<400> 29
   Asn Tyr Leu Ile Glu
   1 5
<210> 30
   <211> 17
   <212> PRT
   <213> Artificial Sequence
<220>
   <223> Synthetic Construct
<400> 30
   Val Ile Asn Pro Gly Ser Gly Asp Thr Tyr Tyr Ser Glu Lys Phe Lys
   1               5                   10                  15
   Gly
<210> 31
   <211> 17
   <212> PRT
   <213> Artificial Sequence
<220>
   <223> Synthetic Construct
<400> 31
   Val Ile Asn Pro Gly Ser Gly Asp Ala Tyr Tyr Ser Glu Lys Phe Lys
   1               5                   10                  15
   Gly
<210> 32
   <211> 17
   <212> PRT
   <213> Artificial Sequence
<220>
   <223> Synthetic Construct
<400> 32
   Val Ile Asn Pro Gly Ser Gly Asp Gln Tyr Tyr Ser Glu Lys Phe Lys
   1               5                   10                  15
   Gly
<210> 33
   <211> 5
   <212> PRT
   <213> Artificial Sequence
<220>
   <223> Synthetic Construct
<400> 33
   Asp Arg Leu Asp Tyr
   1               5
<210> 34
   <211> 5
   <212> PRT
   <213> Artificial Sequence
<220>
   <223> Synthetic Construct
<400> 34
   Ala Arg Leu Asp Tyr
   1               5
<210> 35
   <211> 5
   <212> PRT
   <213> Artificial Sequence
<220>
   <223> Synthetic Construct
<400> 35
   Asp Ala Leu Asp Tyr
   1               5
<210> 36
   <211> 5
   <212> PRT
   <213> Artificial Sequence
<220>
   <223> Synthetic Construct
<400> 36
   Asp Arg Ala Asp Tyr
   1               5
<210> 37
   <211> 11
   <212> PRT
   <213> Artificial Sequence
<220>
   <223> Synthetic Construct
<400> 37
   His Ala Ser Gln Asp Ile Ser Ser Tyr Ile Val
   1               5                   10
<210> 38
   <211> 7
   <212> PRT
   <213> Artificial Sequence
<220>
   <223> Synthetic Construct
<400> 38
   His Gly Thr Asn Leu Glu Asp
   1 5
<210> 39
   <211> 7
   <212> PRT
   <213> Artificial Sequence
<220>
   <223> Synthetic Construct
<400> 39
   His Gly Thr Asn Leu Glu Ser
   1 5
<210> 40
   <211> 7
   <212> PRT
   <213> Artificial Sequence
<220>
   <223> Synthetic Construct
<400> 40
   His Gly Thr Asn Leu Glu Glu
   1 5
<210> 41
   <211> 7
   <212> PRT
   <213> Artificial Sequence
<220>
   <223> Synthetic Construct
<400> 41
   His Gly Thr Asn Leu Glu Gln
   1 5
<210> 42
   <211> 9
   <212> PRT
   <213> Artificial Sequence
<220>
   <223> Synthetic Construct
<400> 42
   Val His Tyr Ala Gln Phe Pro Tyr Thr
   1 5
<210> 43
   <211> 9
   <212> PRT
   <213> Artificial Sequence
<220>
   <223> Synthetic Construct
<400> 43
   Ala His Tyr Ala Gln Phe Pro Tyr Thr
   1 5
<210> 44
   <211> 9
   <212> PRT
   <213> Artificial Sequence
<220>
   <223> Synthetic Construct
<400> 44
   Val Ala Tyr Ala Gln Phe Pro Tyr Thr
   1 5
<210> 45
   <211> 9
   <212> PRT
   <213> Artificial Sequence
<220>
   <223> Synthetic Construct
<400> 45
   Val His Ala Ala Gln Phe Pro Tyr Thr
   1 5
<210> 46
   <211> 9
   <212> PRT
   <213> Artificial Sequence
<220>
   <223> Synthetic Construct
<400> 46
   Val His Tyr Ala Ala Phe Pro Tyr Thr
   1 5
<210> 47
   <211> 9
   <212> PRT
   <213> Artificial Sequence
<220>
   <223> Synthetic Construct
<400> 47
   Val His Tyr Ala Gln Ala Pro Tyr Thr
   1 5
<210> 48
   <211> 9
   <212> PRT
   <213> Artificial Sequence
<220>
   <223> Synthetic Construct
<400> 48
   Val His Tyr Ala Gln Phe Ala Tyr Thr
   1 5
<210> 49
   <211> 9
   <212> PRT
   <213> Artificial Sequence
<220>
   <223> Synthetic Construct
<400> 49
   Val His Tyr Ala Gln Phe Pro Ala Thr
   1 5
<210> 50
   <211> 5
   <212> PRT
   <213> Artificial Sequence
<220>
   <223> Synthetic Construct
<400> 50
   Asp Tyr Gly Val Leu
   1 5
<210> 51
   <211> 16
   <212> PRT
   <213> Artificial Sequence
<220>
   <223> Synthetic Construct
<400> 51
   Met Ile Trp Ser Gly Gly Thr Thr Asp Tyr Asn Ala Ala Phe Ile Ser
   1 5 10 15
<210> 52
   <211> 5
   <212> PRT
   <213> Artificial Sequence
<220>
   <223> Synthetic Construct
<400> 52
   Glu Glu Met Asp Tyr
   1               5
<210> 53
   <211> 11
   <212> PRT
   <213> Artificial Sequence
<220>
   <223> Synthetic Construct
<400> 53
   Arg Ala Ser Gln Asp Ile Ser Asn Phe Leu Asn
   1               5                   10
<210> 54
   <211> 7
   <212> PRT
   <213> Artificial Sequence
<220>
   <223> Synthetic Construct
<400> 54
   Tyr Thr Ser Arg Leu His Ser
   1               5
<210> 55
   <211> 9
   <212> PRT
   <213> Artificial Sequence
<220>
   <223> Synthetic Construct
<400> 55
   Gln Gln Gly Asn Thr Leu Pro Trp Thr
   1               5
<210> 56
   <211> 117
   <212> PRT
   <213> Artificial Sequence
<220>
   <223> Synthetic Construct
<400> 56
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 57
   <211> 107
   <212> PRT
   <213> Artificial Sequence
<220>
   <223> Synthetic Construct
<400> 57
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 58
   <211> 117
   <212> PRT
   <213> Artificial Sequence
<220>
   <223> Synthetic Construct
<400> 58
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Ser
   20 25 30
   Tyr Met Ser Trp Val Arg Gln Ala Pro Gly Gln Gly Leu Glu Trp Ile
   35 40 45
   Gly Asp Met Tyr Pro Asp Asn Gly Asp Ser Ser Tyr Asn Gln Lys Phe
   50 55 60
   Arg Glu Arg Val Thr Ile Thr Val Asp Thr Ser Thr Ser Thr Ala Tyr
   65 70 75 80
   Leu Glu Leu Ser Ser Leu Arg Ser Glu Asp Thr Ala Val Tyr Tyr Cys
   85 90 95
   Val Leu Ala Pro Arg Trp Tyr Phe Ser Val Trp Gly Gln Gly Thr Leu
   100 105 110
   Val Thr Val Ser Ser
   115
<210> 59
   <211> 107
   <212> PRT
   <213> Artificial Sequence
<220>
   <223> Synthetic Construct
<400> 59
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Ser Asn Tyr
   20 25 30
   Leu Asn Trp Tyr Gln Gln Lys Pro Gly Lys Ala Pro Lys Leu Leu Ile
   35 40 45
   Tyr Tyr Thr Ser Arg Leu Arg Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Gly His Thr Leu Pro Pro
   85 90 95
   Thr Phe Gly Gln Gly Thr Lys Val Glu Ile Lys
   100 105
<210> 60
   <211> 117
   <212> PRT
   <213> Artificial Sequence
<220>
   <223> Synthetic Construct
<400> 60
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Leu Thr Val Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 61
   <211> 107
   <212> PRT
   <213> Artificial Sequence
<220>
   <223> Synthetic Construct
<400> 61
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 62
   <211> 117
   <212> PRT
   <213> Artificial Sequence
<220>
   <223> Synthetic Construct
<400> 62
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Val Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 63
   <211> 107
   <212> PRT
   <213> Artificial Sequence
<220>
   <223> Synthetic Construct
<400> 63
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Thr Val Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 64
   <211> 117
   <212> PRT
   <213> Artificial Sequence
<220>
   <223> Synthetic Construct
<400> 64
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Val Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 65
   <211> 107
   <212> PRT
   <213> Artificial Sequence
<220>
   <223> Synthetic Construct
<400> 65
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Thr Val Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 66
   <211> 117
   <212> PRT
   <213> Artificial Sequence
<220>
   <223> Synthetic Construct
<400> 66
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
   35 40 45
   Gly Asp Met Tyr Pro Asp Asn Gly Asp Ser Ser Tyr Asn Gln Lys Phe
   50 55 60
   Arg Glu Arg Val Thr Ile Thr Val Asp Thr Ser Thr Ser Thr Ala Tyr
   65 70 75 80
   Leu Glu Leu Ser Ser Leu Arg Ser Glu Asp Thr Ala Val Tyr Tyr Cys
   85 90 95
   Val Leu Ala Pro Arg Trp Tyr Phe Ser Val Trp Gly Gln Gly Thr Leu
   100 105 110
   Val Thr Val Ser Ser
   115
<210> 67
   <211> 107
   <212> PRT
   <213> Artificial Sequence
<220>
   <223> Synthetic Construct
<400> 67
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Ser Asn Tyr
   20 25 30
   Leu Asn Trp Tyr Gln Gln Lys Pro Gly Lys Thr Val Lys Leu Leu Ile
   35 40 45
   Tyr Tyr Thr Ser Arg Leu Arg Ser Gly Val Pro Ser Arg Phe Ser Gly
   50 55 60
   Ser Gly Ser Gly Lys Asp Tyr Thr Leu Thr Ile Ser Ser Leu Gln Pro
   65 70 75 80
   Glu Asp Phe Ala Thr Tyr Phe Cys Gln Gln Gly His Thr Leu Pro Pro
   85 90 95
   Thr Phe Gly Gln Gly Thr Lys Val Glu Ile Lys
   100 105
<210> 68
   <211> 117
   <212> PRT
   <213> Artificial Sequence
<220>
   <223> Synthetic Construct
<400> 68
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Val Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
                           115
<210> 69
   <211> 107
   <212> PRT
   <213> Artificial Sequence
<220>
   <223> Synthetic Construct
<400> 69
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Thr Val Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Lys Asp Tyr Thr Leu Thr Ile Ser Ser Leu Gln Pro
   65                  70                  75                  80
   Glu Asp Phe Ala Thr Tyr Phe Cys Gln Gln Gly His Thr Leu Pro Pro
                   85                  90                  95
   Thr Phe Gly Gln Gly Thr Lys Val Glu Ile Lys
               100                 105
<210> 70
   <211> 117
   <212> PRT
   <213> Artificial Sequence
<220>
   <223> Synthetic Construct
<400> 70
   Glu Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Ala
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 71
   <211> 107
   <212> PRT
   <213> Artificial Sequence
<220>
   <223> Synthetic Construct
<400> 71
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 72
   <211> 117
   <212> PRT
   <213> Artificial Sequence
<220>
   <223> Synthetic Construct
<400> 72
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Glu Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 73
   <211> 107
   <212> PRT
   <213> Artificial Sequence
<220>
   <223> Synthetic Construct
<400> 73
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 74
   <211> 117
   <212> PRT
   <213> Artificial Sequence
<220>
   <223> Synthetic Construct
<400> 74
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Ala Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 75
   <211> 107
   <212> PRT
   <213> Artificial Sequence
<220>
   <223> Synthetic Construct
<400> 75
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 76
   <211> 117
   <212> PRT
   <213> Artificial Sequence
<220>
   <223> Synthetic Construct
<400> 76
   Glu Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Ala Asp Ala Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 77
   <211> 107
   <212> PRT
   <213> Artificial Sequence
<220>
   <223> Synthetic Construct
<400> 77
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 78
   <211> 117
   <212> PRT
   <213> Artificial Sequence
<220>
   <223> Synthetic Construct
<400> 78
   Glu Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ala Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 79
   <211> 107
   <212> PRT
   <213> Artificial Sequence
<220>
   <223> Synthetic Construct
<400> 79
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 80
   <211> 117
   <212> PRT
   <213> Artificial Sequence
<220>
   <223> Synthetic Construct
<400> 80
   Glu Val Gln Leu Val Gln Ser Gly Ala Glu Val Lys Lys Pro Gly Ala
    1               5                   10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Ser Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 81
   <211> 107
   <212> PRT
   <213> Artificial Sequence
<220>
   <223> Synthetic Construct
<400> 81
   Asp Ile Gln Met Thr Gln Ser Pro Ser Ser Leu Ser Ala Ser Val Gly
    1               5                   10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 82
   <211> 117
   <212> PRT
   <213> Artificial Sequence
<220>
   <223> Synthetic Construct
<400> 82
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Ser Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 83
   <211> 107
   <212> PRT
   <213> Artificial Sequence
<220>
   <223> Synthetic Construct
<400> 83
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 84
   <211> 117
   <212> PRT
   <213> Artificial Sequence
<220>
   <223> Synthetic Construct
<400> 84
   Glu Val Gln Leu Val Gln Ser Gly Ala Glu Val Lys Lys Pro Gly Ala
    1               5                   10                  15
   Ser Val Lys Val Ser Cys Lys Ala Ser Gly Tyr Thr Phe Thr Asp Ala
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Ala Asp Ala Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 85
   <211> 107
   <212> PRT
   <213> Artificial Sequence
<220>
   <223> Synthetic Construct
<400> 85
   Asp Ile Gln Met Thr Gln Ser Pro Ser Ser Leu Ser Ala Ser Val Gly
    1               5                   10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
                       100                 105
<210> 86
   <211> 117
   <212> PRT
   <213> Artificial Sequence
<220>
   <223> Synthetic Construct
<400> 86
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 87
   <211> 107
   <212> PRT
   <213> Artificial Sequence
<220>
   <223> Synthetic Construct
<400> 87
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Ala
                   85                  90                  95
   Thr Phe Gly Gln Gly Thr Lys Val Glu Ile Lys
               100                 105
<210> 88
   <211> 117
   <212> PRT
   <213> Artificial Sequence
<220>
   <223> Synthetic Construct
<400> 88
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 89
   <211> 107
   <212> PRT
   <213> Artificial Sequence
<220>
   <223> Synthetic Construct
<400> 89
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Ala Pro Pro
                   85                  90                  95
   Thr Phe Gly Gln Gly Thr Lys Val Glu Ile Lys
               100                 105
<210> 90
   <211> 117
   <212> PRT
   <213> Artificial Sequence
<220>
   <223> Synthetic Construct
<400> 90
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 91
   <211> 107
   <212> PRT
   <213> Artificial Sequence
<220>
   <223> Synthetic Construct
<400> 91
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly Ala Thr Leu Pro Pro
                   85                  90                  95
   Thr Phe Gly Gln Gly Thr Lys Val Glu Ile Lys
               100                 105
<210> 92
   <211> 117
   <212> PRT
   <213> Artificial Sequence
<220>
   <223> Synthetic Construct
<400> 92
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 93
   <211> 107
   <212> PRT
   <213> Artificial Sequence
<220>
   <223> Synthetic Construct
<400> 93
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Ala Leu Pro Pro
                   85                  90                  95
   Thr Phe Gly Gln Gly Thr Lys Val Glu Ile Lys
               100                 105
<210> 94
   <211> 117
   <212> PRT
   <213> Artificial Sequence
<220>
   <223> Synthetic Construct
<400> 94
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 95
   <211> 107
   <212> PRT
   <213> Artificial Sequence
<220>
   <223> Synthetic Construct
<400> 95
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ala His Thr Leu Pro Pro
                   85                  90                  95
   Thr Phe Gly Gln Gly Thr Lys Val Glu Ile Lys
               100                 105
<210> 96
   <211> 117
   <212> PRT
   <213> Artificial Sequence
<220>
   <223> Synthetic Construct
<400> 96
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 97
   <211> 107
   <212> PRT
   <213> Artificial Sequence
<220>
   <223> Synthetic Construct
<400> 97
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Ala Pro
                   85                  90                  95
   Thr Phe Gly Gln Gly Thr Lys Val Glu Ile Lys
               100                 105
<210> 98
   <211> 117
   <212> PRT
   <213> Artificial Sequence
<220>
   <223> Synthetic Construct
<400> 98
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Ser
   20 25 30
   Tyr Met Ser Trp Val Arg Gln Ala Pro Gly Gln Gly Leu Glu Trp Ile
   35 40 45
   Gly Asp Met Tyr Pro Asp Asn Gly Asp Ser Ser Tyr Asn Gln Lys Phe
   50 55 60
   Arg Glu Arg Val Thr Ile Thr Arg Asp Thr Ser Thr Ser Thr Ala Tyr
   65 70 75 80
   Leu Glu Leu Ser Ser Leu Arg Ser Glu Asp Thr Ala Val Tyr Tyr Cys
   85 90 95
   Val Leu Ala Pro Arg Trp Tyr Phe Ser Val Trp Gly Gln Gly Thr Leu
   100 105 110
   Val Thr Val Ser Ser
   115
<210> 99
   <211> 107
   <212> PRT
   <213> Artificial Sequence
<220>
   <223> Synthetic Construct
<400> 99
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Ser Asn Tyr
   20 25 30
   Leu Asn Trp Tyr Gln Gln Lys Pro Gly Lys Ala Pro Lys Leu Leu Ile
   35 40 45
   Tyr Tyr Thr Ser Arg Leu Arg Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Ala Gly His Thr Leu Pro Pro
   85 90 95
   Thr Phe Gly Gln Gly Thr Lys Val Glu Ile Lys
   100 105
<210> 100
   <211> 117
   <212> PRT
   <213> Artificial Sequence
<220>
   <223> Synthetic Construct
<400> 100
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Ala Trp Gly Gln Gly Thr Leu
               100                 105                 110
   Val Thr Val Ser Ser
           115
<210> 101
   <211> 107
   <212> PRT
   <213> Artificial Sequence
<220>
   <223> Synthetic Construct
<400> 101
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 102
   <211> 117
   <212> PRT
   <213> Artificial Sequence
<220>
   <223> Synthetic Construct
<400> 102
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Ser
   20 25 30
   Tyr Met Ser Trp Val Arg Gln Ala Pro Gly Gln Gly Leu Glu Trp Ile
   35 40 45
   Gly Asp Met Tyr Pro Asp Asn Gly Asp Ser Ser Tyr Asn Gln Lys Phe
   50 55 60
   Arg Glu Arg Val Thr Ile Thr Arg Asp Thr Ser Thr Ser Thr Ala Tyr
   65 70 75 80
   Leu Glu Leu Ser Ser Leu Arg Ser Glu Asp Thr Ala Val Tyr Tyr Cys
   85 90 95
   Val Leu Ala Pro Arg Trp Tyr Ala Ser Val Trp Gly Gln Gly Thr Leu
   100 105 110
   Val Thr Val Ser Ser
   115
<210> 103
   <211> 107
   <212> PRT
   <213> Artificial Sequence
<220>
   <223> Synthetic Construct
<400> 103
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Ser Asn Tyr
   20 25 30
   Leu Asn Trp Tyr Gln Gln Lys Pro Gly Lys Ala Pro Lys Leu Leu Ile
   35 40 45
   Tyr Tyr Thr Ser Arg Leu Arg Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Gly His Thr Leu Pro Pro
   85 90 95
   Thr Phe Gly Gln Gly Thr Lys Val Glu Ile Lys
   100 105
<210> 104
   <211> 117
   <212> PRT
   <213> Artificial Sequence
<220>
   <223> Synthetic Construct
<400> 104
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Ala Phe Ser Val Trp Gly Gln Gly Thr Leu
               100                 105                 110
   Val Thr Val Ser Ser
           115
<210> 105
   <211> 107
   <212> PRT
   <213> Artificial Sequence
<220>
   <223> Synthetic Construct
<400> 105
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 106
   <211> 117
   <212> PRT
   <213> Artificial Sequence
<220>
   <223> Synthetic Construct
<400> 106
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Ser
   20 25 30
   Tyr Met Ser Trp Val Arg Gln Ala Pro Gly Gln Gly Leu Glu Trp Ile
   35 40 45
   Gly Asp Met Tyr Pro Asp Asn Gly Asp Ser Ser Tyr Asn Gln Lys Phe
   50 55 60
   Arg Glu Arg Val Thr Ile Thr Arg Asp Thr Ser Thr Ser Thr Ala Tyr
   65 70 75 80
   Leu Glu Leu Ser Ser Leu Arg Ser Glu Asp Thr Ala Val Tyr Tyr Cys
   85 90 95
   Val Leu Ala Pro Ala Trp Tyr Phe Ser Val Trp Gly Gln Gly Thr Leu
   100 105 110
   Val Thr Val Ser Ser
   115
<210> 107
   <211> 107
   <212> PRT
   <213> Artificial Sequence
<220>
   <223> Synthetic Construct
<400> 107
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Ser Asn Tyr
   20 25 30
   Leu Asn Trp Tyr Gln Gln Lys Pro Gly Lys Ala Pro Lys Leu Leu Ile
   35 40 45
   Tyr Tyr Thr Ser Arg Leu Arg Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Gly His Thr Leu Pro Pro
   85 90 95
   Thr Phe Gly Gln Gly Thr Lys Val Glu Ile Lys
   100 105
<210> 108
   <211> 117
   <212> PRT
   <213> Artificial Sequence
<220>
   <223> Synthetic Construct
<400> 108
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asp Ser
   20 25 30
   Tyr Met Ser Trp Val Arg Gln Ala Pro Gly Gln Gly Leu Glu Trp Ile
   35 40 45
   Gly Asp Met Tyr Pro Asp Asn Gly Asp Ser Ser Tyr Asn Gln Lys Phe
   50 55 60
   Arg Glu Arg Val Thr Ile Thr Arg Asp Thr Ser Thr Ser Thr Ala Tyr
   65 70 75 80
   Leu Glu Leu Ser Ser Leu Arg Ser Glu Asp Thr Ala Val Tyr Tyr Cys
   85 90 95
   Val Leu Ala Pro Arg Trp Tyr Phe Ala Val Trp Gly Gln Gly Thr Leu
   100 105 110
   Val Thr Val Ser Ser
   115
<210> 109
   <211> 107
   <212> PRT
   <213> Artificial Sequence
<220>
   <223> Synthetic Construct
<400> 109
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Ser Asn Tyr
   20 25 30
   Leu Asn Trp Tyr Gln Gln Lys Pro Gly Lys Ala Pro Lys Leu Leu Ile
   35 40 45
   Tyr Tyr Thr Ser Arg Leu Arg Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Gly His Thr Leu Pro Pro
   85 90 95
   Thr Phe Gly Gln Gly Thr Lys Val Glu Ile Lys
   100 105
<210> 110
   <211> 117
   <212> PRT
   <213> Artificial Sequence
<220>
   <223> Synthetic Construct
<400> 110
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Ala Tyr Phe Ser Val Trp Gly Gln Gly Thr Leu
               100                 105                 110
   Val Thr Val Ser Ser
           115
<210> 111
   <211> 107
   <212> PRT
   <213> Artificial Sequence
<220>
   <223> Synthetic Construct
<400> 111
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 112
   <211> 117
   <212> PRT
   <213> Artificial Sequence
<220>
   <223> Synthetic Construct
<400> 112
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Ala Arg Trp Tyr Phe Ser Val Trp Gly Gln Gly Thr Leu
               100                 105                 110
   Val Thr Val Ser Ser
           115
<210> 113
   <211> 107
   <212> PRT
   <213> Artificial Sequence
<220>
   <223> Synthetic Construct
<400> 113
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 114
   <211> 117
   <212> PRT
   <213> Artificial Sequence
<220>
   <223> Synthetic Construct
<400> 114
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Leu Ala Pro Arg Trp Tyr Phe Ser Val Trp Gly Gln Gly Thr Leu
               100                 105                 110
   Val Thr Val Ser Ser
           115
<210> 115
   <211> 107
   <212> PRT
   <213> Artificial Sequence
<220>
   <223> Synthetic Construct
<400> 115
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 116
   <211> 117
   <212> PRT
   <213> Artificial Sequence
<220>
   <223> Synthetic Construct
<400> 116
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Ala Ala Pro Arg Trp Tyr Phe Ser Val Trp Gly Gln Gly Thr Leu
               100                 105                 110
   Val Thr Val Ser Ser
           115
<210> 117
   <211> 107
   <212> PRT
   <213> Artificial Sequence
<220>
   <223> Synthetic Construct
<400> 117
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly His Thr Leu Pro Pro
                   85                  90                  95
   Thr Phe Gly Gln Gly Thr Lys Val Glu Ile Lys
               100                 105
<210> 118
   <211> 114
   <212> PRT
   <213> Artificial Sequence
<220>
   <223> Synthetic Construct
<400> 118
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Ile Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 119
   <211> 107
   <212> PRT
   <213> Artificial Sequence
<220>
   <223> Synthetic Construct
<400> 119
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ala Pro Lys Leu Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 120
   <211> 114
   <212> PRT
   <213> Artificial Sequence
<220>
   <223> Synthetic Construct
<400> 120
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Ile Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 121
   <211> 107
   <212> PRT
   <213> Artificial Sequence
<220>
   <223> Synthetic Construct
<400> 121
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ala Pro Lys Leu Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 122
   <211> 114
   <212> PRT
   <213> Artificial Sequence
<220>
   <223> Synthetic Construct
<400> 122
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 123
   <211> 107
   <212> PRT
   <213> Artificial Sequence
<220>
   <223> Synthetic Construct
<400> 123
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ala Pro Lys Leu Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 124
   <211> 114
   <212> PRT
   <213> Artificial Sequence
<220>
   <223> Synthetic Construct
<400> 124
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Ile Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 125
   <211> 107
   <212> PRT
   <213> Artificial Sequence
<220>
   <223> Synthetic Construct
<400> 125
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 126
   <211> 114
   <212> PRT
   <213> Artificial Sequence
<220>
   <223> Synthetic Construct
<400> 126
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 127
   <211> 107
   <212> PRT
   <213> Artificial Sequence
<220>
   <223> Synthetic Construct
<400> 127
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 128
   <211> 114
   <212> PRT
   <213> Artificial Sequence
<220>
   <223> Synthetic Construct
<400> 128
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Ala Phe Thr Asn Tyr
   20 25 30
   Leu Ile Glu Trp Val Arg Gln Ala Pro Gly Gln Gly Leu Glu Trp Ile
   35 40 45
   Gly Val Ile Asn Pro Gly Ser Gly Asp Thr Tyr Tyr Ser Glu Lys Phe
   50 55 60
   Lys Gly Arg Val Thr Leu Thr Ala Asp Thr Ser Thr Ser Thr Ala Tyr
   65 70 75 80
   Leu Glu Leu Ser Ser Leu Arg Ser Glu Asp Thr Ala Val Tyr Tyr Cys
   85 90 95
   Ala Arg Asp Arg Leu Asp Tyr Trp Gly Gln Gly Thr Leu Val Thr Val
   100 105 110
   Ser Ser
<210> 129
   <211> 107
   <212> PRT
   <213> Artificial Sequence
<220>
   <223> Synthetic Construct
<400> 129
   Asp Ile Gln Met Thr Gln Ser Pro Ser Ser Leu Ser Ala Ser Val Gly
   1 5 10 15
   Asp Arg Val Thr Ile Thr Cys His Ala Ser Gln Asp Ile Ser Ser Tyr
   20 25 30
   Ile Val Trp Tyr Gln Gln Lys Pro Gly Lys Ser Phe Lys Gly Leu Ile
   35 40 45
   Tyr His Gly Thr Asn Leu Glu Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Val His Tyr Ala Gln Phe Pro Tyr
   85 90 95
   Thr Phe Gly Gln Gly Thr Lys Val Glu Ile Lys
   100 105
<210> 130
   <211> 114
   <212> PRT
   <213> Artificial Sequence
<220>
   <223> Synthetic Construct
<400> 130
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 131
   <211> 107
   <212> PRT
   <213> Artificial Sequence
<220>
   <223> Synthetic Construct
<400> 131
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Glu Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 132
   <211> 114
   <212> PRT
   <213> Artificial Sequence
<220>
   <223> Synthetic Construct
<400> 132
   Glu Val Gln Leu Val Gln Ser Gly Ala Glu Val Lys Lys Pro Gly Ala
   1               5                   10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 133
   <211> 107
   <212> PRT
   <213> Artificial Sequence
<220>
   <223> Synthetic Construct
<400> 133
   Asp Ile Gln Met Thr Gln Ser Pro Ser Ser Leu Ser Ala Ser Val Gly
   1               5                   10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Gln Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 134
   <211> 114
   <212> PRT
   <213> Artificial Sequence
<220>
   <223> Synthetic Construct
<400> 134
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Ile Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 135
   <211> 107
   <212> PRT
   <213> Artificial Sequence
<220>
   <223> Synthetic Construct
<400> 135
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Ala Asp Tyr Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 136
   <211> 114
   <212> PRT
   <213> Artificial Sequence
<220>
   <223> Synthetic Construct
<400> 136
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 137
   <211> 107
   <212> PRT
   <213> Artificial Sequence
<220>
   <223> Synthetic Construct
<400> 137
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Ala Asp Tyr Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 138
   <211> 114
   <212> PRT
   <213> Artificial Sequence
<220>
   <223> Synthetic Construct
<400> 138
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Ala Phe Thr Asn Tyr
   20 25 30
   Leu Ile Glu Trp Val Arg Gln Ala Pro Gly Gln Gly Leu Glu Trp Ile
   35 40 45
   Gly Val Ile Asn Pro Gly Ser Gly Asp Thr Tyr Tyr Ser Glu Lys Phe
   50 55 60
   Lys Gly Arg Val Thr Leu Thr Arg Asp Thr Ser Thr Ser Thr Ala Tyr
   65 70 75 80
   Leu Glu Leu Ser Ser Leu Arg Ser Glu Asp Thr Ala Val Tyr Tyr Cys
   85 90 95
   Ala Arg Asp Arg Leu Asp Tyr Trp Gly Gln Gly Thr Leu Val Thr Val
   100 105 110
   Ser Ser
<210> 139
   <211> 107
   <212> PRT
   <213> Artificial Sequence
<220>
   <223> Synthetic Construct
<400> 139
   Asp Ile Gln Met Thr Gln Ser Pro Ser Ser Leu Ser Ala Ser Val Gly
   1 5 10 15
   Asp Arg Val Thr Ile Thr Cys His Ala Ser Gln Asp Ile Ser Ser Tyr
   20 25 30
   Ile Val Trp Tyr Gln Gln Lys Pro Gly Lys Ser Phe Lys Gly Leu Ile
   35 40 45
   Tyr His Gly Thr Asn Leu Glu Asp Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Val His Tyr Ala Gln Phe Pro Tyr
   85 90 95
   Thr Phe Gly Gln Gly Thr Lys Val Glu Ile Lys
   100 105
<210> 140
   <211> 114
   <212> PRT
   <213> Artificial Sequence
<220>
   <223> Synthetic Construct
<400> 140
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Ala Phe Thr Asn Tyr
   20 25 30
   Leu Ile Glu Trp Val Arg Gln Ala Pro Gly Gln Gly Leu Glu Trp Ile
   35 40 45
   Gly Val Ile Asn Pro Gly Ser Gly Asp Thr Tyr Tyr Ser Glu Lys Phe
   50 55 60
   Lys Gly Arg Val Thr Leu Thr Arg Asp Thr Ser Thr Ser Thr Ala Tyr
   65 70 75 80
   Leu Glu Leu Ser Ser Leu Arg Ser Glu Asp Thr Ala Val Tyr Tyr Cys
   85 90 95
   Ala Arg Asp Arg Leu Asp Tyr Trp Gly Gln Gly Thr Leu Val Thr Val
   100 105 110
   Ser Ser
<210> 141
   <211> 107
   <212> PRT
   <213> Artificial Sequence
<220>
   <223> Synthetic Construct
<400> 141
   Asp Ile Gln Met Thr Gln Ser Pro Ser Ser Leu Ser Ala Ser Val Gly
   1 5 10 15
   Asp Arg Val Thr Ile Thr Cys His Ala Ser Gln Asp Ile Ser Ser Tyr
   20 25 30
   Ile Val Trp Tyr Gln Gln Lys Pro Gly Lys Ser Pro Lys Leu Leu Ile
   35 40 45
   Tyr His Gly Thr Asn Leu Glu Asp Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Val His Tyr Ala Gln Phe Pro Tyr
   85 90 95
   Thr Phe Gly Gln Gly Thr Lys Val Glu Ile Lys
   100 105
<210> 142
   <211> 114
   <212> PRT
   <213> Artificial Sequence
<220>
   <223> Synthetic Construct
<400> 142
   Glu Val Gln Leu Val Gln Ser Gly Ala Glu Val Lys Lys Pro Gly Ala
    1               5                   10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Arg Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 143
   <211> 107
   <212> PRT
   <213> Artificial Sequence
<220>
   <223> Synthetic Construct
<400> 143
   Asp Ile Gln Met Thr Gln Ser Pro Ser Ser Leu Ser Ala Ser Val Gly
    1               5                   10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ala Phe Lys Leu Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 144
   <211> 114
   <212> PRT
   <213> Artificial Sequence
<220>
   <223> Synthetic Construct
<400> 144
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Ala Phe Thr Asn Tyr
   20 25 30
   Leu Ile Glu Trp Val Arg Gln Ala Pro Gly Gln Gly Leu Glu Trp Ile
   35 40 45
   Gly Val Ile Asn Pro Gly Ser Gly Asp Thr Tyr Tyr Ser Glu Lys Phe
   50 55 60
   Lys Gly Arg Val Thr Leu Thr Arg Asp Thr Ser Thr Ser Thr Ala Tyr
   65 70 75 80
   Leu Glu Leu Ser Ser Leu Arg Ser Glu Asp Thr Ala Val Tyr Tyr Cys
   85 90 95
   Ala Arg Asp Arg Leu Asp Tyr Trp Gly Gln Gly Thr Leu Val Thr Val
   100 105 110
   Ser Ser
<210> 145
   <211> 107
   <212> PRT
   <213> Artificial Sequence
<220>
   <223> Synthetic Construct
<400> 145
   Asp Ile Gln Met Thr Gln Ser Pro Ser Ser Leu Ser Ala Ser Val Gly
   1 5 10 15
   Asp Arg Val Thr Ile Thr Cys His Ala Ser Gln Asp Ile Ser Ser Tyr
   20 25 30
   Ile Val Trp Tyr Gln Gln Lys Pro Gly Lys Ala Pro Lys Gly Leu Ile
   35 40 45
   Tyr His Gly Thr Asn Leu Glu Asp Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Val His Tyr Ala Gln Phe Pro Tyr
   85 90 95
   Thr Phe Gly Gln Gly Thr Lys Val Glu Ile Lys
   100 105
<210> 146
   <211> 114
   <212> PRT
   <213> Artificial Sequence
<220>
   <223> Synthetic Construct
<400> 146
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Ala Phe Thr Asn Tyr
                       20                      25                      30
   Leu Ile Glu Trp Val Arg Gln Ala Pro Gly Gln Gly Leu Glu Trp Ile
               35                      40                      45
   Gly Val Ile Asn Pro Gly Ser Gly Asp Thr Tyr Tyr Ser Glu Lys Phe
       50                      55                      60
   Lys Gly Arg Val Thr Leu Thr Ala Asp Thr Ser Thr Ser Thr Ala Tyr
   65                      70                      75                      80
   Leu Glu Leu Ser Ser Leu Arg Ser Glu Asp Thr Ala Val Tyr Tyr Cys
                       85                      90                      95
   Ala Arg Asp Arg Leu Asp Tyr Trp Gly Gln Gly Thr Leu Val Thr Val
               100                     105                     110
   Ser Ser
<210> 147
   <211> 107
   <212> PRT
   <213> Artificial Sequence
<220>
   <223> Synthetic Construct
<400> 147
   Asp Ile Gln Met Thr Gln Ser Pro Ser Ser Leu Ser Ala Ser Val Gly
   1               5                   10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Ala His Tyr Ala Gln Phe Pro Tyr
                   85                  90                  95
   Thr Phe Gly Gln Gly Thr Lys Val Glu Ile Lys
               100                 105
<210> 148
   <211> 114
   <212> PRT
   <213> Artificial Sequence
<220>
   <223> Synthetic Construct
<400> 148
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 149
   <211> 107
   <212> PRT
   <213> Artificial Sequence
<220>
   <223> Synthetic Construct
<400> 149
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val Ala Tyr Ala Gln Phe Pro Tyr
                   85                  90                  95
   Thr Phe Gly Gln Gly Thr Lys Val Glu Ile Lys
               100                 105
<210> 150
   <211> 114
   <212> PRT
   <213> Artificial Sequence
<220>
   <223> Synthetic Construct
<400> 150
   Glu Val Gln Leu Val Gln Ser Gly Ala Glu Val Lys Lys Pro Gly Ala
    1               5                   10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 151
   <211> 107
   <212> PRT
   <213> Artificial Sequence
<220>
   <223> Synthetic Construct
<400> 151
   Asp Ile Gln Met Thr Gln Ser Pro Ser Ser Leu Ser Ala Ser Val Gly
    1               5                   10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Ala Ala Gln Phe Pro Tyr
                   85                  90                  95
   Thr Phe Gly Gln Gly Thr Lys Val Glu Ile Lys
               100                 105
<210> 152
   <211> 114
   <212> PRT
   <213> Artificial Sequence
<220>
   <223> Synthetic Construct
<400> 152
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 153
   <211> 107
   <212> PRT
   <213> Artificial Sequence
<220>
   <223> Synthetic Construct
<400> 153
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Ala Phe Pro Tyr
                   85                  90                  95
   Thr Phe Gly Gln Gly Thr Lys Val Glu Ile Lys
               100                 105
<210> 154
   <211> 114
   <212> PRT
   <213> Artificial Sequence
<220>
   <223> Synthetic Construct
<400> 154
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 155
   <211> 107
   <212> PRT
   <213> Artificial Sequence
<220>
   <223> Synthetic Construct
<400> 155
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Ala Pro Tyr
                   85                  90                  95
   Thr Phe Gly Gln Gly Thr Lys Val Glu Ile Lys
               100                 105
<210> 156
   <211> 114
   <212> PRT
   <213> Artificial Sequence
<220>
   <223> Synthetic Construct
<400> 156
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 157
   <211> 107
   <212> PRT
   <213> Artificial Sequence
<220>
   <223> Synthetic Construct
<400> 157
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Ala Tyr
                   85                  90                  95
   Thr Phe Gly Gln Gly Thr Lys Val Glu Ile Lys
               100                 105
<210> 158
   <211> 114
   <212> PRT
   <213> Artificial Sequence
<220>
   <223> Synthetic Construct
<400> 158
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Leu Asp Tyr Trp Gly Gln Gly Thr Leu Val Thr Val
               100                 105                 110
   Ser Ser
<210> 159
   <211> 107
   <212> PRT
   <213> Artificial Sequence
<220>
   <223> Synthetic Construct
<400> 159
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Ala
                   85                  90                  95
   Thr Phe Gly Gln Gly Thr Lys Val Glu Ile Lys
               100                 105
<210> 160
   <211> 114
   <212> PRT
   <213> Artificial Sequence
<220>
   <223> Synthetic Construct
<400> 160
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Ala Arg Leu Asp Tyr Trp Gly Gln Gly Thr Leu Val Thr Val
               100                 105                 110
   Ser Ser
<210> 161
   <211> 107
   <212> PRT
   <213> Artificial Sequence
<220>
   <223> Synthetic Construct
<400> 161
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 162
   <211> 114
   <212> PRT
   <213> Artificial Sequence
<220>
   <223> Synthetic Construct
<400> 162
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Ala Leu Asp Tyr Trp Gly Gln Gly Thr Leu Val Thr Val
               100                 105                 110
   Ser Ser
<210> 163
   <211> 107
   <212> PRT
   <213> Artificial Sequence
<220>
   <223> Synthetic Construct
<400> 163
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 164
   <211> 114
   <212> PRT
   <213> Artificial Sequence
<220>
   <223> Synthetic Construct
<400> 164
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Ala Phe Thr Asn Tyr
               20                  25                  30
   Leu Ile Glu Trp Val Arg Gln Ala Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Arg Val Thr Leu Thr Ala Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Ala Arg Asp Arg Ala Asp Tyr Trp Gly Gln Gly Thr Leu Val Thr Val
               100                 105                 110
   Ser Ser
<210> 165
   <211> 107
   <212> PRT
   <213> Artificial Sequence
<220>
   <223> Synthetic Construct
<400> 165
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Asp Ile Ser Ser Tyr
               20                  25                  30
   Ile Val Trp Tyr Gln Gln Lys Pro Gly Lys Ser Phe Lys Gly Leu Ile
           35                  40                  45
   Tyr His Gly Thr Asn Leu Glu Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Val His Tyr Ala Gln Phe Pro Tyr
                   85                  90                  95
   Thr Phe Gly Gln Gly Thr Lys Val Glu Ile Lys
               100                 105
<210> 166
   <211> 113
   <212> PRT
   <213> Artificial Sequence
<220>
   <223> Synthetic Construct
<400> 166
   Glu Val Gln Leu Val Glu Ser Gly Pro Gly Leu Val Lys Pro Ser Glu
    1               5                  10                  15
   Thr Leu Ser Leu Thr Cys Thr Val Ser Gly Phe Ser Leu Thr Asp Tyr
               20                  25                  30
   Gly Val Leu Trp Ile Arg Gln Pro Pro Gly Lys Gly Leu Glu Trp Ile
           35                  40                  45
   Gly Met Ile Trp Ser Gly Gly Thr Thr Asp Tyr Asn Ala Ala Phe Ile
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Val
                   85                  90                  95
   Arg Glu Glu Met Asp Tyr Trp Gly Gln Gly Thr Leu Val Thr Val Ser
               100                 105                 110
   Ser
<210> 167
   <211> 107
   <212> PRT
   <213> Artificial Sequence
<220>
   <223> Synthetic Construct
<400> 167
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Phe
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys
               100                 105
<210> 168
   <211> 113
   <212> PRT
   <213> Artificial Sequence
<220>
   <223> Synthetic Construct
<400> 168
   Glu Val Gln Leu Val Glu Ser Gly Pro Gly Leu Val Lys Pro Ser Glu
    1               5                  10                  15
   Thr Leu Ser Leu Thr Cys Thr Val Ser Gly Phe Ser Leu Thr Asp Tyr
               20                  25                  30
   Gly Val Leu Trp Ile Arg Gln Pro Pro Gly Lys Gly Leu Glu Trp Ile
           35                  40                  45
   Gly Met Ile Trp Ser Gly Gly Thr Thr Asp Tyr Asn Ala Ala Phe Ile
       50                  55                  60
   Ser Arg Val Thr Ile Ser Lys Asp Thr Ser Lys Asn Gln Val Ser Leu
   65                  70                  75                  80
   Lys Leu Ser Ser Val Thr Ala Ala Asp Thr Ala Val Tyr Tyr Cys Val
                   85                  90                  95
   Arg Glu Glu Met Asp Tyr Trp Gly Gln Gly Thr Leu Val Thr Val Ser
               100                 105                 110
   Ser
<210> 169
   <211> 107
   <212> PRT
   <213> Artificial Sequence
<220>
   <223> Synthetic Construct
<400> 169
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Phe
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys
               100                 105
<210> 170
   <211> 113
   <212> PRT
   <213> Artificial Sequence
<220>
   <223> Synthetic Construct
<400> 170
   Glu Val Gln Leu Val Glu Ser Gly Pro Gly Leu Val Lys Pro Ser Glu
    1               5                  10                  15
   Thr Leu Ser Leu Thr Cys Thr Val Ser Gly Phe Ser Leu Thr Asp Tyr
               20                  25                  30
   Gly Val Leu Trp Val Arg Gln Pro Pro Gly Lys Gly Leu Glu Trp Leu
           35                  40                  45
   Gly Met Ile Trp Ser Gly Gly Thr Thr Asp Tyr Asn Ala Ala Phe Ile
       50                  55                  60
   Ser Arg Leu Thr Ile Ser Lys Asp Thr Ser Lys Asn Gln Val Ser Leu
   65                  70                  75                  80
   Lys Leu Ser Ser Val Thr Ala Ala Asp Thr Ala Val Tyr Tyr Cys Val
                   85                  90                  95
   Arg Glu Glu Met Asp Tyr Trp Gly Gln Gly Thr Leu Val Thr Val Ser
               100                 105                 110
   Ser
<210> 171
   <211> 107
   <212> PRT
   <213> Artificial Sequence
<220>
   <223> Synthetic Construct
<400> 171
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Ser Asn Phe
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys
               100                 105
<210> 172
   <211> 5
   <212> PRT
   <213> Artificial Sequence
<220>
   <223> Synthetic Construct
<220>
   <221> VARIANT
   <222> 1
   <223> Xaa = D or E
<220>
   <221> VARIANT
   <222> 2
   <223> Xaa = S or A
<400> 172
   Xaa Xaa Tyr Met Ser
    1               5
<210> 173
   <211> 17
   <212> PRT
   <213> Artificial Sequence
<220>
   <223> Synthetic Construct
<220>
   <221> VARIANT
   <222> 6
   <223> Xaa = N or S
<220>
   <221> VARIANT
   <222> 7
   <223> Xaa = A or G
<220>
   <221> VARIANT
   <222> 8
   <223> Xaa = D or S
<220>
   <221> VARIANT
   <222> 9
   <223> Xaa = A or S
<400> 173
   Asp Met Tyr Pro Asp Xaa Xaa Xaa Xaa Ser Tyr Asn Gln Lys Phe Arg
    1               5                  10                  15
   Glu
<210> 174
   <211> 8
   <212> PRT
   <213> Artificial Sequence
<220>
   <223> Synthetic Construct
<220>
   <221> VARIANT
   <222> 5
   <223> Xaa = Y or A
<220>
   <221> VARIANT
   <222> 6
   <223> Xaa = A or F
<220>
   <221> VARIANT
   <222> 7
   <223> Xaa = S or A
<220>
   <221> VARIANT
   <222> 8
   <223> Xaa = A or V
<400> 174
   Ala Pro Arg Trp Xaa Xaa Xaa Xaa
    1               5
<210> 175
   <211> 9
   <212> PRT
   <213> Artificial Sequence
<220>
   <223> Synthetic Construct
<220>
   <221> VARIANT
   <222> 2
   <223> Xaa = A or Q
<220>
   <221> VARIANT
   <222> 3
   <223> Xaa = A or G
<220>
   <221> VARIANT
   <222> 4
   <223> Xaa = A or H
<220>
   <221> VARIANT
   <222> 5
   <223> Xaa = A or T
<220>
   <221> VARIANT
   <222> 6
   <223> Xaa = A or L
<220>
   <221> VARIANT
   <222> 7
   <223> Xaa = A or P
<220>
   <221> VARIANT
   <222> 8
   <223> Xaa = A or P
<400> 175
   Gln Xaa Xaa Xaa Xaa Xaa Xaa Xaa Thr
   1 5
<210> 176
   <211> 17
   <212> PRT
   <213> Artificial Sequence
<220>
   <223> Synthetic Construct
<220>
   <221> VARIANT
   <222> 9
   <223> Xaa = T, A or Q
<400> 176
   Val Ile Asn Pro Gly Ser Gly Asp Xaa Tyr Tyr Ser Glu Lys Phe Lys
   1 5 10 15
   Gly
<210> 177
   <211> 7
   <212> PRT
   <213> Artificial Sequence
<220>
   <223> Synthetic Construct
<220>
   <221> VARIANT
   <222> 7
   <223> Xaa = S, E, or Q
<400> 177
   His Gly Thr Asn Leu Glu Xaa
   1 5
<210> 178
   <211> 9
   <212> PRT
   <213> Artificial Sequence
<220>
   <223> Synthetic Construct
<220>
   <221> VARIANT
   <222> 1
   <223> Xaa = V or A
<220>
   <221> VARIANT
   <222> 2
   <223> Xaa = H or A
<220>
   <221> VARIANT
   <222> 9
   <223> Xaa = Y or A
<400> 178
   Xaa Xaa Tyr Ala Gln Phe Pro Tyr Xaa
   1               5
<210> 179
   <211> 107
   <212> PRT
   <213> Artificial Sequence
<220>
   <223> Synthetic Construct
<400> 179
   Asp Ile Gln Met Thr Gln Thr Thr Ser Ser Leu Ser Ala Ser Leu Gly
   1               5                   10                  15
   Asp Arg Val Thr Ile Ser Cys Arg Ala Ser Gln Asp Ile Ser Asn Tyr
               20                  25                  30
   Leu Asn Trp Tyr Gln Gln Lys Pro Asp Gly Thr Val Lys Leu Leu Ile
           35                  40                  45
   Tyr Tyr Thr Ser Arg Leu Arg Ser Gly Val Pro Ser Arg Phe Ser Gly
       50                  55                  60
   Ser Gly Ser Gly Lys Asp Tyr Phe Leu Thr Ile Ser Asn Leu Glu Gln
   65                  70                  75                  80
   Glu Asp Val Ala Ala Tyr Phe Cys Gln Gln Gly His Thr Leu Pro Pro
                   85                  90                  95
   Thr Phe Gly Gly Gly Thr Lys Leu Glu Ile Lys
               100                 105
<210> 180
   <211> 117
   <212> PRT
   <213> Artificial Sequence
<220>
   <223> Synthetic Construct
<400> 180
   Glu Val Gln Leu Gln Gln Ser Gly Pro Glu Leu Val Lys Pro Gly Ala
    1               5                  10                  15
   Ser Val Lys Ile Ser Cys Lys Ala Ser Gly Tyr Thr Phe Thr Asp Ser
               20                  25                  30
   Tyr Met Ser Trp Val Lys Gln Ser His Gly Lys Thr Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Lys Val Thr Leu Thr Val Asp Lys Ser Ser Thr Thr Ala Tyr
   65                  70                  75                  80
   Met Glu Phe Arg Ser Leu Thr Ser Glu Asp Ser Ala Val Tyr Tyr Cys
                   85                  90                  95
   Val Leu Ala Pro Arg Trp Tyr Phe Ser Val Trp Gly Thr Gly Thr Thr
               100                 105                 110
   Val Thr Val Ser Ser
           115
<210> 181
   <211> 107
   <212> PRT
   <213> Artificial Sequence
<220>
   <223> Synthetic Construct
<400> 181
   Asp Ile Leu Met Thr Gln Ser Pro Ser Ser Met Ser Val Ser Leu Gly
    1               5                  10                  15
   Asp Thr Val Ser Ile Thr Cys His Ala Ser Gln Asp Ile Ser Ser Tyr
               20                  25                  30
   Ile Val Trp Leu Gln Gln Lys Pro Gly Lys Ser Phe Arg Gly Leu Ile
           35                  40                  45
   Tyr His Gly Thr Asn Leu Glu Asp Gly Ile Pro Ser Arg Phe Ser Gly
       50                  55                  60
   Ser Gly Ser Gly Ala Asp Tyr Ser Leu Thr Ile Ser Ser Leu Glu Ser
   65                  70                  75                  80
   Glu Asp Phe Ala Asp Tyr Tyr Cys Val His Tyr Ala Gln Phe Pro Tyr
                   85                  90                  95
   Thr Phe Gly Gly Gly Thr Lys Leu Glu Ile Lys
               100                 105
<210> 182
   <211> 114
   <212> PRT
   <213> Artificial Sequence
<220>
   <223> Synthetic Construct
<400> 182
   Gln Val Gln Leu Gln Gln Ser Gly Ala Glu Leu Val Arg Pro Gly Thr
    1               5                  10                  15
   Ser Val Lys Val Ser Cys Lys Ala Ser Gly Tyr Ala Phe Thr Asn Tyr
               20                  25                  30
   Leu Ile Glu Trp Val Lys Gln Arg Pro Gly Gln Gly Leu Glu Trp Ile
           35                  40                  45
   Gly Val Ile Asn Pro Gly Ser Gly Asp Thr Tyr Tyr Ser Glu Lys Phe
       50                  55                  60
   Lys Gly Lys Val Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Asp Arg Leu Asp Tyr Trp Gly Gln Gly Thr Thr Leu Thr Val
               100                 105                 110
   Ser Ser
<210> 183
   <211> 117
   <212> PRT
   <213> Artificial Sequence
<220>
   <223> Synthetic Construct
<400> 183
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Leu Thr Val Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 184
   <211> 117
   <212> PRT
   <213> Artificial Sequence
<220>
   <223> Synthetic Construct
<400> 184
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Ser
               20                  25                  30
   Tyr Met Ser Trp Val Arg Gln Ala Pro Gly Gln Gly Leu Glu Trp Ile
           35                  40                  45
   Gly Asp Met Tyr Pro Asp Asn Gly Asp Ser Ser Tyr Asn Gln Lys Phe
       50                  55                  60
   Arg Glu Arg Val Thr Leu Thr Val Asp Thr Ser Thr Ser Thr Ala Tyr
   65                  70                  75                  80
   Leu Glu Leu Ser Ser Leu Arg Ser Glu Asp Thr Ala Val Tyr Tyr Cys
                   85                  90                  95
   Val Leu Ala Pro Arg Trp Tyr Phe Ser Val Trp Gly Gln Gly Thr Leu
               100                 105                 110
   Val Thr Val Ser Ser
           115
<210> 185
   <211> 25
   <212> PRT
   <213> Artificial Sequence
<220>
   <223> Synthetic Construct
<400> 185
<210> 186
   <211> 13
   <212> PRT
   <213> Artificial Sequence
<220>
   <223> Synthetic Construct
<400> 186
   Trp Val Arg Gln Ala Pro Gly Lys Gly Leu Glu Trp Val
    1               5                  10
<210> 187
   <211> 30
   <212> PRT
   <213> Artificial Sequence
<220>
   <223> Synthetic Construct
<400> 187
   Arg Phe Thr Ile Ser Arg Asp Asn Ser Lys Asn Thr Leu Tyr Leu Gln
    1               5                  10                  15
   Met Asn Ser Leu Arg Ala Glu Asp Thr Ala Val Tyr Tyr Cys
               20                  25                  30
<210> 188
   <211> 11
   <212> PRT
   <213> Artificial Sequence
<220>
   <223> Synthetic Construct
<400> 188
   Trp Gly Gln Gly Thr Leu Val Thr Val Ser Ser
    1               5                   10
<210> 189
   <211> 23
   <212> PRT
   <213> Artificial Sequence
<220>
   <223> Synthetic Construct
<400> 189
   Asp Ile Gln Met Thr Gln Ser Pro Ser Ser Leu Ser Ala Ser Val Gly
    1               5                   10                  15
   Asp Arg Val Thr Ile Thr Cys
               20
<210> 190
   <211> 15
   <212> PRT
   <213> Artificial Sequence
<220>
   <223> Synthetic Construct
<400> 190
   Trp Tyr Gln Gln Lys Pro Gly Lys Ala Pro Lys Leu Leu Ile Tyr
    1               5                   10                  15
<210> 191
   <211> 32
   <212> PRT
   <213> Artificial Sequence
<220>
   <223> Synthetic Construct
<400> 191
   Gly Val Pro Ser Arg Phe Ser Gly Ser Gly Ser Gly Thr Asp Phe Thr
    1               5                   10                  15
   Leu Thr Ile Ser Ser Leu Gln Pro Glu Asp Phe Ala Thr Tyr Tyr Cys
               20                  25                  30
<210> 192
   <211> 10
   <212> PRT
   <213> Artificial Sequence
<220>
   <223> Synthetic Construct
<400> 192
   Phe Gly Gln Gly Thr Lys Val Glu Ile Lys
   1 5 10
<210> 193
   <211> 17
   <212> PRT
   <213> Artificial Sequence
<220>
   <223> Synthetic Construct
<400> 193
   Asp Met Tyr Pro Asp Ala Ala Ala Ala Ser Tyr Asn Gln Lys Phe Arg
   1 5 10 15
   Glu
<210> 194
   <211> 8
   <212> PRT
   <213> Artificial Sequence
<220>
   <223> Synthetic Construct
<400> 194
   Ala Pro Arg Trp Ala Ala Ala Ala
   1 5
<210> 195
   <211> 9
   <212> PRT
   <213> Artificial Sequence
<220>
   <223> Synthetic Construct
<400> 195
   Gln Ala Ala Ala Ala Ala Ala Ala Thr
   1 5
<210> 196
   <211> 10
   <212> PRT
   <213> Artificial Sequence
<220>
   <223> Synthetic Construct
<400> 196
   Ala Leu Ala Pro Arg Trp Tyr Phe Ser Val
   1 5 10
<210> 197
   <211> 7
   <212> PRT
   <213> Artificial Sequence
<220>
   <223> Synthetic Construct
<400> 197
   Ala Arg Ala Arg Leu Asp Tyr
   1 5
<210> 198
   <211> 7
   <212> PRT
   <213> Artificial Sequence
<220>
   <223> Synthetic Construct
<400> 198
   Ala Arg Asp Ala Leu Asp Tyr
   1 5
<210> 199
   <211> 7
   <212> PRT
   <213> Artificial Sequence
<220>
   <223> Synthetic Construct
<400> 199
   Ala Arg Asp Arg Ala Asp Tyr
   1 5
<210> 200
   <211> 7
   <212> PRT
   <213> Artificial Sequence
<220>
   <223> Synthetic Construct
<400> 200
   Ala Arg Asp Arg Leu Asp Tyr
   1 5
<210> 201
   <211> 10
   <212> PRT
   <213> Artificial Sequence
<220>
   <223> Synthetic Construct
<400> 201
   Val Ala Ala Pro Arg Trp Tyr Phe Ser Val
    1               5                   10
<210> 202
   <211> 10
   <212> PRT
   <213> Artificial Sequence
<220>
   <223> Synthetic Construct
<400> 202
   Val Leu Ala Ala Arg Trp Tyr Phe Ser Val
    1               5                   10
<210> 203
   <211> 10
   <212> PRT
   <213> Artificial Sequence
<220>
   <223> Synthetic Construct
<400> 203
   Val Leu Ala Pro Ala Trp Tyr Phe Ser Val
    1               5                   10
<210> 204
   <211> 10
   <212> PRT
   <213> Artificial Sequence
<220>
   <223> Synthetic Construct
<400> 204
   Val Leu Ala Pro Arg Ala Tyr Phe Ser Val
    1               5                   10
<210> 205
   <211> 10
   <212> PRT
   <213> Artificial Sequence
<220>
   <223> Synthetic Construct
<400> 205
   Val Leu Ala Pro Arg Trp Ala Phe Ser Val
   1 5 10
<210> 206
   <211> 10
   <212> PRT
   <213> Artificial Sequence
<220>
   <223> Synthetic Construct
<400> 206
   Val Leu Ala Pro Arg Trp Tyr Ala Ser Val
   1 5 10
<210> 207
   <211> 10
   <212> PRT
   <213> Artificial Sequence
<220>
   <223> Synthetic Construct
<400> 207
   Val Leu Ala Pro Arg Trp Tyr Phe Ala Val
   1 5 10
<210> 208
   <211> 10
   <212> PRT
   <213> Artificial Sequence
<220>
   <223> Synthetic Construct
<400> 208
   Val Leu Ala Pro Arg Trp Tyr Phe Ser Ala
   1 5 10
<210> 209
   <211> 10
   <212> PRT
   <213> Artificial Sequence
<220>
   <223> Synthetic Construct
<400> 209
   Val Leu Ala Pro Arg Trp Tyr Phe Ser Val
   1 5 10

## Description

### FIELD OF THE INVENTION

The present invention relates to anti-OX40 antibodies and methods of using the same.

### BACKGROUND

OX40 (also known as CD34, TNFRSF4 and ACT35) is a member of the tumor necrosis factor receptor superfamily. OX40 is not constitutively expressed on naive T cells, but is induced after engagement of the T cell receptor (TCR). The ligand for OX40, OX40L, is predominantly expressed on antigen presenting cells. OX40 is highly expressed by activated CD4+ T cells, activated CD8+ T cells, memory T cells, and regulatory T cells. OX40 signaling can provide costimulatory signals to CD4 and CD8 T cells, leading to enhanced cell proliferation, survival, effector function and migration. OX40 signaling also enhances memory T cell development and function.

Regulatory T cells (Treg) cells are highly enriched in tumors and tumor draining lymph nodes derived from multiple cancer indications, including melanoma, NSCLC, renal, ovarian, colon, pancreatic, hepatocellular, and breast cancer. In a subset of these indications, increased intratumoral T reg cell densities are associated with poor patient prognosis, suggesting that these cells play an important role in suppressing antitumor immunity. OX40 positive tumor infiltrating lymphocytes have been described.

WO 2009/079335 discloses agonistic OX40 antibody 11D4 that binds with high affinity to the extracellular domain of the OX40R and on whole T cells. Two agonistic antibodies 1F7 and 2G2 are described in Xie et al., Tissue Antigens 2006, 67(4), 307-317. A group of agonistic OX40 scFvs can be found in WO 03/106498. OX40 antibodies 119-122 and 106-122 and their biological activity are described in Voo et al., Journal of Immunology 2013, 191(7), 3641-3650.

The administration of an agonistic OX40 antibody 9B12 to patients with solid malignancies is described in Curti et al., Cancer Research 2013, 73(24), 7189-7198 and the use of an agonistic OX40 antibody in combination with a PD-1 antagonist is disclosed in Guo et al., PLOS ONE 2014, 9(2), e89350.

It is clear that there continues to be a need for agents that have clinical attributes that are optimal for development as therapeutic agents. The invention described herein meets this need and provides other benefits.

### SUMMARY

In one aspect, provided are isolated antibodies that bind to human OX40 comprising a heavy chain variable domain (VH) comprising the amino acid sequence of SEQ ID NO:56 and a light chain variable domain (VL) comprising the amino acid sequence of SEQ ID NO:57.

In some embodiments, these antibodies deplete CD4+ effector T cells. In some embodiments, these antibodies deplete regulatory T cells (Treg). In some embodiments, the depleting is by ADCC and/or phagocytosis. In some embodiments, the depleting is by ADCC.

In another aspect, provided are anti-human OX40 agonist antibodies that bind human OX40 with an affinity of less than or equal to about 0.45 nM as determined using radioimmunoassay. In some embodiments, the antibody binds human OX40 and cynomolgus OX40. In some embodiments, binding to human and cynomolgus OX40 is determined using a FACS assay. In some embodiments, binding to human OX40 has an EC50 of less than or equal to 0.3 µg/ml or lower. In some embodiments, binding to cynomolgus OX40 has an EC50 of less than or equal to 1.5 µg/ml. In some embodiments, binding to cynomolgus OX40 has an EC50 of less than or equal to 1.4 µg/ml.

In another aspect, provided are isolated nucleic acids encoding any of the anti-human OX40 antibodies (e.g., agonist antibodies) provided herein.

In another aspect, provided are host cell comprising the nucleic acid encoding any of the anti-human OX40 antibodies (e.g., agonist antibodies) provided herein.

In another aspect, provided are methods of producing an antibody comprising culturing the host cell so that the antibody is produced. In some embodiments, the methods further comprise recovering the antibody from the host cell.

In another aspect, provided are immunoconjugates comprising any of the anti-human OX40 antibodies (e.g., agonist antibodies) provided herein and a cytotoxic agent.

In another aspect, provided are pharmaceutical formulations comprising any of the anti-human OX40 antibodies (e.g., agonist antibodies) provided herein and a pharmaceutically acceptable carrier. In some embodiments, the pharmaceutical formulation comprises (a) any of the anti-human OX40 agonist antibodies described herein at a concentration between about 10 mg/mL and about 100 mg/mL, (b) a polysorbate, wherein the polysorbate concentration is about 0.02% to about 0.06%; (c) a histidine buffer at about pH 5.0 to about pH 6.0; and (d) a saccharide, wherein the saccharide concentration is about 120mM to about 320 mM.

In another aspect, an anti-human OX0 agonist antibodies provided herein is for use as a medicament.

In another aspect, an anti-human OX0 agonist antibody provided herein is for use in treating cancer.

In another aspect, provided is an anti-human OX0 agonist antibody for use in methods of treating an individual having cancer comprising administering to the individual an effective amount of any of the anti-human OX40 agonist antibodies provided herein. In some embodiments, the methods further comprise administering an additional therapeutic agent. In some embodiments, the additional therapeutic agent comprises a chemotherapeutic agent. In some embodiments, the additional therapeutic agent comprises a PD-1 axis binding antagonist.

### BRIEF DESCRIPTION OF THE FIGURES

**FIG. 1****:** Humanized OX40 antibody variants were analyzed by FACS to evaluate antibody binding to huOX40 expressed on the surface of Hut78 cells.
**FIG. 2****:** OX40 agonist antibody 1A7.gr.1 bound with high affinity to human and cynomolgus monkey T cells.
**FIGS. 3A** and **3B:** (**FIG. 3A**) Mab 1A7.gr.1 had no effect on T cell proliferation in the absence of crosslinking. Increasing concentration of mab 1A7.gr.1 costimulated CD4+ memory T cell proliferation in response to anti-CD3 crosslinking. The calculated EC50 for the costimulatory effect of mab1A7.gr.1 was 9.96 ng/mL (n=2). (**FIG. 3B**) Increasing concentrations of mab 1A7.gr.1 costimulated CD4+ memory T cell production of interferon gamma in response to anti-CD3 crosslinking.
**FIG. 4A****:** In the presence of plate-bound anti-CD3, plate-bound mab 1A7 costimulated effector T cell proliferation. By contrast, costimulatory activity was abrogated when mab 1A7 was provided in soluble form in the presence of plate-bound anti-CD3, to a similar level as that observed with a plate-bound isotype control antibody in the presence of plate-bound anti-CD3.
**FIG. 4B****:** MAb 1A7 gr.1 harboring the N297G mutation failed to costimulate Teff cell proliferation. By contrast, wild type (un-mutated) mab 1A7 gr.1 costimulated anti-CD3 induced Teff cell proliferation.
**FIG. 5****:** Treatment with OX40 agonist antibody inhibited Treg cell-mediated suppression of naive CD4+ T cells. Naive CD4+ T cell (Tn) when cultured alone were inhibited by the addition of Treg cells and an isotype control antibody. Treg cell mediated inhibition of native CD4+ T cell proliferation was abrogated in cultures containing anti-OX40 antibody, mab 1A7.gr1. The data represented the average of 3 independent experiments.
**FIG. 6****:** Treatment with mab 1A7.gr.1 impaired the suppressive function of Treg cells.
**FIGS. 7A** and **7B:** (**FIG. 7A**) Treatment with mab 1A7.gr.1 induced ADCC of OX40-expressing T cells. (**FIG. 7B**) Treatment with mab A7.gr1 (IgG1) induced greater ADCC of OX40-expressing CD4+ T cells compared to level of ADCC induced by mab 1A7.gr1 (IgG4).
**FIGS. 8A** and **8B****:** BT474-human OX40 transgenic clones expressed different levels of human OX40. **FIG. 8A****,** low OX40 expressing BT474 cells. **FIG. 8B****,** high OX40 expressing BT474 cells.
**FIG. 9****:** Treatment with OX40 agonist antibody induced antibody dependent cell-mediated phagocytosis of cell lines expressing human OX40, and level of antibody dependent cell-mediated phagocytosis was sensitive to level of OX40 expression in the target cells.
**FIG. 10****:** Treatment with OX40 agonist antibody 1A7.gr1 induced ADCC in OX40-expressing cells.
**FIGS. 11A****-I:** Amino acid sequences of variable regions of anti-OX40 antibodies. Heavy chain HVR - H1, -H2, and -H3, and light chain HVR -LI, -L2, and -L3 sequences are marked. Amino acid positions are numbered according to the Kabat numbering system as described herein.
**FIG. 12A****:** Anti-human OX40 mab 1A7gr1 bound to Hut78-hOX40 cells in a dose dependent fashion, with 70% of maximum binding observed at about 200 ng/mL of antibody (indicated by the dotted square).
**FIG. 12B****:** OX40L-flag demonstrated dose dependent binding to Hut78-hOX40 cells.
**FIG. 12C****:** Binding of anti-human OX40 mab 1A7.gr.1 to Hut78-hOX40 cells decreased as the concentration of OX40L-flag increased.
**FIG. 12D****:** Presence of control DR5-flag had no impact on mab 1A7.gr.1 binding.
**FIG. 13****:** Pharmacokinetics (PK) of 1A7.gr1 dosed at 1mg/kg or 10mg/kg in SCID mice.
**FIGS. 14A** and **14B****:** Administration of 1A7.gr1 in cynomolgus monkeys resulted in minimal or transient increases in c-reactive protein (CRP). (**FIG. 14A**) CRP levels over time observed in monkeys given 0mg/kg or 0.01mg/kg doses. (**FIG. 14B**) CRP levels over time observed in monkeys given 0.3mg/kg or 10mg/kg doses.
**FIGS. 15A** and **15B****:** Administration of 1A7.gr1 in cynomolgus monkeys resulted in minimal or transient increases in a mixed subset of cytokines. (**FIG. 15A**) Levels of pro-inflammatory cytokines IL6 and MCP1 over time. (**FIG. 15B**) Levels of anti-inflammatory cytokines IL10 and IL1ra over time. In **FIGS. 15A** and **15B****,** individual monkeys in the 10mg/kg dose group that demonstrated transient increases in cytokine levels are labeled with arrows.
**FIGS. 16A** and **16B****:** Exposure of cynomolgus monkeys to 1A7.gr1 was confirmed by serum PK and peripheral receptor occupancy. (**FIG. 16A**) Serum PK of monkeys administered 0.01, 0.3, or 10mg/kg of 1A7.gr1. (**FIG. 16B**) OX40 receptor occupancy on peripheral CD4+ T cells over time in monkeys administered 0.01, 0.3, or 10mg/kg of 1A7.gr1.
**FIG. 17****:** Pharmacokinetics (PK) of 1A7.gr1 dosed at 0.5, 5, or 30mg/kg in cynomolgus monkeys.
**FIG. 18****:** OX40 receptor occupancy over time in monkeys administered 0, 0.5, 5, or 30mg/kg of 1A7.gr1. Arrows indicate days on which samples were obtained.
**FIG. 19****:** MCP-1 levels over time in monkeys administered 0, 0.5, 5, or 30mg/kg of 1A7.gr1.
**FIG. 20****:** No significant activation or proliferation of peripheral T cells was observed in monkeys administered 0, 0.5, 5, or 30mg/kg of 1A7.gr1.

### DETAILED DESCRIPTION OF EMBODIMENTS OF THE INVENTION

### I. DEFINITIONS

The term "dysfunction" in the context of immune dysfunction, refers to a state of reduced immune responsiveness to antigenic stimulation.

The term "dysfunctional", as used herein, also includes refractory or unresponsive to antigen recognition, specifically, impaired capacity to translate antigen recognition into downstream T-cell effector functions, such as proliferation, cytokine production (e.g., gamma interferon) and/or target cell killing.

"Enhancing T cell function" means to induce, cause or stimulate an effector or memory T cell to have a renewed, sustained or amplified biological function. Examples of enhancing T-cell function include: increased secretion of γ-interferon from CD8⁺ effector T cells, increased secretion of γ-interferon from CD4+ memory and/or effector T-cells, increased proliferation of CD4+ effector and/or memory T cells, increased proliferation of CD8+ effector T-cells, increased antigen responsiveness (*e.g.*, clearance), relative to such levels before the intervention. In one aspect, the level of enhancement is at least 50%, alternatively 60%, 70%, 80%, 90%, 100%, 120%, 150%, 200%. The manner of measuring this enhancement is known to one of ordinary skill in the art.

"Tumor immunity" refers to the process in which tumors evade immune recognition and clearance. Thus, as a therapeutic concept, tumor immunity is "treated" when such evasion is attenuated, and the tumors are recognized and attacked by the immune system. Examples of tumor recognition include tumor binding, tumor shrinkage and tumor clearance.

"Immunogenicity" refers to the ability of a particular substance to provoke an immune response. Tumors are immunogenic and enhancing tumor immunogenicity aids in the clearance of the tumor cells by the immune response.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some aspects, the number of amino acid changes are 10 or less, 9 or less, 8 or less, 7 or less, 6 or less, 5 or less, 4 or less, 3 or less, or 2 or less. In some aspects, the VL acceptor human framework is identical in sequence to the VL human immunoglobulin framework sequence or human consensus framework sequence.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary aspects for measuring binding affinity are described in the following.

An "agonist antibody," as used herein, is an antibody which activates a biological activity of the antigen it binds.

An "anti-angiogenic agent" refers to a compound which blocks, or interferes with to some degree, the development of blood vessels. An anti-angiogenic agent may, for instance, be a small molecule or antibody that binds to a growth factor or growth factor receptor involved in promoting angiogenesis. In one aspect, an anti-angiogenic agent is an antibody that binds to vascular endothelial growth factor (VEGF), such as bevacizumab (AVASTIN).

"Antibody-dependent cell-mediated cytotoxicity" or "ADCC" refers to a form of cytotoxicity in which secreted immunoglobulin bound onto Fc receptors (FcRs) present on certain cytotoxic cells (*e.g.* NK cells, neutrophils, and macrophages) enable these cytotoxic effector cells to bind specifically to an antigen-bearing target cell and subsequently kill the target cell with cytotoxins. The primary cells for mediating ADCC, NK cells, express FcγRIII only, whereas monocytes express FcγRI, FcγRII, and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 or U.S. Patent No. 6,737,056 (Presta), may be performed. Useful effector cells for such assays include PBMC and NK cells. Alternatively, or additionally, ADCC activity of the molecule of interest may be assessed *in vivo, e.g.,* in an animal model such as that disclosed in Clynes et al. PNAS (USA) 95:652-656 (1998). An exemplary assay for assessing ADCC activity is provided in the examples herein.

The terms "anti-OX40 antibody" and "an antibody that binds to OX40" refer to an antibody that is capable of binding OX40 with sufficient affinity such that the antibody is useful as a diagnostic and/or therapeutic agent in targeting OX40. In one aspect, the extent of binding of an anti-OX40 antibody to an unrelated, non-OX40 protein is less than about 10% of the binding of the antibody to OX40 as measured, e.g., by a radioimmunoassay (RIA). In certain aspects, an antibody that binds to OX40 has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M). In certain aspects, an anti-OX40 antibody binds to an epitope of OX40 that is conserved among OX40 from different species.

As used herein, the term "binds", "specifically binds to" or is "specific for" refers to measurable and reproducible interactions such as binding between a target and an antibody, which is determinative of the presence of the target in the presence of a heterogeneous population of molecules including biological molecules. For example, an antibody that binds to or specifically binds to a target (which can be an epitope) is an antibody that binds this target with greater affinity, avidity, more readily, and/or with greater duration than it binds to other targets. In one aspect, the extent of binding of an antibody to an unrelated target is less than about 10% of the binding of the antibody to the target as measured, e.g., by a radioimmunoassay (RIA). In certain embodiments, an antibody that specifically binds to a target has a dissociation constant (Kd) of ≤ 1µM, ≤ 100 nM, ≤ 10 nM, ≤ 1 nM, or ≤ 0.1 nM. In certain aspects, an antibody specifically binds to an epitope on a protein that is conserved among the protein from different species. In another aspect, specific binding can include, but does not require exclusive binding.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "binding domain" refers to the region of a polypeptide that binds to another molecule. In the case of an FcR, the binding domain can comprise a portion of a polypeptide chain thereof (*e.g.* the alpha chain thereof) which is responsible for binding an Fc region. One useful binding domain is the extracellular domain of an FcR alpha chain.

A polypeptide with a variant IgG Fc with "altered" FcR, ADCC or phagocytosis activity is one which has either enhanced or diminished FcR binding activity (e.g, FcγR) and/or ADCC activity and/or phagocytosis activity compared to a parent polypeptide or to a polypeptide comprising a native sequence Fc region.

The term "OX40," as used herein, refers to any native OX40 from any vertebrate source, including mammals such as primates (e.g. humans) and rodents (e.g., mice and rats), unless otherwise indicated. The term encompasses "full-length," unprocessed OX40 as well as any form of OX40 that results from processing in the cell. The term also encompasses naturally occurring variants of OX40, e.g., splice variants or allelic variants. The amino acid sequence of an exemplary human OX40 is shown in SEQ ID NO:1.

"OX40 activation" refers to activation, of the OX40 receptor. Generally, OX40 activation results in signal transduction.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia or lymphoid malignancies. More particular examples of such cancers include, but not limited to, squamous cell cancer (*e.g.*, epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer and gastrointestinal stromal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, superficial spreading melanoma, lentigo maligna melanoma, acral lentiginous melanomas, nodular melanomas, multiple myeloma and B-cell lymphoma; chronic lymphocytic leukemia (CLL); acute lymphoblastic leukemia (ALL); hairy cell leukemia; chronic myeloblastic leukemia; and post-transplant lymphoproliferative disorder (PTLD), as well as abnormal vascular proliferation associated with phakomatoses, edema (such as that associated with brain tumors), Meigs' syndrome, brain, as well as head and neck cancer, and associated metastases. In certain aspects, cancers that are amenable to treatment by the antibodies of the invention include breast cancer, colorectal cancer, rectal cancer, non-small cell lung cancer, glioblastoma, non-Hodgkins lymphoma (NHL), renal cell cancer, prostate cancer, liver cancer, pancreatic cancer, soft-tissue sarcoma, kaposi's sarcoma, carcinoid carcinoma, head and neck cancer, ovarian cancer, mesothelioma, and multiple myeloma. In some aspects, the cancer is selected from: non-small cell lung cancer, glioblastoma, neuroblastoma, melanoma, breast carcinoma (e.g. triple-negative breast cancer), gastric cancer, colorectal cancer (CRC), and hepatocellular carcinoma. Yet, in some aspects, the cancer is selected from: non-small cell lung cancer, colorectal cancer, glioblastoma and breast carcinoma (e.g. triple-negative breast cancer), including metastatic forms of those cancers.

The terms "cell proliferative disorder" and "proliferative disorder" refer to disorders that are associated with some degree of abnormal cell proliferation. In one aspect, the cell proliferative disorder is cancer.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, *e.g.,* as described in Gazzano-Santoro et al., J. Immunol. Methods 202:163 (1996), may be performed. Polypeptide variants with altered Fc region amino acid sequences (polypeptides with a variant Fc region) and increased or decreased C1q binding capability are described, *e.g.,* in US Patent No. 6,194,551 B1 and WO 1999/51642. See also, *e.g.,* Idusogie et al. J. Immunol. 164: 4178-4184 (2000).

The term "cytostatic agent" refers to a compound or composition which arrests growth of a cell either in vitro or in vivo. Thus, a cytostatic agent may be one which significantly reduces the percentage of cells in S phase. Further examples of cytostatic agents include agents that block cell cycle progression by inducing G0/G1 arrest or M-phase arrest. The humanized anti-Her2 antibody trastuzumab (HERCEPTIN®) is an example of a cytostatic agent that induces G0/G1 arrest. Classical M-phase blockers include the vincas (vincristine and vinblastine), taxanes, and topoisomerase II inhibitors such as doxorubicin, epirubicin, daunorubicin, etoposide, and bleomycin. Certain agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in Mendelsohn and Israel, eds., The Molecular Basis of Cancer, Chapter 1, entitled "Cell cycle regulation, oncogenes, and antineoplastic drugs" by Murakami et al. (W.B. Saunders, Philadelphia, 1995), e.g., p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

A "depleting anti-OX40 antibody," is an anti-OX40 antibody that kills or depletes OX40-expressing cells. Depletion of OX40 expressing cells can be achieved by various mechanisms, such as antibody-dependent cell-mediated cytotoxicity and/or phagocytosis. Depletion of OX40-expressing cells may be assayed in vitro, and exemplary methods for in vitro ADCC and phagocytosis assays are provided herein. In some embodiments, the OX40-expressing cell is a human CD4+ effector T cell. In some embodiments, the OX40-expressing cell is a transgenic BT474 cell that expresses human OX40.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

"Fc receptor" or "FcR" describes a receptor that binds to the Fc region of an antibody. In some embodiments, an FcR is a native human FcR. In some aspects, an FcR is one which binds an IgG antibody (a gamma receptor) and includes receptors of the FcγRI, FcγRII, and FcγRIII subclasses, including allelic variants and alternatively spliced forms of those receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see, *e.g.,* Daëron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known (see, *e.g.,* Ghetie and Ward., Immunol. Today 18(12):592-598 (1997); Ghetie et al., Nature Biotechnology, 15(7):637-640 (1997); Hinton et al., J. Biol. Chem. 279(8):6213-6216 (2004); WO 2004/92219 (Hinton et al.). Binding to human FcRn *in vivo* and serum half life of human FcRn high affinity binding polypeptides can be assayed, *e.g.,* in transgenic mice or transfected human cell lines expressing human FcRn, or in primates to which the polypeptides with a variant Fc region are administered. WO 2000/42072 (Presta) describes antibody variants with improved or diminished binding to FcRs. See also, *e.g.,* Shields et al. J. Biol. Chem. 9(2):6591-6604 (2001).

The term "Fc region" herein is used to define a C-terminal region of an immunoglobulin heavy chain that contains at least a portion of the constant region. The term includes native sequence Fc regions and variant Fc regions. In one aspect, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

A "functional Fc region" possesses an "effector function" of a native sequence Fc region. Exemplary "effector functions" include C1q binding; CDC; Fc receptor binding; ADCC; phagocytosis; down regulation of cell surface receptors (e.g. B cell receptor; BCR), etc. Such effector functions generally require the Fc region to be combined with a binding domain (*e.g*., an antibody variable domain) and can be assessed using various assays as disclosed, for example, in definitions herein.

"Human effector cells" refer to leukocytes that express one or more FcRs and perform effector functions. In certain embodiments, the cells express at least FcγRIII and perform ADCC effector function(s). Examples of human leukocytes which mediate ADCC include peripheral blood mononuclear cells (PBMC), natural killer (NK) cells, monocytes, cytotoxic T cells, and neutrophils. The effector cells may be isolated from a native source, e.g., from blood.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one aspect, for the VL, the subgroup is subgroup kappa I as in Kabat et al., *supra.* In one aspect, for the VH, the subgroup is subgroup III as in Kabat et al., *supra.*

A "humanized" antibody refers to a chimeric antibody comprising amino acid residues from non-human HVRs and amino acid residues from human FRs. In certain aspects, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (HI, H2, H3), and three in the VL (LI, L2, L3). Exemplary HVRs herein include:
(a) hypervariable loops occurring at amino acid residues 26-32 (LI), 50-52 (L2), 91-96 (L3), 26-32 (HI), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (HI), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (LI), 46-55 (L2), 89-96 (L3), 30-35b (HI), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (HI), 26-35b (HI), 49-65 (H2), 93-102 (H3), and 94-102 (H3).

Unless otherwise indicated, HVR residues and other residues in the variable domain (e.g., FR residues) are numbered herein according to Kabat et al., *supra.*

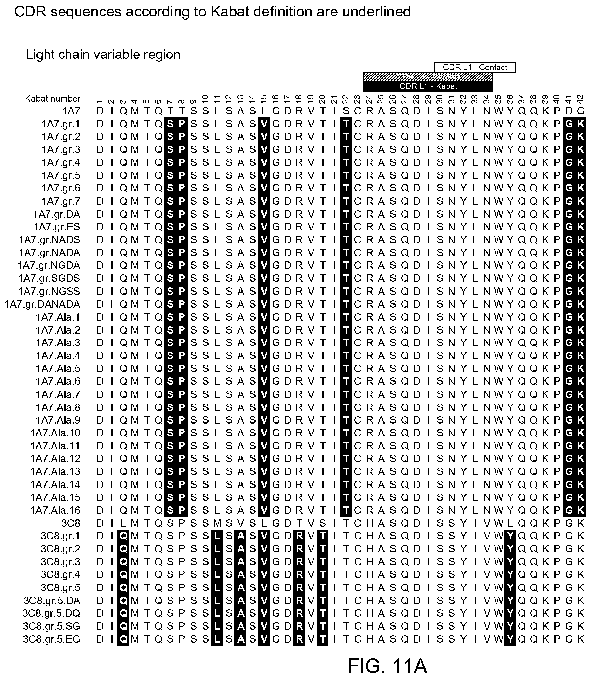

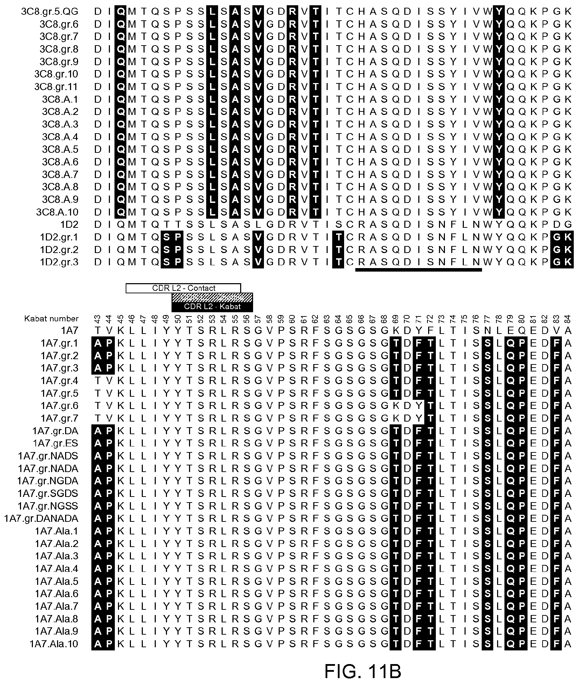

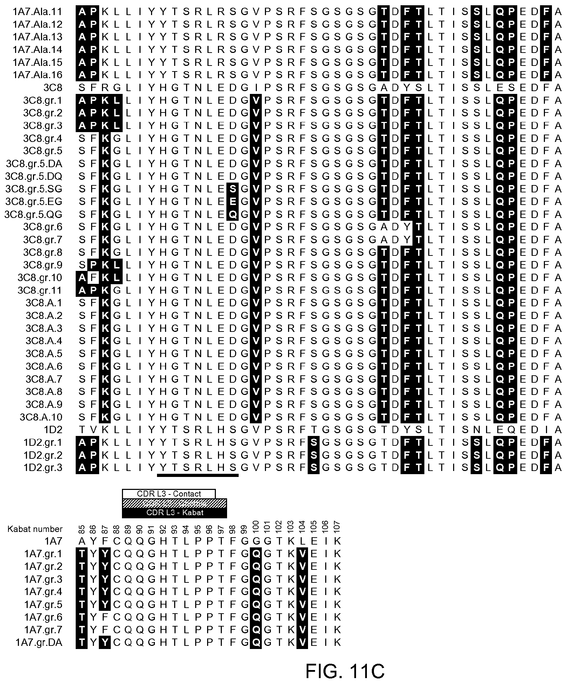

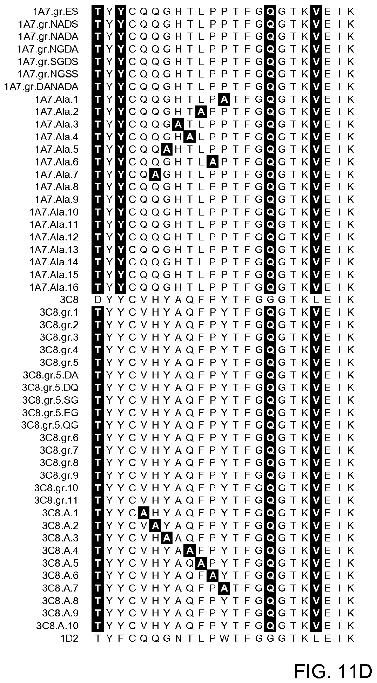

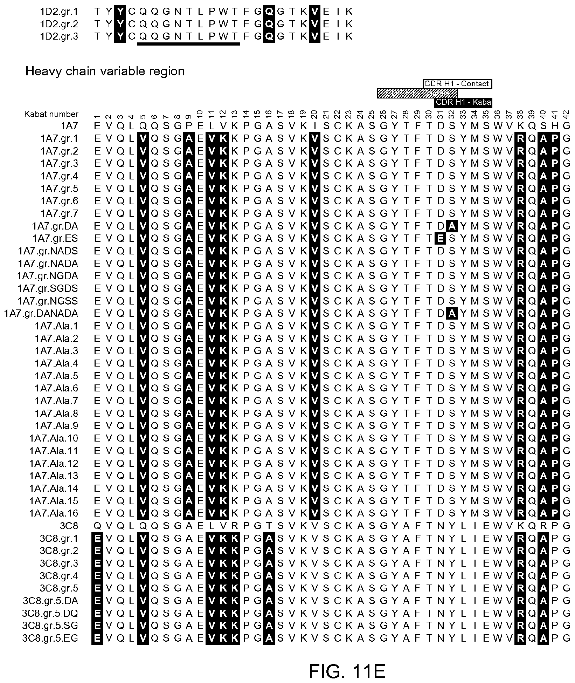

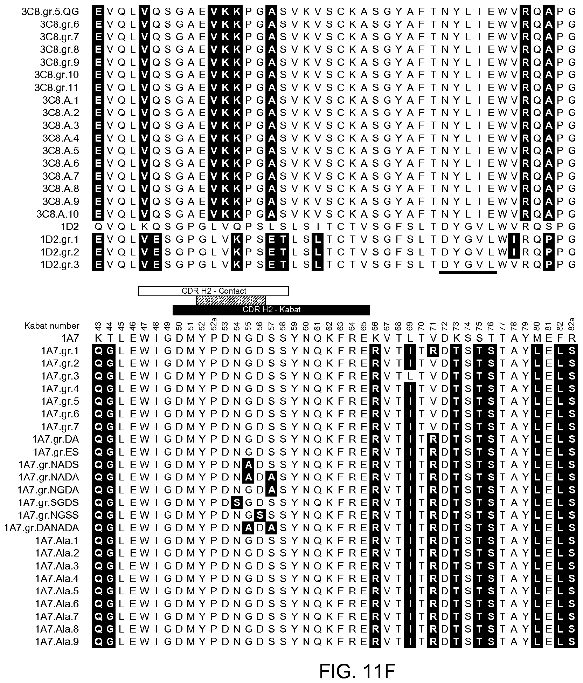

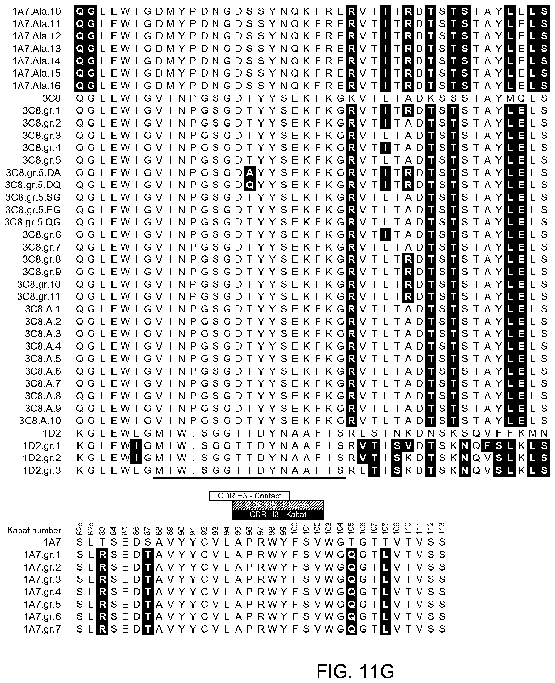

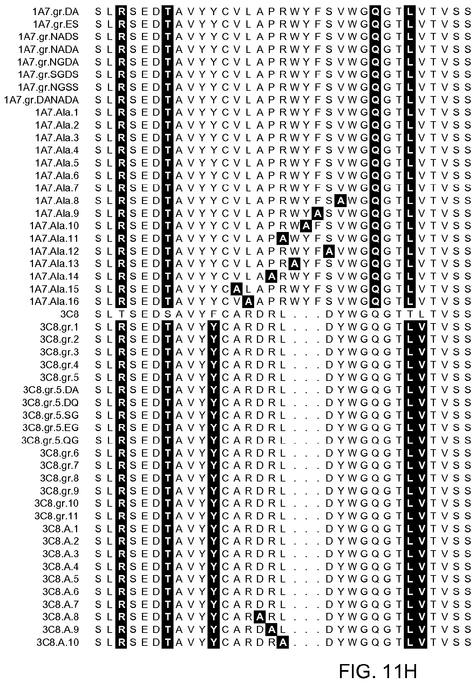

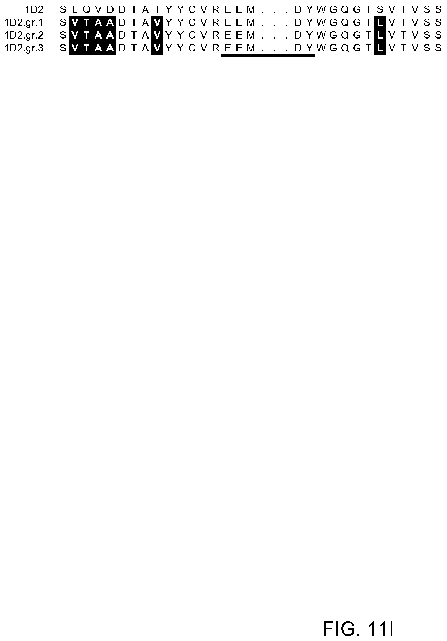

In one aspect, HVR residues comprise those identified in FIGS. 11A-I or elsewhere in the specification.

An "immunoconjugate" is an antibody conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain aspects, the individual or subject is a human.

"Promoting cell growth or proliferation" means increasing a cell's growth or proliferation by at least 10%, 20%, 30%, 40%, 50%, 60%, 70%, 80%, 90%, 95%, or 100%.

An "isolated" antibody is one which has been separated from a component of its natural environment. In some aspects, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-OX40 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain. A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some aspects, antibodies of the invention are used to delay development of a disease or to slow the progression of a disease.

The term "tumor" refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

A "variant Fc region" comprises an amino acid sequence which differs from that of a native sequence Fc region by virtue of at least one amino acid modification, preferably one or more amino acid substitution(s). Preferably, the variant Fc region has at least one amino acid substitution compared to a native sequence Fc region or to the Fc region of a parent polypeptide, e.g. from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

A "VH subgroup III consensus framework" comprises the consensus sequence obtained from the amino acid sequences in variable heavy subgroup III of Kabat et al. In one embodiment, the VH subgroup III consensus framework amino acid sequence comprises at least a portion or all of each of the following sequences: EVQLVESGGGLVQPGGSLRLSCAAS (SEQ ID NO:185)-H1-WVRQAPGKGLEWV (SEQ ID NO: 186)-H2-RFTISRDNSKNTLYLQMNSLRAEDTAVYYC (SEQ ID NO:187)-H3-WGQGTLVTVSS (SEQ ID NO:188).

A "VL subgroup I consensus framework" comprises the consensus sequence obtained from the amino acid sequences in variable light kappa subgroup I of Kabat et al. In one embodiment, the VH subgroup I consensus framework amino acid sequence comprises at least a portion or all of each of the following sequences:

The term "cytotoxic agent" as used herein refers to a substance that inhibits or prevents a cellular function and/or causes cell death or destruction. Cytotoxic agents include, but are not limited to, radioactive isotopes (e.g., At211, 1131, 1125, Y90, Re186, Re188, Sm¹⁵³, Bi212, P32, Pb212 and radioactive isotopes of Lu); chemotherapeutic agents; growth inhibitory agents; enzymes and fragments thereof such as nucleolytic enzymes; and toxins such as small molecule toxins or enzymatically active toxins of bacterial, fungal, plant or animal origin, including fragments and/or variants thereof. Exemplary cytotoxic agents can be selected from anti-microtubule agents, platinum coordination complexes, alkylating agents, antibiotic agents, topoisomerase II inhibitors, antimetabolites, topoisomerase I inhibitors, hormones and hormonal analogues, signal transduction pathway inhibitors, non-receptor tyrosine kinase angiogenesis inhibitors, immunotherapeutic agents, proapoptotic agents, inhibitors of LDH-A; inhibitors of fatty acid biosynthesis; cell cycle signalling inhibitors; HDAC inhibitors, proteasome inhibitors; and inhibitors of cancer metabolism.

In one aspect the cytotoxic agent is selected from anti-microtubule agents, platinum coordination complexes, alkylating agents, antibiotic agents, topoisomerase II inhibitors, antimetabolites, topoisomerase I inhibitors, hormones and hormonal analogues, signal transduction pathway inhibitors, non-receptor tyrosine kinase angiogenesis inhibitors, immunotherapeutic agents, proapoptotic agents, inhibitors of LDH-A, inhibitors of fatty acid biosynthesis, cell cycle signalling inhibitors, HDAC inhibitors, proteasome inhibitors, and inhibitors of cancer metabolism. In one aspect the cytotoxic agent is a taxane. In one aspect the taxane is paclitaxel or docetaxel. In one aspect the cytotoxic agent is a platinum agent. In one aspect the cytotoxic agent is an antagonist of EGFR. In one aspect the antagonist of EGFR is N-(3-ethynylphenyl)-6,7-bis(2-methoxyethoxy)quinazolin-4-amine (e.g., erlotinib). In one aspect the cytotoxic agent is a RAF inhibitor. In one aspect, the RAF inhibitor is a BRAF and/or CRAF inhibitor. In one aspect the RAF inhibitor is vemurafenib. In one aspect the cytotoxic agent is a PI3K inhibitor.

"Chemotherapeutic agent" includes chemical compounds useful in the treatment of cancer. Examples of chemotherapeutic agents include erlotinib (TARCEVA®, Genentech/OSI Pharm.), bortezomib (VELCADE®, Millennium Pharm.), disulfiram, epigallocatechin gallate , salinosporamide A, carfilzomib, 17-AAG (geldanamycin), radicicol, lactate dehydrogenase A (LDH-A), fulvestrant (FASLODEX®, AstraZeneca), sunitib (SUTENT®, Pfizer/Sugen), letrozole (FEMARA®, Novartis), imatinib mesylate (GLEEVEC®, Novartis), finasunate (VATALANIB®, Novartis), oxaliplatin (ELOXATIN®, Sanofi), 5-FU (5-fluorouracil), leucovorin, Rapamycin (Sirolimus, RAPAMUNE®, Wyeth), Lapatinib (TYKERB®, GSK572016, Glaxo Smith Kline), Lonafamib (SCH 66336), sorafenib (NEXAVAR®, Bayer Labs), gefitinib (IRESSA®, AstraZeneca), AG1478, alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including topotecan and irinotecan); bryostatin; callystatin; CC-1065 (including its adozelesin, carzelesin and bizelesin synthetic analogs); cryptophycins (particularly cryptophycin 1 and cryptophycin 8); adrenocorticosteroids (including prednisone and prednisolone); cyproterone acetate; 5α-reductases including finasteride and dutasteride); vorinostat, romidepsin, panobinostat, valproic acid, mocetinostat dolastatin; aldesleukin, talc duocarmycin (including the synthetic analogs, KW-2189 and CB1-TM1); eleutherobin; pancratistatin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin γ1I and calicheamicin ω1I (Angew Chem. Intl. Ed. Engl. 1994 33:183-186); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® (doxorubicin), morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidamnol; nitraerine; pentostatin; phenamet; pirarubicin; losoxantrone; podophyllinic acid; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL (paclitaxel; Bristol-Myers Squibb Oncology, Princeton, N.J.), ABRAXANE® (Cremophor-free), albumin-engineered nanoparticle formulations of paclitaxel (American Pharmaceutical Partners, Schaumberg, Ill.), and TAXOTERE® (docetaxel, doxetaxel; Sanofi-Aventis); chloranmbucil; GEMZAR® (gemcitabine); 6-thioguanine; mercaptopurine; methotrexate; platinum analogs such as cisplatin and carboplatin; vinblastine; etoposide (VP-16); ifosfamide; mitoxantrone; vincristine; NAVELBINE® (vinorelbine); novantrone; teniposide; edatrexate; daunomycin; aminopterin; capecitabine (XELODA®); ibandronate; CPT-11; topoisomerase inhibitor RFS 2000; difluoromethylornithine (DMFO); retinoids such as retinoic acid; and pharmaceutically acceptable salts, acids and derivatives of any of the above.

Chemotherapeutic agent also includes (i) anti-hormonal agents that act to regulate or inhibit hormone action on tumors such as anti-estrogens and selective estrogen receptor modulators (SERMs), including, for example, tamoxifen (including NOLVADEX®; tamoxifen citrate), raloxifene, droloxifene, iodoxyfene , 4-hydroxytamoxifen, trioxifene, keoxifene, LY117018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RIVISOR® (vorozole), FEMARA® (letrozole; Novartis), and ARIMIDEX® (anastrozole; AstraZeneca); (iii) anti-androgens such as flutamide, nilutamide, bicalutamide, leuprolide and goserelin; buserelin, tripterelin, medroxyprogesterone acetate, diethylstilbestrol, premarin, fluoxymesterone, all transretionic acid, fenretinide, as well as troxacitabine (a 1,3-dioxolane nucleoside cytosine analog); (iv) protein kinase inhibitors; (v) lipid kinase inhibitors; (vi) antisense oligonucleotides, particularly those which inhibit expression of genes in signaling pathways implicated in aberrant cell proliferation, such as, for example, PKC-alpha, Ralf and H-Ras; (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAXID®; PROLEUKIN®, rIL-2; a topoisomerase 1 inhibitor such as LURTOTECAN®; ABARELIX® rmRH; and (ix) pharmaceutically acceptable salts, acids and derivatives of any of the above.

Chemotherapeutic agent also includes antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), pertuzumab (OMNITARG®, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth). Additional humanized monoclonal antibodies with therapeutic potential as agents in combination with the compounds of the invention include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti-interleukin-12 (ABT-874/J695, Wyeth Research and Abbott Laboratories) which is a recombinant exclusively human-sequence, full-length IgG1 λ antibody genetically modified to recognize interleukin-12 p40 protein.

Chemotherapeutic agent also includes "EGFR inhibitors," which refers to compounds that bind to or otherwise interact directly with EGFR and prevent or reduce its signaling activity, and is alternatively referred to as an "EGFR antagonist." Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn et al.) and variants thereof, such as chimerized 225 (C225 or Cetuximab; ERBUTIX®) and reshaped human 225 (H225) (see, WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF or Panitumumab (see WO98/50433, Abgenix/Amgen); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding (EMD/Merck); human EGFR antibody, HuMax-EGFR (GenMab); fully human antibodies known as E1.1, E2.4, E2.5, E6.2, E6.4, E2.11, E6. 3 and E7.6. 3 and described in US 6,235,883; MDX-447 (Medarex Inc); and mAb 806 or humanized mAb 806 (Johns et al., J. Biol. Chem. 279(29):30375-30384 (2004)). The anti-EGFR antibody may be conjugated with a cytotoxic agent, thus generating an immunoconjugate (see, e.g., EP659,439A2, Merck Patent GmbH). EGFR antagonists include small molecules such as compounds described in US Patent Nos: 5,616,582, 5,457,105, 5,475,001, 5,654,307, 5,679,683, 6,084,095, 6,265,410, 6,455,534, 6,521,620, 6,596,726, 6,713,484, 5,770,599, 6,140,332, 5,866,572, 6,399,602, 6,344,459, 6,602,863, 6,391,874, 6,344,455, 5,760,041, 6,002,008, and 5,747,498, as well as the following PCT publications: WO98/14451, WO98/50038, WO99/09016, and WO99/24037. Particular small molecule EGFR antagonists include OSI-774 (CP-358774, erlotinib, TARCEVA® Genentech/OSI Pharmaceuticals); PD 183805 (CI 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSA®) 4-(3'-Chloro-4'-fluoroanilino)-7-methoxy-6-(3-morpholinopropoxy)quinazoline, AstraZeneca); ZM 105180 ((6-amino-4-(3-methylphenyl-amino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4-[4-[(1-phenylethyl)amino]-1H-pyrrolo[2,3-d]pyrimidin-6-yl]-phenol);(R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl]-2-butynamide); EKB-569 (N-[4-[(3-chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); dual EGFR/HER2 tyrosine kinase inhibitors such as lapatinib (TYKERB®, GSK572016 or N-[3-chloro-4-[(3 fluorophenyl)methoxy]phenyl]-6[5[[[2methylsulfonyl)ethyl]amino]methyl]-2-furanyl]-4-quinazolinamine).

Chemotherapeutic agents also include "tyrosine kinase inhibitors" including the EGFR-targeted drugs noted in the preceding paragraph; small molecule HER2 tyrosine kinase inhibitor such as TAK165 available from Takeda; CP-724,714, an oral selective inhibitor of the ErbB2 receptor tyrosine kinase (Pfizer and OSI); dual-HER inhibitors such as EKB-569 (available from Wyeth) which preferentially binds EGFR but inhibits both HER2 and EGFR-overexpressing cells; lapatinib (GSK572016; available from Glaxo-SmithKline), an oral HER2 and EGFR tyrosine kinase inhibitor; PKI-166 (available from Novartis); pan-HER inhibitors such as canertinib (CI-1033; Pharmacia); Raf-1 inhibitors such as antisense agent ISIS-5132 available from ISIS Pharmaceuticals which inhibit Raf-1 signaling; non-HER targeted TK inhibitors such as imatinib mesylate (GLEEVEC®, available from Glaxo SmithKline); multi-targeted tyrosine kinase inhibitors such as sunitinib (SUTENT®, available from Pfizer); VEGF receptor tyrosine kinase inhibitors such as vatalanib (PTK787/ZK222584, available from Novartis/Schering AG); MAPK extracellular regulated kinase I inhibitor CI-1040 (available from Pharmacia); quinazolines, such as PD 153035,4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis (4-fluoroanilino)phthalimide); tyrphostines containing nitrothiophene moieties; PD-0183805 (Warner-Lamber); antisense molecules (e.g. those that bind to HER-encoding nucleic acid); quinoxalines (US Patent No. 5,804,396); tryphostins (US Patent No. 5,804,396); ZD6474 (Astra Zeneca); PTK-787 (Novartis/Schering AG); pan-HER inhibitors such as CI-1033 (Pfizer); Affinitac (ISIS 3521; Isis/Lilly); imatinib mesylate (GLEEVEC®); PKI 166 (Novartis); GW2016 (Glaxo SmithKline); CI-1033 (Pfizer); EKB-569 (Wyeth); Semaxinib (Pfizer); ZD6474 (AstraZeneca); PTK-787 (Novartis/Schering AG); INC-1C11 (Imclone), rapamycin (sirolimus, RAPAMUNE®); or as described in any of the following patent publications: US Patent No. 5,804,396; WO 1999/09016 (American Cyanamid); WO 1998/43960 (American Cyanamid); WO 1997/38983 (Warner Lambert); WO 1999/06378 (Warner Lambert); WO 1999/06396 (Warner Lambert); WO 1996/30347 (Pfizer, Inc); WO 1996/33978 (Zeneca); WO 1996/3397 (Zeneca) and WO 1996/33980 (Zeneca).

Chemotherapeutic agents also include dexamethasone, interferons, colchicine, metoprine, cyclosporine, amphotericin, metronidazole, alemtuzumab, alitretinoin, allopurinol, amifostine, arsenic trioxide, asparaginase, BCG live, bevacuzimab, bexarotene, cladribine, clofarabine, darbepoetin alfa, denileukin, dexrazoxane, epoetin alfa, elotinib, filgrastim, histrelin acetate, ibritumomab, interferon alfa-2a, interferon alfa-2b, lenalidomide, levamisole, mesna, methoxsalen, nandrolone, nelarabine, nofetumomab, oprelvekin, palifermin, pamidronate, pegademase, pegaspargase, pegfilgrastim, pemetrexed disodium, plicamycin, porfimer sodium, quinacrine, rasburicase, sargramostim, temozolomide, VM-26, 6-TG, toremifene, tretinoin, ATRA, valrubicin, zoledronate, and zoledronic acid, and pharmaceutically acceptable salts thereof.

Chemotherapeutic agents also include hydrocortisone, hydrocortisone acetate, cortisone acetate, tixocortol pivalate, triamcinolone acetonide, triamcinolone alcohol, mometasone, amcinonide, budesonide, desonide, fluocinonide, fluocinolone acetonide, betamethasone, betamethasone sodium phosphate, dexamethasone, dexamethasone sodium phosphate, fluocortolone, hydrocortisone-17-butyrate, hydrocortisone-17-valerate, aclometasone dipropionate, betamethasone valerate, betamethasone dipropionate, prednicarbate, clobetasone-17-butyrate, clobetasol-17-propionate, fluocortolone caproate, fluocortolone pivalate and fluprednidene acetate; immune selective anti-inflammatory peptides (ImSAIDs) such as phenylalanine-glutamine-glycine (FEG) and its D-isomeric form (feG) (IMULAN BioTherapeutics, LLC); anti-rheumatic drugs such as azathioprine, ciclosporin (cyclosporine A), D-penicillamine, gold salts, hydroxychloroquine, leflunomideminocycline, sulfasalazine, tumor necrosis factor alpha (TNFα) blockers such as etanercept (Enbrel), infliximab (Remicade), adalimumab (Humira), certolizumab pegol (Cimzia), golimumab (Simponi), Interleukin 1 (IL-1) blockers such as anakinra (Kineret), T cell costimulation blockers such as abatacept (Orencia), Interleukin 6 (IL-6) blockers such as tocilizumab (ACTEMERA®); Interleukin 13 (IL-13) blockers such as lebrikizumab; Interferon alpha (IFN) blockers such as Rontalizumab; Beta 7 integrin blockers such as rhuMAb Beta7; IgE pathway blockers such as Anti-M1 prime; Secreted homotrimeric LTa3 and membrane bound heterotrimer LTa1/β2 blockers such as Anti-lymphotoxin alpha (LTa); radioactive isotopes (e.g., At211, 1131, 1125, Y90, Re186, Re188, Sm¹⁵³, Bi212, P32, Pb212 and radioactive isotopes of Lu); miscellaneous investigational agents such as thioplatin, PS-341, phenylbutyrate, ET-18- OCH3, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; autophagy inhibitors such as chloroquine; delta-9-tetrahydrocannabinol (dronabinol, MARINOL®); beta-lapachone; lapachol; colchicines; betulinic acid; acetylcamptothecin, scopolectin, and 9-aminocamptothecin); podophyllotoxin; tegafur (UFTORAL®); bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); and epidermal growth factor receptor (EGF-R); vaccines such as THERATOPE® vaccine; perifosine, COX-2 inhibitor (e.g. celecoxib or etoricoxib), proteosome inhibitor (e.g. PS341); CCI-779; tipifarnib (R11577); orafenib, ABT510; Bcl-2 inhibitor such as oblimersen sodium (GENASENSE®); pixantrone; farnesyltransferase inhibitors such as lonafarnib (SCH 6636, SARASARTM); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATINTM) combined with 5-FU and leucovorin.

Chemotherapeutic agents also include non-steroidal anti-inflammatory drugs with analgesic, antipyretic and anti-inflammatory effects. NSAIDs include non-selective inhibitors of the enzyme cyclooxygenase. Specific examples of NSAIDs include aspirin, propionic acid derivatives such as ibuprofen, fenoprofen, ketoprofen, flurbiprofen, oxaprozin and naproxen, acetic acid derivatives such as indomethacin, sulindac, etodolac, diclofenac, enolic acid derivatives such as piroxicam, meloxicam, tenoxicam, droxicam, lornoxicam and isoxicam, fenamic acid derivatives such as mefenamic acid, meclofenamic acid, flufenamic acid, tolfenamic acid, and COX-2 inhibitors such as celecoxib, etoricoxib, lumiracoxib, parecoxib, rofecoxib, rofecoxib, and valdecoxib. NSAIDs can be indicated for the symptomatic relief of conditions such as rheumatoid arthritis, osteoarthritis, inflammatory arthropathies, ankylosing spondylitis, psoriatic arthritis, Reiter's syndrome, acute gout, dysmenorrhoea, metastatic bone pain, headache and migraine, postoperative pain, mild-to-moderate pain due to inflammation and tissue injury, pyrexia, ileus, and renal colic.

The term "cytokine" is a generic term for proteins released by one cell population that act on another cell as intercellular mediators. Examples of such cytokines are lymphokines, monokines; interleukins (ILs) such as IL-1, IL-1a, IL-2, IL-3, IL-4, IL-5, IL-6, IL-7, IL-8, IL-9, IL-11, IL-12, IL-15; a tumor necrosis factor such as TNF-α or TNF-β; and other polypeptide factors including LIF and kit ligand (KL) and gamma interferon. As used herein, the term cytokine includes proteins from natural sources or from recombinant cell culture and biologically active equivalents of the native-sequence cytokines, including synthetically produced small-molecule entities and pharmaceutically acceptable derivatives and salts thereof.

The term "PD-1 axis binding antagonist" is a molecule that inhibits the interaction of a PD-1 axis binding partner with either one or more of its binding partner, so as to remove T-cell dysfunction resulting from signaling on the PD-1 signaling axis - with a result being to restore or enhance T-cell function (e.g., proliferation, cytokine production, target cell killing). As used herein, a PD-1 axis binding antagonist includes a PD-1 binding antagonist, a PD-L1 binding antagonist and a PD-L2 binding antagonist.

The term "PD-1 binding antagonists" is a molecule that decreases, blocks, inhibits, abrogates or interferes with signal transduction resulting from the interaction of PD-1 with one or more of its binding partners, such as PD-L1, PD-L2. In some aspects, the PD-1 binding antagonist is a molecule that inhibits the binding of PD-1 to its binding partners. In a specific aspect, the PD-1 binding antagonist inhibits the binding of PD-1 to PD-L1 and/or PD-L2. For example, PD-1 binding antagonists include anti-PD-1 antibodies, antigen binding fragments thereof, immunoadhesins, fusion proteins, oligopeptides and other molecules that decrease, block, inhibit, abrogate or interfere with signal transduction resulting from the interaction of PD-1 with PD-L1 and/or PD-L2. In one aspect, a PD-1 binding antagonist reduces the negative co-stimulatory signal mediated by or through cell surface proteins expressed on T lymphocytes mediated signaling through PD-1 so as render a dysfunctional T-cell less dysfunctional (e.g. , enhancing effector responses to antigen recognition). In some aspects, the PD-1 binding antagonist is an anti-PD-1 antibody. In a specific aspect, a PD-1 binding antagonist is MDX- 1 106 described herein. In another specific aspect, a PD-1 binding antagonist is Merck 3745 described herein. In another specific aspect, a PD-1 binding antagonist is CT-011 described herein.

The term "PD-L1 binding antagonists" is a molecule that decreases, blocks, inhibits, abrogates or interferes with signal transduction resulting from the interaction of PD-L1 with either one or more of its binding partners, such as PD-1, B7-1. In some aspects, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some aspects, the PD-L1 binding antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, B7- 1. In one aspect, a PD-L1 binding antagonist reduces the negative co-stimulatory signal mediated by or through cell surface proteins expressed on T lymphocytes mediated signaling through PD-L1 so as to render a dysfunctional T-cell less dysfunctional (e.g. , enhancing effector responses to antigen recognition). In some embodiments, a PD-L1 binding antagonist is an anti-PD-L1 antibody. In a specific aspect, an anti-PD-L1 antibody is YW243.55.S70 described herein. In another specific aspect, an anti-PD-L1 antibody is MDX- 1 105 described herein. In still another specific aspect, an anti-PD-L1 antibody is MPDL3280A described herein.

The term "PD-L2 binding antagonists" is a molecule that decreases, blocks, inhibits, abrogates or interferes with signal transduction resulting from the interaction of PD-L2 with either one or more of its binding partners, such as PD-1. In some aspects, a PD-L2 binding antagonist is a molecule that inhibits the binding of PD-L2 to its binding partners. In a specific aspect, the PD-L2 binding antagonist inhibits binding of PD-L2 to PD-1. In some aspects, the PD-L2 antagonists include anti-PD-L2 antibodies, antigen binding fragments thereof, immunoadhesins, fusion proteins, oligopeptides and other molecules that decrease, block, inhibit, abrogate or interfere with signal transduction resulting from the interaction of PD-L2 with either one or more of its binding partners, such as PD-1. In one aspect, a PD-L2 binding antagonist reduces the negative co-stimulatory signal mediated by or through cell surface proteins expressed on T lymphocytes mediated signaling through PD-L2 so as render a dysfunctional T-cell less dysfunctional (e.g., enhancing effector responses to antigen recognition). In some embodiments, a PD-L2 binding antagonist is an immunoadhesin.

The term "phagocytosis" means the internalization of cells or particulate matter by cells. In some embodiments, the phagocytic cells or phagocytes are macrophages or neutrophils. In some aspects, the cells are cells that express human OX40. Methods for assaying phagocytosis are known in the art and include use of microscopy to detect the presence of cells internalized within another cells. In other aspects, phagocytosis is detected using FACS, e.g., by detecting presence of a detectably labeled cell within another cell (which may be detectably labeled, e.g., with a different label than the first cell).

The phrase "does not possess substantial activity" or "substantially no activity" with respect to an antibody, as used herein, means the antibody does not exhibit an activity that is above background level (in some embodiments, that is above background level that is statistically significant). The phrase "little to no activity" with respect to an antibody, as used herein, means the antibody does not display a biologically meaningful amount of a function. The function can be measured or detected according to any assay or technique known in the art, including, e.g., those described herein. In some aspects, antibody function is stimulation of effector T cell proliferation and/or cytokine secretion.

The term "biomarker" or "marker" as used herein refers generally to a molecule, including a gene, mRNA, protein, carbohydrate structure, or glycolipid, the expression of which in or on a tissue or cell or secreted can be detected by known methods (or methods disclosed herein) and is predictive or can be used to predict (or aid prediction) for a cell, tissue, or patient's responsiveness to treatment regimes.

By "patient sample" is meant a collection of cells or fluids obtained from a cancer patient. The source of the tissue or cell sample may be solid tissue as from a fresh, frozen and/or preserved organ or tissue sample or biopsy or aspirate; blood or any blood constituents; bodily fluids such as cerebrospinal fluid, amniotic fluid, peritoneal fluid, or interstitial fluid; cells from any time in gestation or development of the subject. The tissue sample may contain compounds which are not naturally intermixed with the tissue in nature such as preservatives, anticoagulants, buffers, fixatives, nutrients, antibiotics, or the like. Examples of tumor samples herein include, but are not limited to, tumor biopsy, fine needle aspirate, bronchiolar lavage, pleural fluid, sputum, urine, a surgical specimen, circulating tumor cells, serum, plasma, circulating plasma proteins, ascitic fluid, primary cell cultures or cell lines derived from tumors or exhibiting tumor-like properties, as well as preserved tumor samples, such as formalin-fixed, paraffin-embedded tumor samples or frozen tumor samples.

The phrase "based on expression of" when used herein means that information about expression level or presence or absence of expression (e.g., presence or absence or prevalence of (e.g., percentage of cells displaying) of the one or more biomarkers herein (e.g., presence or absence of or amount or prevelance of FcR-expressing cells, or e.g., presence or absence or amount or prevelance of human effector cells) is used to inform a treatment decision, information provided on a package insert, or marketing/promotional guidance etc.

A cancer or biological sample which "has human effector cells" is one which, in a diagnostic test, has human effector cells present in the sample (e.g., infiltrating human effector cells).

A cancer or biological sample which "has FcR-expressing cells" is one which, in a diagnostic test, has FcR-expressing present in the sample (e.g., infiltrating FcR-expressing cells). In some embodiments, FcR is FcγR. In some embodiments, FcR is an activating FcγR.

The phrase "recommending a treatment" as used herein refers to using the information or data generated relating to the level or presence of c-met in a sample of a patient to identify the patient as suitably treated or not suitably treated with a therapy. In some aspects the therapy may comprise c-met antibody (e.g., onartuzumab). In some aspects, the therapy may comprise VEGF antagonist (e.g., bevacizumab). In some aspects, the therapy may comprise anti-human OX40 agonist antibody. The information or data may be in any form, written, oral or electronic. In some aspects, using the information or data generated includes communicating, presenting, reporting, storing, sending, transferring, supplying, transmitting, delivering, dispensing, or combinations thereof. In some aspects, communicating, presenting, reporting, storing, sending, transferring, supplying, transmitting, delivering, dispensing, or combinations thereof are performed by a computing device, analyzer unit or combination thereof. In some further aspects, communicating, presenting, reporting, storing, sending, transferring, supplying, transmitting, dispensing, or combinations thereof are performed by an individual (e.g., a laboratory or medical professional). In some aspects, the information or data includes a comparison of the amount or prevelance of FcR expressing cells to a reference level. In some aspects, the information or data includes a comparison of the amount or prevelance of human effector cells to a reference level. In some aspects, the information or data includes an indication that human effector cells or FcR-expressing cells are present or absent in the sample. In some aspects, the information or data includes an indication that FcR-expressing cells and/or human effector cells are present in a particular percentage of cells (e.g., high prevelance). In some aspects, the information or data includes an indication that the patient is suitably treated or not suitably treated with a therapy comprising anti-human OX40 agonist antibody.

### II. COMPOSITIONS AND METHODS

In one aspect, the disclosure is based, in part, on identification of a variety of OX40 binding agents. In certain aspects, antibodies (e.g., agonist antibodies) that bind to human OX40 are provided. Antibodies of the disclosure are useful, e.g., for the diagnosis or treatment of cancer and other disorders associated with OX40 expression and/or activity.

### A. Exemplary Anti-OX40 Antibodies

In one aspect, the provided are isolated antibodies that bind to human OX40.

In some aspects, the anti-human OX40 agonist antibody binds human OX40 with an affinity of less than or equal to about 0.45 nM. In some aspects, the anti-human OX40 antibody binds human OX40 with an affinity of less than or equal to about 0.4 nM. In some aspects, the anti-human OX40 antibody binds human OX40 with an affinity of less than or equal to about 0.5nM. In some aspects, the binding affinity is determined using radioimmunoassay.

In some aspects, the anti-human OX40 agonist antibody binds human OX40 and cynomolgus OX40. In some aspects, binding is determined using a FACS assay. In some aspects, binding to human OX40 has an EC50 of about 0.2 µg/ml. In some aspects, binding to human OX40 has an EC50 of about 0.3 µg/ml or lower. In some aspects, binding to cynomolgus OX40 has an EC50 of about 1.5 µg/ml. In some aspects, binding to cynomolgus OX40 has an EC50 of about 1.4 µg/ml.

In some aspects, the anti-human OX40 agonist antibody does not bind to rat OX40 or mouse OX40.

In some aspects, the anti-human OX40 agonist antibody is a depleting anti-human OX40 antibody (e.g., depletes cells that express human OX40). In some aspects, the human OX40 expressing cells are CD4+ effector T cells. In some aspects, the human OX40 expressing cells are Treg cells. In some aspects, depleting is by ADCC and/or phagocytosis. In some aspects, the antibody mediates ADCC by binding FcγR expressed by a human effector cell and activating the human effector cell function. In some aspects, the antibody mediates phagocytosis by binding FcγR expressed by a human effector cell and activating the human effector cell function. Exemplary human effector cells include, e.g., macrophage, natural killer (NK) cells, monocytes, neutrophils. In some embodiments, the human effector cell is macrophage. In some aspects, the human effector cell is NK cells. In some aspects, depletion is not by apoptosis.

In some aspects, the anti-human OX40 agonist antibody has a functional Fc region. In some aspects, effector function of a functional Fc region is ADCC. In some aspects, effector function of a functional Fc region is phagocytosis. In some aspects, effector function of a functional Fc region is ADCC and phagocytosis. In some aspects, the Fc region is human IgG1. In some aspects, the Fc region is human IgG4.

In some aspects, the anti-human OX40 agonist antibody does not induce apoptosis in OX40-expressing cells (e.g., Treg). In some aspects, apoptosis is assayed using an antibody concentration of 30 µg/ml, e.g., by determining whether apoptosis has occurred using annexin V and proprodium iodide stained Treg.

In some aspects, the anti-human OX40 agonist antibody enhances CD4+ effector T cell function, for example, by increasing CD4+ effector T cell proliferation and/or increasing gamma interferon production by the CD4+ effector T cell (for example, as compared to proliferation and/or cytokine production prior to treatment with anti-human OX40 agonist antibody). In some aspects, the cytokine is gamma interferon. In some aspects, the anti-human OX40 agonist antibody increases number of intratumoral (infiltrating) CD4+ effector T cells (e.g., total number of CD4+ effector T cells, or e.g., percentage of CD4+ cells in CD45+ cells), e.g., as compared to number of intratumoral (infiltrating) CD4+ T cells prior to treatment with anti-human OX40 agonist antibody. In some aspects, the anti-human OX40 agonist antibody increases number of intratumoral (infiltrating) CD4+ effector T cells that express gamma interferon (e.g., total gamma interferon expressing CD4+ cells, or e.g., percentage of gamma interferon expressing CD4+ cells in total CD4+ cells), e.g., as compared to number of intratumoral (infiltrating) CD4+ T cells that express gamma interferon prior to treatment with anti-human OX40 agonist antibody.

In some aspects, the anti-human OX40 agonist antibody increases number of intratumoral (infiltrating) CD8+ effector T cells (e.g., total number of CD8+ effector T cells, or e.g., percentage of CD8+ in CD45+ cells), e.g., as compared to number of intratumoral (infiltrating) CD8+ T effector cells prior to treatment with anti-human OX40 agonist antibody. In some aspects, the anti-human OX40 agonist antibody increases number of intratumoral (infiltrating) CD8+ effector T cells that express gamma interferon (e.g., percentage of CD8+ cells that express gamma interferon in total CD8+ cells), e.g., compared to number of intratumoral (infiltrating) CD8+ T cells that express gamma interferon prior to treatment with anti-human OX40 agonist antibody.

In some aspects, the anti-human OX40 agonist antibody enhances memory T cell function, for example by increasing memory T cell proliferation and/or increasing cytokine production by the memory cell. In some aspects, the cytokine is gamma interferon.

In some aspects, the anti-human OX40 agonist antibody inhibits Treg function, for example, by decreasing Treg suppression of effector T cell function (e.g., effector T cell proliferation and/or effector T cell cytokine secretion). In some aspects, the effector T cell is a CD4+ effector T cell. In some aspects, the anti-human OX40 agonist antibody reduces the number of intratumoral (infiltrating) Treg (e.g., total number of Treg or e.g., percentage of Fox3p+ cells in CD4+ cells).

In some aspects, the anti-human OX40 agonist antibody is engineered to increase effector function (e.g., compared to effector function in a wild-type IgG1). In some aspects, the antibody has increased binding to a Fcγ receptor. In some aspects, the antibody lacks fucose attached (directly or indirectly) to the Fc region. For example, the amount of fucose in such antibody may be from 1% to 80%, from 1% to 65%, from 5% to 65% or from 20% to 40%. In some aspects, the Fc region comprises bisected oligosaccharides, e.g., in which a biantennary oligosaccharide attached to the Fc region of the antibody is bisected by GlcNAc. In some aspects, the antibody comprises an Fc region with one or more amino acid substitutions which improve ADCC, e.g., substitutions at positions 298, 333, and/or 334 of the Fc region (EU numbering of residues).

In some aspects, the anti-human OX40 agonist antibody increases OX40 signal transduction in a target cell that expresses OX40. In some aspects, OX40 signal transduction is detected by monitoring NFkB downstream signaling.

In some aspects, the anti-human OX40 agonist antibody is stable after treatment at 40C for two weeks.

In some aspects, the anti-human OX40 agonist antibody binds human effector cells, e.g., binds FcγR (e.g., an activating FcγR) expressed by human effector cells. In some aspects, the human effector cell performs (is capable of performing) ADCC effector function. In some aspects, the human effector cell performs (is capable of performing) phagocytosis effector function.

In some aspects, the anti-human OX40 agonist antibody comprising a variant IgG1 Fc polypeptide comprising a mutation that eliminates binding to human effector cells (e.g., a DANA mutation) has diminished activity (e.g., CD4+ effector T cell function, e.g., proliferation), relative to anti-human OX40 agonist antibody comprising native sequence IgG1 Fc portion. In some embodiment, the anti-human OX40 agonist antibody comprising a variant IgG1 Fc polypeptide comprising a mutation that eliminates binding to human effector cells (e.g., a DANA mutation) does not possess substantial activity (e.g., CD4+ effector T cell function, e.g., proliferation).

In some aspects, antibody cross-linking is required for anti-human OX40 agonist antibody function. In some aspects, function is stimulation of CD4+ effector T cell proliferation. In some aspects, antibody cross-linking is determined by providing anti-human OX40 agonist antibody adhered on a solid surface (e.g., a cell culture plate). In some aspects, antibody cross-linking is determined by introducing a mutation in the antibody's IgG1 Fc portion (e.g., a DANA mutation) and testing function of the mutant antibody.

In some aspects, the anti-human OX40 agonist antibody competes for binding to human OX40 with OX40L. In some aspects, addition of OX40L does not enhance anti-human OX40 antibody function in an in vitro assay.

According to another embodiment, the anti-human OX40 agonist antibodies include any one, any combination, or all of the following properties: (1) binds human OX40 with an affinity of less than or equal to about 0.45 nM, in some aspects, binds human OX40 with an affinity of less than or equal to about 0.4 nM, in some aspects, binds human OX40 with an affinity of less than or equal to about 0.5nM, in some aspects, the binding affinity is determined using radioimmunoassay; (2) binds human OX40 and cynomolgus OX40, in some aspects, binding is determined using a FACS assay, (3) binds human OX40 with an EC50 of about 0.2 ug/ml, in some aspects, binds to human OX40 has an EC50 of about 0.3 ug/ml or lower, in some aspects, binds to cynomolgus OX40 with an EC50 of about 1.5 ug/ml, in some aspects, binds to cynomolgus OX40 has an EC50 of about 1.4 ug/ml, (4) does not substantially bind to rat OX40 or mouse OX40, (6) is a depleting anti-human OX40 antibody (e.g., depletes cells that express human OX40), in some aspects, the cells are CD4+ effector T cells and/or Treg cells, (7) enhances CD4+ effector T cell function, for example, by increasing CD4+ effector T cell proliferation and/or increasing gamma interferon production by the CD4+ effector T cell (for example, as compared to proliferation and/or cytokine production prior to treatment with anti-human OX40 agonist antibody), (8) enhances memory T cell function, for example by increasing memory T cell proliferation and/or increasing cytokine production by the memory cell, (9) inhibits Treg function, for example, by decreasing Treg suppression of effector T cell function (e.g., effector T cell proliferation and/or effector T cell cytokine secretion). In some aspects, the effector T cell is a CD4+ effector T cell, (10) increases OX40 signal transduction in a target cell that expresses OX40 (in some aspects, OX40 signal transduction is detected by monitoring NFkB downstream signaling), (11) is stable after treatment at 40C for two weeks, (12) binds human effector cells, e.g., binds FcγR expressed by human effector cells, (13) anti-human OX40 agonist antibody comprising a variant IgG1 Fc polypeptide comprising a mutation that eliminates binding to human effector cells (e.g., N297G) has diminished activity (e.g., CD4+ effector T cell function, e.g., proliferation), relative to anti-human OX40 agonist antibody comprising native sequence IgG1 Fc portion, in some embodiment, the anti-human OX40 agonist antibody comprising a variant IgG1 Fc polypeptide comprising a mutation that eliminates binding to human effector cells (e.g., N297G) does not possess substantial activity (e.g., CD4+ effector T cell function, e.g., proliferation), (14) antibody cross-linking (e.g., by Fc receptor binding) is required for anti-human OX40 agonist antibody function.

In one aspect, the disclosed is an anti-human OX40 agonist antibody comprising at least one, two, three, four, five, or six HVRs selected from (a) HVR-H1 comprising the amino acid sequence of SEQ ID NO:2; (b) HVR-H2 comprising the amino acid sequence of SEQ ID NO:3; (c) HVR-H3 comprising the amino acid sequence of SEQ ID NO:4; (d) HVR-L1 comprising the amino acid sequence of SEQ ID NO:5; (e) HVR-L2 comprising the amino acid sequence of SEQ ID NO:6; and (f) HVR-L3 comprising the amino acid sequence of SEQ ID NO:7.

In one aspect, the provided is an anti-human OX40 agonist antibody comprising at least one, at least two, or all three VH HVR sequences selected from (a) HVR-H1 comprising the amino acid sequence of SEQ ID NO:2; (b) HVR-H2 comprising the amino acid sequence of SEQ ID NO:3; and (c) HVR-H3 comprising the amino acid sequence of SEQ ID NO:4. In one embodiment, the antibody comprises HVR-H3 comprising the amino acid sequence of SEQ ID NO:4. In another embodiment, the antibody comprises HVR-H3 comprising the amino acid sequence of SEQ ID NO:4 and HVR-L3 comprising the amino acid sequence of SEQ ID NO:7. In a further embodiment, the antibody comprises HVR-H3 comprising the amino acid sequence of SEQ ID NO:4, HVR-L3 comprising the amino acid sequence of SEQ ID NO:7, and HVR-H2 comprising the amino acid sequence of SEQ ID NO:3. In a further aspect, the antibody comprises (a) HVR-H1 comprising the amino acid sequence of SEQ ID NO:2; (b) HVR-H2 comprising the amino acid sequence of SEQ ID NO:3; and (c) HVR-H3 comprising the amino acid sequence of SEQ ID NO:4.

In another aspect, the provided is an anti-human OX40 agonist antibody comprising at least one, at least two, or all three VL HVR sequences selected from (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:7. In one embodiment, the antibody comprises (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:7.

In another aspect, an anti-human OX40 agonist antibody comprises (a) a VH domain comprising at least one, at least two, or all three VH HVR sequences selected from (i) HVR-H1 comprising the amino acid sequence of SEQ ID NO:2, (ii) HVR-H2 comprising the amino acid sequence of SEQ ID NO:3, and (iii) HVR-H3 comprising an amino acid sequence selected from SEQ ID NO:4; and (b) a VL domain comprising at least one, at least two, or all three VL HVR sequences selected from (i) HVR-L1 comprising the amino acid sequence of SEQ ID NO:5, (ii) HVR-L2 comprising the amino acid sequence of SEQ ID NO:6, and (c) HVR-L3 comprising the amino acid sequence of SEQ ID NO:7.

In another aspect, the disclosed is an anti-human OX40 agonist antibody comprising (a) HVR-H1 comprising the amino acid sequence of SEQ ID NO:2; (b) HVR-H2 comprising the amino acid sequence of SEQ ID NO:3; (c) HVR-H3 comprising the amino acid sequence of SEQ ID NO:4; (d) HVR-L1 comprising the amino acid sequence of SEQ ID NO:5; (e) HVR-L2 comprising the amino acid sequence of SEQ ID NO:6; and (f) HVR-L3 comprising an amino acid sequence selected from SEQ ID NO:7.

In one aspect, disclosed is an anti-human OX40 agonist antibody comprising at least one, two, three, four, five, or six HVRs selected from (a) HVR-H1 comprising the amino acid sequence of SEQ ID NO:2; (b) HVR-H2 comprising the amino acid sequence of SEQ ID NO:3; (c) HVR-H3 comprising the amino acid sequence of SEQ ID NO:4; (d) HVR-L1 comprising the amino acid sequence of SEQ ID NO:5; (e) HVR-L2 comprising the amino acid sequence of SEQ ID NO:6; and (f) HVR-L3 comprising the amino acid sequence of SEQ ID NO:26.

In another aspect of the disclosure, the antibody comprises HVR-H3 comprising the amino acid sequence of SEQ ID NO:4 and HVR-L3 comprising the amino acid sequence of SEQ ID NO:26. In a further embodiment, the antibody comprises HVR-H3 comprising the amino acid sequence of SEQ ID NO:4, HVR-L3 comprising the amino acid sequence of SEQ ID NO:26, and HVR-H2 comprising the amino acid sequence of SEQ ID NO:3.

In another aspect, an antibody comprises (a) a VH domain comprising at least one, at least two, or all three VH HVR sequences selected from (i) HVR-H1 comprising the amino acid sequence of SEQ ID NO:2, (ii) HVR-H2 comprising the amino acid sequence of SEQ ID NO:3, and (iii) HVR-H3 comprising an amino acid sequence selected from SEQ ID NO:4; and (b) a VL domain comprising at least one, at least two, or all three VL HVR sequences selected from (i) HVR-L1 comprising the amino acid sequence of SEQ ID NO:5, (ii) HVR-L2 comprising the amino acid sequence of SEQ ID NO:6, and (c) HVR-L3 comprising the amino acid sequence of SEQ ID NO:26.

In another aspect, disclosed is an antibody comprising (a) HVR-H1 comprising the amino acid sequence of SEQ ID NO:2; (b) HVR-H2 comprising the amino acid sequence of SEQ ID NO:3; (c) HVR-H3 comprising the amino acid sequence of SEQ ID NO:4; (d) HVR-L1 comprising the amino acid sequence of SEQ ID NO:5; (e) HVR-L2 comprising the amino acid sequence of SEQ ID NO:6; and (f) HVR-L3 comprising an amino acid sequence selected from SEQ ID NO:26.

In one aspect, disclosed is an anti-human OX40 agonist antibody comprising at least one, two, three, four, five, or six HVRs selected from (a) HVR-H1 comprising the amino acid sequence of SEQ ID NO:2; (b) HVR-H2 comprising the amino acid sequence of SEQ ID NO:3; (c) HVR-H3 comprising the amino acid sequence of SEQ ID NO:4; (d) HVR-L1 comprising the amino acid sequence of SEQ ID NO:5; (e) HVR-L2 comprising the amino acid sequence of SEQ ID NO:6; and (f) HVR-L3 comprising the amino acid sequence of SEQ ID NO:27.

In another aspect, the antibody comprises HVR-H3 comprising the amino acid sequence of SEQ ID NO:4 and HVR-L3 comprising the amino acid sequence of SEQ ID NO:27. In a further embodiment, the antibody comprises HVR-H3 comprising the amino acid sequence of SEQ ID NO:4, HVR-L3 comprising the amino acid sequence of SEQ ID NO:27, and HVR-H2 comprising the amino acid sequence of SEQ ID NO:3.

In another aspect, an antibody disclosed herein comprises (a) a VH domain comprising at least one, at least two, or all three VH HVR sequences selected from (i) HVR-H1 comprising the amino acid sequence of SEQ ID NO:2, (ii) HVR-H2 comprising the amino acid sequence of SEQ ID NO:3, and (iii) HVR-H3 comprising an amino acid sequence selected from SEQ ID NO:4; and (b) a VL domain comprising at least one, at least two, or all three VL HVR sequences selected from (i) HVR-L1 comprising the amino acid sequence of SEQ ID NO:5, (ii) HVR-L2 comprising the amino acid sequence of SEQ ID NO:6, and (c) HVR-L3 comprising the amino acid sequence of SEQ ID NO:27.

In another aspect, disclosed is an antibody comprising (a) HVR-H1 comprising the amino acid sequence of SEQ ID NO:2; (b) HVR-H2 comprising the amino acid sequence of SEQ ID NO:3; (c) HVR-H3 comprising the amino acid sequence of SEQ ID NO:4; (d) HVR-L1 comprising the amino acid sequence of SEQ ID NO:5; (e) HVR-L2 comprising the amino acid sequence of SEQ ID NO:6; and (f) HVR-L3 comprising an amino acid sequence selected from SEQ ID NO:27.

In one aspect, disclosed is an anti-human OX40 agonist antibody comprising at least one, two, three, four, five, or six HVRs selected from (a) HVR-H1 comprising the amino acid sequence of SEQ ID NO:2, 8 or 9; (b) HVR-H2 comprising the amino acid sequence of SEQ ID NO:3, 10, 11, 12, 13 or 14; (c) HVR-H3 comprising the amino acid sequence of SEQ ID NO:4, 15, or 19; (d) HVR-L1 comprising the amino acid sequence of SEQ ID NO:5; (e) HVR-L2 comprising the amino acid sequence of SEQ ID NO:6; and (f) HVR-L3 comprising the amino acid sequence of SEQ ID NO:7, 22, 23, 24, 25, 26, 27, or 28.

In one aspect, disclosed is an antibody comprising at least one, at least two, or all three VH HVR sequences selected from (a) HVR-H1 comprising the amino acid sequence of SEQ ID NO: 2, 8 or 9; (b) HVR-H2 comprising the amino acid sequence of SEQ ID NO: 3, 10, 11, 12, 13 or 14; and (c) HVR-H3 comprising the amino acid sequence of SEQ ID NO: 4, 15, or 19. In one aspect, the antibody comprises HVR-H3 comprising the amino acid sequence of SEQ ID NO: 4, 15, or 19. In another embodiment, the antibody comprises HVR-H3 comprising the amino acid sequence of SEQ ID NO:4, 15, or 19 and HVR-L3 comprising the amino acid sequence of SEQ ID NO: 7, 22, 23, 24, 25, 26, 27, or 28. In a further aspect, the antibody comprises HVR-H3 comprising the amino acid sequence of SEQ ID NO: 4, 15, or 19, HVR-L3 comprising the amino acid sequence of SEQ ID NO: 7, 22, 23, 24, 25, 26, 27, or 28, and HVR-H2 comprising the amino acid sequence of SEQ ID NO: 3, 10, 11, 12, 13 or 14. In a further aspect, the antibody comprises (a) HVR-H1 comprising the amino acid sequence of SEQ ID NO: 2, 8 or 9; (b) HVR-H2 comprising the amino acid sequence of SEQ ID NO: 3, 10, 11, 12, 13 or 14; and (c) HVR-H3 comprising the amino acid sequence of SEQ ID NO: 4, 15, or 19.

In another aspect, disclosed is an antibody comprising at least one, at least two, or all three VL HVR sequences selected from (a) HVR-L1 comprising the amino acid sequence of SEQ ID NO: 5; (b) HVR-L2 comprising the amino acid sequence of SEQ ID NO:6; and (c) HVR-L3 comprising the amino acid sequence of SEQ ID NO: 7, 22, 23, 24, 25, 26, 27, or 28. In one embodiment, the antibody comprises (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO: 7, 22, 23, 24, 25, 26, 27, or 28.

In another aspect, an antibody comprises (a) a VH domain comprising at least one, at least two, or all three VH HVR sequences selected from (i) HVR-H1 comprising the amino acid sequence of SEQ ID NO: 2, 8 or 9, (ii) HVR-H2 comprising the amino acid sequence of SEQ ID NO: 3, 10, 11, 12, 13 or 14, and (iii) HVR-H3 comprising an amino acid sequence selected from SEQ ID NO: 4, 15, or 19; and (b) a VL domain comprising at least one, at least two, or all three VL HVR sequences selected from (i) HVR-L1 comprising the amino acid sequence of SEQ ID NO:5, (ii) HVR-L2 comprising the amino acid sequence of SEQ ID NO:6, and (c) HVR-L3 comprising the amino acid sequence of SEQ ID NO: 7, 22, 23, 24, 25, 26, 27, or 28.

In another aspect, the disclosed is an antibody comprising (a) HVR-H1 comprising the amino acid sequence of SEQ ID NO: 2, 8 or 9; (b) HVR-H2 comprising the amino acid sequence of SEQ ID NO: 3, 10, 11, 12, 13 or 14; (c) HVR-H3 comprising the amino acid sequence of SEQ ID NO: 4, 15, or 19; (d) HVR-L1 comprising the amino acid sequence of SEQ ID NO:5; (e) HVR-L2 comprising the amino acid sequence of SEQ ID NO:6; and (f) HVR-L3 comprising an amino acid sequence selected from SEQ ID NO: 7, 22, 23, 24, 25, 26, 27, or 28.

In one aspect, disclosed is an anti-human OX40 agonist antibody comprising at least one, two, three, four, five, or six HVRs selected from (a) HVR-H1 comprising the amino acid sequence of SEQ ID NO:172; (b) HVR-H2 comprising the amino acid sequence of SEQ ID NO:173; (c) HVR-H3 comprising the amino acid sequence of SEQ ID NO:174; (d) HVR-L1 comprising the amino acid sequence of SEQ ID NO:5; (e) HVR-L2 comprising the amino acid sequence of SEQ ID NO:6; and (f) HVR-L3 comprising the amino acid sequence of SEQ ID NO:175. In some aspect, HVR-H2 is not DMYPDAAAASYNQKFRE (SEQ ID NO:193).In some aspects, HVR-H3 is not APRWAAAA (SEQ ID NO:194). In some aspects, HVR-L3 is not QAAAAAAAT (SEQ ID NO:195).

In one aspect, disclosed is an antibody comprising at least one, at least two, or all three VH HVR sequences selected from (a) HVR-H1 comprising the amino acid sequence of SEQ ID NO:172; (b) HVR-H2 comprising the amino acid sequence of SEQ ID NO:173; and (c) HVR-H3 comprising the amino acid sequence of SEQ ID NO:174. In one aspect, the antibody comprises HVR-H3 comprising the amino acid sequence of SEQ ID NO:174. In another aspect, the antibody comprises HVR-H3 comprising the amino acid sequence of SEQ ID NO:174 and HVR-L3 comprising the amino acid sequence of SEQ ID NO:175. In a further aspect, the antibody comprises HVR-H3 comprising the amino acid sequence of SEQ ID NO:174, HVR-L3 comprising the amino acid sequence of SEQ ID NO:175, and HVR-H2 comprising the amino acid sequence of SEQ ID NO:173. In a further aspect, the antibody comprises (a) HVR-H1 comprising the amino acid sequence of SEQ ID NO:172; (b) HVR-H2 comprising the amino acid sequence of SEQ ID NO:173; and (c) HVR-H3 comprising the amino acid sequence of SEQ ID NO:174. In some aspect, HVR-H2 is not DMYPDAAAASYNQKFRE (SEQ ID NO:193).In some aspects, HVR-H3 is not APRWAAAA (SEQ ID NO:194). In some aspects, HVR-L3 is not QAAAAAAAT (SEQ ID NO:195).

In another aspect, disclosed is an antibody comprising (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:175. In some aspects, HVR-L3 is not QAAAAAAAT (SEQ ID NO:195).

In another aspect, an antibody as disclosed herein comprises (a) a VH domain comprising at least one, at least two, or all three VH HVR sequences selected from (i) HVR-H1 comprising the amino acid sequence of SEQ ID NO:172, (ii) HVR-H2 comprising the amino acid sequence of SEQ ID NO:173, and (iii) HVR-H3 comprising an amino acid sequence selected from SEQ ID NO:174; and (b) a VL domain comprising at least one, at least two, or all three VL HVR sequences selected from (i) HVR-L1 comprising the amino acid sequence of SEQ ID NO:5, (ii) HVR-L2 comprising the amino acid sequence of SEQ ID NO:6, and (c) HVR-L3 comprising the amino acid sequence of SEQ ID NO:175.

In another aspect, disclosed is an antibody comprising (a) HVR-H1 comprising the amino acid sequence of SEQ ID NO:172; (b) HVR-H2 comprising the amino acid sequence of SEQ ID NO:173; (c) HVR-H3 comprising the amino acid sequence of SEQ ID NO:174; (d) HVR-L1 comprising the amino acid sequence of SEQ ID NO:5; (e) HVR-L2 comprising the amino acid sequence of SEQ ID NO:6; and (f) HVR-L3 comprising an amino acid sequence selected from SEQ ID NO:175. In some aspect, HVR-H2 is not DMYPDAAAASYNQKFRE (SEQ ID NO:193).In some aspects, HVR-H3 is not APRWAAAA (SEQ ID NO:194). In some aspects, HVR-L3 is not QAAAAAAAT (SEQ ID NO:195).

All possible combinations of the above substitutions are encompassed by the consensus sequences of SEQ ID NO:172, 173, 174 and 175.

In any of the above aspects, an anti-OX40 agonist antibody is humanized. In one aspect, an anti-OX40 antibody comprises HVRs as in any of the above aspects or for any of the aspects in FIGS. 11A-I, and further comprises an acceptor human framework, e.g. a human immunoglobulin framework or a human consensus framework. In another aspect, an anti-OX40 antibody comprises HVRs as in any of the above aspects, and further comprises a VH and/or VL comprising an FR sequence shown in FIGS. 11A-I.

In another aspect, an anti-human OX40 agonist antibody as disclosed herein comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:56, 58, 60, 62, 64, 66, 68, 70, 72, 74, 76, 78, 80, 82, 84, 86, 88, 90, 92, 94, 96, 98, 100, 108, 114, 116, 183, or 184. In certain aspects, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO:56, 58, 60, 62, 64, 66, 68, 70, 72, 74, 76, 78, 80, 82, 84, 86, 88, 90, 92, 94, 96, 98, 100, 108, 114, 116, 183, or 184. In certain aspects, substitutions, insertions, or deletions occur in regions outside the HVRs (i.e., in the FRs). Optionally, the anti-human OX40 agonist antibody comprises the VH sequence in SEQ ID NO: SEQ ID NO:56, 58, 60, 62, 64, 66, 68, 70, 72, 74, 76, 78, 80, 82, 84, 86, 88, 90, 92, 94, 96, 98, 100, 108, 114, 116, 183, or 184, including post-translational modifications of that sequence. In a particular aspect, the VH comprises one, two or three HVRs selected from: (a) HVR-H1 comprising the amino acid sequence of SEQ ID NO:2, (b) HVR-H2 comprising the amino acid sequence of SEQ ID NO:3, and (c) HVR-H3 comprising the amino acid sequence of SEQ ID NO:4.

In another aspect, an anti-human OX40 agonist antibody is disclosed, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:57, 59, 61, 63, 65, 67, 69, 71, 73, 75, 77, 79, 81, 83, 85, 87, 89, 91, 93, 95, 97, 99, 101, 109, 115 or 117. In certain aspects, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO: 57, 59, 61, 63, 65, 67, 69, 71, 73, 75, 77, 79, 81, 83, 85, 87, 89, 91, 93, 95, 97, 99, 101, 109, 115 or 117. In certain aspects, the substitutions, insertions, or deletions occur in regions outside the HVRs (i.e., in the FRs). Optionally, the anti-human OX40 agonist antibody comprises the VL sequence in SEQ ID NO: 57, 59, 61, 63, 65, 67, 69, 71, 73, 75, 77, 79, 81, 83, 85, 87, 89, 91, 93, 95, 97, 99, 101, 109, 115 or 117, including post-translational modifications of that sequence. In a particular aspect, the VL comprises one, two or three HVRs selected from (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:7.

In another aspect, an anti-human OX40 agonist antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:56. In certain aspects, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO:56. In certain aspects, substitutions, insertions, or deletions occur in regions outside the HVRs (i.e., in the FRs). Optionally, the anti-human OX40 agonist antibody comprises the VH sequence in SEQ ID NO:56, including post-translational modifications of that sequence. In a particular embodiment, the VH comprises one, two or three HVRs selected from: (a) HVR-H1 comprising the amino acid sequence of SEQ ID NO:2, (b) HVR-H2 comprising the amino acid sequence of SEQ ID NO:3, and (c) HVR-H3 comprising the amino acid sequence of SEQ ID NO:4.

In another aspect, an anti-human OX40 agonist antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:57. In certain aspects, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO: 57. In certain aspects, the substitutions, insertions, or deletions occur in regions outside the HVRs (i.e., in the FRs). Optionally, the anti-human OX40 agonist antibody comprises the VL sequence in SEQ ID NO: 57, including post-translational modifications of that sequence. In a particular aspect, the VL comprises one, two or three HVRs selected from (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:7.

In another aspect, an anti-human OX40 agonist antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:180. In certain aspects, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO:180. In certain aspects, substitutions, insertions, or deletions occur in regions outside the HVRs (i.e., in the FRs). Optionally, the anti-human OX40 agonist antibody comprises the VH sequence in SEQ ID NO:180, including post-translational modifications of that sequence. In a particular aspect, the VH comprises one, two or three HVRs selected from: (a) HVR-H1 comprising the amino acid sequence of SEQ ID NO:2, (b) HVR-H2 comprising the amino acid sequence of SEQ ID NO:3, and (c) HVR-H3 comprising the amino acid sequence of SEQ ID NO:4.

In another aspect, an anti-human OX40 agonist antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:179. In certain aspects, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO: 179. In certain aspects, the substitutions, insertions, or deletions occur in regions outside the HVRs (i.e., in the FRs). Optionally, the anti-human OX40 agonist antibody comprises the VL sequence in SEQ ID NO: 179, including post-translational modifications of that sequence. In a particular embodiment, the VL comprises one, two or three HVRs selected from (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:7.

In another aspect, an anti-human OX40 agonist antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:94. In certain aspects, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO:94. In certain aspects, substitutions, insertions, or deletions occur in regions outside the HVRs (i.e., in the FRs). Optionally, the anti-human OX40 agonist antibody comprises the VH sequence in SEQ ID NO:94, including post-translational modifications of that sequence. In a particular aspect, the VH comprises one, two or three HVRs selected from: (a) HVR-H1 comprising the amino acid sequence of SEQ ID NO:2, (b) HVR-H2 comprising the amino acid sequence of SEQ ID NO:3, and (c) HVR-H3 comprising the amino acid sequence of SEQ ID NO:4.

In another aspect, an anti-human OX40 agonist antibody is disclosed, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:95. In certain aspects, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO:95. In certain aspects, the substitutions, insertions, or deletions occur in regions outside the HVRs (i.e., in the FRs). Optionally, the anti-human OX40 agonist antibody comprises the VL sequence in SEQ ID NO:95, including post-translational modifications of that sequence. In a particular embodiment, the VL comprises one, two or three HVRs selected from (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:26.

In another aspect, an anti-human OX40 agonist antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:96. In certain aspects, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO:96. In certain aspects, substitutions, insertions, or deletions occur in regions outside the HVRs (i.e., in the FRs). Optionally, the anti-human OX40 agonist antibody comprises the VH sequence in SEQ ID NO:96, including post-translational modifications of that sequence. In a particular embodiment, the VH comprises one, two or three HVRs selected from: (a) HVR-H1 comprising the amino acid sequence of SEQ ID NO:2, (b) HVR-H2 comprising the amino acid sequence of SEQ ID NO:3, and (c) HVR-H3 comprising the amino acid sequence of SEQ ID NO:4.

In another aspect, an anti-human OX40 agonist antibody is disclosed, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:97. In certain aspects, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO:97. In certain aspects, the substitutions, insertions, or deletions occur in regions outside the HVRs (i.e., in the FRs). Optionally, the anti-human OX40 agonist antibody comprises the VL sequence in SEQ ID NO:97, including post-translational modifications of that sequence. In a particular aspect, the VL comprises one, two or three HVRs selected from (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:27.

In another aspect, an anti-human OX40 agonist antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO: 118, 120, 122, 124, 126, 128, 130, 132, 134, 136, 138, 140, 142, 144, 146, 148. In certain aspects, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO: 118, 120, 122, 124, 126, 128, 130, 132, 134, 136, 138, 140, 142, 144, 146, 148. In certain aspects, substitutions, insertions, or deletions occur in regions outside the HVRs (i.e., in the FRs). Optionally, the anti-human OX40 agonist antibody comprises the VH sequence in SEQ ID NO: SEQ ID NO: 118, 120, 122, 124, 126, 128, 130, 132, 134, 136, 138, 140, 142, 144, 146, 148, including post-translational modifications of that sequence. In a particular embodiment, the VH comprises one, two or three HVRs selected from: (a) HVR-H1 comprising the amino acid sequence of SEQ ID NO: 29, (b) HVR-H2 comprising the amino acid sequence of SEQ ID NO:30, and (c) HVR-H3 comprising the amino acid sequence of SEQ ID NO:33.

In another aspect, an anti-human OX40 agonist antibody is disclosed, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO: 119, 121, 123, 125, 127, 129, 131, 133, 135, 137, 139, 141, 143, 145, 147, 149. In certain aspects, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO: 119, 121, 123, 125, 127, 129, 131, 133, 135, 137, 139, 141, 143, 145, 147, 149. In certain aspects, the substitutions, insertions, or deletions occur in regions outside the HVRs (i.e., in the FRs). Optionally, the anti-human OX40 agonist antibody comprises the VL sequence in SEQ ID NO: 119, 121, 123, 125, 127, 129, 131, 133, 135, 137, 139, 141, 143, 145, 147, 149, including post-translational modifications of that sequence. In a particular aspect, the VL comprises one, two or three HVRs selected from (a) HVR-L1 comprising the amino acid sequence of SEQ ID NO:37; (b) HVR-L2 comprising the amino acid sequence of SEQ ID NO:39; and (c) HVR-L3 comprising the amino acid sequence of SEQ ID NO:42.

In one aspect, the antibody comprises the VH and VL sequences in SEQ ID NO:56 and SEQ ID NO:57, respectively, including post-translational modifications of those sequences. In one aspect, the antibody comprises the VH and VL sequences in SEQ ID NO:58 and SEQ ID NO:59, respectively, including post-translational modifications of those sequences. In one aspect, the antibody comprises the VH and VL sequences in SEQ ID NO:60 and SEQ ID NO:61, respectively, including post-translational modifications of those sequences. In one aspect, the antibody comprises the VH and VL sequences in SEQ ID NO:62 and SEQ ID NO:63, respectively, including post-translational modifications of those sequences. In one aspect, the antibody comprises the VH and VL sequences in SEQ ID NO:64 and SEQ ID NO:65, respectively, including post-translational modifications of those sequences. In one aspect, the antibody comprises the VH and VL sequences in SEQ ID NO:66 and SEQ ID NO:67, respectively, including post-translational modifications of those sequences. In one aspect, the antibody comprises the VH and VL sequences in SEQ ID NO:68 and SEQ ID NO:69, respectively, including post-translational modifications of those sequences. In one aspect, the antibody comprises the VH and VL sequences in SEQ ID NO:70 and SEQ ID NO:71, respectively, including post-translational modifications of those sequences. In one aspect, the antibody comprises the VH and VL sequences in SEQ ID NO:72 and SEQ ID NO:73, respectively, including post-translational modifications of those sequences. In one aspect, the antibody comprises the VH and VL sequences in SEQ ID NO:74 and SEQ ID NO:75, respectively, including post-translational modifications of those sequences. In one aspect, the antibody comprises the VH and VL sequences in SEQ ID NO:76 and SEQ ID NO:77, respectively, including post-translational modifications of those sequences. In one aspect, the antibody comprises the VH and VL sequences in SEQ ID NO:78 and SEQ ID NO:79, respectively, including post-translational modifications of those sequences. In one aspect, the antibody comprises the VH and VL sequences in SEQ ID NO:80 and SEQ ID NO:81, respectively, including post-translational modifications of those sequences. In one aspect, the antibody comprises the VH and VL sequences in SEQ ID NO:82 and SEQ ID NO:83, respectively, including post-translational modifications of those sequences. In one aspect, the antibody comprises the VH and VL sequences in SEQ ID NO:84 and SEQ ID NO:85, respectively, including post-translational modifications of those sequences. In one aspect, the antibody comprises the VH and VL sequences in SEQ ID NO:86 and SEQ ID NO:87, respectively, including post-translational modifications of those sequences. In one aspect, the antibody comprises the VH and VL sequences in SEQ ID NO:88 and SEQ ID NO:89, respectively, including post-translational modifications of those sequences. In one aspect, the antibody comprises the VH and VL sequences in SEQ ID NO:90 and SEQ ID NO:91, respectively, including post-translational modifications of those sequences. In one aspect, the antibody comprises the VH and VL sequences in SEQ ID NO:92 and SEQ ID NO:93, respectively, including post-translational modifications of those sequences. In one aspect, the antibody comprises the VH and VL sequences in SEQ ID NO:94 and SEQ ID NO:95, respectively, including post-translational modifications of those sequences. In one aspect, the antibody comprises the VH and VL sequences in SEQ ID NO:96 and SEQ ID NO:97, respectively, including post-translational modifications of those sequences. In one aspect, the antibody comprises the VH and VL sequences in SEQ ID NO:98 and SEQ ID NO:99, respectively, including post-translational modifications of those sequences. In one aspect, the antibody comprises the VH and VL sequences in SEQ ID NO:100 and SEQ ID NO:101, respectively, including post-translational modifications of those sequences. In one aspect, the antibody comprises the VH and VL sequences in SEQ ID NO:108 and SEQ ID NO:109, respectively, including post-translational modifications of those sequences. In one aspect, the antibody comprises the VH and VL sequences in SEQ ID NO:114 and SEQ ID NO:115, respectively, including post-translational modifications of those sequences. In one aspect, the antibody comprises the VH and VL sequences in SEQ ID NO:116 and SEQ ID NO:117, respectively, including post-translational modifications of those sequences. In one aspect, the antibody comprises the VH and VL sequences in SEQ ID NO:183 and SEQ ID NO:65, respectively, including post-translational modifications of those sequences. In one aspect, the antibody comprises the VH and VL sequences in SEQ ID NO:184 and SEQ ID NO:69, respectively, including post-translational modifications of those sequences.

In another aspect, an anti-human OX40 agonist antibody is provided, wherein the antibody comprises a VH as in any of the aspects provided above, and a VL as in any of the aspects provided above.

In a further aspect, the invention provides an antibody that binds to the same epitope as an anti-human OX40 antibody provided herein. In some aspects, the antibody is an anti-human OX40 agonist antibody.

In a further aspect, an anti-OX40 antibody according to any of the above aspects is a monoclonal antibody, including a chimeric, humanized or human antibody. In one aspect, an anti-OX40 antibody is an antibody fragment, e.g., a Fv, Fab, Fab', scFv, diabody, or F(ab')₂ fragment. In another aspect, the antibody is a full length antibody, e.g., an intact IgG1 antibody or other antibody class or isotype as defined herein. In some aspects, the antibody is a full length intact IgG4 antibody.

In a further aspect, an anti-OX40 antibody according to any of the above aspects may incorporate any of the features, singly or in combination, as described in Sections 1-7 below:

### 1. Antibody Affinity

In certain aspects, an antibody provided herein has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

In one aspect, Kd is measured by a radiolabeled antigen binding assay (RIA). In one aspect, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER® multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20®) in PBS. When the plates have dried, 150 µl/well of scintillant (MICROSCINT-20 ™; Packard) is added, and the plates are counted on a TOPCOUNT ™ gamma counter (Packard) for ten minutes. Concentrations of each Fab that give less than or equal to 20% of maximal binding are chosen for use in competitive binding assays.

According to another aspect, Kd is measured using a BIACORE® surface plasmon resonance assay. For example, an assay using a BIACORE®-2000 or a BIACORE ®-3000 (BIAcore, Inc., Piscataway, NJ) is performed at 25°C with immobilized antigen CM5 chips at -10 response units (RU). In one aspect, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (-0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 µl/min. Association rates (kₒₙ) and dissociation rates (k_{off}) are calculated using a simple one-to-one Langmuir binding model (BIACORE ® Evaluation Software version 3.2) by simultaneously fitting the association and dissociation sensorgrams. The equilibrium dissociation constant (Kd) is calculated as the ratio k_{off}/k_{on.} See, e.g., Chen et al., J. Mol. Biol. 293:865-881 (1999). If the on-rate exceeds 106 M-1 s-1 by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25oC of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO ™ spectrophotometer (ThermoSpectronic) with a stirred cuvette.

### 2. Antibody Fragments

In certain aspects, an antibody provided herein is an antibody fragment. Antibody fragments include, but are not limited to, Fab, Fab', Fab'-SH, F(ab')₂, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain aspects, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

### 3. Chimeric and Humanized Antibodies

In certain aspects, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain aspects, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some aspects, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Human Antibodies

In certain aspects, an antibody is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain aspects, an antibody is a multispecific antibody, e.g. a bispecific antibody. Multispecific antibodies are monoclonal antibodies that have binding specificities for at least two different sites. In certain aspects, one of the binding specificities is for OX40 and the other is for any other antigen. In certain aspects, bispecific antibodies may bind to two different epitopes of OX40. Bispecific antibodies may also be used to localize cytotoxic agents to cells which express OX40. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to OX40 as well as another, different antigen (see, US 2008/0069820, for example).

### 7. Antibody Variants

In certain aspects, amino acid sequence variants of the antibodies are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

In certain aspects, antibody variants having one or more amino acid substitutions are disclosed. Sites of interest for substitutional mutagenesis include the HVRs and FRs. Conservative substitutions are shown in Table A under the heading of "preferred substitutions." More substantial changes are provided in Table A under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE A**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g*. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some aspects of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain aspects, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain aspects of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### b) Glycosylation variants

In certain aspects, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some aspects, modifications of the oligosaccharide in an antibody of the invention may be made in order to create antibody variants with certain improved properties.

In one aspect, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al.*,* especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further disclosed with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.)*.* Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### c) Fc region variants

In certain aspects, one or more amino acid modifications may be introduced into the Fc region of an antibody provided herein, thereby generating an Fc region variant. The Fc region variant may comprise a human Fc region sequence (*e.g.,* a human IgG1, IgG2, IgG3 or IgG4 Fc region) comprising an amino acid modification (*e.g*. a substitution) at one or more amino acid positions.

In certain aspects, the invention contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain aspects, an antibody variant comprises an Fc region with one or more amino acid substitutions which improve ADCC, e.g., substitutions at positions 298, 333, and/or 334 of the Fc region (EU numbering of residues).

In some aspects, alterations are made in the Fc region that result in altered (*i.e.,* either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### d) Cysteine engineered antibody variants

In certain aspects, it may be desirable to create cysteine engineered antibodies, e.g., "thioMAbs," in which one or more residues of an antibody are substituted with cysteine residues. In particular aspects, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain aspects, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### e) Antibody Derivatives

In certain aspects, an antibody may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another aspect, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### B. Recombinant Methods and Compositions

Antibodies may be produced using recombinant methods and compositions, e.g., as described in U.S. Patent No. 4,816,567. In one embodiment, isolated nucleic acid encoding an anti-OX40 antibody described herein is provided. Such nucleic acid may encode an amino acid sequence comprising the VL and/or an amino acid sequence comprising the VH of the antibody (e.g., the light and/or heavy chains of the antibody). In a further aspect, one or more vectors (e.g., expression vectors) comprising such nucleic acid are provided. In a further aspect, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one aspect, the host cell is eukaryotic, e.g. a Chinese Hamster Ovary (CHO) cell or lymphoid cell (e.g., Y0, NS0, Sp20 cell). In one aspect, a method of making an anti-OX40 antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-OX40 antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.*) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N. Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

### C. Assays

Anti-OX40 antibodies provided herein may be identified, screened for, or characterized for their physical/chemical properties and/or biological activities by various assays known in the art.

### 1. Binding assays and other assays

In one aspect, an antibody of the invention is tested for its antigen binding activity, e.g., by known methods such as ELISA, Western blot, etc. OX40 binding may be determined using methods known in the art and exemplary methods are disclosed herein. In one embodiment, binding is measured using radioimmunoassay. An exemplary radioimmunassay is exemplified in the Examples. OX40 antibody is iodinated, and competition reaction mixtures are prepared containing a fixed concentration of iodinated antibody and decreasing concentrations of serially diluted, unlabeled OZ X40 antibody. Cells expressing OX40 (e.g., BT474 cells stably transfected with human OX40) are added to the reaction mixture. Following an incubation, cells are washed to separate the free iodinated OX40 antibody from the OX40 antibody bound to the cells. Level of bound iodinated OX40 antibody is determined, e.g., by counting radioactivity associated with cells, and binding affinity determined using standard methods. In another aspect, ability of OX40 antibody to bind to surface-expressed OX40 (e.g., on T cell subsets) is assessed using flow cytometry. Peripheral white blood cells are obtained (e.g., from human, cynomolgus monkey, rat or mouse) and cells are blocked with serum. Labeled OX40 antibody is added in serial dilutions, and T cells are also stained to identify T cell subsets (using methods known in the art). Following incubation of the samples and washing, the cells are sorted using flow cytometer, and data analyzed using methods well known in the art. In another aspect, OX40 binding may be analyzed using surface plasmon resonance. An exemplary surface plasmon resonance method is exemplified in the Examples.

In another aspect, competition assays may be used to identify an antibody that competes with any of the anti-OX40 antibodies disclosed herein for binding to OX40. In certain aspects, such a competing antibody binds to the same epitope (e.g., a linear or a conformational epitope) that is bound by any of the anti-OX40 antibodies disclosed herein. Detailed exemplary methods for mapping an epitope to which an antibody binds are provided in Morris (1996) "Epitope Mapping Protocols," in Methods in Molecular Biology vol. 66 (Humana Press, Totowa, NJ). A competition assay is exemplified in the Examples.

In an exemplary competition assay, immobilized OX40 is incubated in a solution comprising a first labeled antibody that binds to OX40 (e.g., mab 1A7.gr.1, mab 3C8.gr5) and a second unlabeled antibody that is being tested for its ability to compete with the first antibody for binding to OX40. The second antibody may be present in a hybridoma supernatant. As a control, immobilized OX40 is incubated in a solution comprising the first labeled antibody but not the second unlabeled antibody. After incubation under conditions permissive for binding of the first antibody to OX40, excess unbound antibody is removed, and the amount of label associated with immobilized OX40 is measured. If the amount of label associated with immobilized OX40 is substantially reduced in the test sample relative to the control sample, then that indicates that the second antibody is competing with the first antibody for binding to OX40. See Harlow and Lane (1988) Antibodies: A Laboratory Manual ch.14 (Cold Spring Harbor Laboratory, Cold Spring Harbor, NY).

### 2. Activity assays

In one aspect, assays are provided for identifying anti-OX40 antibodies thereof having biological activity. Biological activity may include, e.g., binding OX40 (e.g., binding human and/or cynomolgus OX40), increasing OX40-mediated signal transduction (e.g., increasing NFkB-mediated transcription), depleting cells that express human OX40 (e.g., T cells), depleting cells that express human OX40 by ADCC and/or phagocytosis, enhancing T effector cell function (e.g., CD4+ effector T cell), e.g., by increasing effector T cell proliferation and/or increasing cytokine production (e.g., gamma interferon) by effector T cells, enhancing memory T cell function (e.g., CD4+ memory T cell), e.g., by increasing memory T cell proliferation and/or increasing cytokine production by memory T cells (e.g., gamma interferon), inhibiting regulatory T cell function (e.g., by decreasing Treg suppression of effector T cell function (e.g., CD4+ effector T cell function), binding human effector cells. Antibodies having such biological activity in vivo and/or in vitro are also provided.

In certain aspects, an antibody of the invention is tested for such biological activity.

T cell costimulation may be assayed using methods known in the art and exemplary methods are disclosed herein. For example, T cells (e.g., memory or effector T cells) may be obtained from peripheral white blood cells (e.g., isolated from human whole blood using Ficoll gradient centrifugation). Memory T cells (e.g., CD4+ memory T cells) or effector T cells (e.g. CD4+ Teff cells) may be isolated from PBMC using methods known in the art. For example, the Miltenyi CD4+ memory T cell isolation kit or Miltenyi naive CD4+ T cell isolation kit may be used. Isolated T cells are cultured in the presence of antigen presenting cells (e.g., irradiated L cells that express CD32 and CD80), and activated by addition of anti-CD3 antibody in the presence or absence of OX40 agonist antibody. Effect of agonist OX40 antibody of T cell proliferation may be measured using methods well known in the art. For example, the CellTiter Glo kit (Promega) may be used, and results read on a Multilabel Reader (Perkin Elmer). Effect of agonist OX40 antibody on T cell function may also be determined by analysis of cytokines produced by the T cell. In one embodiment, production of interferon gamma by CD4+ T cells is determined, e.g., by measurement of interferon gamma in cell culture supernatant. Methods for measuring interferon gamma are well-known in the art.

Treg cell function may be assayed using methods known in the art and exemplary methods are disclosed herein. In one example, the ability of Treg to suppress effector T cell proliferation is assayed. T cells are isolated from human whole blood using methods known in the art (e.g., isolating memory T cells or naive T cells). Purified CD4+ naive T cells are labeled (e.g., with CFSE) and purified Treg cells are labeled with a different reagent. Irradiated antigen presenting cells (e.g., L cells expressing CD32 and CD80) are co-cultured with the labeled purified naive CD4+ T cells and purified Tregs. The co-cultures are activated using anti-CD3 antibody and tested in the presence or absence of agonist OX40 antibody. Following a suitable time (e.g., 6 days of coculture), level of CD4+ naive T cell proliferation is tracked by dye dilution in reduced label staining (e.g., reduced CFSE label staining) using FACS analysis.

OX40 signaling may be assayed using methods well known in the art and exemplary methods are disclosed herein. In one aspect, transgenic cells are generated that express human OX40 and a reporter gene comprising the NFkB promoter fused to a reporter gene (e.g., beta luciferase). Addition of OX40 agonist antibody to the cells results in increased NFkB transcription, which is detected using an assay for the reporter gene.

Phagocytosis may be assayed, e.g., by using monocyte-derived macrophages, or U937 cells (a human histiocytic lymphoma cells line with the morphology and characteristics of mature macrophages). OX40 expressing cells are added to the monocyte-derived macrophages or U937 cells in the presence or absence of anti-OX40 agonist antibody. Following culturing of the cells for a suitable period of time, the percentage of phagocytosis is determined by examining percentage of cells that double stain for markers of 1) the macrophage or U937 cell and 2) the OX40 expressing cell, and dividing this by the total number of cells that show markers of the OX40 expressing cell (e.g., GFP). Analysis may be done by flow cytometry. In another aspect, analysis may be done by fluorescent microscopy analysis.

ADCC may be assayed, e.g., using methods well known in the art. Exemplary methods are described in the definition section and an exemplary assay is disclosed in the Examples. In some aspects, level of OX40 is characterized on an OX40 expressing cell that is used for testing in an ADCC assay. The cell may be stained with a detectably labeled anti-OX40 antibody (e.g., PE labeled), then level of fluorescence determined using flow cytometry, and results presented as median fluorescence intensity (MFI). In another aspect, ADCC may be analyzed by CellTiter Glo assay kit and cell viability/cytotoxicity may be determined by chemioluminescence.

The binding affinities of various antibodies to FcyRIA, FcγRIIA, FcγRIIB, and two allotypes of FcyRIIIA (F158 and V158) may be measured in ELISA-based ligand-binding assays using the respective recombinant Fcγ receptors. Purified human Fcγ receptors are expressed as fusion proteins containing the extracellular domain of the receptor γ chain linked to a Gly/6xHis/glutathione S-transferase (GST) polypeptide tag at the C-terminus. The binding affinities of antibodies to those human Fcγ receptors are assayed as follows. For the low-affinity receptors, i.e. FcγRIIA (CD32A), FcγRIIB (CD32B), and the two allotypes of FcγRIIIA (CD16), F-158 and V-158, antibodies may be tested as multimers by cross-linking with a F(ab')2 fragment of goat anti-human kappa chain (ICN Biomedical; Irvine, CA) at an approximate molar ratio of 1:3 antibody:cross-linking F(ab')₂. Plates are coated with an anti-GST antibody (Genentech) and blocked with bovine serum albumin (BSA). After washing with phosphate-buffered saline (PBS) containing 0.05% Tween-20 with an ELx405™ plate washer (Biotek Instruments; Winooski, VT), Fcγ receptors are added to the plate at 25 ng/well and incubated at room temperature for 1 hour. After the plates are washed, serial dilutions of test antibodies are added as multimeric complexes and the plates were incubated at room temperature for 2 hours. Following plate washing to remove unbound antibodies, the antibodies bound to the Fcγ receptor are detected with horseradish peroxidase (HRP)-conjugated F(ab')₂ fragment of goat anti-human F(ab')₂ (Jackson ImmunoResearch Laboratories; West Grove, PA) followed by the addition of substrate, tetramethylbenzidine (TMB) (Kirkegaard & Perry Laboratories; Gaithersburg, MD). The plates are incubated at room temperature for 5-20 minutes, depending on the Fcγ receptors tested, to allow color development. The reaction is terminated with 1 M H₃PO₄ and absorbance at 450 nm was measured with a microplate reader (SpectraMax®190, Molecular Devices; Sunnyvale, CA). Dose-response binding curves are generated by plotting the mean absorbance values from the duplicates of antibody dilutions against the concentrations of the antibody. Values for the effective concentration of the antibody at which 50% of the maximum response from binding to the Fcγ receptor is detected (EC₅₀) were determined after fitting the binding curve with a four-parameter equation using SoftMax Pro (Molecular Devices).

To select for antibodies which induce cell death, loss of membrane integrity as indicated by, e.g., propidium iodide (PI), trypan blue or 7AAD uptake may be assessed relative to control. A PI uptake assay can be performed in the absence of complement and immune effector cells. OX40 expressing cells are incubated with medium alone or medium containing of the appropriate monoclonal antibody at e.g., about 10µg/ml. The cells are incubated for a time period (e.g., 1 or 3 days). Following each treatment, cells are washed and aliquoted. In some aspects, cells are aliquoted into 35 mm strainer-capped 12 x 75 tubes (1ml per tube, 3 tubes per treatment group) for removal of cell clumps. Tubes then receive PI (10µg/ml). Samples may be analyzed using a FACSCAN™ flow cytometer and FACSCONVERT™ CellQuest software (Becton Dickinson).

Cells for use in any of the above in vitro assays include cells or cell lines that naturally express OX40 or that have been engineered to express OX40. Such cells include activated T cells, Treg cells and activated memory T cells that naturally express OX40. Such cells also include cell lines that express OX40 and cell lines that do not normally express OX40 but have been transfected with nucleic acid encoding OX40. Exemplary cell lines provided herein for use in any of the above in vitro assays include transgenic BT474 cells (a human breast cancer cell line) that express human OX40

It is understood that any of the above assays may be carried out using an immunoconjugate of the invention in place of or in addition to an anti-OX40 antibody.

It is understood that any of the above assays may be carried out using anti-OX40 antibody and an additional therapeutic agent.

### D. Immunoconjugates

The invention also provides immunoconjugates comprising an anti-OX40 antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one aspect, an immunoconjugate is an antibody-drug conjugate (ADC) in which an antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another aspect, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another aspect, an immunoconjugate comprises an antibody as described herein conjugated to a radioactive atom to form a radioconjugate. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example tc99m or 1123, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### E. Methods and Compositions for Diagnostics and Detection

In certain aspects, any of the anti-OX40 antibodies provided herein is useful for detecting the presence of OX40 in a biological sample. The term "detecting" as used herein encompasses quantitative or qualitative detection. In certain aspects, a biological sample comprises a cell or tissue, such as a sample of a tumor (e.g., NSCLC or breast tumor).

In one aspect, an anti-OX40 antibody for use in a method of diagnosis or detection is provided. In a further aspect, a method of detecting the presence of OX40 in a biological sample is provided. In certain aspects, the method comprises contacting the biological sample with an anti-OX40 antibody as described herein under conditions permissive for binding of the anti-OX40 antibody to OX40, and detecting whether a complex is formed between the anti-OX40 antibody and OX40. Such method may be an in vitro or in vivo method. In one aspect, an anti-OX40 antibody is used to select subjects eligible for therapy with an anti-OX40 antibody, e.g. where OX40 is a biomarker for selection of patients.

In some aspects, the anti-OX40 antibody for use in a method of diagnosis or detection is an anti-human OX40 antibody comprising at least one, two, three, four, five, or six HVRs selected from (a) HVR-H1 comprising the amino acid sequence of SEQ ID NO:2; (b) HVR-H2 comprising the amino acid sequence of SEQ ID NO:3; (c) HVR-H3 comprising the amino acid sequence of SEQ ID NO:4; (d) HVR-L1 comprising the amino acid sequence of SEQ ID NO:5; (e) HVR-L2 comprising the amino acid sequence of SEQ ID NO:6; and (f) HVR-L3 comprising the amino acid sequence of SEQ ID NO:7. In some aspects, the anti-OX40 antibody comprises (a) a VH domain comprising at least one, at least two, or all three VH HVR sequences selected from (i) HVR-H1 comprising the amino acid sequence of SEQ ID NO:2, (ii) HVR-H2 comprising the amino acid sequence of SEQ ID NO:3, and (iii) HVR-H3 comprising an amino acid sequence selected from SEQ ID NO:4; and (b) a VL domain comprising at least one, at least two, or all three VL HVR sequences selected from (i) HVR-L1 comprising the amino acid sequence of SEQ ID NO:5, (ii) HVR-L2 comprising the amino acid sequence of SEQ ID NO:6, and (c) HVR-L3 comprising the amino acid sequence of SEQ ID NO:7. In some aspects, the OX40 antibody comprises (a) HVR-H1 comprising the amino acid sequence of SEQ ID NO:2; (b) HVR-H2 comprising the amino acid sequence of SEQ ID NO:3; (c) HVR-H3 comprising the amino acid sequence of SEQ ID NO:4; (d) HVR-L1 comprising the amino acid sequence of SEQ ID NO:5; (e) HVR-L2 comprising the amino acid sequence of SEQ ID NO:6; and (f) HVR-L3 comprising an amino acid sequence selected from SEQ ID NO:7. In some aspects, the antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:180. In certain aspects, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO:180. In certain aspects, substitutions, insertions, or deletions occur in regions outside the HVRs (i.e., in the FRs). Optionally, the anti-human OX40 agonist antibody comprises the VH sequence in SEQ ID NO:180, including post-translational modifications of that sequence. In a particular aspect, the VH comprises one, two or three HVRs selected from: (a) HVR-H1 comprising the amino acid sequence of SEQ ID NO:2, (b) HVR-H2 comprising the amino acid sequence of SEQ ID NO:3, and (c) HVR-H3 comprising the amino acid sequence of SEQ ID NO:4. In some aspects, the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:179. In certain aspects, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO: 179. In certain aspects, the substitutions, insertions, or deletions occur in regions outside the HVRs (i.e., in the FRs). Optionally, the anti-human OX40 agonist antibody comprises the VL sequence in SEQ ID NO: 179, including post-translational modifications of that sequence. In a particular aspect, the VL comprises one, two or three HVRs selected from (a) HVR-LI comprising the amino acid sequence of SEQ ID NO:5; (b) HVR-L2 comprising the amino acid sequence of SEQ ID NO:6; and (c) HVR-L3 comprising the amino acid sequence of SEQ ID NO:7.

In some aspects, the anti-OX40 antibody used in the method of diagnosis or detection is an anti-human OX40 antibody comprising at least one, two, three, four, five, or six HVRs selected from (a) HVR-H1 comprising the amino acid sequence of SEQ ID NO:29; (b) HVR-H2 comprising the amino acid sequence of SEQ ID NO:30; (c) HVR-H3 comprising the amino acid sequence of SEQ ID NO:31; (d) HVR-L1 comprising the amino acid sequence of SEQ ID NO:37; (e) HVR-L2 comprising the amino acid sequence of SEQ ID NO:39; and (f) HVR-L3 comprising the amino acid sequence of SEQ ID NO:42. In some aspects, the anti-OX40 antibody comprises (a) a VH domain comprising at least one, at least two, or all three VH HVR sequences selected from (i) HVR-H1 comprising the amino acid sequence of SEQ ID NO:29, (ii) HVR-H2 comprising the amino acid sequence of SEQ ID NO:30, and (iii) HVR-H3 comprising an amino acid sequence selected from SEQ ID NO:31; and (b) a VL domain comprising at least one, at least two, or all three VL HVR sequences selected from (i) HVR-L1 comprising the amino acid sequence of SEQ ID NO:37, (ii) HVR-L2 comprising the amino acid sequence of SEQ ID NO:39, and (c) HVR-L3 comprising the amino acid sequence of SEQ ID NO:42. In some aspects, the anti-OX40 antibody comprises (a) HVR-H1 comprising the amino acid sequence of SEQ ID NO:29; (b) HVR-H2 comprising the amino acid sequence of SEQ ID NO:30; (c) HVR-H3 comprising the amino acid sequence of SEQ ID NO:31; (d) HVR-L1 comprising the amino acid sequence of SEQ ID NO:37; (e) HVR-L2 comprising the amino acid sequence of SEQ ID NO:39; and (f) HVR-L3 comprising an amino acid sequence selected from SEQ ID NO:42. In some embodiment, the anti-OX40 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:182. In certain aspects, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO:182. In certain aspects, substitutions, insertions, or deletions occur in regions outside the HVRs (i.e., in the FRs). Optionally, the anti-human OX40 agonist antibody comprises the VH sequence in SEQ ID NO:182, including post-translational modifications of that sequence. In a particular aspect, the VH comprises one, two or three HVRs selected from: (a) HVR-H1 comprising the amino acid sequence of SEQ ID NO:29, (b) HVR-H2 comprising the amino acid sequence of SEQ ID NO:30, and (c) HVR-H3 comprising the amino acid sequence of SEQ ID NO:31. In some aspects, the anti-OX40 antibody comprises a light chain variable domain (VL) having at least 90%, 91%, 92%, 93%, 94%, 95%, 96%, 97%, 98%, 99%, or 100% sequence identity to the amino acid sequence of SEQ ID NO:181. In certain aspects, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-human OX40 agonist antibody comprising that sequence retains the ability to bind to OX40. In certain aspects, a total of 1 to 10 amino acids have been substituted, inserted and/or deleted in SEQ ID NO: 181. In certain aspects, the substitutions, insertions, or deletions occur in regions outside the HVRs (i.e., in the FRs). Optionally, the anti-human OX40 agonist antibody comprises the VL sequence in SEQ ID NO: 181, including post-translational modifications of that sequence. In a particular embodiment, the VL comprises one, two or three HVRs selected from (a) HVR-L1 comprising the amino acid sequence of SEQ ID NO:37; (b) HVR-L2 comprising the amino acid sequence of SEQ ID NO:39; and (c) HVR-L3 comprising the amino acid sequence of SEQ ID NO:42.

In some aspects, the anti-OX40 antibody comprises a VH sequence of SEQ ID NO: 180. In some aspects, the anti-OX40 antibody comprises a VL sequence of SEQ ID NO: 179. In some aspects, the anti-OX40 antibody comprises a VH sequence of SEQ ID NO:180 and a VL sequence of SEQ ID NO: 179. In some aspects, the anti-OX40 antibody comprises a VH sequence of SEQ ID NO: 182. In some aspects, the anti-OX40 antibody comprises a VL sequence of SEQ ID NO: 181. In some aspects, the anti-OX40 antibody comprises a VH sequence of SEQ ID NO:182 and a VL sequence of SEQ ID NO: 181.

Exemplary disorders that may be diagnosed using an antibody of the invention include cancer.

In certain aspects, labeled anti-OX40 antibodies are provided. Labels include, but are not limited to, labels or moieties that are detected directly (such as fluorescent, chromophoric, electrondense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

In one aspect, the invention provides diagnostic methods, e.g. for identifying a cancer patient who is likely to respond to treatment with an anti-human OX40 agonist antibody.

In some aspects, methods are provided for identifying patients who are likely to respond to treatment with anti-human OX40 agonist antibody, the methods comprising (i) determining presence or absence or amount (e.g., number per given sample size) of cells expressing FcR in a sample of cancer from the patient, and (ii) identifying the patient as likely to respond if the sample comprises cells expressing FcR (e.g., high number of cells expressing FcR). Methods for detecting cells that express FcR are well known in the art, including, e.g., by IHC. In some aspects, FcR is FcγR. In some aspects, FcR is an activating FcγR. In some aspects, the cancer is any cancer described herein. In some aspects, the cancer is non-small cell lung cancer (NSCLC), glioblastoma, neuroblastoma, melanoma, breast carcinoma (e.g. triple-negative breast cancer), gastric cancer, colorectal cancer (CRC), or hepatocellular carcinoma. In some aspects, the method is an in vitro method. In some aspects, the methods further comprise (iii) recommending treatment with the anti-human OX40 agonist antibody (e.g., any of the anti-human OX40 agonist antibodies described herein). In some aspects, the methods further comprise (iv) treating the patient with the anti-human OX40 agonist antibody.

In some aspects, methods are provided for identifying patients who are likely to respond to treatment with anti-human OX40 agonist antibody, the methods comprising (i) determining presence or absence or amount (e.g., number per given sample size) of human effector cells (e.g., infiltrating effector cells) in a sample of cancer from the patient, and (ii) identifying the patient as likely to respond if the sample comprises effector cells (e.g., high number of effector cells). Methods for detecting infiltrating human effector cells are well known in the art, including, e.g., by IHC. In some aspects, human effector cells are one or more of NK cells, macrophages, monocytes. In some aspects, the effector cells express activating FcγR. In some aspects, the method is an in vitro method. In some aspects, the cancer is any cancer described herein. In some aspects, the cancer is non-small cell lung cancer (NSCLC), glioblastoma, neuroblastoma, melanoma, breast carcinoma (e.g. triple-negative breast cancer), gastric cancer, colorectal cancer (CRC), or hepatocellular carcinoma. In some aspects, the methods further comprise (iii) recommending treatment with the anti-human OX40 agonist antibody (e.g., any of the anti-human OX40 agonist antibodies described herein). In some aspects, the methods further comprise (iv) treating the patient with the anti-human OX40 agonist antibody.

Provided are methods of providing a cancer diagnosis comprising: (i) measuring FcR expressing cells (e.g., the level or presence or absence of or prevalence (e.g., percentage of cells expressing FcR, e.g., by IHC) of FcR) in a sample from the patient; (ii) diagnosing the patient as having cancer comprising FcR biomarker (e.g., high FcR biomarker) when the sample has FcR biomarker expression. In some aspects, the method further comprises (iii) selecting a therapy comprising (a) anti-human OX40 agonist antibody or (b) recommending a therapy comprising anti-human OX40 agonist antibody for the patient. In some aspects, the method is an in vitro method.

Provided are methods of providing a cancer diagnosis comprising: (i) measuring human effector cells (e.g., the level or presence or absence of or prevalence (e.g., percentage of human effector cells) of human effector cells) in a sample from the patient; (ii) diagnosing the patient as having cancer comprising human effector cells (e.g., high human effector cells) when the sample has human effector cell biomarker. In some aspects, the method further comprises (iii) selecting a therapy comprising (a) anti-human OX40 agonist antibody or (b) recommending a therapy comprising anti-human OX40 agonist antibody for the patient. In some aspects, the method is an in vitro method.

Provided are methods of recommending a treatment to a cancer patient comprising: (i) measuring FcR expressing cells (e.g., the level or presence or absence of or prevalence (e.g., percentage of cells expressing FcR) of FcR) in a sample from the patient; (ii) recommending treatment with an anti-human OX40 agonist antibody when the sample has FcR expressing cells (in some aspects, high FcR expressing cells). In some aspects, the method further comprises (iii) selecting a therapy comprising anti-human OX40 agonist antibody for the patient. In some aspects, the method is an in vitro method.

Provided are methods of recommending a treatment to a cancer patient comprising: (i) measuring human effector cells (e.g., the level or presence or absence of or prevalence (e.g., percentage of human effector cells) of human effector cells) in a sample from the patient; (ii) recommending treatment with an anti-human OX40 agonist antibody when the sample has human effector cells (in some aspects, high human effector cells). In some aspects, the method further comprises (iii) selecting a therapy comprising anti-human OX40 agonist antibody for the patient. In some aspects, the method is an in vitro method.

In some aspects of any of the inventions provided herein, the sample is obtained prior to treatment with anti-human OX40 agonist antibody. In some aspects, the sample is obtained prior to treatment with a cancer medicament. In some aspects, the sample is obtained after the cancer has metastasized. In some aspects, the sample is formalin fixed and paraffin embedded (FFPE). In some aspects, the sample is of a biopsy (e.g., a core biopsy), a surgical specimen (e.g., a specimen from a surgical resection), or a fine needle aspirate.

### F. Pharmaceutical Formulations

Pharmaceutical formulations of an anti-OX40 antibody as described herein are prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

In some aspects, a "histidine buffer" is a buffer comprising histidine ions. Examples of histidine buffers include histidine chloride, histidine acetate, histidine phosphate, histidine sulfate. The preferred histidine buffer identified in the examples herein was found to be histidine acetate. In the preferred aspect, the histidine acetate buffer is prepared by titrating L-histidine (free base, solid) with acetic acid (liquid). In some aspects, the histidine buffer or histidine-acetate buffer is at pH 5.0 to 6.0, in some aspects, pH 5.3 to 5.8.

In some aspects, a "saccharide" herein comprises the general composition (CH2O)n and derivatives thereof, including monosaccharides, disaccharides, trisaccharides, polysaccharides, sugar alcohols, reducing sugars, nonreducing sugars, etc. Examples of saccharides herein include glucose, sucrose, trehalose, lactose, fructose, maltose, dextran, glycerin, dextran, erythritol, glycerol, arabitol, sylitol, sorbitol, mannitol, mellibiose, melezitose, raffinose, mannotriose, stachyose, maltose, lactulose, maltulose, glucitol, maltitol, lactitol, iso-maltulose, etc. In some aspects, the saccharide is a nonreducing disaccharide, such as trehalose or sucrose.

In some aspects herein, a "surfactant" refers to a surface-active agent, preferably a nonionic surfactant. Examples of surfactants herein include polysorbate (for example, polysorbate 20 and polysorbate 80); poloxamer (e.g. poloxamer 188); Triton; sodium dodecyl sulfate (SDS); sodium laurel sulfate; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palmidopropyl-, or isostearamidopropyl-betaine (e.g. lauroamidopropyl); myristamidopropyl-, palmidopropyl-, or isostearamidopropyl-dimethylamine; sodium methyl cocoyl-, or disodium methyl oleyl-taurate; and the MONAQUAT™ series (Mona Industries, Inc., Paterson, New Jersey); polyethyl glycol, polypropyl glycol, and copolymers of ethylene and propylene glycol (*e.g.* Pluronics, PF68 etc); etc. In some aspects, the surfactant is polysorbate 20. In some aspects, the surfactant is polysorbate 80.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide an additional medicament (examples of which are provided herein). Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for *in vivo* administration are generally sterile. Sterility may be readily accomplished, e.g., by filtration through sterile filtration membranes.

In some aspects, provided herein are pharmaceutical formulations comprising: (a) any of the anti-human OX40 agonist antibodies described herein; (b) a histidine buffer at pH 5.0-6.0.

In some aspects, provided herein are pharmaceutical formulations comprising: (a) any of the anti-human OX40 agonist antibodies described herein; (b) a histidine buffer at pH 5.0-6.0; (c) a saccharide; and (d) a surfactant.

In some aspects of any of the formulations, the anti-human OX40 agonist antibody is present at a concentration between about 10 mg/mL and about 100 mg/mL (*e.g*. about 15 mg/mL, 18 mg/mL, 20 mg/mL, 60 mg/mL, and 75 mg/mL). In some aspects, the anti-human OX40 agonist antibody is present at a concentration of about 20 mg/mL. In some aspects, the anti-human OX40 agonist antibody is present at a concentration of about 50 mg/mL. In some aspects, the anti-human OX40 agonist antibody is present at a concentration of about 60 mg/mL. In some aspects, the anti-human OX40 agonist antibody is present at a concentration of about 70 mg/mL.

In some aspects of any of the formulations, the saccharide is present at a concentration of about 75 mM to about 360 mM (*e.g.,* about 100 mM, about 120 mM, about 240 mM, about 320 mM to about 360 mM). In some aspects, the saccharide is present at a concentration of about 120 mM. In some aspects, the saccharide is present at a concentration of about 240 mM. In some aspects, the saccharide is present at a concentration of about 320 mM. In some aspects, the saccharide is a disaccharide. In some aspects, the disaccharide is trehalose. In some aspects, the disaccharide is sucrose.

In some aspects of any of the formulations, the histidine buffer is at a concentration of about 1 mM to about 50 mM (*e.g*. about 1 mM to about 25 mM). In some aspects, the histidine buffer is at a concentration of about 10 mM. In some aspects, the histidine buffer is at a concentration of about 20 mM. In some aspects, the histidine buffer is at a concentration of about 30 mM. In some aspects, the histidine buffer is histidine acetate.

In some aspects of any of the formulations, the surfactant is polysorbate (e.g., polysorbate 20 or polysorbate 40), poloxamer (*e.g*. poloxamer 188); Triton; sodium dodecyl sulfate (SDS); sodium laurel sulfate; or sodium octyl glycoside.

In some aspects of any of the formulations, the surfactant is polysorbate. In some aspects, the polysorbate is present at a concentration of about 0.005% to about 0.1%. In some aspects, the polysorbate is present at a concentration of about 0.005%. In some aspects, the polysorbate is present at a concentration of about 0.02%. In some aspects, the polysorbate is present at a concentration of about 0.04%. In some aspects, the polysorbate is present at a concentration of about 0.06%. In some aspects, the polysorbate is polysorbate 20. In some aspects, the polysorbate is polysorbate 80.

In some aspects of any of the formulations, the formulation is diluted with a diluent (*e.g.,* 0.9% NaCl). In some aspects, the anti-human OX40 agonist antibody is present at a concentration of about 1 mg/mL.

In particular, provided herein are pharmaceutical formulations comprising (a) any of the anti-human OX40 agonist antibodies described herein, (b) a polysorbate, wherein the polysorbate concentration is about 0.005% to about 0.1%.; and (c) a histidine buffer *(e.g.,* a histidine buffer at a pH between 5.0 and 6.0).

In some aspects, the pharmaceutical formulation comprises (a) any of the anti-human OX40 agonist antibodies described herein (*e.g.,* at a concentration between about 10 mg/mL and about 100 mg/mL), (b) a polysorbate, wherein the polysorbate concentration is about 0.02% to about 0.06%; (c) a histidine buffer (*e.g.,* a histidine buffer at pH 5.0 to 6.0); and a saccharide, wherein the saccharide concentration is about 120mM to about 320 mM. In some aspects, the saccharide is sucrose.

In some aspects, the pharmaceutical formulation comprises (a) any of the anti-human OX40 agonist antibodies described herein at a concentration between about 10 mg/mL and about 100 mg/mL, (b) a polysorbate, wherein the polysorbate concentration is about 0.02% to about 0.06%, wherein the polysorbate is polysorbate 20 or polysorbate 40; (c) a histidine acetate buffer at pH 5.0 to 6.0; and a saccharide (e.g., sucrose) at a concentration of about 120mM to about 320 mM.

In some aspects, the pharmaceutical formulation comprises (a) any of the anti-human OX40 agonist antibodies described herein, (b) polysorbate 20, wherein the polysorbate concentration is about 0.02% to about 0.06%; (c) a histidine acetate buffer (*e.g.,* a histidine acetate buffer at pH 5.0 to 6.0); and (d) sucrose, wherein the sucrose concentration is about 120 mM to about 320 mM.

In some aspects, the pharmaceutical formulation comprises (a) any of the anti-human OX40 agonist antibodies described herein, (b) polysorbate 40, wherein the polysorbate concentration is about 0.02% to about 0.06%; (c) a histidine acetate buffer (*e.g.,* a histidine acetate buffer at a pH between 5.0 and 6.0); and sucrose, wherein the sucrose concentration is about 120 mM to about 320 mM.

In some aspects, the pharmaceutical formulation comprises (a) any of the anti-human OX40 agonist antibodies described herein, (b) polysorbate 20, wherein the polysorbate concentration is about 0.02%; (c) a histidine acetate buffer at pH 6.0; and (d) sucrose, wherein the sucrose concentration is about 320 mM.

In some aspects, the pharmaceutical formulation comprises (a) any of the anti-human OX40 agonist antibodies described herein, (b) polysorbate 20, wherein the polysorbate concentration is about 0.02%; (c) a histidine acetate buffer at pH 5.5; and (d) sucrose, wherein the sucrose concentration is about 240 mM.

In some aspects, the pharmaceutical formulation comprises (a) any of the anti-human OX40 agonist antibodies described herein, (b) polysorbate 20, wherein the polysorbate concentration is about 0.04%; (c) a histidine acetate buffer at pH 6.0; and (d) sucrose, wherein the sucrose concentration is about 120 mM.

In some aspects, the pharmaceutical formulation comprises (a) any of the anti-human OX40 agonist antibodies described herein, (b) polysorbate 40, wherein the polysorbate concentration is about 0.04%; (c) a histidine acetate buffer at pH 5.0; and (d) sucrose, wherein the sucrose concentration is about 240 mM.

In some aspects, the pharmaceutical formulation comprises (a) any of the anti-human OX40 agonist antibodies described herein, (b) polysorbate 40, wherein the polysorbate concentration is about 0.04%; (c) a histidine acetate buffer at pH 6.0; and (d) sucrose, wherein the sucrose concentration is about 120 mM.

In some aspects, the pharmaceutical formulation is a liquid pharmaceutical formulation. In some aspects, the pharmaceutical formulation is a stable pharmaceutical formulation. In some aspects, the pharmaceutical formulation is a stable liquid pharmaceutical formulation.

In some aspects of any of the pharmaceutical formulations described herein, the anti-human OX40 agonist antibody of the pharmaceutical formulation is present at a concentration between about 10 mg/mL and about 100 mg/mL. In some aspects, the concentration of the human OX40 agonist antibody is between about any of 10 mg/mL to 50 mg/mL, 10 mg/mL to 75 mg/mL, 25 mg/mL to 75 mg/mL, 50 mg/mL to 100 mg/mL, 50 mg/mL to 75 mg/mL, and/or 75 mg/mL to 100 mg/mL. In some aspects, the concentration of the human OX40 agonist antibody is greater than about any of 20 mg/mL, 30 mg/mL, 40 mg/mL, 50 mg/mL, 60 mg/mL, 70 mg/mL, or 100 mg/mL.

The pharmaceutical formulation preferably comprises a polysorbate. The polysorbate is generally included in an amount which reduces aggregate formation (such as that which occurs upon shaking or shipping). Examples of polysorbate include, but are not limited to, polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), and/or polysorbate 80 (polyoxyethylene (20) sorbitan monooleate). In some aspects, the polysorbate is polysorbate 20 (polyoxyethylene (20) sorbitan monolaurate). In some aspects of any of the pharmaceutical formulations described herein, the polysorbate concentration is sufficient to minimize aggregation and/or maintain stability upon long term storage and/or during administration (e.g., after dilution in an IV bag). In some aspects, the polysorbate concentration is about 0.005% w/v, about 0.02% w/v, about 0.04% w/v and less than about 0.1% w/v. In some aspects, the polysorbate concentration is greater than 0.01% w/v and less than about 0.1% w/v. In some aspects, the polysorbate concentration is about any of 0.005% w/v, about 0.02% w/v, 0.03% w/v, 0.04% w/v, or 0.05% w/v. In some aspects, the polysorbate is present at a concentration of about 0.04% w/v. In some aspects, the polysorbate is present at a concentration of about 0.02% w/v.

The pharmaceutical formulation preferably comprises a saccharide. Saccharides include monosaccharides, disaccharides, trisaccharides, polysaccharides, sugar alcohols, reducing sugars, nonreducing sugars, etc. Further examples of saccharides include, but are not limited to, glucose, sucrose, trehalose, lactose, fructose, maltose, dextran, glycerin, dextran, erythritol, glycerol, arabitol, sylitol, sorbitol, mannitol, mellibiose, melezitose, raffinose, mannotriose, stachyose, maltose, lactulose, maltulose, glucitol, maltitol, lactitol, iso-maltulose, etc. In some aspects, the saccharide is a disaccharide. In some aspects, the saccharide is a nonreducing disaccharide. In some aspects, the saccharide is trehalose.

The saccharide is generally included in an amount which reduces aggregate formation. In some aspects of any of the pharmaceutical formulations described herein, the saccharide is present at a concentration of between about any of 50 mM to 250 mM, 75 mM to 200 mM, 75 mM to 150 mM, 100 mM to 150 mM, or 110 mM to 130 mM, or 100mM to 320 mM, or 240 mM to 320 mM, or 240 mM to 400mM. In some aspects, the saccharide is present at a concentration greater than about any of 50 mM, 75 mM, 100 mM, 110 mM, or 115 mM. In some aspects, the saccharide is present at a concentration of about any of 100 mM, 110 mM, 120 mM, 130 mM, or 140 mM. In some aspects, the saccharide is present at a concentration of about 120 mM. In some aspects of any of the formulations, the saccharide is present at a concentration of about 75 mM to about 360 mM (e.g., about 100 mM, about 120 mM, about 240 mM, about 320 mM to about 360 mM). In some aspects, the saccharide is present at a concentration of about 240 mM. In some aspects, the saccharide is present at a concentration of about 320 mM.

The pharmaceutical formulation preferably comprises a histidine buffer. Examples of histidine buffers include, but are not limited to, histidine chloride, histidine succinate, histidine acetate, histidine phosphate, histidine sulfate. In some aspects, the histidine buffer is histidine acetate. In some aspects of any of the pharmaceutical formulations described herein, the histidine buffer concentration is between about any of 1 mM to 50 mM, 1 mM to 35 mM, 1 mM to 25 mM, 1 mM to 20 mM, 7.5 mM to 12.5 mM, or 5 mM to 15 mM, 20mM to 30mM, 25 mM to 35 mM. In some aspects, the histidine buffer concentration is about any of 5 mM, 7.5 mM, 10 mM, 12.5 mM, 15 mM, 20 mM, 25 mM, 30 mM, 35 mM or 40 mM. In some aspects, the histidine buffer concentration is about 10 mM. In some aspects, the histidine buffer concentration is about 20 mM. In some aspects, the histidine buffer concentration is about 30 mM. In some aspects, the histidine buffer concentration is about 40 mM. In some aspects of any of the pharmaceutical formulations described herein, the histidine buffer is at a pH of between pH 5.0 and 6.0, for example, about any of pH 5.0, pH 5.1, pH 5.2, pH 5.3, pH 5.4, pH 5.5, pH 5.6, pH 5.7, pH 5.8, pH 5.9 or pH 6.0.. In some aspects, the pH is between pH 4.9 to pH 6.3.

The pharmaceutical formulation herein may also contain more than one active compound as necessary for the particular indication being treated, preferably those with complementary activities that do not adversely affect each other. Such molecules are suitably present in combination in amounts that are effective for the purpose intended.

Further, provided herein are vials and methods of filing a vial comprising a pharmaceutical formulation described herein. In some aspects, the pharmaceutical formulation is provided inside a vial with a stopper pierceable by a syringe, preferably in aqueous form. The vial is desirably stored at about 2-8°C as well as up to 30°C for 24 hours until it is administered to a subject in need thereof. The vial may for example be a 15 cc vial (for example for a 200 mg dose).

The pharmaceutical formulation for administration is preferably a liquid formulation (not lyophilized) and has not been subjected to prior lyophilization. While the pharmaceutical formulation may be lyophilized, preferably it is not. In some aspects of any of the pharmaceutical formulations, the pharmaceutical formulation, the pharmaceutical formulation is a lyophilized pharmaceutical formulation. In some aspects, the pharmaceutical formulation is a liquid formulation. In some aspects, the pharmaceutical formulation does not contain a tonicifying amount of a salt such as sodium chloride. In some aspects of any of the pharmaceutical formulations, the pharmaceutical formulation is diluted.

### G. Therapeutic Methods and Compositions

Any of the anti-human OX40 antibodies provided herein may be used in therapeutic methods.

In one aspect, an anti-human OX40 agonist antibody for use as a medicament is provided. In further aspects, an anti-human OX40 agonist antibody for use in treating cancer is provided. In certain aspects, an anti-human OX40 agonist antibody for use in a method of treatment is provided. In certain aspects, the invention provides an anti-human OX40 agonist antibody for use in a method of treating an individual having cancer comprising administering to the individual an effective amount of the anti-human agonist OX40 antibody. In one such aspect, the method further comprises administering to the individual an effective amount of at least one additional therapeutic agent, e.g., as described below.

In one aspect, disclosed is an anti-human OX40 agonist antibody for use in enhancing immune function (e.g., by upregulating cell-mediated immune responses) in an individual having cancer comprising administering to the individual an effective amount of the anti-human agonist OX40 antibody. In one aspect, disclosed is an anti-human OX40 agonist antibody for use in enhancing T cell function in an individual having cancer comprising administering to the individual an effective amount of the anti-human agonist OX40 antibody. In one aspect, disclosed are an anti-human OX40 agonist antibody for use in depleting human OX40-expressing cells (e.g., OX40 expressing T cells, e.g., OX40 expressing Treg) comprising administering to the individual an effective amount of the anti-human agonist OX40 antibody. In some aspects, depletion is by ADCC. In some aspects, depletion is by phagocytosis. Disclosed is an anti-human OX40 agonist antibody for treating an individual having tumor immunity.

In further aspects, an anti-human OX40 agonist antibody for use in treating infection (e.g., with a bacteria or virus or other pathogen) is disclosed. In certain aspects, disclosed is an anti-human OX40 agonist antibody for use in a method of treating an individual having an infection comprising administering to the individual an effective amount of the anti-human agonist OX40 antibody. In some aspects, the infection is with a virus and/or a bacteria. In some aspects, the infection is with a pathogen.

In a further aspect, disclosed is the use of an anti-OX40 antibody in the manufacture or preparation of a medicament. In one aspect, the medicament is for treatment of cancer. In a further aspect, the medicament is for use in a method of treating cancer comprising administering to an individual having cancer an effective amount of the medicament. In one such aspect, the method further comprises administering to the individual an effective amount of at least one additional therapeutic agent, e.g., as described below.

In one aspect, the medicament is for use in enhancing immune function (e.g., by upregulating cell-mediated immune responses) in an individual having cancer comprising administering to the individual an effective amount of the medicament. In one aspect, the medicament is for use in enhancing T cell function in an individual having cancer comprising administering to the individual an effective amount of the medicament. In some aspects, the T cell dysfunctional disorder is cancer. In one aspect, the medicament is for use in depleting human OX40-expressing cells (e.g., cell expressing high OX40, e.g., OX40 expressing T cells) comprising administering to the individual an effective amount of the medicament. In some aspects, depletion is by ADCC. In some aspects, depletion is by phagocytosis. In one aspect, the medicament is for treating an individual having tumor immunity.

In further aspects, the medicament is for use in treating infection (e.g., with a bacteria or virus or other pathogen). In certain aspects, the medicament is for use in a method of treating an individual having an infection comprising administering to the individual an effective amount of the medicament. In some aspects, the infection is with virus and/or bacteria. In some aspects, the infection is with a pathogen.

In a further aspect, the invention provides a method for treating a cancer. In one aspect, the method comprises administering to an individual having such cancer an effective amount of an anti-OX40 antibody. In one such aspect, the method further comprises administering to the individual an effective amount of at least one additional therapeutic agent, as described below. An "individual" according to any of the above aspects may be a human.

In one aspect, disclosed is a method for enhancing immune function (e.g., by upregulating cell-mediated immune responses) in an individual having cancer comprising administering to the individual an effective amount of the anti-human agonist OX40 antibody. In one aspect, disclosed is a method for enhancing T cell function in an individual having cancer comprising administering to the individual an effective amount of the anti-human agonist OX40 antibody. In one aspect, disclosed is a method for depleting human OX40-expressing cells (e.g., cells that express high level of OX40, e.g., OX40 expressing T cells) comprising administering to the individual an effective amount of the anti-human agonist OX40 antibody. In some aspects, depletion is by ADCC. In some aspects, depletion is by phagocytosis.

In some aspects, examples of cancer further include, but are not limited to, B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), B-cell proliferative disorders, and Meigs' syndrome. More specific examples include, but are not limited to, relapsed or refractory NHL, front line low grade NHL, Stage III/IV NHL, chemotherapy resistant NHL, precursor B lymphoblastic leukemia and/or lymphoma, small lymphocytic lymphoma, B-cell chronic lymphocytic leukemia and/or prolymphocytic leukemia and/or small lymphocytic lymphoma, B-cell prolymphocytic lymphoma, immunocytoma and/or lymphoplasmacytic lymphoma, lymphoplasmacytic lymphoma, marginal zone B-cell lymphoma, splenic marginal zone lymphoma, extranodal marginal zone-MALT lymphoma, nodal marginal zone lymphoma, hairy cell leukemia, plasmacytoma and/or plasma cell myeloma, low grade/follicular lymphoma, intermediate grade/follicular NHL, mantle cell lymphoma, follicle center lymphoma (follicular), intermediate grade diffuse NHL, diffuse large B-cell lymphoma, aggressive NHL (including aggressive front-line NHL and aggressive relapsed NHL), NHL relapsing after or refractory to autologous stem cell transplantation, primary mediastinal large B-cell lymphoma, primary effusion lymphoma, high grade immunoblastic NHL, high grade lymphoblastic NHL, high grade small non-cleaved cell NHL, bulky disease NHL, Burkitt's lymphoma, precursor (peripheral) large granular lymphocytic leukemia, mycosis fungoides and/or Sezary syndrome, skin (cutaneous) lymphomas, anaplastic large cell lymphoma, angiocentric lymphoma.

In some aspects, examples of cancer further include, but are not limited to, B-cell proliferative disorders, which further include, but are not limited to, lymphomas (e.g., B-Cell Non-Hodgkin's lymphomas (NHL)) and lymphocytic leukemias. Such lymphomas and lymphocytic leukemias include e.g. a) follicular lymphomas, b) Small Non-Cleaved Cell Lymphomas/ Burkitt's lymphoma (including endemic Burkitt's lymphoma, sporadic Burkitt's lymphoma and Non-Burkitt's lymphoma), c) marginal zone lymphomas (including extranodal marginal zone B-cell lymphoma (Mucosa-associated lymphatic tissue lymphomas, MALT), nodal marginal zone B-cell lymphoma and splenic marginal zone lymphoma), d) Mantle cell lymphoma (MCL), e) Large Cell Lymphoma (including B-cell diffuse large cell lymphoma (DLCL), Diffuse Mixed Cell Lymphoma, Immunoblastic Lymphoma, Primary Mediastinal B-Cell Lymphoma, Angiocentric Lymphoma-Pulmonary B-Cell Lymphoma), f) hairy cell leukemia, g) lymphocytic lymphoma, Waldenstrom's macroglobulinemia, h) acute lymphocytic leukemia (ALL), chronic lymphocytic leukemia (CLL)/ small lymphocytic lymphoma (SLL), B cell prolymphocytic leukemia, i) plasma cell neoplasms, plasma cell myeloma, multiple myeloma, plasmacytoma, and/or j) Hodgkin's disease.

In some aspects of any of the methods, the cancer is a B-cell proliferative disorder. In some aspects, the B-cell proliferative disorder is lymphoma, non-Hodgkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), or mantle cell lymphoma. In some aspects, the B-cell proliferative disorder is NHL, such as indolent NHL and/or aggressive NHL. In some aspects, the B-cell proliferative disorder is indolent follicular lymphoma or diffuse large B-cell lymphoma.

In a further aspect, the invention provides pharmaceutical formulations comprising the anti-OX40 antibodies provided herein, e.g., for use in any of the above therapeutic methods. In one aspect, a pharmaceutical formulation comprises any of the anti-OX40 antibodies provided herein and a pharmaceutically acceptable carrier. In another aspect, a pharmaceutical formulation comprises any of the anti-OX40 antibodies provided herein and at least one additional therapeutic agent, e.g., as described below.

In some aspects of any of the methods of the invention, the anti-human OX40 agonist antibodies inhibits tumor immunity by inhibiting Treg function (e.g., inhibiting the suppressive function of Tregs), killing OX40 expressing cells (e.g., cells that express high levels of OX40), increasing effector T cell function and/or increasing memory T cell function. In some aspects of any of the methods of the invention, the anti-human OX40 agonist antibodies treat cancer by inhibiting Treg function (e.g., inhibiting the suppressive function of Tregs), killing OX40 expressing cells (e.g., cells that express high levels of OX40), increasing effector T cell function and/or increasing memory T cell function. In some aspects of any of the methods of the invention, the anti-human OX40 agonist antibodies enhance immune function by inhibiting Treg function (e.g., inhibiting the suppressive function of Tregs), killing OX40 expressing cells (e.g., cells that express high levels of OX40), increasing effector T cell function and/or increasing memory T cell function. In some aspects of any of the methods of the invention, the anti-human OX40 agonist antibodies enhance T cell function by inhibiting Treg function (e.g., inhibiting the suppressive function of Tregs), killing OX40 expressing cells (e.g., cells that express high levels of OX40), increasing effector T cell function and/or increasing memory T cell function.

In some aspects of any of the methods, the anti-human OX40 agonist antibody is a depleting anti-human agonist antibody. In some aspects, treatment with the anti-human OX40 agonist antibody results in cell depletion (e.g., depletion of OX40-expressing cells, e.g., depletion of cells that express high levels of OX40). In some aspects, depletion is by ADCC. In some aspects, depletion is by phagocytosis.

In some aspects of any of the methods, the anti-human OX40 agonist antibody inhibits Treg function, e.g., by inhibiting Treg suppression of effector and/or memory T cell function (in some aspects, effector T cell and/or memory T cell proliferation and/or cytokine secretion), relative to Treg function prior to administration of the OX40 agonist antibody. In some aspects of any of the methods, the anti-human OX40 agonist antibody increases effector T cell proliferation, relative to effector T cell proliferation prior to administration of the OX40 agonist antibody. In some aspects of any of the methods, the anti-human OX40 agonist antibody increases memory T cell proliferation, relative to memory T cell proliferation prior to administration of the OX40 agonist antibody. In some aspects of any of the methods, the anti-human OX40 agonist antibody increases effector T cell cytokine production (e.g., gamma interferon production), relative to effector T cell cytokine production prior to administration of the OX40 agonist antibody. In some aspects of any of the methods, the anti-human OX40 agonist antibody increases memory T cell cytokine production (e.g., gamma interferon production), relative to memory T cell cytokine production prior to administration of the OX40 agonist antibody. In some aspects of any of the methods, the anti-human OX40 agonist antibody increases CD4+ effector T cell proliferation and/or CD8+ effector T cell proliferation relative to CD4+ effector T cell proliferation and/or CD8+ effector T cell proliferation prior to administration of the OX40 agonist antibody. In some aspects of any of the methods, the anti-human OX40 agonist antibody increases memory T cell proliferation (e.g., CD4+ memory T cell proliferation), relative to memory T cell proliferation prior to administration of the OX40 agonist antibody. In some aspects, the CD4+ effector T cells in the individual have enhanced proliferation, cytokine secretion and/or cytolytic activity relative to proliferation, cytokine secretion and/or cytolytic activity prior to the administration of the anti-human OX40 agonist antibody.

In some aspects of any of the methods of the invention, the number of CD4+ effector T cells is elevated relative to prior to administration of the anti-human OX40 agonist antibody. In some aspects, CD4+ effector T cell cytokine secretion is elevated relative to prior to administration of the anti-human OX40 agonist antibody. In some aspects of any of the methods, the CD8+ effector T cells in the individual have enhanced proliferation, cytokine secretion and/or cytolytic activity relative to prior to the administration of the anti-human OX40 agonist antibody. In some aspects, the number of CD8+ effector T cells is elevated relative to prior to administration of the anti-human OX40 agonist antibody. In some aspects, CD8+ effector T cell cytokine secretion is elevated relative to prior to administration of the anti-human OX40 agonist antibody.

In some aspects of any of the methods of the invention, the anti-human OX40 agonist antibody binds human effector cells, e.g., binds FcγR expressed by human effector cells. In some aspects, the human effector cell performs ADCC effector function. In some aspects, the human effector cell performs phagocytosis effector function.

In some aspects of any of the methods of the invention, the anti-human OX40 agonist antibody comprising a variant IgG1 Fc polypeptide comprising a mutation that eliminates binding to human effector cells (e.g., a DANA or N297G mutation) has diminished activity (e.g., CD4+ effector T cell function, e.g., proliferation), relative to anti-human OX40 agonist antibody comprising native sequence IgG1 Fc portion. In some aspect, the anti-human OX40 agonist antibody comprising a variant IgG1 Fc polypeptide comprising a mutation that eliminates binding to human effector cells (e.g., a DANA or N297G mutation) does not possess substantial activity (e.g., CD4+ effector T cell function, e.g., proliferation).

In some aspects of any of the methods of the invention, antibody cross-linking is required for anti-human OX40 agonist antibody function. In some aspects, function is stimulation of CD4+ effector T cell proliferation. In some aspects, antibody cross-linking is determined by providing anti-human OX40 agonist antibody adhered on a solid surface (e.g., a cell culture plate). In some aspects, antibody cross-linking is determined by introducing a mutation in the antibody's IgG1 Fc portion (e.g., a DANA or N297S mutation) and testing function of the mutant antibody.

In some aspects of any of the methods, the memory T cells in the individual have enhanced proliferation and/or cytokine secretion relative to prior to the administration of the anti-human OX40 agonist antibody. In some aspects, the number of memory T cells is elevated relative to prior to administration of the anti-human OX40 agonist antibody. In some aspects, memory T cell cytokine secretion (level) is elevated relative to prior to administration of the anti-human OX40 agonist antibody. In some aspects of any of the methods, the Treg in the individual have decreased inhibition of effector T cell function (e.g., proliferation and/or cytokine secretion) relative to prior to the administration of the anti-human OX40 agonist antibody. In some aspects, the number of effector T cells is elevated relative to prior to administration of the anti-human OX40 agonist antibody. In some aspects, effector T cell cytokine secretion (level) is elevated relative to prior to administration of the anti-human OX40 agonist antibody.

In some aspects of any of the methods of the invention, the number of intratumoral (infiltrating) CD4+ effector T cells (e.g., total number of CD4+ effector T cells, or e.g., percentage of CD4+ cells in CD45+ cells) is elevated relative to prior to administration of the anti-human OX40 agonist antibody. In some aspects of any of the methods of the invention, number of intratumoral (infiltrating) CD4+ effector T cells that express gamma interferon (e.g., total gamma interferon expressing CD4+ cells, or e.g., percentage of gamma interferon expressing CD4+ cells in total CD4+ cells) is elevated relative to prior to administration anti-human OX40 agonist antibody.

In some aspects of any of the methods of the invention, the number of intratumoral (infiltrating) CD8+ effector T cells (e.g., total number of CD8+ effector T cells, or e.g., percentage of CD8+ in CD45+ cells) is elevated relative to prior to administration of anti-human OX40 agonist antibody. In some aspects of any of the methods of the invention, number of intratumoral (infiltrating) CD8+ effector T cells that express gamma interferon (e.g., percentage of CD8+ cells that express gamma interferon in total CD8+ cells) is increased relative to prior to administration of anti-human OX40 agonist antibody.

In some aspects of any of the methods of the invention, the number of intratumoral (infiltrating) Treg (e.g., total number of Treg or e.g., percentage of Fox3p+ cells in CD4+ cells) is reduced relative to prior to administration of anti-human OX40 agonist antibody.

In some aspects of any of the methods of the invention, administration of anti-human OX40 agonist antibody is in combination with administration of a tumor antigen. In some aspects, the tumor antigen comprises protein. In some aspects, the tumor antigen comprises nucleic acid. In some aspects, the tumor antigen is a tumor cell.

In some aspects of any of the methods of the invention, the cancer displays human effector cells (e.g., is infiltrated by human effector cells). Methods for detecting human effector cells are well known in the art, including, e.g., by IHC. In some aspects, the cancer display high levels of human effector cells. In some aspects, human effector cells are one or more of NK cells, macrophages, monocytes. In some aspects, the cancer is any cancer described herein. In some aspects, the cancer is non-small cell lung cancer (NSCLC), glioblastoma, neuroblastoma, melanoma, breast carcinoma (e.g. triple-negative breast cancer), gastric cancer, colorectal cancer (CRC), or hepatocellular carcinoma.

In some aspects of any of the methods of the invention, the cancer displays cells expressing FcR (e.g., is infiltrated by cells expressing FcR). Methods for detecting FcR are well known in the art, including, e.g., by IHC. In some aspects, the cancer display high levels of cells expressing FcR. In some aspects, FcR is FcγR. In some aspects, FcR is activating FcγR. In some aspects, the cancer is non-small cell lung cancer (NSCLC), glioblastoma, neuroblastoma, melanoma, breast carcinoma (e.g. triple-negative breast cancer), gastric cancer, colorectal cancer (CRC), or hepatocellular carcinoma.

An "individual" according to any of the above aspects is preferably a human.

Antibodies of the invention can be used either alone or in combination with other agents in a therapy. For instance, an antibody of the invention may be co-administered with at least one additional therapeutic agent.

Such combination therapies noted above encompass combined administration (where two or more therapeutic agents are included in the same or separate formulations), and separate administration, in which case, administration of the antibody of the invention can occur prior to, simultaneously, and/or following, administration of the additional therapeutic agent or agents. In one embodiment, administration of the anti-OX40 antibody and administration of an additional therapeutic agent occur within about one month, or within about one, two or three weeks, or within about one, two, three, four, five, or six days, of each other. Antibodies of the invention can also be used in combination with radiation therapy.

In some aspects, an anti-human OX40 agonist antibody may be administered in conjunction with a chemotherapy or chemotherapeutic agent. In some aspects, an anti-human OX40 agonist antibody may be administered in conjunction with a radiation therapy or radiotherapeutic agent. In some aspects, an anti-human OX40 agonist antibody may be administered in conjunction with a targeted therapy or targeted therapeutic agent. In some aspects, an anti-human OX40 agonist antibody may be administered in conjunction with an immunotherapy or immunotherapeutic agent, for example a monoclonal antibody.

In some aspects, an anti-human OX40 agonist antibody may be administered in conjunction with a PARP inhibitor (e.g., Olaparanib, Rucaparib, Niraparib, Cediranib, BMN673, Veliparib), Trabectedin, nab-paclitaxel (albumen-bound paclitaxel, ABRAXANE), Trebananib, Pazopanib, Cediranib, Palbociclib, everolimus, fluoropyrimidine (e.g., FOLFOX, FOLFIRI), IFL, regorafenib, Reolysin, Alimta, Zykadia, Sutent, Torisel (temsirolimus), Inlyta (axitinib, Pfizer), Afinitor (everolimus, Novartis), Nexavar (sorafenib, Onyx / Bayer), Votrient, Pazopanib, axitinib, IMA-901, AGS-003, cabozantinib, Vinflunine, Hsp90 inhibitor (e.g., apatorsin), Ad-GM-CSF (CT-0070), Temazolomide, IL-2, IFNa, vinblastine, Thalomid, dacarbazine, cyclophosphamide, lenalidomide, azacytidine, lenalidomide, bortezomid (VELCADE), amrubicine, carfilzomib, pralatrexate, and/or enzastaurin.

In some aspects, an anti-human OX40 agonist antibody may be administered in conjunction with a PD-1 axis binding antagonist. A PD-1 axis binding antagonist includes but is not limited to a PD-1 binding antagonist, a PD-L1 binding antagonist and a PD-L2 binding antagonist. Alternative names for "PD-1" include CD279 and SLEB2. Alternative names for "PD-L1" include B7-H1, B7-4, CD274, and B7-H. Alternative names for "PD-L2" include B7-DC, Btdc, and CD273. In some aspects, PD-1, PD-L1 , and PD-L2 are human PD-1 , PD-L1 and PD-L2. In some aspects, the PD-1 binding antagonist is a molecule that inhibits the binding of PD-1 to its ligand binding partners. In a specific aspect the PD-1 ligand binding partners are PD-L1 and/or PD-L2. In another embodiment, a PD-L1 binding antagonist is a molecule that inhibits the binding of PD-L1 to its binding partners. In a specific aspect, PD-L1 binding partners are PD-1 and/or B7- 1. In another embodiment, the PD-L2 binding antagonist is a molecule that inhibits the binding of PD-L2 to its binding partners. In a specific aspect, a PD-L2 binding partner is PD-1. The antagonist may be an antibody, an antigen binding fragment thereof, an immunoadhesin, a fusion protein, or oligopeptide. In some aspects, the PD-1 binding antagonist is an anti-PD-1 antibody (e.g., a human antibody, a humanized antibody, or a chimeric antibody). In some aspects, the anti-PD-1 antibody is selected from the group consisting of MDX-1106 (nivolumab, OPDIVO), Merck 3475 (MK-3475, pembrolizumab, KEYTRUDA) and CT-011 (Pidilizumab). In some aspects, the PD-1 binding antagonist is an immunoadhesin (e.g., an immunoadhesin comprising an extracellular or PD-1 binding portion of PD-L1 or PD-L2 fused to a constant region (e.g., an Fc region of an immunoglobulin sequence). In some aspects, the PD-1 binding antagonist is AMP-224. In some aspects, the PD-L1 binding antagonist is anti-PD-Ll antibody. In some aspects, the anti-PD-Ll binding antagonist is selected from the group consisting of YW243.55.S70, MPDL3280A, MEDI4736 and MDX-1105. MDX-1105, also known as BMS-936559, is an anti-PD-Ll antibody described in WO2007/005874. Antibody YW243.55.S70 (heavy and light chain variable region sequences shown in SEQ ID Nos. 20 and 21, respectively) is an anti-PD-L1 described in WO 2010/077634 A1. MDX-1106, also known as MDX-1106-04, ONO-4538, BMS-936558 or nivolumab, is an anti-PD-1 antibody described in WO2006/121168. Merck 3475, also known as MK-3475, SCH-900475 or pembrolizumab, is an anti-PD-1 antibody described in WO2009/114335. CT-011, also known as hBAT, hBAT-1 or pidilizumab, is an anti- PD-1 antibody described in WO2009/101611. AMP-224, also known as B7-DCIg, is a PD-L2- Fc fusion soluble receptor described in WO2010/027827 and WO201 1/066342. In some aspects, the anti-PD-1 antibody is MDX- 1106. Alternative names for "MDX- 1106" include MDX-1 106-04, ONO-4538, BMS-936558 or nivolumab. In some aspects, the anti-PD-1 antibody is nivolumab (CAS Registry Number: 946414-94-4).

In some aspects, an anti-human OX40 agonist antibody may be administered in conjunction with an agonist directed against an activating co-stimulatory molecule. In some aspects, an activating co-stimulatory molecule may include CD40, CD226, CD28, GITR, CD137, CD27, HVEM, or CD127. In some aspects, the agonist directed against an activating co-stimulatory molecule is an agonist antibody that binds to CD40, CD226, CD28, OX40, GITR, CD137, CD27, HVEM, or CD127. In some aspects, an anti-human OX40 agonist antibody may be administered in conjunction with an antagonist directed against an inhibitory co-stimulatory molecule. In some aspects, an inhibitory co-stimulatory molecule may include CTLA-4 (also known as CD152), PD-1, TIM-3, BTLA, VISTA, LAG-3, B7-H3, B7-H4, IDO, TIGIT, MICA/B, or arginase. In some aspects, the antagonist directed against an inhibitory co-stimulatory molecule is an antagonist antibody that binds to CTLA-4, PD-1, TIM-3, BTLA, VISTA, LAG-3 (e.g., LAG-3-IgG fusion protein (IMP321)), B7-H3, B7-H4, IDO, TIGIT, MICA/B, or arginase.

In some aspects, an anti-human OX40 agonist antibody may be administered in conjunction with an antagonist directed against CTLA-4 (also known as CD152), *e.g.,* a blocking antibody. In some aspects, an anti-human OX40 agonist antibody may be administered in conjunction with ipilimumab (also known as MDX-010, MDX-101, or Yervoy®). In some aspects, an anti-human OX40 agonist antibody may be administered in conjunction with tremelimumab (also known as ticilimumab or CP-675,206). In some aspects, an anti-human OX40 agonist antibody may be administered in conjunction with an antagonist directed against B7-H3 (also known as CD276), *e.g*., a blocking antibody. In some aspects, an anti-human OX40 agonist antibody may be administered in conjunction with MGA271. In some aspects, an anti-human OX40 agonist antibody may be administered in conjunction with an antagonist directed against a TGF beta, *e.g*., metelimumab (also known as CAT-192), fresolimumab (also known as GC1008), or LY2157299.

In some aspects, an anti-human OX40 agonist antibody may be administered in conjunction with a treatment comprising adoptive transfer of a T cell (*e.g.,* a cytotoxic T cell or CTL) expressing a chimeric antigen receptor (CAR). In some aspects, an anti-human OX40 agonist antibody may be administered in conjunction with UCART19. In some aspects, an anti-human OX40 agonist antibody may be administered in conjunction with WT128z. In some aspects, an anti-human OX40 agonist antibody may be administered in conjunction with KTE-C19 (Kite). In some aspects, an anti-human OX40 agonist antibody may be administered in conjunction with CTL019 (Novartis). In some aspects, an anti-human OX40 agonist antibody may be administered in conjunction with a treatment comprising adoptive transfer of a T cell comprising a dominant-negative TGF beta receptor, *e.g,* a dominant-negative TGF beta type II receptor. In some aspects, an anti-human OX40 agonist antibody may be administered in conjunction with a treatment comprising a HERCREEM protocol (see, e.g., ClinicalTrials.gov Identifier NCT00889954).

In some aspects, an anti-human OX40 agonist antibody may be administered in conjunction with an antagonist directed against CD19. In some aspects, an anti-human OX40 agonist antibody may be administered in conjunction with MOR00208. In some aspects, an anti-human OX40 agonist antibody may be administered in conjunction with an antagonist directed against CD38. In some aspects, an anti-human OX40 agonist antibody may be administered in conjunction with daratumumab.

In some aspects, an anti-human OX40 agonist antibody may be administered in conjunction with an agonist directed against CD137 (also known as TNFRSF9, 4-1BB, or ILA), *e.g.,* an activating antibody. In some aspects, an anti-human OX40 agonist antibody may be administered in conjunction with urelumab (also known as BMS-663513). In some aspects, an anti-human OX40 agonist antibody may be administered in conjunction with an agonist directed against CD40, *e.g.,* an activating antibody. In some aspects, an anti-human OX40 agonist antibody may be administered in conjunction with CP-870893. In some aspects, an anti-human OX40 agonist antibody may be administered in conjunction with an agonist directed against OX40 (also known as CD134), *e.g.,* an activating antibody. In some aspects, an anti-human OX40 agonist antibody may be administered in conjunction with a different anti-OX40 antibody (e.g., AgonOX). In some aspects, an anti-human OX40 agonist antibody may be administered in conjunction with an agonist directed against CD27, *e.g.,* an activating antibody. In some aspects, an anti-human OX40 agonist antibody may be administered in conjunction with CDX-1127. In some aspects, an anti-human OX40 agonist antibody may be administered in conjunction with an antagonist directed against indoleamine-2,3-dioxygenase (IDO). In some aspects, with the IDO antagonist is 1-methyl-D-tryptophan (also known as 1-D-MT).

In some aspects, an anti-human OX40 agonist antibody may be administered in conjunction with an agonist directed against CD137 (also known as TNFRSF9, 4-1BB, or ILA), *e.g.,* an activating antibody. In some aspects, an anti-human OX40 agonist antibody may be administered in conjunction with urelumab (also known as BMS-663513). In some aspects, an anti-human OX40 agonist antibody may be administered in conjunction with an agonist directed against CD40, *e.g.,* an activating antibody. In some aspects, an anti-human OX40 agonist antibody may be administered in conjunction with CP-870893 or RO7009789. In some aspects, an anti-human OX40 agonist antibody may be administered in conjunction with an agonist directed against OX40 (also known as CD134), *e.g.,* an activating antibody.). In some aspects, an anti-human OX40 agonist antibody may be administered in conjunction with an agonist directed against CD27, *e.g.,* an activating antibody. In some aspects, an anti-human OX40 agonist antibody may be administered in conjunction with CDX-1127 (also known as varlilumab). In some aspects, an anti-human OX40 agonist antibody may be administered in conjunction with an antagonist directed against indoleamine-2,3-dioxygenase (IDO). In some aspects, with the IDO antagonist is 1-methyl-D-tryptophan (also known as 1-D-MT). In some aspects, the IDO antagonist is an IDO antagonist shown in WO2010/005958 (the contents of which are expressly incorporated by record herein). In some aspects the IDO antagonist is 4-({2-[(Aminosulfonyl)amino]ethyl}amino)-*N*-(3-bromo-4-fluorophenyl)-*N'*-hydroxy-1,2,5-oxadiazole-3-carboximidamide (e.g., as described in Example 23 of WO2010/005958). In some aspects the IDO antagonist is 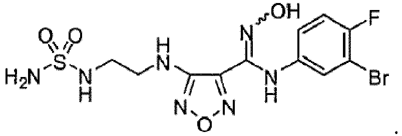 In some aspects, the IDO antagonist is INCB24360. In some aspects, the IDO antagonist is Indoximod (the D isomer of 1-methyl-tryptophan). In some aspects, an anti-human OX40 agonist antibody may be administered in conjunction with an antibody-drug conjugate. In some aspects, the antibody-drug conjugate comprises mertansine or monomethyl auristatin E (MMAE). In some aspects, an anti-human OX40 agonist antibody may be administered in conjunction with an anti-NaPi2b antibody-MMAE conjugate (also known as DNIB0600A, RG7599 or lifastuzumab vedotin). In some aspects, an anti-human OX40 agonist antibody may be administered in conjunction with trastuzumab emtansine (also known as T-DM1, ado-trastuzumab emtansine, or KADCYLA®, Genentech). In some aspects, an anti-human OX40 agonist antibody may be administered in conjunction with an anti-MUC16 antibody-MMAE conjugate, DMUC5754A. In some aspects, an anti-human OX40 agonist antibody may be administered in conjunction with an anti-MUC16 antibody-MMAE conjugate, DMUC4064A. In some aspects, an anti-human OX40 agonist antibody may be administered in conjunction with an antibody-drug conjugate targeting the endothelin B receptor (EDNBR), e.g., an antibody directed against EDNBR conjugated with MMAE. In some aspects, an anti-human OX40 agonist antibody may be administered in conjunction with an antibody-drug conjugate targeting the lymphocyte antigen 6 complex, locus E (Ly6E), e.g., an antibody directed against Ly6E conjugated with MMAE, (also known as DLYE5953A). In some aspects, an anti-human OX40 agonist antibody may be administered in conjunction with polatuzumab vedotin. In some aspects, an anti-human OX40 agonist antibody may be administered in conjunction with an antibody-drug conjugate targeting CD30. In some aspects, an anti-human OX40 agonist antibody may be administered in conjunction with ADCETRIS (also known as brentuximab vedotin). In some aspects, an anti-human OX40 agonist antibody may be administered in conjunction with polatuzumab vedotin.

In some aspects, an anti-human OX40 agonist antibody may be administered in conjunction with an angiogenesis inhibitor. In some aspects, an anti-human OX40 agonist antibody may be administered in conjunction with an antibody directed against a VEGF, *e.g*., VEGF-A. In some aspects, an anti-human OX40 agonist antibody may be administered in conjunction with bevacizumab (also known as AVASTIN®, Genentech). In some aspects, an anti-human OX40 agonist antibody may be administered in conjunction with an antibody directed against angiopoietin 2 (also known as Ang2). In some aspects, an anti-human OX40 agonist antibody may be administered in conjunction with MEDI3617. In some aspects, an anti-human OX40 agonist antibody may be administered in conjunction with an antibody directed against VEGFR2. In some aspects, an anti-human OX40 agonist antibody may be administered in conjunction with ramucirumab. In some aspects, an anti-human OX40 agonist antibody may be administered in conjunction with a VEGF Receptor fusion protein. In some aspects, an anti-human OX40 agonist antibody may be administered in conjunction with aflibercept. In some aspects, an anti-human OX40 agonist antibody may be administered in conjunction with ziv-aflibercept (also known as VEGF Trap or Zaltrap®). In some aspects, an anti-human OX40 agonist antibody may be administered in conjunction with a bispecific antibody directed against VEGF and Ang2. In some aspects, an anti-human OX40 agonist antibody may be administered in conjunction with RG7221 (also known as vanucizumab). In some aspects, an anti-human OX40 agonist antibody may be administered in conjunction with an angiogenesis inhibitor and in conjunction with a PD-1 axis binding antagonist (e.g., a PD-1 binding antagonist such as an anti-PD-1 antibody, a PD-L1 binding antagonist such as an anti-PD-Ll antibody, and a PD-L2 binding antagonist such as an anti-PD-L2 antibody). In some aspects, an anti-human OX40 agonist antibody may be administered in conjunction with bevacizumab and a PD-1 axis binding antagonist (e.g., a PD-1 binding antagonist such as an anti-PD-1 antibody, a PD-L1 binding antagonist such as an anti-PD-Ll antibody, and a PD-L2 binding antagonist such as an anti-PD-L2 antibody). In some aspects, an anti-human OX40 agonist antibody may be administered in conjunction with bevacizumab and MDX-1106 (nivolumab, OPDIVO). In some aspects, an anti-human OX40 agonist antibody may be administered in conjunction with bevacizumab and Merck 3475 (MK-3475, pembrolizumab, KEYTRUDA). In some aspects, an anti-human OX40 agonist antibody may be administered in conjunction with bevacizumab and CT- 011 (Pidilizumab). In some aspects, an anti-human OX40 agonist antibody may be administered in conjunction with bevacizumab and YW243.55.S70. In some aspects, an anti-human OX40 agonist antibody may be administered in conjunction with bevacizumab and MPDL3280A. In some aspects, an anti-human OX40 agonist antibody may be administered in conjunction with bevacizumab and MEDI4736. In some aspects, an anti-human OX40 agonist antibody may be administered in conjunction with bevacizumab and MDX-1105.

In some aspects, an anti-human OX40 agonist antibody may be administered in conjunction with an antineoplastic agent. In some aspects, an anti-human OX40 agonist antibody may be administered in conjunction with an agent targeting CSF-1R (also known as M-CSFR or CD115). In some aspects, an anti-human OX40 agonist antibody may be administered in conjunction with anti-CSF-1R antibody (also known as IMC-CS4 or LY3022855) In some aspects, an anti-human OX40 agonist antibody may be administered in conjunction with anti-CSF-1R antibody, RG7155 (also known as RO5509554 or emactuzumab). In some aspects, an anti-human OX40 agonist antibody may be administered in conjunction with an interferon, for example interferon alpha or interferon gamma. In some aspects, an anti-human OX40 agonist antibody may be administered in conjunction with Roferon-A (also known as recombinant Interferon alpha-2a). In some aspects, an anti-human OX40 agonist antibody may be administered in conjunction with GM-CSF (also known as recombinant human granulocyte macrophage colony stimulating factor, rhu GM-CSF, sargramostim, or Leukine®). In some aspects, an anti-human OX40 agonist antibody may be administered in conjunction with IL-2 (also known as aldesleukin or Proleukin®). In some aspects, an anti-human OX40 agonist antibody may be administered in conjunction with IL-12. In some aspects, an anti-human OX40 agonist antibody may be administered in conjunction with IL27. In some aspects, an anti-human OX40 agonist antibody may be administered in conjunction with IL-15. In some aspects, an anti-human OX40 agonist antibody may be administered in conjunction with ALT-803. In some aspects, an anti-human OX40 agonist antibody may be administered in conjunction with an antibody targeting CD20. In some aspects, the antibody targeting CD20 is obinutuzumab (also known as GA101 or Gazyva®) or rituximab. In some aspects, an anti-human OX40 agonist antibody may be administered in conjunction with an antibody targeting GITR. In some aspects, the antibody targeting GITR is TRX518. In some aspects, the antibody targeting GITR is MK04166 (Merck).

In some aspects, an anti-human OX40 agonist antibody may be administered in conjunction with an inhibitor of Bruton's tyrosine kinase (BTK). In some aspects, an anti-human OX40 agonist antibody may be administered in conjunction with ibrutinib. In some aspects, an anti-human OX40 agonist antibody may be administered in conjunction with an inhibitor of Isocitrate dehydrogenase 1 (IDH1) and/or Isocitrate dehydrogenase 2 (IDH2). In some aspects, an anti-human OX40 agonist antibody may be administered in conjunction with AG-120 (Agios).

In some aspects, an anti-human OX40 agonist antibody may be administered in conjunction with obinutuzumab and a PD-1 axis binding antagonist (e.g., a PD-1 binding antagonist such as an anti-PD-1 antibody, a PD-L1 binding antagonist such as an anti-PD-Ll antibody, and a PD-L2 binding antagonist such as an anti-PD-L2 antibody).

In some aspects, an anti-human OX40 agonist antibody may be administered in conjunction with a cancer vaccine. In some aspects, the cancer vaccine is a peptide cancer vaccine, which in some aspects is a personalized peptide vaccine. In some aspects the peptide cancer vaccine is a multivalent long peptide, a multi-peptide, a peptide cocktail, a hybrid peptide, or a peptide-pulsed dendritic cell vaccine (see, e.g., Yamada et al., Cancer Sci, 104:14-21, 2013). In some aspects, an anti-human OX40 agonist antibody may be administered in conjunction with an adjuvant. In some aspects, an anti-human OX40 agonist antibody may be administered in conjunction with a treatment comprising a TLR agonist, *e.g*., Poly-ICLC (also known as Hiltonol®), LPS, MPL, or CpG ODN. In some aspects, an anti-human OX40 agonist antibody may be administered in conjunction with tumor necrosis factor (TNF) alpha. In some aspects, an anti-human OX40 agonist antibody may be administered in conjunction with IL-1. In some aspects, an anti-human OX40 agonist antibody may be administered in conjunction with HMGB1. In some aspects, an anti-human OX40 agonist antibody may be administered in conjunction with an IL-10 antagonist. In some aspects, an anti-human OX40 agonist antibody may be administered in conjunction with an IL-4 antagonist. In some aspects, an anti-human OX40 agonist antibody may be administered in conjunction with an IL-13 antagonist. In some aspects, an anti-human OX40 agonist antibody may be administered in conjunction with an IL-17 antagonist. In some aspects, an anti-human OX40 agonist antibody may be administered in conjunction with an HVEM antagonist. In some aspects, an anti-human OX40 agonist antibody may be administered in conjunction with an ICOS agonist, *e.g*., by administration of ICOS-L, or an agonistic antibody directed against ICOS. In some aspects, an anti-human OX40 agonist antibody may be administered in conjunction with a treatment targeting CX3CL1. In some aspects, an anti-human OX40 agonist antibody may be administered in conjunction with a treatment targeting CXCL9. In some aspects, an anti-human OX40 agonist antibody may be administered in conjunction with a treatment targeting CXCL10. In some aspects, an anti-human OX40 agonist antibody may be administered in conjunction with a treatment targeting CCL5. In some aspects, an anti-human OX40 agonist antibody may be administered in conjunction with an LFA-1 or ICAM1 agonist. In some aspects, an anti-human OX40 agonist antibody may be administered in conjunction with a Selectin agonist.

In some aspects, an anti-human OX40 agonist antibody may be administered in conjunction with an inhibitor of B-Raf. In some aspects, an anti-human OX40 agonist antibody may be administered in conjunction with vemurafenib (also known as Zelboraf®). In some aspects, an anti-human OX40 agonist antibody may be administered in conjunction with dabrafenib (also known as Tafinlar®). In some aspects, an anti-human OX40 agonist antibody may be administered in conjunction with encorafenib (LGX818).

In some aspects, an anti-human OX40 agonist antibody may be administered in conjunction with an EGFR inhibitor. In some aspects, an anti-human OX40 agonist antibody may be administered in conjunction with erlotinib (also known as Tarceva®). In some aspects, an anti-human OX40 agonist antibody may be administered in conjunction with an inhibitor of EGFR-T790M. In some aspects, an anti-human OX40 agonist antibody may be administered in conjunction with gefitinib. In some aspects, an anti-human OX40 agonist antibody may be administered in conjunction with afatinib. In some aspects, an anti-human OX40 agonist antibody may be administered in conjunction with cetuximab (also known as Erbitux®). In some aspects, an anti-human OX40 agonist antibody may be administered in conjunction with panitumumab (also known as Vectibix®). In some aspects, an anti-human OX40 agonist antibody may be administered in conjunction with rociletinib. In some aspects, an anti-human OX40 agonist antibody may be administered in conjunction with AZD9291. In some aspects, an anti-human OX40 agonist antibody may be administered in conjunction with an inhibitor of a MEK, such as MEK1 (also known as MAP2K1) and/or MEK2 (also known as MAP2K2). In some aspects, an anti-human OX40 agonist antibody may be administered in conjunction with cobimetinib (also known as GDC-0973 or XL-518). In some aspects, an anti-human OX40 agonist antibody may be administered in conjunction with trametinib (also known as Mekinist®). In some aspects, an anti-human OX40 agonist antibody may be administered in conjunction with binimetinib.

In some aspects, an anti-human OX40 agonist antibody may be administered in conjunction an inhibitor of B-Raf (e.g., vemurafenib or dabrafenib) and an inhibitor of MEK (e.g., MEK1 and/or MEK2 (e.g., cobimetinib or trametinib). In some aspects, an anti-human OX40 agonist antibody may be administered in conjunction with an inhibitor of ERK (e.g., ERK1/2). In some aspects, an anti-human OX40 agonist antibody may be administered in conjunction with GDC-0994). In some aspects, an anti-human OX40 agonist antibody may be administered in conjunction with an inhibitor of B-Raf, an inhibitor of MEK, and an inhibitor of ERK1/2. In some aspects, an anti-human OX40 agonist antibody may be administered in conjunction with an inhibitor of EGFR, an inhibitor of MEK, and an inhibitor of ERK1/2. In some aspects, an anti-human OX40 agonist antibody may be administered in conjunction with one or more MAP kinase pathway inhibitor. In some aspects, an anti-human OX40 agonist antibody may be administered in conjunction with CK127. In some aspects, an anti-human OX40 agonist antibody may be administered in conjunction with an inhibitor of K-Ras.

In some aspects, an anti-human OX40 agonist antibody may be administered in conjunction with an inhibitor of c-Met. In some aspects, an anti-human OX40 agonist antibody may be administered in conjunction with onartuzumab (also known as MetMAb). In some aspects, an anti-human OX40 agonist antibody may be administered in conjunction with an inhibitor of anaplatic lymphoma kinase (ALK). In some aspects, an anti-human OX40 agonist antibody may be administered in conjunction with AF802 (also known as CH5424802 or alectinib). In some aspects, an anti-human OX40 agonist antibody may be administered in conjunction with crizotinib. In some aspects, an anti-human OX40 agonist antibody may be administered in conjunction with ceritinib. In some aspects, an anti-human OX40 agonist antibody may be administered in conjunction with an inhibitor of a phosphatidylinositol 3-kinase (PI3K). In some aspects, an anti-human OX40 agonist antibody may be administered in conjuction with buparlisib (BKM-120). In some aspects, an anti-human OX40 agonist antibody may be administered in conjunction with pictilisib (also known as GDC-0941). In some aspects, an anti-human OX40 agonist antibody may be administered in conjunction with buparlisib (also known as BKM-120). In some aspects, an anti-human OX40 agonist antibody may be administered in conjunction with perifosine (also known as KRX-0401). In some aspects, an anti-human OX40 agonist antibody may be administered in conjunction with a delta-selective inhibitor of a phosphatidylinositol 3-kinase (PI3K). In some aspects, an anti-human OX40 agonist antibody may be administered in conjunction with idelalisib (also known as GS-1101 or CAL-101). In some aspects, an anti-human OX40 agonist antibody may be administered in conjunction with taselisib (also known as GDC-0032). In some aspects, an anti-human OX40 agonist antibody may be administered in conjunction with BYL-719. In some aspects, an anti-human OX40 agonist antibody may be administered in conjunction with an inhibitor of an Akt. In some aspects, an anti-human OX40 agonist antibody may be administered in conjunction with MK2206. In some aspects, an anti-human OX40 agonist antibody may be administered in conjunction with GSK690693. In some aspects, an anti-human OX40 agonist antibody may be administered in conjunction with ipatasertib (also known as GDC-0068). In some aspects, an anti-human OX40 agonist antibody may be administered in conjunction with an inhibitor of mTOR. In some aspects, an anti-human OX40 agonist antibody may be administered in conjunction with sirolimus (also known as rapamycin). In some aspects, an anti-human OX40 agonist antibody may be administered in conjunction with temsirolimus (also known as CCI-779 or Torisel®). In some aspects, an anti-human OX40 agonist antibody may be administered in conjunction with everolimus (also known as RAD001). In some aspects, an anti-human OX40 agonist antibody may be administered in conjunction with ridaforolimus (also known as AP-23573, MK-8669, or deforolimus). In some aspects, an anti-human OX40 agonist antibody may be administered in conjunction with OSI-027. In some aspects, an anti-human OX40 agonist antibody may be administered in conjunction with AZD8055. In some aspects, an anti-human OX40 agonist antibody may be administered in conjunction with INK128. In some aspects, an anti-human OX40 agonist antibody may be administered in conjunction with a dual PI3K/mTOR inhibitor. In some aspects, an anti-human OX40 agonist antibody may be administered in conjunction with XL765. In some aspects, an anti-human OX40 agonist antibody may be administered in conjunction with GDC-0980. In some aspects, an anti-human OX40 agonist antibody may be administered in conjunction with BEZ235 (also known as NVP-BEZ235). In some aspects, an anti-human OX40 agonist antibody may be administered in conjunction with BGT226. In some aspects, an anti-human OX40 agonist antibody may be administered in conjunction with GSK2126458. In some aspects, an anti-human OX40 agonist antibody may be administered in conjunction with PF-04691502. In some aspects, an anti-human OX40 agonist antibody may be administered in conjunction with PF-05212384 (also known as PKI-587).

In some aspects, an anti-human OX40 agonist antibody may be administered in conjunction with an agent that selectively degrades the estrogen receptor. In some aspects, an anti-human OX40 agonist antibody may be administered in conjunction with GDC-0927. In some aspects, an anti-human OX40 agonist antibody may be administered in conjunction with an inhibitor of HER3. In some aspects, an anti-human OX40 agonist antibody may be administered in conjunction with duligotuzumab. In some aspects, an anti-human OX40 agonist antibody may be administered in conjunction with an inhibitor of LSD1. In some aspects, an anti-human OX40 agonist antibody may be administered in conjunction with an inhibitor of MDM2. In some aspects, an anti-human OX40 agonist antibody may be administered in conjunction with an inhibitor of BCL2. In some aspects, an anti-human OX40 agonist antibody may be administered in conjunction with venetoclax. In some aspects, an anti-human OX40 agonist antibody may be administered in conjunction with an inhibitor of CHK1. In some aspects, an anti-human OX40 agonist antibody may be administered in conjunction with GDC-0575. In some aspects, an anti-human OX40 agonist antibody may be administered in conjunction with an inhibitor of activated hedgehog signaling pathway. In some aspects, an anti-human OX40 agonist antibody may be administered in conjunction with ERIVEDGE.

In some aspects, an anti-human OX40 agonist antibody may be administered in conjunction with radiation therapy. In some aspects, an anti-human OX40 agonist antibody may be administered in conjunction with gemcitabine. In some aspects, an anti-human OX40 agonist antibody may be administered in conjunction with nab-paclitaxel (ABRAXANE). In some aspects, an anti-human OX40 agonist antibody may be administered in conjunction with trastuzumab. In some aspects, an anti-human OX40 agonist antibody may be administered in conjunction with TVEC. In some aspects, an anti-human OX40 agonist antibody may be administered in conjunction with IL27. In some aspects, an anti-human OX40 agonist antibody may be administered in conjunction with cyclophosphamide. In some aspects, an anti-human OX40 agonist antibody may be administered in conjunction with an agent that recruits T cells to the tumor. In some aspects, an anti-human OX40 agonist antibody may be administered in conjunction with lirilumab (IPH2102/BMS-986015). In some aspects, an anti-human OX40 agonist antibody may be administered in conjunction with Idelalisib. In some aspects, an anti-human OX40 agonist antibody may be administered in conjunction with an antibody that targets CD3 and CD20. In some aspects, an anti-human OX40 agonist antibody may be administered in conjunction with REGN1979. In some aspects, an anti-human OX40 agonist antibody may be administered in conjunction with an antibody that targets CD3 and CD19. In some aspects, an anti-human OX40 agonist antibody may be administered in conjunction with blinatumomab.

In some aspects, an anti-human OX40 agonist antibody may be administered in conjunction with an oncolytic virus. In some aspects, an anti-human OX40 agonist antibody may be administered in conjunction with carboplatin and nab-paclitaxel. In some aspects, an anti-human OX40 agonist antibody may be administered in conjunction with carboplatin and paclitaxel. In some aspects, an anti-human OX40 agonist antibody may be administered in conjunction with cisplatin and pemetrexed. In some aspects, an anti-human OX40 agonist antibody may be administered in conjunction with cisplatin and gemcitabine. In some aspects, an anti-human OX40 agonist antibody may be administered in conjunction with FOLFOX. In some aspects, an anti-human OX40 agonist antibody may be administered in conjunction with FOLFIRI.

Such combination therapies noted above encompass combined administration (where two or more therapeutic agents are included in the same or separate formulations), and separate administration, in which case, administration of the antibody of the invention can occur prior to, simultaneously, and/or following, administration of the additional therapeutic agent and/or adjuvant. Antibodies of the invention can also be used in combination with radiation therapy.

An antibody as disclosed herein (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies as disclosed herein would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody as disclosed herein (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 µg/kg to 40 mg/kg of antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. Such doses may be administered intermittently, e.g. every week or every three weeks (*e.g*. such that the patient receives from about two to about twenty, or *e.g.* about six doses of the antibody). An initial higher loading dose, followed by one or more lower doses may be administered. However, other dosage regimens may be useful. The progress of this therapy is easily monitored by conventional techniques and assays.

It is understood that any of the above formulations or therapeutic methods may be carried out using an immunoconjugate of the invention in place of or in addition to an anti-OX40 antibody.

### III. ARTICLES OF MANUFACTURE AND KITS

In another aspect, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

In some aspects, disclosed is a kit comprising a medicament comprising an anti-human OX40 agonist antibody described herein and an optional pharmaceutically acceptable carrier. In some aspects, the kit further comprises instructions for administration of the medicament for treatment of cancer.

It is understood that any of the above articles of manufacture may include an immunoconjugate of the invention in place of or in addition to an anti-OX40 antibody.

### Sequences

| Name | SEQUENCE | SEQ ID NO: |
|---|---|---|
| Human OX40 (lacking the signal peptide) | | 1 |
| HVR-H1- | | 2 |
| 1A7.gr.1 | | |
| 1A7.gr.2 | | |
| 1A7.gr.3 | | |
| 1A7.gr.4 | | |
| 1A7.gr.5 | | |
| 1A7.gr.5' | | |
| 1A7.gr.6 | | |
| 1A7.gr.7 | | |
| 1A7.gr.7' | | |
| 1A7.gr.NADS | | |
| 1A7.gr.NADA | | |
| 1A7.gr.NGDA | | |
| 1A7.gr.SGDS | | |
| 1A7.gr.NGSS | | |
| 1A7.Ala.1 | | |
| 1A7.Ala.2 | | |
| 1A7.Ala.3 | | |
| 1A7.Ala.4 | | |
| 1A7.Ala.5 | | |
| 1A7.Ala.6 | | |
| 1A7.Ala.7 | | |
| 1A7.Ala.8 | | |
| 1A7.Ala.9 | | |
| 1A7.Ala.10 | | |
| 1A7.Ala.11 | | |
| 1A7.Ala.12 | | |
| 1A7.Ala.13 | | |
| 1A7.Ala.14 | | |
| 1A7.Ala.15 | | |
| 1A7.Ala.16 | DSYMS | |
| HVR-H2- | | 3 |
| 1A7.gr.1 | | |
| 1A7.gr.2 | | |
| 1A7.gr.3 | | |
| 1A7.gr.4 | | |
| 1A7.gr.5 | | |
| 1A7.gr.5' | | |
| 1A7.gr.6 | | |
| 1A7.gr.7 | | |
| 1A7.gr.7' | DMYPDNGDSSYNQKFRE | |
| 1A7.gr.DA | | |
| 1A7.gr.ES | | |
| 1A7.Ala.1 | | |
| 1A7.Ala.2 | | |
| 1A7.Ala.3 | | |
| 1A7.Ala.4 | | |
| 1A7.Ala.5 | | |
| 1A7.Ala.6 | | |
| 1A7.Ala.7 | | |
| 1A7.Ala.8 | | |
| 1A7.Ala.9 | | |
| 1A7.Ala.10 | | |
| 1A7.Ala.11 | | |
| 1A7.Ala.12 | | |
| 1A7.Ala.13 | | |
| 1A7.Ala.14 | | |
| 1A7.Ala.15 | | |
| 1A7.Ala.16 | | |
| HVR-H3- | | 4 |
| 1A7.gr.1 | | |
| 1A7.gr.2 | | |
| 1A7.gr.3 | | |
| 1A7.gr.4 | | |
| 1A7.gr.5 | | |
| 1A7.gr.5' | | |
| 1A7.gr.6 | | |
| 1A7.gr.7 | | |
| 1A7.gr.7' | | |
| 1A7.gr.DA | | |
| 1A7.gr.ES | | |
| 1A7.gr.NADS | | |
| 1A7.gr.NADA | | |
| 1A7.gr.NGDA | | |
| 1A7.gr.SGDS | | |
| 1A7.gr.NGSS | | |
| 1A7.gr.DANAD | | |
| A | | |
| 1A7.Ala.1 | | |
| 1A7.Ala.2 | | |
| 1A7.Ala.3 | | |
| 1A7.Ala.4 | | |
| 1A7.Ala.5 | | |
| 1A7.Ala.6 | | |
| 1A7.Ala.7 | | |
| 1A7-Ala.15 | | |
| 1A7.Ala.16 | APRWYFSV | |
| HVR-L1- | | 5 |
| 1A7.gr.1 | | |
| 1A7.gr.2 | | |
| 1A7.gr.3 | | |
| 1A7.gr.4 | | |
| 1A7.r.5 | RASQDISNYLN | |
| 1A7.gr.5' | | |
| 1A7.gr.6 | | |
| 1A7.gr.7 | | |
| 1A7.gr.7' | | |
| 1A7.gr.DA | | |
| 1A7.gr.ES | | |
| 1A7.gr.NADS | | |
| 1A7.gr.NADA | | |
| 1A7.gr.NGDA | | |
| 1A7.gr.SGDS | | |
| 1A7.gr.NGSS | | |
| 1A7.gr.DANAD | | |
| A | | |
| 1A7.Ala.1 | | |
| 1A7.Ala.2 | | |
| 1A7.Ala.3 | | |
| 1A7.Ala.4 | | |
| 1A7.Ala.5 | | |
| 1A7.Ala.6 | | |
| 1A7.Ala.7 | | |
| 1A7.Ala.8 | | |
| 1A7.Ala.9 | | |
| 1A7.Ala.10 | | |
| 1A7.Ala.11 | | |
| 1A7.Ala.12 | | |
| 1A7.Ala.13 | | |
| 1A7.Ala.14 | | |
| 1A7.Ala.15 | | |
| 1A7.Ala.16 | | |
| HVR-L2- | | 6 |
| 1A7.gr.1 | | |
| 1A7.gr.2 | | |
| 1A7.gr.3 | | |
| 1A7.gr.4 | | |
| 1A7.gr.5 | | |
| 1A7.gr.5' | | |
| 1A7.gr.6 | | |
| 1A7.gr.7 | | |
| 1A7.gr.7' | | |
| 1A7.gr.DA | | |
| 1A7.gr.ES | | |
| 1A7.gr.NADS | | |
| 1A7.gr.NADA | | |
| 1A7.gr.NGDA | | |
| 1A7.gr.SGDS | | |
| 1A7.gr.NGSS | | |
| 1A7.gr.DANAD | | |
| A | | |
| 1A7.Ala.1 | | |
| 1A7.Ala.2 | | |
| 1A7.Ala.3 | | |
| 1A7.Ala.4 | YTSRLRS | |
| 1A7.Ala.5 | | |
| 1A7.Ala.6 | | |
| 1A7.Ala.7 | | |
| 1A7.Ala.8 | | |
| 1A7.Ala.9 | | |
| 1A7.Ala.10 | | |
| 1A7.Ala.11 | | |
| 1A7.Ala.12 | | |
| 1A7.Ala.13 | | |
| 1A7.Ala.14 | | |
| 1A7.Ala.15 | | |
| 1A7.Ala.16 | | |
| HVR-L3- | | 7 |
| 1A7.gr.1 | | |
| 1A7.gr.2 | | |
| 1A7.gr.3 | | |
| 1A7.gr.4 | | |
| 1A7.gr.5 | | |
| 1A7.gr.5' | | |
| 1A7.gr.6 | | |
| 1A7.gr.7 | | |
| 1A7.gr.7' | | |
| 1A7.gr.DA | | |
| 1A7.gr.ES | | |
| 1A7.gr.NADS | | |
| 1A7.gr.NADA | | |
| 1A7.gr.NGDA | | |
| 1A7.gr.SGDS | | |
| 1A7.gr.NGSS | | |
| 1A7.gr.DANAD | | |
| A | | |
| 1A7.Ala.8 | | |
| 1A7.Ala.9 | | |
| 1A7.Ala.10 | | |
| 1A7.Ala.11 | | |
| 1A7.Ala.12 | | |
| 1A7.Ala.13 | | |
| 1A7.Ala.14 | | |
| 1A7.Ala.15 | | |
| 1A7.Ala.16 | QQGHTLPPT | |
| HVR-H1- 1A7.gr.DA | DAYMS | 8 |
| HVR-H1- | | 9 |
| 1A7.gr.ES | | |
| 1A7.gr.DANAD | | |
| A | ESYMS | |
| HVR-H2- | | 10 |
| 1A7.gr.NADS | | |
| | DMYPDNADSSYNQKFRE | |
| HVR-H2- | | 11 |
| 1A7.gr.NADA | | |
| 1A7.gr.DANADA | DMYPDNADASYNQKFRE | |
| HVR-H2-1A7.gr.NGDA | DMYPDNGDASYNQKFRE | 12 |
| HVR-H2-1A7.gr.SGDS | DMYPDSGDSSYNQKFRE | 13 |
| HVR-H2-1A7.gr.NGSS | DMYPDNGSSSYNQKFRE | 14 |
| HVR-H3-1A7.Ala.8 | APRWYFSA | 15 |
| HVR-H3-1A7.Ala.9 | APRWYASV | 16 |
| HVR-H3-1A7.Ala.10 | APRWAFSV | 17 |
| HVR-H3-1A7.Ala.11 | APAWYFSV | 18 |
| HVR-H3-1A7.Ala.12 | APRWYFAV | 19 |
| HVR-H3-1A7.Ala.13 | APRAYFSV | 20 |
| HVR-H3-1A7.Ala.14 | AARWYFSV | 21 |
| HVR-L3-1A7.Ala.1 | QQGHTLPAT | 22 |
| HVR-L3-1A7.Ala.2 | QQGHTAPPT | 23 |
| HVR-L3-1A7.Ala.3 | QQGATLPPT | 24 |
| HVR-L3-1A7.Ala.4 | QQGHALPPT | 25 |
| HVR-L3-1A7.Ala.5 | QQAHTLPPT | 26 |
| HVR-L3-1A7.Ala.6 | QQGHTLAPT | 27 |
| HVR-L3-1A7.Ala.7 | QAGHTLPPT | 28 |
| HVR-H1- | | 29 |
| 3C8.gr.1 | | |
| 3C8.gr.2 | | |
| 3C8.gr.3 | | |
| 3C8.gr.4 | | |
| 3C8.gr.5 | | |
| 3C8.gr.5.SG | | |
| 3C8.gr.5.EG | | |
| 3C8.gr.5.QG | | |
| 3C9.gr.5.DQ | | |
| 3C8.gr.5.DA | | |
| 3C8.gr.6 | | |
| 3C8.gr.7 | | |
| 3C8.gr.8 | | |
| 3C8.gr.9 | | |
| 3C8.gr.10 | | |
| 3C8.gr.11 | | |
| 3C8.A.1 | | |
| 3C8.A.2 | | |
| 3C8.A.3 | | |
| 3C8.A.4 | | |
| 3C8.A.5 | | |
| 3C8.A.6 | | |
| 3C8.A.7 | | |
| 3C8.A.8 | | |
| 3C8.A.9 | | |
| 3C8.A.10 | | |
| | NYLIE | |
| HVR-H2- | | 30 |
| 3C8.gr.1 | | |
| 3C8.gr.2 | | |
| 3C8.gr.3 | | |
| 3C8.gr.4 | | |
| 3C8.gr.5 | | |
| 3C8.gr.5.SG | | |
| 3C8.gr.5.EG | | |
| 3C8.gr.5.QG | | |
| 3C8.gr.6 | | |
| 3C8.gr.7 | | |
| 3C8.gr.8 | | |
| 3C8.gr.9 | | |
| 3C8.gr.10 | | |
| 3C8.gr.11 | | |
| 3C8.A.1 | | |
| 3C8.A.2 | | |
| 3C8.A.3 | | |
| 3C8.A.4 | | |
| 3C8.A.5 | | |
| 3C8.A.6 | | |
| 3C8.A.7 | VINPGSGDTYYSEKFKG | |
| 3C8.A.8 | | |
| 3C8.A.9 | | |
| 3C8.A.10 | | |
| HVR-H2-3C8.gr.5.DA | VINPGSGDAYYSEKFKG | 31 |
| HVR-H2-3C8.gr.5.DQ | VINPGSGDQYYSEKFKG | 32 |
| HVR-H3- | | 33 |
| 3C8.gr.1 | | |
| 3C8.gr.2 | | |
| 3C8.gr.3 | | |
| 3C8.gr.4 | | |
| 3C8.gr.5 | | |
| 3C8.gr.5.SG | | |
| 3C8.gr.5.EG | | |
| 3C8.gr.5.QG | | |
| 3C8.gr.5.DA | | |
| 3C8.gr.5.DQ | | |
| 3C8.gr.6 | | |
| 3C8.gr.7 | | |
| 3C8.gr.8 | | |
| 3C8.gr.9 | | |
| 3C8.gr.10 | | |
| 3C8.gr.11 | | |
| 3C8.A.1 | | |
| 3C8.A.2 | | |
| 3C8.A.3 | | |
| 3C8.A.4 | | |
| 3C8.A.5 | | |
| 3C8.A.6 | | |
| 3C8.A.7 | | |
| | DRLDY | |
| HVR-H3-3C8.A.8 | ARLDY | 34 |
| HVR-H3-3C8.A.9 | DALDY | 35 |
| HVR-H3-3C8.A.10 | DRADY | 36 |
| HVR-L1- | | 37 |
| 3C8.gr.1 | | |
| 3C8.gr.2 | | |
| 3C8.gr.3 | | |
| 3C8.gr.4 | | |
| 3C8.gr.5 | | |
| 3C8.gr.5.SG | | |
| 3C8.gr.5.EG | | |
| 3C8.gr.5.QG | | |
| 3C8.gr.5.DA | | |
| 3C8.gr.5.DQ | | |
| 3C8.gr.6 | HASQDISSYIV | |
| 3C8.gr.7 | | |
| 3C8.gr.8 | | |
| 3C8.gr.9 | | |
| 3C8.gr.10 | | |
| 3C8.gr.11 | | |
| 3C8.A.1 | | |
| 3C8.A.2 | | |
| 3C8.A.3 | | |
| 3C8.A.4 | | |
| 3C8.A.5 | | |
| 3C8.A.6 | | |
| 3C8.A.7 | | |
| 3C8.A.8 | | |
| 3C8.A.9 | | |
| 3C8.A.10 | | |
| HVR-L2- | | 38 |
| 3C8.gr.1 | | |
| 3C8.gr.2 | | |
| 3C8.gr.3 | | |
| 3C8.gr.4 | | |
| 3C8.gr.5 | | |
| 3C8.gr.5.DA | | |
| 3C8.gr.5.DQ | | |
| 3C8.gr.6 | | |
| 3C8.gr.7 | | |
| 3C8.gr.8 | | |
| 3C8.gr.9 | | |
| 3C8.gr.10 | | |
| 3C8.gr.11 | | |
| 3C8.A.1 | | |
| 3C8.A.2 | | |
| 3C8.A.3 | | |
| 3C8.A.4 | | |
| 3C8.A.5 | | |
| 3C8.A.6 | | |
| 3C8.A.7 | | |
| 3C8.A.8 | | |
| 3C8.A.9 | | |
| 3C8.A.10 | HGTNLED | |
| HVR-L2-3C8.gr5.SG | HGTNLES | 39 |
| HVR-L2-3C8.gr.5.EG | HGTNLEE | 40 |
| HVR-L2-3C8.gr.5.QG | HGTNLEQ | 41 |
| HVR-L3 3C8.gr.1 3C8.r.2 | VHYAQFPYT | 42 |
| 3C8.gr.3 | | |
| 3C8.gr.4 | | |
| 3C8.gr.5 | | |
| 3C8.gr.5.SG | | |
| 3C8.gr.5.EG | | |
| 3C8.gr.5.QG | | |
| 3C8.gr.5.DA | | |
| 3C8.gr.5.DQ | | |
| 3C8.gr.6 | | |
| 3C8.gr.7 | | |
| 3C8.gr.8 | | |
| 3C8.gr.9 | | |
| 3C8.gr.10 | | |
| 3C8.gr.11 | | |
| 3C8.A.8 | | |
| 3C8.A.9 | | |
| 3C8.A.10 | | |
| HVR-L3-3C8.A.1 | AHYAQFPYT | 43 |
| HVR-L3-3C8.A.2 | VAYAQFPYT | 44 |
| HVR-L3-3C8.A.3 | VHAAQFPYT | 45 |
| HVR-L3-3C8.A.4 | VHY AAFPYT | 46 |
| HVR-L3-3C8.A.5 | VHYAQAPYT | 47 |
| HVR-L3-3C8.A.6 | VHYAQFAYT | 48 |
| HVR-L3-3C8.A.7 | VHYAQFPAT | 49 |
| HVR-H1- | | 50 |
| 1D2.gr.1 | | |
| 1D2.gr.2 | | |
| 1D2.gr.3 | DYGVL | |
| HVR-H2- | | 51 |
| 1D2.gr.1 | | |
| 1D2.gr.2 | | |
| 1D2.gr.3 | MIWSGGTTDYNAAFIS | |
| HVR-H3- | | 52 |
| 1D2.gr.1 | | |
| 1D2.gr.2 | | |
| 1D2.gr.3 | EEMDY | |
| HVR-L1-1D2.gr. 1 | RASQDISNFLN | 53 |
| 1D2.gr.2 | | |
| 1D2.gr.3 | | |
| HVR-L2- | | 54 |
| 1D2.gr.1 | | |
| 1D2.gr.2 | | |
| 1D2.gr.3 | YTSRLHS | |
| HVR-L3- | | 55 |
| 1D2.gr.1 | | |
| 1D2.gr.2 | | |
| 1D2.gr.3 | QQGNTLPWT | |
| 1A7.gr.1 V_{H} | | 56 |
| 1A7.gr.1 V_{L} | | 57 |
| 1A7.gr.2 V_{H} | | 58 |
| 1A7.gr.2 V_{L} | | 59 |
| 1A7.gr.3 V_{H} | | 60 |
| 1A7.gr.3 V_{L} | | 61 |
| 1A7.gr.4 V_{H} | | 62 |
| 1A7.gr.4 V_{L} | | 63 |
| 1A7.gr.5 V_{H} | | 64 |
| 1A7.gr.5 V_{L} | | 65 |
| 1A7.gr.6 V_{H} | | 66 |
| 1A7.gr.6 V_{L} | | 67 |
| 1A7.gr.7 V_{H} | | 68 |
| 1A7.gr.7 V_{L} | | 69 |
| 1A7.gr.DA V_{H} | | 70 |
| | | |
| 1A7.gr.DA V_{L} | | 71 |
| 1A7.gr.ES V_{H} | | 72 |
| 1A7.gr.ES V_{L} | | 73 |
| 1A7.gr.NADS V_{H} | | 74 |
| 1A7.gr.NADS V_{L} | | 75 |
| 1A7.gr.NADA V_{H} | | 76 |
| 1A7.gr.NADA V_{L} | | 77 |
| 1A7.gr.NGDA V_{H} | | 78 |
| 1A7.gr.NGDA V_{L} | | 79 |
| 1A7.gr.SGDS V_{H} | | 80 |
| 1A7.gr.SGDS V_{L} | | 81 |
| 1A7.gr.NGSS V_{H} | | 82 |
| 1A7.gr.NGSS V_{L} | | 83 |
| 1A7.gr.DANAD A V_{H} | | 84 |
| 1A7.gr.DANAD A V_{L} | | 85 |
| 1A7.Ala.1 V_{H} | | 86 |
| 1A7.Ala.1 V_{L} | | 87 |
| 1A7.Ala.2 V_{H} | | 88 |
| 1A7.Ala.2 V_{L} | | 89 |
| 1A7.Ala.3 V_{H} | | 90 |
| 1A7.Ala.3 V_{L} | | 91 |
| 1A7.Ala.4 V_{H} | | 92 |
| 1A7.Ala.4 V_{L} | | 93 |
| 1A7.Ala.5 V_{H} | | 94 |
| 1A7.Ala.5 V_{L} | | 95 |
| 1A7.Ala.6 V_{H} | | 96 |
| 1A7.Ala.6 V_{L} | | 97 |
| 1A7.Ala.7 V_{H} | | 98 |
| 1A7.Ala.7 V_{L} | | 99 |
| 1A7.Ala.8 V_{H} | | 100 |
| 1A7.Ala.8 V_{L} | | 101 |
| 1A7.Ala.9 V_{H} | | 102 |
| 1A7.Ala.9 V_{L} | | 103 |
| 1A7.Ala.10 V_{H} | | 104 |
| 1A7.Ala.10 V_{L} | | 105 |
| | | |
| 1A7.Ala.11 V_{H} | | 106 |
| 1A7.Ala.11 V_{L} | | 107 |
| 1A7.Ala.12 V_{H} | | 108 |
| 1A7.Ala.12 V_{L} | | 109 |
| 1A7.Ala.13 V_{H} | | 110 |
| 1A7.Ala.13 V_{L} | | 111 |
| 1A7.Ala.14 V_{H} | | 112 |
| 1A7.Ala.14 V_{L} | | 113 |
| 1A7.Ala.15 V_{H} | | 114 |
| 1A7.Ala.15 V_{L} | | 115 |
| 1A7.Ala.16 V_{H} | | 116 |
| 1A7.Ala.16 V_{L} | | 117 |
| 3C8.gr.1 V_{H} | | 118 |
| 3C8.gr.1 V_{L} | | 119 |
| 3C8.gr.2 V_{H} | | 120 |
| 3C8.gr.2 V_{L} | | 121 |
| 3C8.gr.3 V_{H} | | 122 |
| 3C8.gr.3 V_{L} | | 123 |
| 3C8.gr.4 V_{H} | | 124 |
| 3C8.gr.4 V_{L} | | 125 |
| 3C8.gr.5 V_{H} | | 126 |
| 3C8.gr.5 V_{L} | | 127 |
| 3C8.gr.5.SG V_{H} | | 128 |
| 3C8.gr.5.SG V_{L} | | 129 |
| 3C8.gr.5.EG V_{H} | | 130 |
| 3C8.gr.5.EG V_{L} | | 131 |
| 3C8.gr.5.QG V_{H} | | 132 |
| 3C8.gr.5.QG V_{L} | | 133 |
| 3C8.gr.6 V_{H} | | 134 |
| 3C8.gr.6 V_{L} | | 135 |
| 3C8.gr.7 V_{H} | | 136 |
| 3C8.gr.7 V_{L} | | 137 |
| 3C8.gr.8 V_{H} | | 138 |
| 3C8.gr.8 V_{L} | | 139 |
| 3C8.gr.9 V_{H} | | 140 |
| | | |
| 3C8.gr.9 V_{L} | | 141 |
| 3C8.gr.10 V_{H} | | 142 |
| 3C8.gr.10 V_{L} | | 143 |
| 3C8.gr.11 V_{H} | | 144 |
| 3C8.gr.11 V_{L} | | 145 |
| 3C8.A.1 V_{H} | | 146 |
| 3C8.A.1 V_{L} | | 147 |
| 3C8.A.2 V_{H} | | 148 |
| 3C8.A.2 V_{L} | | 149 |
| 3C8.A.3 V_{H} | | 150 |
| 3C8.A.3 V_{L} | | 151 |
| 3C8.A.4 V_{H} | | 152 |
| 3C8.A.4 V_{L} | | 153 |
| 3C8.A.5 V_{H} | | 154 |
| 3C8.A.5 V_{L} | | 155 |
| 3C8.A.6 V_{H} | | 156 |
| 3C8.A.6 V_{L} | | 157 |
| | | |
| 3C8.A.7 V_{H} | | 158 |
| 3C8.A.7 V_{L} | | 159 |
| 3C8.A.8 V_{H} | | 160 |
| 3C8.A.8 V_{L} | | 161 |
| 3C8.A.9 V_{H} | | 162 |
| 3C8.A.9 V_{L} | | 163 |
| 3C8.A.10 V_{H} | | 164 |
| 3C8.A.10 V_{L} | | 165 |
| 1D2.gr.1 V_{H} | | 166 |
| 1D2.gr.1 V_{L} | | 167 |
| 1D2.gr.2 V_{H} | | 168 |
| 1D2.gr.2 V_{L} | | 169 |
| 1D2.gr.3 V_{H} | | 170 |
| 1D2.gr.3 V_{L} | | 171 |
| CON1 (1A7)HVR-H1 | X₁X₂YMS, wherein X₁ is D or E, and X₂ is S or A | 172 |
| CON1 (1A7) HVR-H2 | DMYPDX₁X₂X₃X₄SYNQKFRE, wherein X₁ is N or S, X₁ is A or G, X₃ is D or S, and X₄ is A or S | 173 |
| CON1 (1A7) HVR-H3 | APRWX₁X₂X₃X₄, wherein X₁ is Y or A, X₂ is A or F, X₃ is S or A, and X₄ is A or V. | 174 |
| CON1 (1A7) HVR-L3 | QX₁X₂X₃X₄X₅X₆X₇T, wherein X₁ is A or Q, X₂ is A or G, X₃ is A or H, X₄ is A or T, X₅ is A or L, X₆ is A or P, and X₇ is A or P. | 175 |
| CON2 (3C8) HVR-H2 | VINPGSGDX₁YYSEKFKG, wherein X₁ is T, A or Q. | 176 |
| CON2 (3C8) HVR-L2 | HGTNLEX₁, wherein X₁ is S, E, or Q. | 177 |
| CON2 (3C8) HVR-L3 | X₁X₂YAQFPYX₃, wherein X₁ is V or A, X₂ is H or A, and X₃ is Y or A. | 178 |
| 1A7 V_{L} | | 179 |
| 1A7 V_{H} | | 180 |
| 3C8 V_{L} | | 181 |
| 3C8 V_{H} | | 182 |
| 1A7.gr.5' V_{H} | | 183 |
| 1A7.gr.7' V_{H} | | 184 |

### EXAMPLES

### MATERIALS AND METHODS

**Surface Plasmon resonance:** The binding kinetics of the anti-OX40 antibodies was measured using surface plasmon resonance (SPR) on a Biacore 3000 instrument (GE Healthcare). Anti-human Fc (GE Healthcare) was immobilized on a CM5 sensor chip via amine-based coupling using manufacturer provided protocol. Anti-OX40 antibody was captured at a level of 300-400 resonance units (RU). Antibody binding was measured to human OX40 (Sino Biological Inc). Two-fold concentration series of huOX40 with a range of either 18.75 to 300 nM or 6.25 to 200nM were used for the experiments. Sensorgrams for binding of OX40 were recorded using an injection time of 2 minutes with a flow rate of 30 µl/min, at a temperature of 25°C, and with a running buffer of 10mM HEPES, pH 7.4, 150 mM NaCl, and 0.005% Tween 20. After injection, disassociation of the ligand from the antibody was monitored for 600 seconds in running buffer. The surface was regenerated between binding cycles with a 40 µl injection of 3 M Magnesium Chloride. After subtraction of a blank which contained running buffer only, sensorgrams observed for OX40 binding to anti-OX40 antibodies were analyzed using a 1:1 Langmuir binding model with software supplied by the manufacturer to calculate the kinetics and binding constants.
**Cross-blocking:** Cross-blocking experiments were performed using surface plasmon resonance (SPR) on a Biacore 3000 instrument (GE Healthcare) as described above. Two formats were used for analysis. For the first, the hybridoma derived parent antibodies 1A7, 3C8 and 1D2 were captured using an Anti-murine Fc (GE Healthcare) on different flow cells. Human OX40 (Sino Biological Inc) was then flowed across the antibodies for 2 minutes allowing it to bind. This was followed by a 2.5 minute injection of anti-OX40 antibody clones 1A7.gr.1, 3C8.gr.5, and 1D2.chimera. An increase in resonance units (RUs) indicates no cross-blocking and no change in RU indicates competition. For the second format, the antibody format was reversed and the humanized variants were captured using anti-human Fc (GE Healthcare). This was followed by a 2 minute injection of huOX40 and then the murine antibodies. Each of the clones was able to bind in the presence of the other two clones and was only competed from binding by itself.
**Generation of antibody variants:** Variants of humanized anti-OX40 agonist antibody clones 1A7, 3C8 and 1D2 were generated using Kunkel mutagenesis. Variant antibodies were expressed transiently in 293 cells and then tested for binding and function.
**FACS analysis:** The human T cell line Hut78 was stably transfected with human OX40, and humanized anti-OX40 antibody clones were tested by FACS using the Hut78-OX40 cells to assess whether variants retained binding cell-surface expressed OX40. After blocking with FACS Blocking Buffer (1X PBS, 0.5% BSA, 0.05% Na Azide), cells were stained for 30 minutes on ice with either 0.1, 1.0, or 10.0 µg/ml of antibody. After staining, cells were washed 2x with cold FACS Buffer (1X PBS, 0.5% BSA, 0.05% Na Azide). Cells were then stained with Anti-huFc phycoerythrin (PE) or Anti-muFc PE for 30 minutes on ice. Stained cells were washed 3x with cold FACS Buffer before reading on a FACSCalibur, followed by analysis using Flowjo software.
**Stability testing:** anti-OX40 agonist antibodies were subjected to stability analysis, including heat stress testing and chemical stress testing. For heat testing, samples of formulated antibody were treated at 40C for two weeks, then subjected to analysis, e.g., for presence of isomerized or deaminated amino acid residues in CDR residues as determined using tryptic mapping using LC-MS/MS. For chemical testing, samples of formulated antibody were subjected to oxidizing conditions (e.g., using AAPH as an oxidizing agent), then analyzed for presence of oxidized residues (e.g., M or W) in CDRs using tryptic mapping using LC-MS/MS.

Equilibrium binding analysis of OX40 agonist antibody 1A7.gr.1 to recombinant human OX40 Protein expressed on transfected BT-474 Cells: Mab 1A7.gr.1 was iodinated using the Iodogen method (Fraker and Speck 1978). The radiolabeled mab 1A7.gr.1 was purified from free ¹²⁵I-Na by gel filtration using a NAP-5 column; the purified antibody had a specific activity of 24.3 µCi/µg. Competition reaction mixtures of 50 µL volume containing a fixed concentration of iodinated mab 1A7.gr.1 and decreasing concentrations of serially diluted, unlabeled mab 1A7.gr.1 were placed into 96-well plates. Stably transfected BT-474 cells expressing human OX40 were cultured at 37°C in 5% CO2. Cells were detached from the flask using Cell Dissociation Solution (Sigma-Aldrich; St. Louis, MO) and were washed with binding buffer (DMEM with 2% FBS, 50 mM HEPES, pH 7.2, 2 mM sodium azide). The washed cells were added at an approximate density of 5 × 104 cells in 0.2 mL of binding buffer to the 96-well plates containing the 50 µL competition reaction mixtures. The final concentration of the iodinated antibody in each competition reaction with cells was approximately 100 pM (approximately 16.0 × 104 cpm per 0.25 mL) and the final concentration of the unlabeled antibody in the competition reaction with cells varied, starting at 500 nM and decreasing in 3-fold steps for 10 concentrations, followed by a no-added unlabeled antibody step using binding buffer only. Competition reactions with cells were incubated for 2 hours at room temperature. The competition reaction with cells at each concentration of unlabeled antibody was assayed in triplicate. After 2 hours of incubation, the competition reactions were transferred to a Millipore Multiscreen® filter plate (Millipore; Billerica, MA) and washed four times with binding buffer to separate the free from bound iodinated antibody. The filters were counted on a Wallac Wizard 1470 gamma counter (PerkinElmer Life and Analytical Sciences Inc.; Wellesley, MA). The binding data were evaluated using NewLigand software (Genentech), which uses the fitting algorithm of Munson and Rodbard to determine the binding affinity of the antibody (Munson and Rodbard 1980).

Binding of OX40 agonist antibody 1A7.gr.1 to cell surface-expressed OX40 on human, cynomolgus monkey, rat and mouse T cells through flow cytometry: Peripheral white blood cells (PBMC) were obtained from human, Mauritius cynomolgus monkey, rat or mouse whole blood by lysis of red blood cells using erythrocyte lysis buffer (BD Bioscience; San Jose, CA). Cells were blocked with 10% human, cynomolgus monkey, rat or mouse serum respectively in PBS for 15 minutes at 4°C and plated in a 96 well plate at the equivalent of 100 µL of whole blood per reaction.

Mab 1A7.gr.1 labeled with Alexa Fluor® 647 (or trastuzumab antibody control labeled with Alexa Fluor® 647) was titrated and bound to cells starting at 9 µg/mL in 3-fold serial dilutions for human, rat and mouse samples, and 8 µg/mL in 2-fold serial dilutions for Mauritius cynomolgus monkey samples. PBMCs were co-stained for 30 minutes in the dark at 4°C to identify T cell subset populations as follows: Human, anti-CD3-PE-Cy7, anti-PerCDP-Cy5.5, anti-CD8-BV510 (BD Biosciences); Cynomolgus monkey, anti- CD3-PE-Cy7, anti-CD4-PerCP-Cy5.5, anti-CD8-APC-H7 (BD Biosciences); Rat, anti-CD3-PE, anti-CD4-FITC (BD Biosciences); Mouse, anti-CD3-FITC, anti-CD4-PE (eBioscience, San Diego, CA).

Cells were then washed twice with FACS Stain Buffer (BD Bioscience), and fixed with 1% paraformaldehyde (EMS) in PBS prior to sample acquisition on a flow cytometer.

All samples were run on a BD Biosciences FACSCanto II. Background staining from trastuzumab control antibody on T cells was used to set the analysis gate and normalize the mab 1A7.gr.1 -Alexa 647 staining. Acquired data were analyzed using BD FACSDiva software.

### Anti-OX40 agonist antibody 1A7.gr.1 mediated effector T cell costimulation:

Peripheral white blood cells (PBMC) were recovered from human whole blood using a Trima Accel apheresis device and further enriched by Ficoll-Hypaque gradient centrifugation. (GE healthcare). CD4+ memory T cells were isolated from PBMC using Miltenyi CD4+ memory T cell isolation kit (Miltenyi) according to the manufacturer's instructions. L cells that express CD32 and CD80 were irradiated at 5000 Rads and employed as antigen presenting cells.

Irradiated CD32+ CD80+ L cells (5000 cells/well) were mixed with CD4+ memory T cells (50000 cells/well) and cultured at 37°C in 5% CO2 in a 96-well plate. Culture media consisted of RPMI 1640 with 10% FBS, Gluta-MAX, sodium pyruvate, penicillin/streptomycin, non-essential amino acids, and beta-mercaptoethanol.

CD4+ Memory T cells were activated in CD32+ CD80+ L cell-cocultures by adding 40 ng/mL of anti-CD3 (clone SP34) and (BD Biosciences) and variable amounts of mab 1A7.gr.1 or isotype control (anti-HSV glycoprotein D, anti-gD) (serially diluted two-fold from 320 ng/mL to 0.16 ng/mL).. IFN-gamma levels in the supernatant were assessed by Luminex (Bio-Rad) at day 5 of culture, and T cell proliferation was measured by Cell Titer-Glo luminescence (Promega) and read on EnVision Multilabel Reader (Perkin Elmer) at day 7 of culture.

### RESULTS:

OX40 agonist antibodies bound human OX40 with sub-nanomolar affinity. Surface plasmon resonance analysis was used to measure the binding kinetics of anti-OX40 antibodies binding to human OX40. Variants of humanized antibody 1A7 showed high affinity binding to human OX40 as measured by Biacore. The results of this analysis are shown in Table 2.

**Table 2: Kinetics Constants for Humanized Anti-OX40 antibody 1A7 Variants Binding to Human OX40**

| | ka (1/Ms) | kd (1/s) | KD (nM) |
|---|---|---|---|
| 1A7.gr.1 | 4.38e5 | 1.52e-4 | 0.529 |
| 1A7.gr.2 | 4.44e5 | 2.33e-4 | 0.524 |
| 1A7.gr.3 | 4.62e5 | 2.43e-4 | 0.525 |
| 1A7.gr.4 | 3.45e5 | 1.63e-4 | 0.472 |
| 1A7.gr.5' | 2.78e5 | 4.92e-4 | 1.77 |
| 1A7.gr.6 | 3.91e5 | 6.93e-5 | 0.177 |
| 1A7.gr.7' | 4.01e5 | 1.45e-4 | 0.360 |

Variants of humanized antibody 3C8 showed high affinity to huOX40. The results of this analysis are shown in Table 3.

**Table 3: Kinetics Constants for Humanized Anti-OX40 antibody 3C8 Variants Binding to human OX40**

| | ka (1/Ms) | kd (1/s) | KD (nM) |
|---|---|---|---|
| 3C8.gr.1 | 4.12e5 | 2.67e-3 | 6.49 |
| 3C8.gr.2 | 4.66e5 | 2.55e-3 | 5.48 |
| 3C8.gr.3 | 4.98e5 | 1.96e-3 | 3.94 |
| 3C8.gr.4 | 4.2e5 | 9.23e-4 | 2.20 |
| 3C8.gr.5 | 4.57e5 | 6.19e-4 | 1.35 |
| 3C8.gr.6 | 3.95e5 | 1.22e-3 | 3.08 |
| 3C8.gr.7 | 4.86e5 | 6.85e-4 | 1.41 |
| 3C8.gr.8 | 3.29e5 | 7.09e-4 | 2.16 |
| 3C8.gr.9 | 4.04e5 | 1.73e-3 | 4.28 |
| 3C8.gr.10 | 3.82e5 | 1.08e-3 | 2.84 |
| 3C8.gr.11 | 3.21e5 | 9.17e-4 | 2.86 |
| 3C8.gr.12 | 5.5e5 | 1.58e-3 | 2.87 |
| 3C8.gr.13 | 4.65e5 | 9.17e-4 | 1.97 |
| 3C8.gr.14 | 5.12e5 | 8.58e-4 | 1.68 |
| 3C8.gr.15 | 5.2e5 | 7.83e-4 | 1.50 |

Humanized variants of antibody 1D2 showed reduced binding to huOX40 as compared to the parent hybridoma antibody. The results of this analysis are shown in Table 4.

**Table 4: Kinetics Constants for Humanized Anti-OX40 antibody 1D2 Variants Binding to human OX40**

| | ka (1/Ms) | kd (1/s) | KD (nM) |
|---|---|---|---|
| 1D2.gr.1 | Fast on | Fast off | |
| 1D2.gr.2 | 3.19e5 | 1.78e-2 | 55.9 |
| 1D2.gr.3 | 2.73e5 | 1.63e-2 | 59.5 |

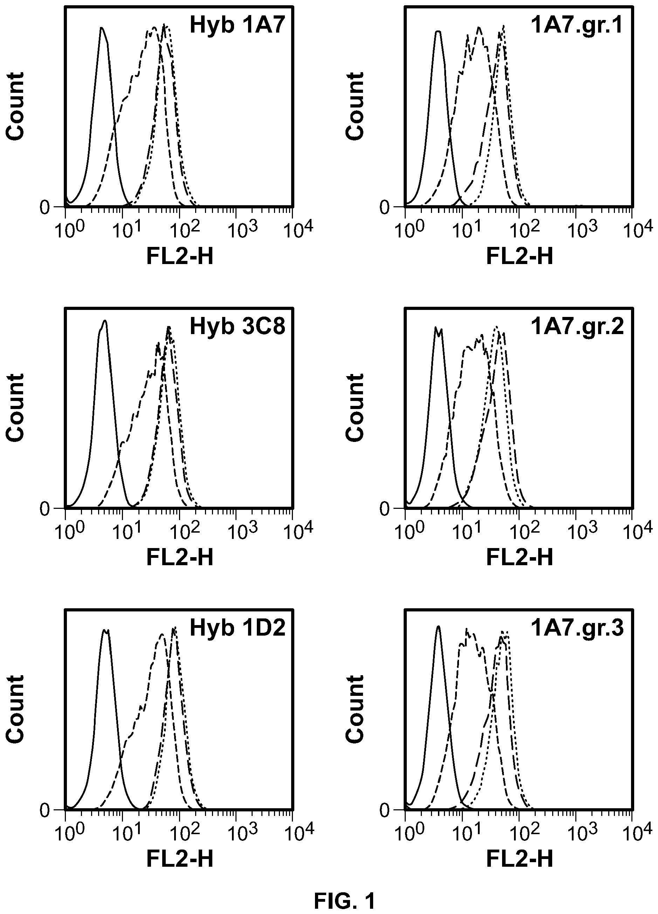

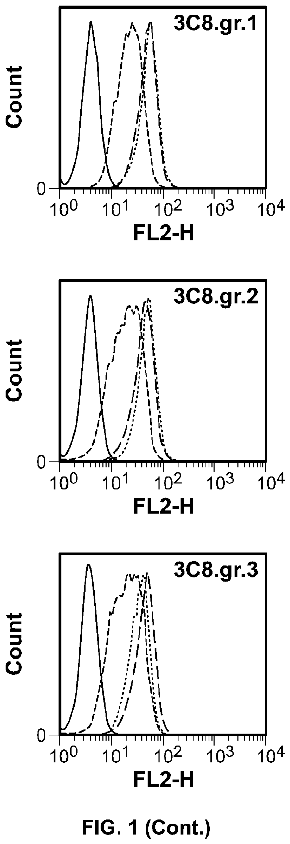

**FACS analysis:** Humanized OX40 antibody variants were analyzed by FACS to evaluate antibody binding to huOX40 expressed on the surface of Hut78 cells. The results of this analysis are shown in **FIG. 1****.** Humanized anti-OX40 agonist antibodies 1A7.gr.1, 1A7.gr.2, 1A7.gr.3, 3C8.gr.1, 3C8.gr.2, and 3C8.gr.3 bound well to human OX40 expressed on the cell surface.

**Cross-blocking experiments:** Each of the antibody clones were able to bind in the presence of the other two clones, and was only competed for binding to human OX40 by itself, suggesting that antibodies 1A7, 3C8 and 1D2 do not compete for binding to human OX40 with each other.

**Generation and characterization of variant OX40 antibodies:** Variants of anti-OX40 clones 1A7.gr.1 and 3C8.gr.5 were generated by kunkel mutagenesis to mutate CDR residue motifs that were potentially labile (e.g., under heat stress conditions). For antibody 1A7.gr1, sequences DS (D is in CDRH1; DS is potentially subject to increased isomerization) and NGDS (in CDR H2; NG is potentially subject to increased isomerization) were identified as potentially unstable residues, and were mutated to as shown in Table 5. Variants were tested by Biacore as described above to evaluate binding kinetics and in co-stimulation assays to determine biological activity.

**Table 5: Mutations introduced into variants of mab 1A7.gr.1**

| Antibody | Sequence change (original sequence → mutated sequence) |
|---|---|
| 1A7.gr.DA | CDR H1 DS→DA |
| 1A7.gr.ES | CDR H1 DS→ES |
| 1A7.gr.NADS | CDR H2 NGDS→NADS |
| 1A7.gr.NADA | CDR H2 NGDS→NADA |
| 1A7.gr.NGDA | CDR H2 NGDS→NGDA |
| 1A7.gr.SGDS | CDR H2 NGDS→SGDS |
| 1A7.gr.NGSS | CDR H2 NGDS→NGSS |

Mutation of residues DS (D is in CDR H1) to either DA or ES did not significantly alter binding affinity for huOX40 (Table 6). Mutation of residues NGDS in CDR H2 to NADS, NADA, NGDA, and NGSS also did not significantly alter binding affinity for human OX40. Mutation of NGDS (in CDR H2) to SGDS resulted in two fold loss of binding affinity (Table 6). Variant antibodies were analyzed using a T cell co-stimulation assay and level of co-stimulation of T effector cells was not significantly altered in the variants as compared to the 1A7.gr1 antibody (data not shown).

**Table 6: Kinetics Constants for Anti-OX40 1A7 variants binding to human OX40**

| | | ka (1/Ms) | kd (1/s) | KD (nM) |
|---|---|---|---|---|
| 1A7.gr.1 | | 3.23e5 | 1.29e-4 | 0.398 |
| 1A7.gr.DA | CDR H1 DS→DA | 3.21e5 | 8.32e-5 | 0.259 |
| 1A7.gr.ES | CDR H1 DS→ES | 3.41e5 | 1.09e-4 | 0.319 |
| 1A7.gr.NADS | CDR H2 NGDS→NADS | 2.75e5 | 1.21e-4 | 0.442 |
| 1A7.gr.NADA | CDR H2 NGDS→NADA | 3.04e5 | 1.08e-4 | 0.355 |
| 1A7.gr.NGDA | CDR H2 NGDS→NGDA | 3.37e5 | 1.09e-4 | 0.322 |
| 1A7.gr.SGDS | CDR H2 NGDS→SGDS | 2.30e5 | 2.28e-4 | 0.993 |
| 1A7.gr.NGSS | CDR H2 NGDS→NGSS | 3.49e5 | 1.08e-4 | 0.311 |
| 1A7.gr.DANADA | CDR H2 NGDS→NADA CDR H1 DS→DA | 3.87e5 | 2.15e-4 | 0.557 |

For antibody 3C8.gr5, sequences DT (in CDRH2) and DG (in CDR L2) (both potential isomerization sites) were identified as potentially unstable residues in antibody 3C8.gr.5. Mutations were made in each of these sequences, as shown in Table 7.

**Table 7: Mutations introduced into variants of mab 3C8.gr.5**

| Antibody clone | Sequence change (original sequence → mutated sequence) |
|---|---|
| 3C8.gr.DA | CDR H2 DT→DA |
| 3C8.gr.DQ | CDR H2 DT→DQ |
| 3C8.gr.5.EG | CDR L2 DG→EG |
| 3C8.gr.5.QG | CDR L2 DG→QG |
| 3C8.gr.5.SG | CDR L2 DG→SG |

Mutation of residues DT in CDR H2 to either DA or DQ resulted in a loss in affinity for human OX40 (as compared to affinity of the parent antibody) (Table 8). Mutation of residues DG in CDR L2 to either EG, QG, or SG did not significantly alter binding affinity (Table 8).

**Table 8: Kinetics Constants for Anti-OX40 3C8 Stability Variants Binding to human OX40**

| | | ka (1/Ms) | kd (1/s) | KD (nM) |
|---|---|---|---|---|
| 3C8.gr.5 | | 4.29e5 | 6.13e-4 | 1.43 |
| 3C8.gr.DA | CDR H2 DT→DA | 3.88e5 | 5.02e-3 | 12.9 |
| 3C8.gr.DQ | CDR H2 DT→DQ | 3.81e5 | 3.09e-3 | 8.12 |
| 3C8.gr.5.EG | CDR L2 DG→EG | 5.80e5 | 5.69e-4 | 0.98 |
| 3C8.gr.5.QG | CDR L2 DG→QG | 4.57e5 | 5.34e-4 | 1.17 |
| 3C8.gr.5.SG | CDR L2 DG→SG | 4.50e5 | 6.82e-4 | 1.51 |

**Stability testing:** Antibody 1A7.gr.1 was subjected to stability testing as described above, and sequences DS (D is in CDRH1) and NGDS (in CDR H2) were stable under thermal stress testing conditions. In addition, M and W residues (in CDR-H1, -H2 and -H3) did not show unacceptable levels of oxidation during chemical stress testing.

Antibody 3C8.gr.5 was also subjected to stability testing. While residues DT were stable under thermal testing conditions, residues DG (CDR-L2) were not stable under thermal testing conditions.

**Generation and characterization of OX40 antibody alanine scanning mutants:** Alanine scan mutants of 1A7.gr.1 and 3C8.gr.5 to were generated, e.g., to examine impact of mutations on binding affinity. In addition, impact of binding affinity on antibody activity was determined. Mutants were generated using kunkel mutagenesis to change amino acid residues in the third CDR of the light chain and the heavy chain. The variant antibodies were expressed transiently in 293 cells, and tested by Biacore 3000 for binding to human OX40.

The results of this experiment are shown in Table 9. Alanine substitutions into heavy chain CDR 3 at positions 96 (1A7.Ala.14), 97 (1A7.Ala.11), 98 (1A7.Ala.13), 99 (1A7.Ala.10), and 100 (1A7.Ala.9) showed reduced binding to huOX40. These results suggest that the residues at position 96, 97, 98, 99 and 100 may be important for antibody binding to OX40. Functional characterization of Ala scan mutants 1A7.Ala9, 1A7.Ala10, 1A7.Ala11, 1A7.Ala13, and 1A7.Ala14 in an in vitro T cell co-stimulation assay showed that decreased OX40 antibody affinity correlated with reduced ability of antibodies to co-stimulation effector T cell proliferation activity.

**Table 9: Kinetics Constants for Anti-OX40 1A7.gr.1 Alanine Scan Variants Binding to human OX40 (substituted alanine is underlined)**

| | LC-CDR 3 | HC-CDR 3 | KD (nM) |
|---|---|---|---|
| 1A7.gr.1 | QQGHTLPPT (SEQ ID NO:7) | VLAPRWYFSV (SEQ ID NO:209) | 0.62 |
| 1A7.Ala.7 | QAGHTLPPT (SEQ ID NO:28) | VLAPRWYFSV (SEQ ID NO:209) | 0.91 |
| 1A7.Ala.5 | QQAHTLPPT (SEQ ID NO:26) | VLAPRWYFSV (SEQ ID NO:209) | 0.39 |
| 1A7.Ala.3 | QQGATLPPT (SEQ ID NO:24) | VLAPRWYFSV (SEQ ID NO:209) | ∼0.5 |
| 1A7.Ala.4 | QQGHALPPT (SEQ ID NO:25) | VLAPRWYFSV (SEQ ID NO:209) | 0.67 |
| 1A7.Ala.2 | QQGHTAPPT (SEQ ID NO:23) | VLAPRWYFSV (SEQ ID NO:209) | ∼0.5 |
| 1A7.Ala.6 | QQGHTLAPT (SEQ ID NO:27) | VLAPRWYFSV (SEQ ID NO:209) | 0.16 |
| 1A7.Ala.1 | QQGHTLPAT (SEQ ID NO:22) | VLAPRWYFSV (SEQ ID NO:209) | ∼0.5 |
| 1A7.Ala.15 | QQGHTLPPT (SEQ ID NO:7) | ALAPRWYFSV (SEQ ID NO:196) | 0.66 |
| 1A7.Ala.16 | QQGHTLPPT (SEQ ID NO:7) | VAAPRWYFSV (SEQ ID NO:201) | 0.54 |
| 1A7.Ala.14 | QQGHTLPPT (SEQ ID NO:7) | VLAARWYFSV (SEQ ID NO:202) | 6.88 |
| 1A7.Ala.11 | QQGHTLPPT (SEQ ID NO:7) | VLAPAWYFSV (SEQ ID NO:203) | 16.7 |
| 1A7.Ala.13 | QQGHTLPPT (SEQ ID NO:7) | VLAPRAYFSV (SEQ ID NO:204) | Weak binding |
| 1A7.Ala.10 | QQGHTLPPT (SEQ ID NO:7) | VLAPRWAFSV (SEQ ID NO:205) | 5.94 |
| 1A7.Ala.9 | QQGHTLPPT (SEQ ID NO:7) | VLAPRWYASV (SEQ ID NO:206) | 5.32 |
| 1A7.Ala.12 | QQGHTLPPT (SEQ ID NO:7) | VLAPRWYFAV (SEQ ID NO:207) | 0.41 |
| 1A7.Ala.8 | QQGHTLPPT (SEQ ID NO:7) | VLAPRWYFSA (SEQ ID NO:208) | 0.41 |

Analysis of antibody 3C8.gr5 alanine scan variants is shown in Table 10. Variant antibodies comprising alanine substitutions into light chain heavy chain CDR 3 at positions 91, 93, 94, and 95 showed significantly reduced binding to huOX40. All three positions (95, 96 and 97) in heavy chain CDR3 when mutated to alanine showed reduced binding to huOX40.

**Table 10: Kinetics Constants for Anti-OX40 3C8.gr.5 Alanine Scan Variants Binding to human OX40 (alanine substitution is underlined)**

| | LC-CDR 3 | HC-CDR 3 | KD (nM) |
|---|---|---|---|
| 3C8.gr.5 | VHYAQFPYT (SEQ ID NO:42) | ARDRLDY (SEQ ID NO:200) | 1.28 |
| 3C8.A.1 | AHYAQFPYT (SEQ ID NO:43) | ARDRLDY (SEQ ID NO:200) | 2.7 |
| 3C8.A.2 | VAYAQFPYT (SEQ ID NO:44) | ARDRLDY (SEQ ID NO:200) | 2.6 |
| 3C8.A.3 | VHAAQFPYT (SEQ ID NO:45) | ARDRLDY (SEQ ID NO:200) | >100 |
| 3C8.A.4 | VHYAAFPYT (SEQ ID NO:46) | ARDRLDY (SEQ ID NO:200) | 1.27 |
| 3C8.A.5 | VHYAQAPYT (SEQ ID NO:47) | ARDRLDY (SEQ ID NO:200) | 27.9 |
| 3C8.A.6 | VHYAQFAYT (SEQ ID NO:48) | ARDRLDY (SEQ ID NO:200) | >100 |
| 3C8.A.7 | VHYAQFPAT (SEQ ID NO:49) | ARDRLDY (SEQ ID NO:200) | weak binding |
| 3C8.A.8 | VHYAQFPYT (SEQ ID NO:42) | ARARLDY (SEQ ID NO:197) | No binding |
| 3C8.A.9 | VHYAQFPYT (SEQ ID NO:42) | ARDALDY (SEQ ID NO:198) | 32.5 |
| 3C8.A.10 | VHYAQFPYT (SEQ ID NO:42) | ARDRADY (SEQ ID NO:199) | 15.8 |

**Mab 1A7.gr.1 bound with high affinity to human OX40, analyzed using radioimmunoassay analysis:** Equilibrium binding studies were performed as described to determine the affinity of mab 1A7.gr.1 to human OX40 expressed on transfected BT-474 cells. The Kd values are summarized in Table 11. The average measured binding affinity was 0.45 nM (n=3, individual values 0.43, 0.44, 0.49).

**Table 11 Equilibrium Binding Analysis of mab 1A7.gr.1 binding to BT-474 cells Expressing Recombinant human OX40**

| Antibody | Assay | Kd (nM) | Average K_{d} (nM) (± SD) |
|---|---|---|---|
| | 1 | 0.43 | |
| 1A7.gr.1 | 2 | 0.44 | 0.45 ± 0.03 |
| | 3 | 0.49 | |

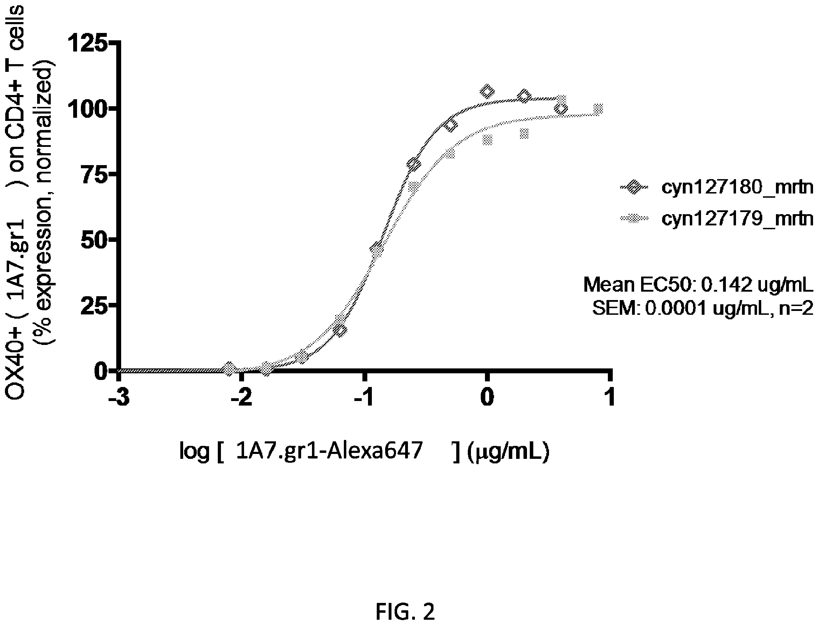

**Mab 1A7.gr.1 bound with high affinity to OX40 expressed on human, cynomolgus monkey, rat and mouse T cells:** Binding of mab 1A7.gr.1 to OX40 expressed on human, cynomolgus monkey, rat and mouse T cells was assessed in a flow cytometry-based assay. The average EC50 values for mab 1A7.gr.1 binding to human and cynomolgus monkey T cells are summarized in Table 12, and binding curves are shown in **FIG. 2****.** The results indicate that mab1A7.gr.1 bound to human and cynomolgus monkey T cells with similar high affinity, with EC50 values of 0.220 ± 0.026 (1.47 nM) and 0.142 ± 0.0001 µg/mL (0.946 nM) for human and cynomolgus monkey T cells respectively. No measurable binding of mab1A7.gr.1 to rat or mouse T cells was detected.

**Table 12: Mab 1A7.gr.1 Binding to Human and Cynomolgus Monkey T Cells**

| | Assay | EC₅₀ (µg/mL) | Average EC₅₀ (µg/mL) (± SD) |
|---|---|---|---|
| Cynomolgus monkey | 1 | 0.143 | 0.142 ± 0.00014 |
| | 2 | 0.142 | |
| Human | 1 | 0.196 | 0.220 ± 0.053 |
| | 2 | 0.184 | |
| | 3 | 0.201 | |
| | 4 | 0.298 | |

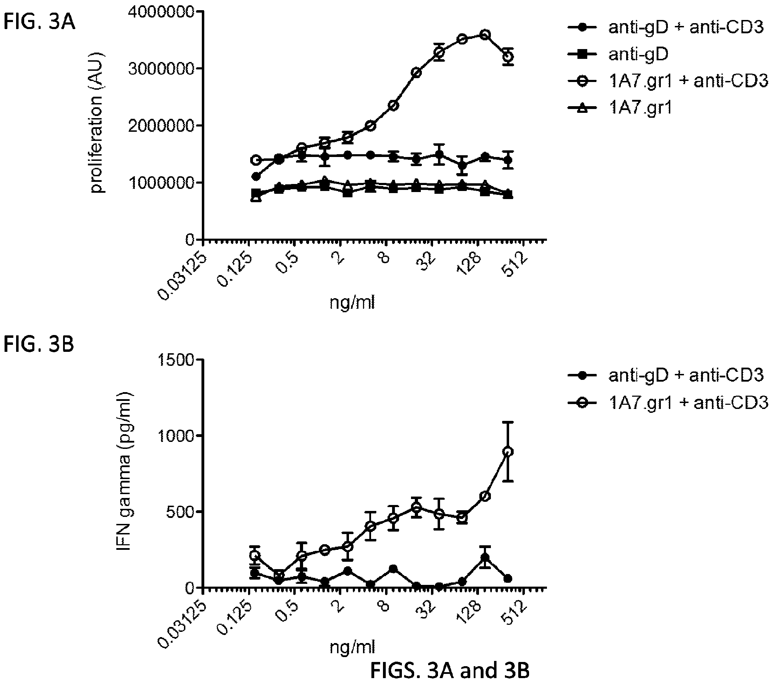

**Treatment with OX40 agonist antibody mab1A7.gr.1 costimulated T effector cell proliferation and interferon gamma production following T cell receptor engagement in a dose dependent manner:** CD4+ memory T cells were stimulated with a fixed concentration of anti-CD3 and variable concentration of anti-OX40 or control antibodies as described. In the absence of anti-CD3 crosslinking, mab 1A7.gr.1 had no effect on T cell proliferation (**FIG. 3A**)**.** Increasing concentration of mab 1A7.gr.1 costimulated CD4+ memory T cell proliferation in response to anti-CD3 crosslinking. The calculated EC50 for the costimulatory effect of mab1A7.gr.1 was 9.96 ng/mL (n=2, **FIG. 3A**)**.** Increasing concentrations of mab1A7.gr.1 costimulated CD4+ memory T cell production of interferon gamma in response to anti-CD3 crosslinking (**FIG. 3B**)**.** These results demonstrated that agonist OX40 mab1A7.gr.1 costimulated CD4+ memory T cell proliferation and interferon gamma production.

### MATERIALS AND METHODS II

**1. Antibodies:** Purified anti-human OX40 (PE) (clone ACT35), mouse IgG1 (PE), anti-human CD3 (clone SP34), anti-human CD4 (FITC), anti-human CD25 (PE), anti-human CD45RA (APC), anti-human CD3-APC and purified mouse anti-human CD11b antibody were purchased from BD biosciences. Alemtuzumab (Campath) was purchased from Genzyme. Anti-human CD52 (APC) was purchased from Sigma. Goat anti-mouse IgG Alexa Fluor 647 was purchased from Invitrogen. Anti-human CD127 (eFluor 450) and rat anti-mouse OX40 (PE, clone OX86) were purchased from eBiosciences. Anti-human OX40 antibody IA7gr1, Rituximab (chimeric anti-CD20, IgG1) and anti-HSV glycoprotein D (anti-gD antibody, human IgG1) were generated at Genentech. Anti-human IgG (PE) was purchased from BD Biosciences. Human CD4 Isolation Kit II was from Miltenyi Biotech. Human NK isolation kit was purchased from Stem Cell Technology.

### 2. Cells and media

**a, Cell lines:** BT474 (a human breast cancer cell line) was acquired from ATCC; 293-based Amphotropic cell line was acquired from Life Technology. BT474 and Amphotropic cells were cultured and maintained in DMEM media supplemented with 10% FBS/Pen/Strep, at 37C/5%CO₂ incubator. L cells expressing CD32a and CD80 were cultured and maintained in the complete RPMI media (RPMI-1640, supplemented with 10% FBS (heat-inactivated)/Pen/strep/Glutamine/Non-essential amino acids/55µM βME), at 37C/5%CO₂ incubator. U937 (a human monocytic cell line) was cultured and maintained in the complete RPMI media at 37C/5%CO₂ incubator.
**b, Generation of human OX40-expressing BT474 clones:** To generate human OX40-MSCV (hOX40-MSCV) plasmid, human OX40 cDNA was cloned as a fusion protein with a 5' HSV glycoprotein D (gD) tag into MSCV IRES GFP retroviral vector (Clontech). Amphotropic Phoenix packaging cells were transfected with hOX40-MSCV plasmid via Fugene HD (Roche). BT474 cells were subsequently infected with viral supernatants from the transfected amphotropic cells. Pools of OX40-expressing BT474 cells were enriched by sorting surface gD+ and GFP+ cells. OX40-expressing clones were generated by limiting dilution analysis of OX40-expressing clones. Clones expressing high and low levels of OX40 were selected based on surface expression of the gD tag and OX40 expression levels by flow cytometry. To determine human OX40 expression levels on BT474 clones, OX40-expressing cells were stained with anti-human OX40-PE (clone ACT35, BD Biosciences), and cell fluorescence was measured on a FACS Canto II (BD Bioscience) and analyzed using Flowjo software (TreeStar).
**c, of normal human peripheral blood mononuclear cells (PBMC), T cells, monocytes and NK cells:** Normal human peripheral blood samples were donated by registered Genentech employees and provided by the Employee Health Service department. Normal human buffy coat was procured from San Francisco Blood Bank via Genentech Employee Health Service department. Peripheral white blood cells (PBMC) were recovered from human whole blood using a Trima Accel apheresis device and further enriched by Ficoll-Hypaque gradient centrifugation (GE Healthcare).

CD4⁺ T cells were isolated from PBMC using a Miltenyi CD4⁺ T cell isolation kit. CD4+ naive T cells were stained with CD4-FITC, CD25-PE, and CD127-e450 (eBiosciences) to sort CD4+CD25-CD127+ cells and CD4⁺CD25⁺CD127⁻ cells. Treg cells were sorted from the same donor with FACS Aria (BD Biosciences). The purity of sorted cells was confirmed by flow cytometric analysis immediately after sorting.

The PBMC of the whole blood samples isolated by Ficoll-Paque gradient centrifugation were used for isolation of monocytes. Monocytes were isolated from PBMC using human monocyte isolation kit (Miltenyi). Isolated monocytes were cultured 7 to 14 days with the complete RPMI media in the presence of 20 ng/mL GM-CSF for the generation of monocyte-derived macrophages (MDM).

Normal human NK cells were isolated from PBMC of buffy coat using the human NK cell enrichment kit (Stem Cell Technology) according to the manufacturer's instructions, and cells were used immediately for the ADCC assay.
**3, Cell labeling reagents:** Purified CD4+ naive T cells were labeled with CFSE using the CellTrace CFSE Cell Proliferation Kit (Life Technology) and purified Treg cells were labeled with PKH26 Red Fluorescent Cell Linker Kit (Sigma). Both kits were used according to the manufacturer's instructions.
**4. Treg suppression assay:** L cells that express CD32 and CD80 were irradiated at 5000 rads and employed as antigen-presenting cells for the CD4+CD25+CD127- cell suppression assay.
   Irradiated CD32+ CD80+ L cells (5000 RAD, 10,000 cells/well) were plated on a flat bottomed 96-well tissue culture plate, and maintained at 37C/5%CO2 incubator overnight. On the following day, CD4+ naive T cell proliferation in the presence or absence of sorted Treg cells was assessed in the following CD4+CD25+CD127- T cell suppression assay.
   To monitor the proliferation of naive CD4+ T cells, 100,000 CFSE (5um) labeled naive CD4+ T cells were added to each well in the assay plate, which already contained L cells seeded the day before. To test the suppressive function of Treg cells, 200,000 PKH26 (2um)-labeled Treg cells were added to each well containing CFSE labeled CD4+ naive T cells. To test the activity of the anti-OX40 antibody 1A7.gr1, 1A7.gr1 or the isotype control antibody anti-CD20 antibody (rituximab) (each at 200ng/ml) was added to the co-culture of naive CD4+ T cells, Treg cells, and irradiated CD32⁺ CD80⁺ L cells. Soluble anti-CD3 was added to cells at a final concentration of 2.5 ng/mL. The assay plate was maintained at 37C/5%CO₂ incubator for 6 days before data acquisition. At day 6, the proliferation of naive CD4 T cells was traced by CFSE dye dilution and cell fluorescence was measured on a FACS Canto II (BD Bioscience) and analyzed using FlowJo software (Treestar).
**5. Antibody dependent cell-mediated phagocytosis (ADCP) assay:** U937 cells were employed as phagocytic effector cells in the ADCP assay. 50,000 U937 cells labeled with PKH26 (2um) were seeded in a 96 well tissue culture plate. Variable amounts of anti-OX40, 1A7.gr1 or anti-HER2 (trastuzumab isotype control) antibodies (2-fold serial dilutions from 1000 to 7.8ng/ml) were added to U937 cells. Either OX40-high or -low expressing BT474 clones were utilized as target cells in the ADCP assay. 5,000 OX40+ BT474 cells were added to U937 cells in the assay plate. The assay plate was incubated at 37C/5%CO₂ incubator for 4 hours. Cells then were harvested for cell fluorescence measurement on a FACS Canto II (BD Bioscience) and analyzed using FlowJo software (Treestar). The percentage of phagocytosis was calculated by dividing GFP+ PKH26+ events (i.e., measured U937 cells that have engulfed BT474 cells) from all GFP⁺ events (all BT474 cells).
**6, Antibody dependent cell-mediate cytotoxicity (ADCC) assay:** To demonstrate that 1A7.gr1 induced ADCC of OX40 expressing T cells, primary NK cells were co-cultured with activated primary CD4+ T cells. CD4+ T cells and NK cells were isolated from the same PBMC donor using the human CD4 T cell isolation kit (Miltenyi kit,) and human NK cell enrichment kit (Stem Cell Technology), repectively. OX40 expression on isolated CD4+ T cells was induced by activating T cells with PHA (5 ug/ml) for 48 hours in complete RPMI-1640 media. Alemtuzumab (anti-CD52 mAb) was used as positive control and anti-gD was used as negative control for the ADCC assay. Activated CD4+ cells (5,000 per well) were incubated with variable amounts of 1A7.gr1 or control antibodies (3-fold serial dilutions from 10000 to 0.01ng/ml) for 30minutes in a 96-well tissue culture assay plate. Subsequently, ADCC assay was initiated by adding NK cells (50, 000 cells) to each well. The assay plate was then incubated for another for 20 hours in a 37C/5%CO₂ incubator. Co-cultures were then stained with anti-human CD4-FITC and 7AAD (BD Biosciences). After washing with PBS, data were acquired by Flow cytometry (BD FACScaliber) and analyzed by Flowjo program (TreeStar). The killing (ADCC) frequency of OX40-expressing CD4 T cells was defined by the percentage of CD4⁺7AAD⁺ cell over total CD4 T cells.

To demonstrate 1A7gr1 induced OX40-sensitive ADCC, high or low human OX40-expressing BT474 (human breast cancer cell line) clones were seeded (5,000 per well) into a black-wall 96-well tissue culture assay plate,to allow attachment and maintained for 24 hours at 37C/5%CO₂ incubator. Then, anti-CD20 (isotype control) or anti-OX40 agonist antibody 1A7.gr1 were added to the BT474 cells. Primary human NK cells from healthy donors were isolated from buffy coat PBMC using the human NK cell enrichment kit (Stem Cell Technology), and 50,000 purified NK cells were then added to the wells containing BT474 clones in the presence of anti-CD20 (Rituximab isotype control) or 1A7.gr1. The assay plate was incubated for 24 hours before CellTiterGlo reagent was added to capture the chemiluminescent signal. The percentage of cells undergoing anti-OX40-mediated ADCC was calculated by dividing the signal of antibody-treated cells with that of non-treated cells. The signal from NK single culture was subtracted to offset the background signal from NK cells.

### RESULTS II

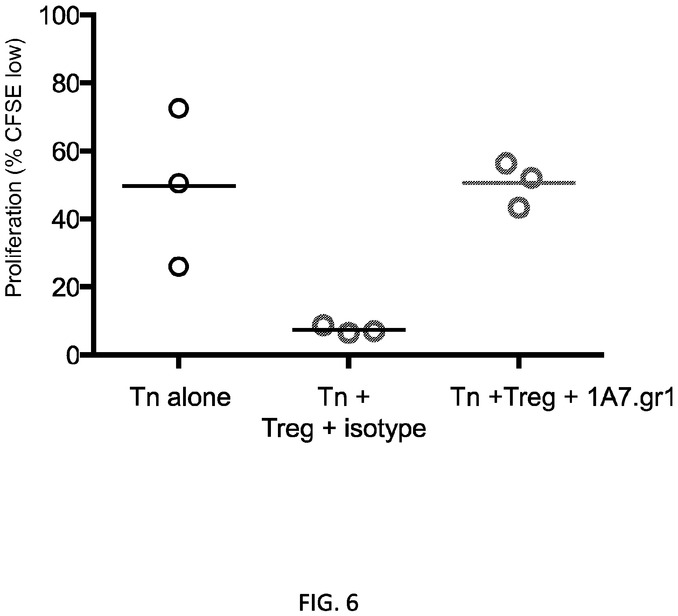

**Mab 1A7.gr.1 inhibited Treg suppression of CD4+ naive T cells:** We tested whether OX40 crosslinking by binding of OX40 agonist antibody to Treg cells will inhibit their capacity to inhibit naive T cell proliferation. Naive CD4⁺ T cell proliferation was induced by anti-CD3 crosslinking in the absence of 1A7.gr1. The addition of Treg cells to naive T cell cultures suppressed anti-CD3-induced naive T cell proliferation. Treatment with mAb 1A7.gr.1 inhibited the suppressive function of regulatory T cells (n=3, **FIG. 6**)**.** Naive T cell proliferation was measured by monitoring CFSE low populations through flow cytometric analysis. This result demonstrated that mAb 1A7.gr.1 inhibited the suppressive function of Treg cells.

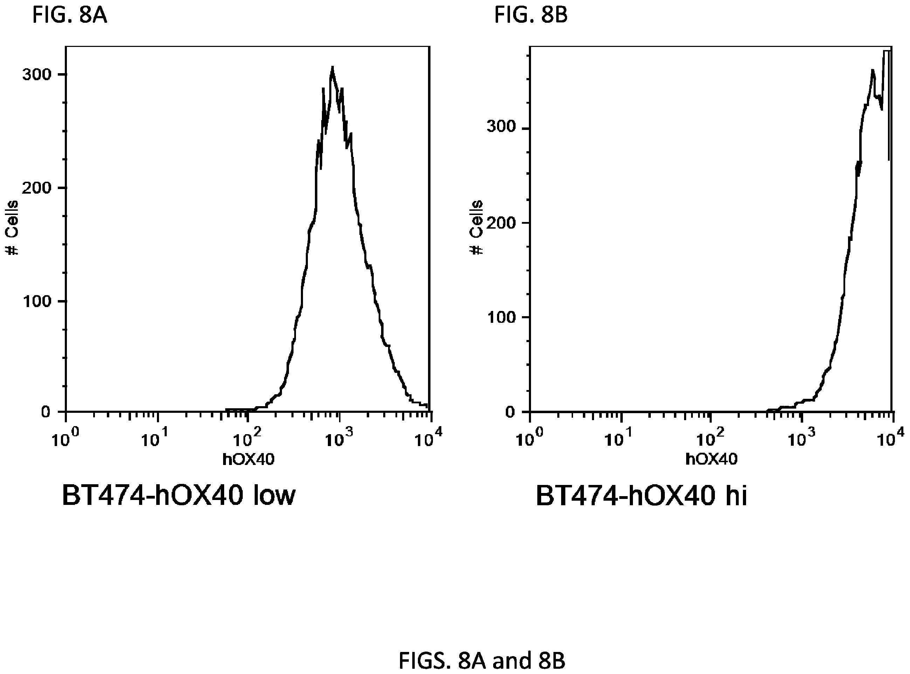

**BT474-hOX40 transgenic clones expressed different levels of hOX40:** Expression level of human OX40 was characterized in transgenic BT474 cell clones using hOX40 surface staining and mean fluorescent intensity (MFI) by flow cytometric analysis. The BT474-human OX40-high clone expressed more than 5-fold higher OX40 than BT474-human OX40 low clone, with the high-OX40 clone showing a MFI of 5243 (**FIG. 8B**) and the low-OX40 clone showing MFI of 994 (**FIG. 8A**)**.** The isotype control antibody, trastuzumab, had an MFI of 10 (data not shown).

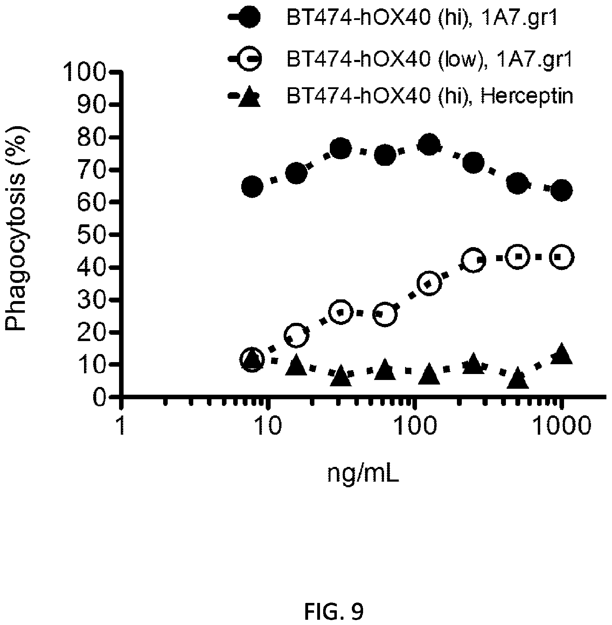

**Treatment with OX40 agonist antibody induced OX40-dependent Antibody Dependent Cell Mediated Phagocytosis (ADCP):** To determine whether anti-human OX40 mab 1A7.gr.1, mediated ADCP, U937 myeloid cells were cultured with OX40-expressing target cells, BT474 clones that expressed high or low levels of OX40. Treatment with OX40 agonist antibody 1A7 induced phagocytosis of BT474-hOX40 clones expressing low and high levels of hOX40, in a dose-dependent manner as assayed using FACS (**FIG. 9**)**.** In addition, higher level of phagocytosis was observed using the BT474-hOX40-high clone than when using the BT474-hOX4-low clone, suggesting that OX40 antibody-mediated cell-mediated phagocytosis was dependent on OX40 expression level on target cells (**FIG. 9**)**.** By contrast, trastuzumab (also termed Herceptin), the isotype control antibody, only mediated 10% phagocytosis as shown with BT474-hOX40 high clone. These data demonstrated that mab 1A7.gr.1 induced phagocytosis of OX40 expressing target cells, and that the levels of antibody dependent cell-mediated phagocytosis were correlated with OX40 levels.

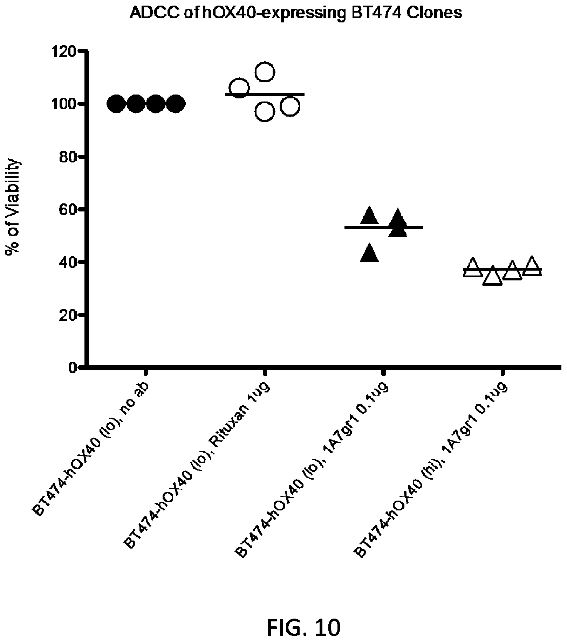

**Treatment with agonist OX40 antibody 1A7.gr1 induced ADCC in BT474 clones expressing human OX40, and level of ADCC was significantly higher in cells expressing high levels of OX40 verses level of ADCC in cells expressing low levels of OX40:** Treatment with OX40 agonist antibody 1A7.gr1 induced ADCC in OX40-expressing cells (**FIG. 10**)**.** To detect ADCC, CellTiterGlo assay was performed. Treatment with anti-OX40 agonist antibody 1A7.gr1 at 0.1 ug/mL induced significant ADCC of hOX40-BT474-low cells as evidenced by reduced target cell viability. Treatment with OX40 agonist antibody 1A7.gr1 at 0.1 ug/ml resulted in even more extensive ADCC of hOX40-BT474 high cells. The difference of ADCC between hOX40-BT474-low and -high clones was statistically significant (p=0.0077). By contrast, treatment with rituximab (control) did not alter viability for both high and low-OX40 expressing BT474 clones. These data demonstrated that agonist OX40 antibody 1A7.gr1 was capable of mediating killing of OX40-expressing cells in vitro. In addition, level of ADCC was significantly higher in cells expressing high levels of OX40 verses level of ADCC in cells expressing low levels of OX40. OX40 is highly expressed in intratumoral T cells, and human intratumoral Treg cells express high levels of human OX40.

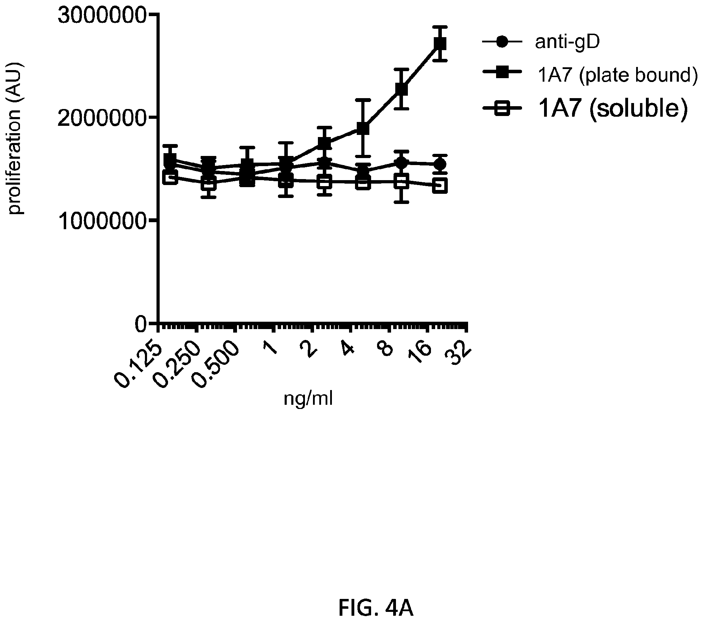

**Activity of agonist OX40 antibody was cross-linking dependent and required Fc effector function to costimulate effector T cells:** To determine whether mab 1A7 was dependent on antibody crosslinking to costimulate effector T cell proliferation, mab 1A7.gr.1 was added to the culture in plate-bound or soluble form. The results of this experiment are shown in **FIG. 4A****.** In the presence of plate-bound anti-CD3, plate-bound mab 1A7.gr.1 costimulated CD4+ effector T cell proliferation. In contrast, costimulatory activity was abrogated when 1A7 was provided in soluble form in the presence of plate-bound anti-CD3, to a similar level as that observed with a plate-bound isotype control antibody in the presence of plate-bound anti-CD3. These results suggest that crosslinking (e.g., provided by adhering mab 1A7.gr.1 to the plate) may be required for mab 1A7 costimulation of effector T cells.

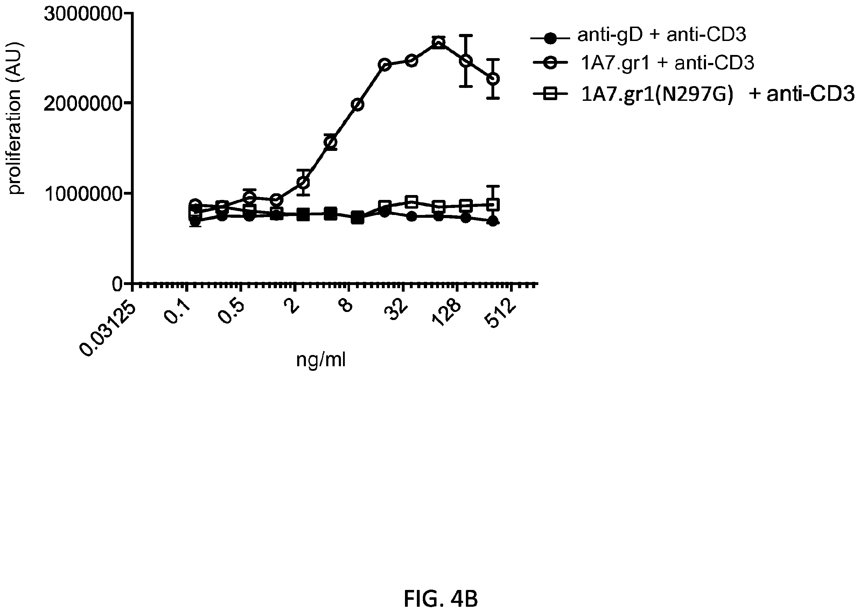

To further interrogate whether mab 1A7.gr.1 required crosslinking for activity, mutant antibody was generated to create an antibody that was unable to bind to Fc gamma receptor presented on cells. Briefly, Fc residue asparagine 297, the site for N-linked glycosylation required for binding to Fc gamma receptors, was mutated to glycine (N297G) to prevent antibody Fc binding to Fc receptors. The results of this experiment are shown in **FIG. 4B****.** MAb 1A7 gr.1 (IgG1) harboring the N297G mutation failed to costimulate Teff cell proliferation. By contrast, wildtype (un-mutated) mab 1A7 gr.1 costimulated anti-CD3 induced Teff cell proliferation. Together with the results shown in **FIG. 4A****,** these results demonstrated that activity of OX40 agonist mab 1A7 gr.1 was cross-linking dependent, requiring Fc effector function to costimulate effector T cells.

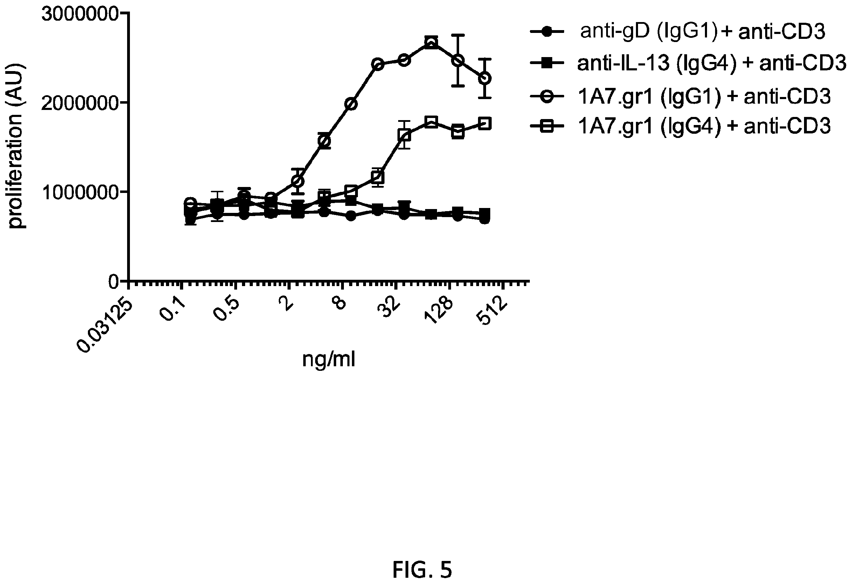

**Mab 1A7 gr.1 IgG1 possessed more potent crosslinking activity than mab 1A7.gr.1** IgG4. Antibody isotypes have differential binding to Fc gamma receptors. To identify the isotype that permitted the most potent effector T cell costimulation activity, activity of mab 1A7.gr.1 having a human IgG1 Fc was compared to activity of mab 1A7 gr.1 on a human IgG4 backbone. The results of this experiment are shown in **FIG. 5****.** Mab 1A7 gr.1-IgG1 costimulated anti-CD3 induced effector T cell proliferation more potently than did mab 1A7 gr.1 -IgG4. This result indicated that mab1A7 gr.1 comprising a human IgG1 Fc possessed more potent crosslinking activity than did mab 1A7.gr1 comprising a human IgG4 Fc.

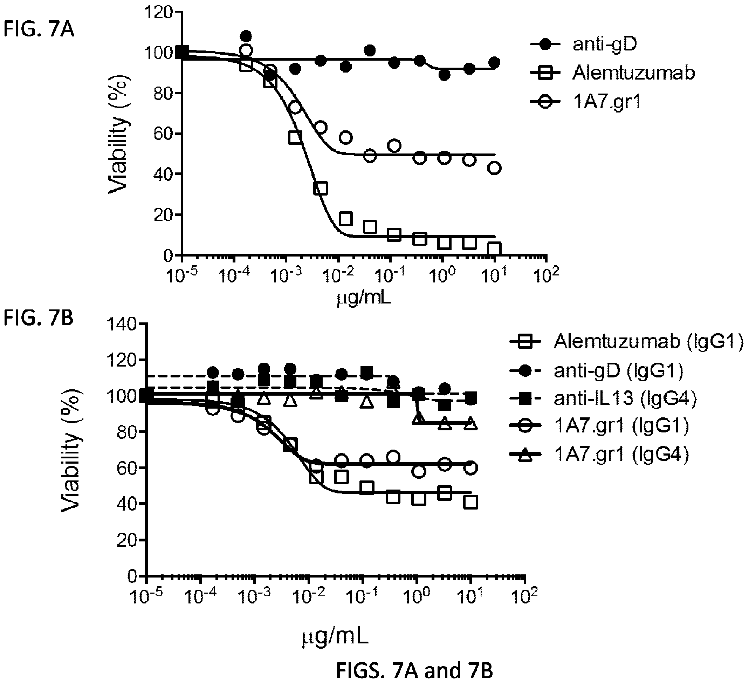

**Treatment with mab 1A7.gr.1 induced ADCC of OX40-expressing T cells.** Antibodies that recognize cell surface antigens have the potential to mediate target cell depletion through antibody dependent cellular cytotoxicity (ADCC). To determine whether mab1A7.gr1 induced ADCC, target cells that express high levels of OX40 were generated by stimulating peripheral blood CD4+ T cells with PHA and contacted with mab 1A7.gr.1 in the presence of human effector cells. The results of this experiment are shown in **FIG. 7A****.** Treatment with mab 1A7.gr.1 induced ADCC of OX40-expressing T cells when NK cells were cultured with stimulated CD4+ T cells (at a ratio of 10 NK cells: 1 activated T cell). This result demonstrated that 1A7.gr1 induced ADCC on T cells that express high levels of OX40. Depletion induced by mab.1A7.gr.1 was less extensive than that induced by treatment with the positive control antibody, Alemtuzumab (anti-CD52 mab). By contrast, treatment with negative control mab anti-gD (isotype control) did not result in induction of ADCC.

To determine whether mab 1A7.gr.1 antibodies with different Fc isotypes demonstrated differential ADCC activity in vitro, activity of mab 1A7.gr1 with the IgG1 isotype was compared to activity of mab 1A7.gr1 with the IgG4 isotype. The results of this experiment are shown in **FIG. 7B****.** Treatment with mab A7.gr1 (IgG1) induced greater ADCC of OX40-expressing CD4+ T cells compared to level of ADCC induced by mab 1A7.gr1 (IgG4).

### MATERIALS AND METHODS III

**OX40L and anti-OX40 binding competition assay.** Anti-human OX40 antibody 1A7gr1 was conjugated to Alexa Fluor647. Human OX40L-flag and human DR5-flag proteins were generated at Genentech. Anti-flag antibody conjugated to Alexa Fluor 555 was purchased from Cell Signaling (Cat# 3768). Transgenic Hut78-hOX40 cells (Hut78-hOX40, a human T cell line, retrovirally transduced for human OX40 expression) were cultured and maintained in the complete RPMI media (RPMI-1640, supplemented with 10% FBS (heat-inactivated)/Pen/strep/Glutamine/Non-essential amino acids/55uM βME) at 37C/5%CO₂ incubator.

First, titration of mab 1A7gr1 binding to OX40 was performed. 1x10⁶ Hut78-hOX40 cells were used and the concentration of mab1A7gr1 was titrated 2 fold down starting from 10 ug/mL. After 30 min of incubation at 4C, cells were washed twice in Wash Buffer (PBS/0.5%FBS/2mM EDTA), followed by data acquisition on FACSCantoII flow cytometer (BD). Data were then analyzed by Flowjo program (TreeStar) and the mean fluorescent intensity (MFI) was captured. The amount of mab 1A7.gr.1 (200ng/mL) that generated around 70% of maximum MFI was chosen for use in the competition assay. A titration of OX40L-flag binding was carried out in a similar fashion, except that OX40L-flag was titrated 3 fold down starting from 10 ug/mL.

The OX40L and anti-OX40 mab binding competition assay was performed as follows: OX40L-flag or control DR5-flag was added at concentrations of 10 ug/ml to 10 ng/mL to 1x10⁶ Hut78-hOX40 cells. After 30 min of incubation at 4C, cells were washed twice in Wash Buffer, and then 10ug/mL of anti-flag -Alexa Fluor 555 and 200 ng/mL mab 1A7.gr.1-AlexaFluor647 were added to test competition between OX40L and anti-hOX40 mab 1A7.gr.1. After 30 min of incubation at 4C, cells were washed twice in Wash Buffer, followed by data acquisition on FACSCantoII flow cytometer (BD). Data were then analyzed by Flowjo program (TreeStar) to capture MFI of 1A7gr1 binding.

### RESULTS III

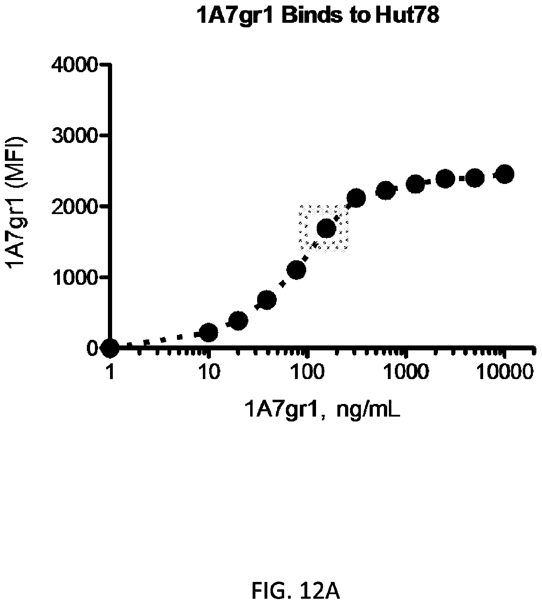

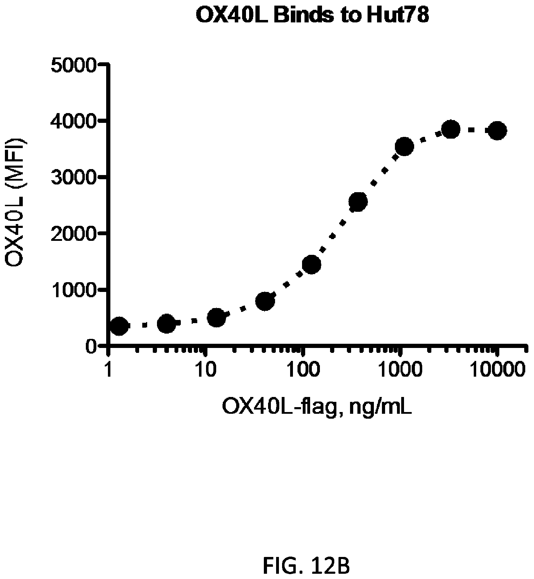

The titration experiment demonstrated that anti-human OX40 mab 1A7gr1 bound to Hut78-hOX40 cells in a dose dependent fashion, with 70% of maximum binding observed at about 200 ng/mL of antibody (**FIG. 12A**)**.** This concentration was chosen for competition experiments. OX40L-flag also demonstrated a dose dependent binding to Hut78-hOX40 cells (**FIG. 12B**)**.**

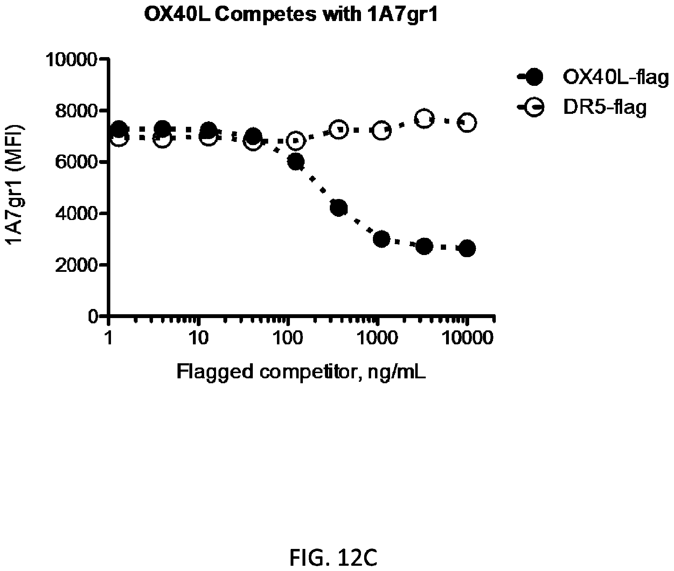

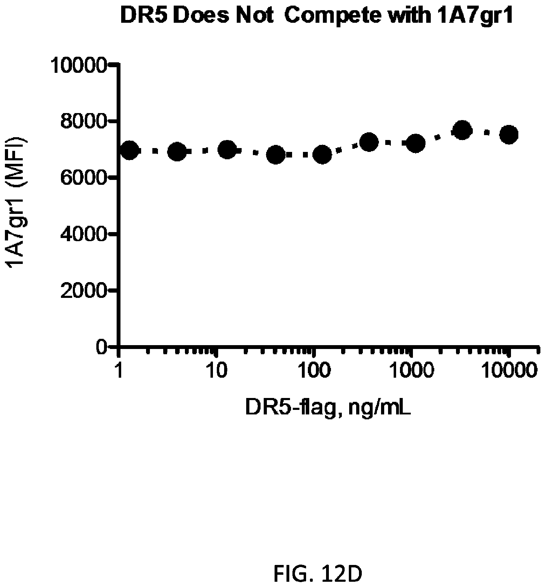

In the competition assay, binding of anti-human OX40 mab 1A7.gr.1 to Hut78-hOX40 cells decreased as the concentration of OX40L-flag increased (**FIG. 12C**)**.** By contrast, presence of control DR5-flag had no impact on mab 1A7.gr.1 binding (**FIG. 12D**)**,** indicating that OX40L binding to Hut78-OX40 cells was specific. These data demonstrated that OX40L competes with anti-human OX40 mab 1A7gr1 for binding to hOX40 on Hut78-OX40 cells. In similar experiments, 3C8.gr5 was also observed to compete for binding with OX40L.

### MATERIALS AND METHODS IV

**Pharmacokinetic (PK) evaluation of anti-OX40 in mice.** Female B6.CB17*-Prkdc^{scid}*/SzJ mice (n=12 animals/group) were given a single IV injection of 1A7.gr1 IgG1 (MOXR0916) at 1mg/kg or 10 mg/kg on Day 0. Each mouse was given a dose volume of 5mL/kg (1mg/kg dose was at a concentration of 0.2mg/mL; 10mg/kg dose was at a concentration of 2mg/mL). Blood samples (125-150µL) were collected from each animal via cardiac puncture (3 timepoints per animal; 3 animals per timepoint) at 5 minutes; 1, 3, 8, and 24 hours; and 2, 4, 7, 10, 14, 21, and 28 days post-dose. No predose samples were collected for the study. The samples were collected into tubes containing serum separator tubes and were processed as described below.

The samples were allowed to clot at ambient temperature for at least 20 minutes prior to centrifugation. Centrifugation began within 1 hour of collection at a relative centrifugal force of 11,000 revolutions per minute for 5 minutes in a centrifuge set to maintain 2°C-8°C. Serum separated from the blood was transferred into pre-labeled, 0.5-mL tubes with caps. Samples were stored in a freezer set to maintain -80°C until analysis.

1A7.gr1 IgG1 was analyzed with a generic ELISA using sheep anti-human IgG as the capturing reagent and goat anti-human IgG horseradish peroxide (HRP) as the detection reagent. The effective limit of detection (LOD) for this assay was 0.08 µg/mL.

The serum concentration versus time data from each animal were analyzed using the IV bolus input model and sparse sampling (Model 201), WinNonlin Pro, version 5.2.1, (Pharsight Corporation; Mountain View, CA). All PK analysis was based on naive pool of individual animal data. The following methods were used to estimate specific PK parameters: Cmax (Maximum concentration), AUCₗₐₛt (Area under the serum concentration versus time curve from time = 0 to time of the last measurable concentration was calculated using the linear trapezoidal rule), AUC_{inf} (Area under the serum concentration versus time curve extrapolated to infinity), Cmax/D (Dose normalized Cmax), AUC_{inf}/D (Dose normalized AUC_{inf}), CL (Clearance; Dose/AUC_{inf}), Vss (Volume of distribution at steady state), and t_{1/2},λ_{z} (Terminal half-life).

### RESULTS IV

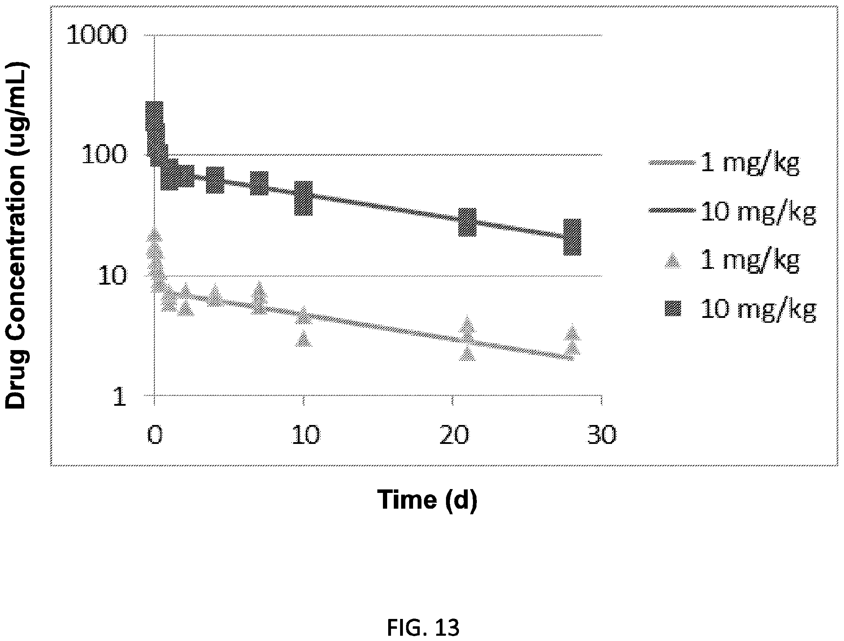

Following the administration of 1 and 10 mg/kg, PK was observed to be dose-proportional for 1A7.gr1 IgG1 (**FIG. 13**). PK parameters are summarized in Table 13 below.

**Table 13: PK parameters**

| **Parameter** | **Estimate** | **Standard Error** |
|---|---|---|
| **CL (ml/kg/day)** | 6.1 | 0.2 |
| **Vc (ml/kg)** | 54.5 | 2.2 |
| **Vp (ml/kg)** | 76.4 | 4.5 |
| **CLD (ml/kg/day)** | 188 | 26.8 |

Linear, dose-proportional PK was observed for 1A7.gr1 in non-tumor bearing SCID mice. These data suggest that the pharmacokinetics of 1A7.gr1 IgG1 at the dose levels tested was as expected for a non-cross-reactive human IgG1 monoclonal antibody in mice and similar to that of typical IgG1 monoclonal antibodies.

### MATERIALS AND METHODS V

**Pilot 4-week toxicity study in cynomolgus monkeys.** Vehicle (10 mM histidine acetate, 6% sucrose, 0.02% Tween 20, pH 5.8) or 1A7.gr1 IgG1 was administered intravenously at 0.01, 0.3, or 10 mg/kg/dose in male cynomolgus monkeys. Dosing regimen was once every 2 weeks for three doses (administered on Days 1, 15, and 29). Terminal necropsy was performed three days after last dose. Safety endpoints included: clinical and daily observations, clinical pathology, coagulation, urinalysis, macroscopic and microscopic analysis, toxicokinetic (TK), and anti-therapeutic antibody (ATA) titers. Pharmacodynamic (PD) analyses included serum cytokines, T cell activation/proliferation/depletion, and OX40 receptor occupancy.

Blood was collected by venipuncture. Additional blood samples were obtained (e.g., due to clotting of non-serum samples) when permissible sampling frequency and blood volume were not exceeded. When other samples types were being collected at the same time as FACS and PD samples, those samples were collected first after venipuncture followed by FACS and PD samples. Urine was collected by drainage from special stainless steel cage pans. After collection, samples were transferred to the appropriate laboratory for processing. Blood samples were processed for serum, and the serum was analyzed for various clinical chemistry parameters.

Individual clinical chemistry values were analyzed with an OLYMPUS® au640e analyzer. C-reactive protein (CRP) was measured by immunoturbidimetry.

Blood samples with K2-EDTA anticoagulant were collected for cytokine analysis at the indicated timepoints. After centrifugation, plasma was collected and stored at -80°C until analysis. Cytokine levels were determined using the LUMINEX® xMAP® assay technology (Luminex, Austin, TX), a bead-based multiplexing technology that enables simultaneous quantitation of multiple cytokines. Samples were analyzed using the MILLIPLEX® MAP Non-Human Primate Cytokine Magnetic Bead Panel Kit (EMD Millipore, Billerica, MA) according to manufacturer's protocol. The standard contained a mixture of recombinant GCSF, GMCSF, IPNγ, IL1beta, IL1ra, IL2, IL4, IL5, IL6, IL8, IL10, IL12/23p40, IL13, IL15, IL17A, IL18, MCP1, MIP1alpha, MIP1beta, sCD40, TGFalpha, TNFalpha, and VEGF. The standard curve ranges were 4.6-3000 pg/mL for IL4; 11.6-7500 pg/mL for IL10 and IL18; and 2.3-1500 pg/mL for all other cytokines. The plasma samples were diluted 1:3 followed by three serial 1:2 dilutions in 96-well plates, and a cocktail of antibody-conjugated fluorescent beads specific for each cytokine was added. The plates were incubated for 30 minutes at room temperature, followed by an overnight incubation at 4°C. The next day, a mixture of biotinylated detection antibodies was added to the samples, and the plates were incubated for 1 hour at room temperature, followed by a 30-minute incubation with streptavidin-phycoerythrin. Fluorescence signals were then assessed using a LUMINEX® FLEXMAP 3D® instrument, with the intensity of the phycoerythrin signal corresponding to the amount of cytokine present. The serum cytokine concentrations were determined from five-parameter fits of the standard curves for each protein.

The concentration of 1A7.gr1 IgG1 in monkey serum was analyzed using a generic ELISA. Sheep anti-human IgG (H+L) (monkey absorbed) was used as the capturing reagent, and goat anti-human IgG (H+L) (monkey absorbed) conjugated to horseradish peroxidase (HRP) was used as the detecting reagent.

Blood samples were collected throughout the study for receptor occupancy analyses using flow cytometry. For flow cytometry assay of staining surface markers (*e.g*., OX40 receptor), antibody cocktails were prepared for each staining panel on each study day. An appropriate aliquot of the cocktail was dispensed into the designated wells of a 96 deep-well plate. For each animal, 100µL whole blood sample were transferred and mixed into the appropriate wells containing the staining cocktail. Stained blood was incubated in the dark at ambient temperature for a minimum of 30 minutes. Red blood cells were lysed with 1.8mL of 1X BD FACS Lyse (BD Biosciences) for at least 6 minutes at room temperature. The samples were centrifuged, and the supernatant was aspirated and discarded. Cell pellets were washed twice with 1.8mL HBSS. Following the second wash, each sample was resuspended in 300µL of 1% paraformaldehyde. Each sample (200µL) was transferred to the appropriate wells of a 96-well U-bottom plate for acquisition on a BD FACS Canto II flow cytometer. Data analysis was performed using FACS Diva software (BD Biosciences). Lymphocytes were identified based on their FSC/SSC profile, and T cells were gated as CD3+/SSC^{low}. Subsequent hierarchical gating of CD3+ T cells was used to identify and enumerate each of the T-cell subsets. Occupancy of OX40 on CD3+CD4+CD8- Th lymphocytes was evaluated using fluorescently labeled test article (anti-OX40 antibody) that does not bind to OX40 in the presence of the administered test article. An unrelated humanized IgG1 antibody was used as an isotype control for 1A7.gr1. The fraction of Th cells that were positive above the isotype gate was classified as %OX40+ cells.

The study design is summarized in Table 14 below.

**Table 14: Pilot toxicity study design.**

| Group | # of male cynos | Test Article | Dose (mg/kg) | Projected Cₘₐₓ (% occupancy) | Projected C_{trough} (% occupancy) |
|---|---|---|---|---|---|
| 1 | 4 | Vehicle | 0 | -- | -- |
| 2 | 4 | 1A7.gr1 | 0.01 | 0.2 µg/mL (80%) | 0.05 µg/mL (50%) |
| 3 | 4 | | 0.3 | 6 µg/mL (99%) | 1.5 µg/mL (96%) |
| 4 | 4 | | 10 | 200 µg/mL (100%) | 60 µg/mL (100%) |

**Good laboratory practice (GLP) toxicity study.** Vehicle (20 mM histidine acetate, 240 mM sucrose, 0.02% polysorbate 20, pH 5.5) or 1A7.gr1 IgG1 was administered via intravenous bolus at 0.5,5, or 30 mg/kg/dose in male and female cynomolgus monkeys (five males and five females per group). Dosing regimen was once every 2 weeks for six weeks, for a total of four doses (administered on Days 1, 15, 29, and 43). Main study animals were designated for terminal necropsy at Day 45. Animals designated for recovery necropsy underwent 6 weeks of recovery after the last dose and were necropsied on Day 85. Safety endpoints included: clinical and daily observations, clinical pathology, coagulation, urinalysis, macroscopic and microscopic analysis, toxicokinetic (TK), anti-therapeutic antibody (ATA) titers, CV safety pharmacology, and neurological evaluations. Pharmacodynamic (PD) analyses included serum cytokines, T cell activation/proliferation/depletion, and OX40 receptor occupancy (peripheral and tissue).

Blood was collected by venipuncture. Urine was collected from/by drainage from special stainless steel cage pans overnight on wet ice prior to blood collection. After collection, samples were transferred to the appropriate laboratory for processing. Animals were fasted prior to clinical chemistry blood collections.

The concentration of 1A7. gr1 IgG1 in monkey serum was analyzed using a generic ELISA.

Receptor occupancy was assessed by evaluation of binding of AF647-labeled 1A7.gr1 on CD3+CD4+ T-helper lymphocytes by flow cytometry. Briefly, 100 µL of whole blood or single cell suspensions were added to the appropriate wells of a 2.2 ml/well 96 well plate along with 1.8 mL of freshly prepared Pharm Lyse. The samples were mixed well and incubated for 20 minutes at room temperature. After lysing, the samples were centrifuged, the supernatants discarded, and the samples washed once with 1.8 mL HBSS. After the washing, the cells were blocked in 100 µL of 10% cynomolgus monkey serum and incubated for a minimum of 15 minutes in the dark in a refrigerator set to maintain 2-8°C. After the blocking step, the appropriate volumes of antibody cocktail were added to the appropriate wells on the plate, the plate was mixed, and then incubated for a minimum of 30 minutes in the dark in a refrigerator set to maintain 2-8°C. After the incubation, the plate was washed twice with 1.8 mL HBSS and the samples were resuspended in 300 µL of 1% parafolrmaldehyde and incubated for 10-15 minutes in the dark in a refrigerator set to maintain 2-8°C. After the incubation, 200 µL of each sample was transferred to a 96-well v-bottom plate and analyzed on the cytometer. The flow cytometry instrument yielded data on the relative abundance of the various cell-types with the marker antigens. Samples were analyzed utilizing the FACSCanto™ II Flow Cytometer and DIVA® 6.0 software. Data generated for each animal at each post dose time point were compared to the corresponding prestudy value as well as the range of control values. The magnitude of increase or decrease was evaluated secondarily to determine the potential biological significance of any observed change.

MCP-1 levels were measured by validated LUMINEX®-based methods. The bead-based multiplex sandwich immunoassay is a solid phase protein assay which uses spectrally encoded beads conjugated to analyte specific capture antibodies as the solid support. Standards and samples were added to the wells, and the analyte (cytokine) present bound the specific capture antibody conjugated to a color-coded bead (each color bead is specific to 1 cytokine). The detection system in the assay was streptavidin conjugated to the fluorescent protein, R-phycoerythrin or streptavidin-phycoerythrin (streptavidin-RPE) mixed with analyte-specific biotinylated detection antibodies. The contents of each microplate well were drawn in the Luminex instrument. By monitoring the spectral properties of the beads and the amount of associated RPE fluorescence, the concentrations of several analytes were determined simultaneously. Samples were centrifuged to pellet debris. Wells of a 96-well multi-screen filter plate were washed with 200 µL of wash buffer. Standards, quality control (QC) samples, and samples were then transferred (25 µL) to appropriate wells containing 25 µL of assay buffer and followed by the addition of 25 µL of the appropriate antibody bead working solution. The plate was incubated in the dark at room temperature on a plate shaker set to vigorously shake for 2 hours (± 5 minutes), then washed twice with 200 µL of wash buffer, followed by the addition of 25 µL of detection antibodies to each well. The plate was incubated in the dark at room temperature for 1 hour (± 2 minutes) on a plate shaker set to shake vigorously. Then, 25 µL of working streptavidin-RPE working solution was added to each well and the plate was incubated in the dark at room temperature, for 30 minutes (± 1 minute) on a plate shaker set to shake vigorously. After the plate was washed twice with 200 µL of wash buffer, 150 µL of sheath fluid was added to each well and the plate was incubated at room temperature for at least 5 minutes in the dark, on a plate shaker set to shake vigorously. The plate was read on the Luminex system. Standards of monkey MCP-1 were prepared in assay diluent covering the concentration range of 37.500 to 6000 pg/mL. The LLOQ and ULOQ of the assay were 75.00 to 6000 pg/mL. QC samples of monkey MCP-1 were prepared in assay diluent at theoretical concentrations of 200, 1000, and 3200 pg/mL. In addition, an endogenous QC (EQC) was Plated Neat and was in the range of the curve.

The study design is summarized in Table 15 below.

**Table 15: GLP toxicity study design.**

| Group | # of cynos | Test Article | Dose (mg/kg) | # of animals terminal necropsy (Day 45) | # of animals recovery necropsy (Day 85) |
|---|---|---|---|---|---|
| 1 | 5F/5M | Vehicle | 0 | 3F/3M | 2F/2M |
| 2 | 5F/5M | 1A7.gr1 | 0.5 | 3F/3M | 2F/2M |
| 3 | 5F/5M | | 5 | 3F/3M | 2F/2M |
| 4 | 5F/5M | | 30 | 3F/3M | 2F/2M |

### RESULTS V

### Pilot toxicity study

The objective of this study was to evaluate the toxicity, toxicokinetic profile, and pharmacodynamics of anti-OX40 (1A7.gr1 IgG1) when administered via intravenous injection to cynomolgus monkeys once every 2 weeks (Days 1, 15, and 29) for a total of 3 doses.

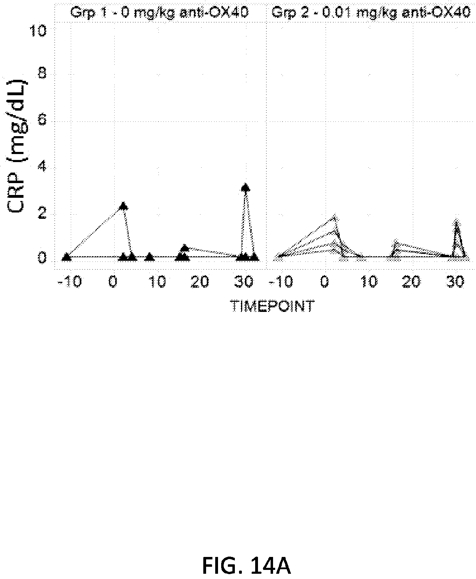

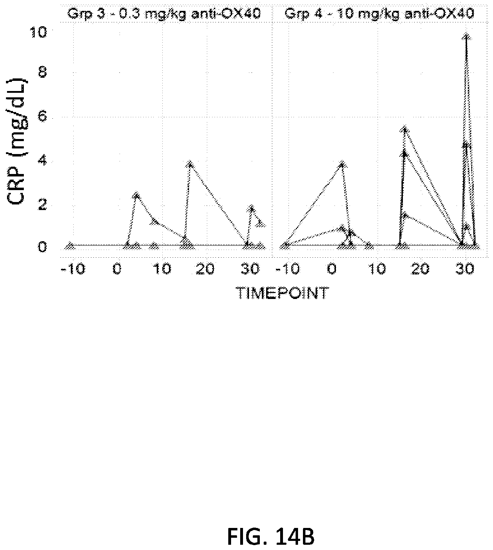

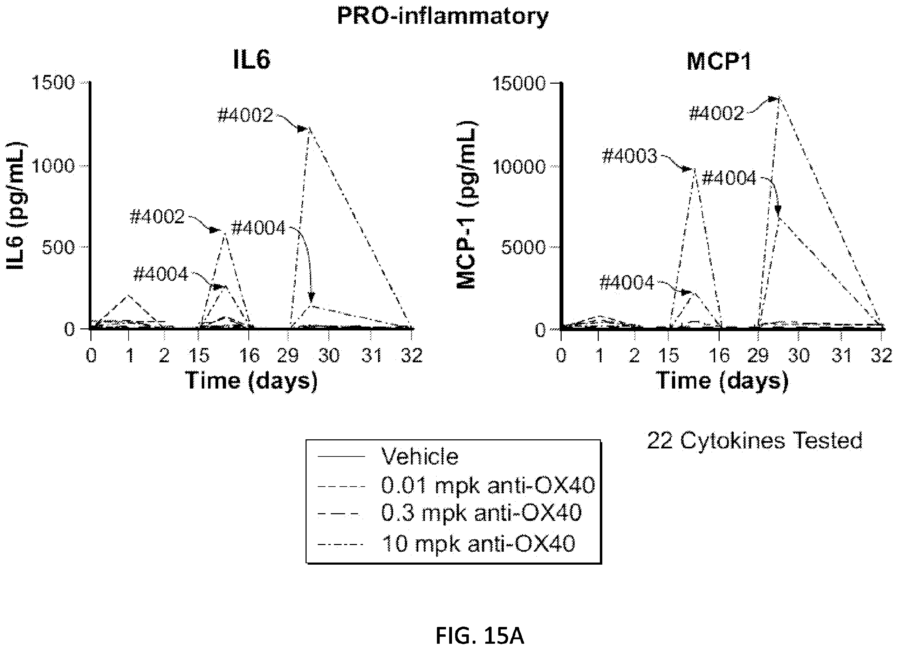

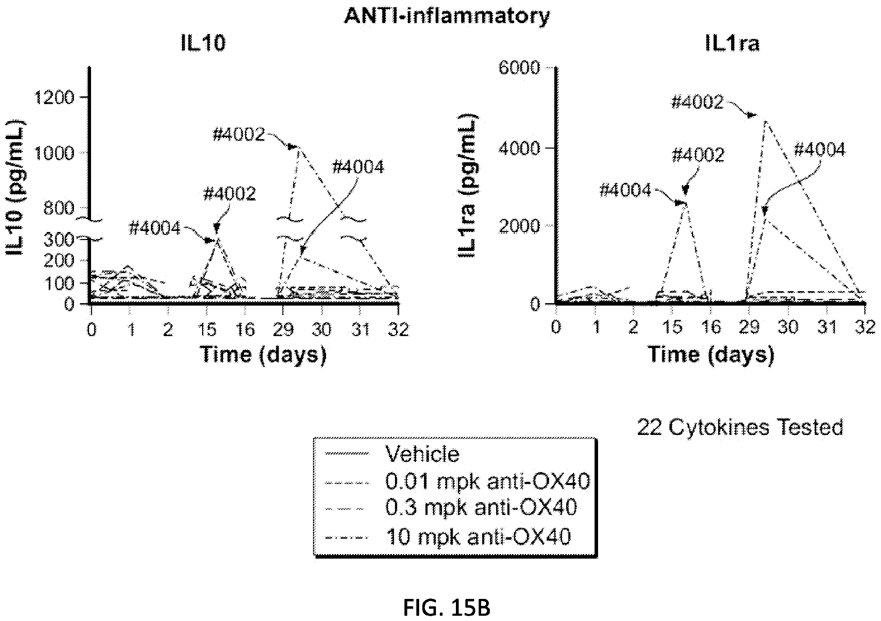

Intravenous administration of 1A7.gr1 IgG1 was well tolerated in cynomolgus monkeys up to 10mg/kg once every 2 weeks for 4 weeks, and all animals survived until the scheduled necropsy. Minimal increases in c-reactive protein (CRP) (**FIGS. 14A** and **B**) were observed, with transient increases in CRP limited to three ATA+ monkeys primarly in the high dose group (10mg/kg). Similarly, transient increases in a mixed subset of cytokines (**FIGS. 15A** and **B**) were limited to the three ATA+ monkeys primarily in the high dose (10mg/kg) group. No clinical pathology changes or cytokine elevations were observed in the remaining ATA+ or ATA- animals. No IFNγ increase was observed, as was expected in the absence of antigen challenge.

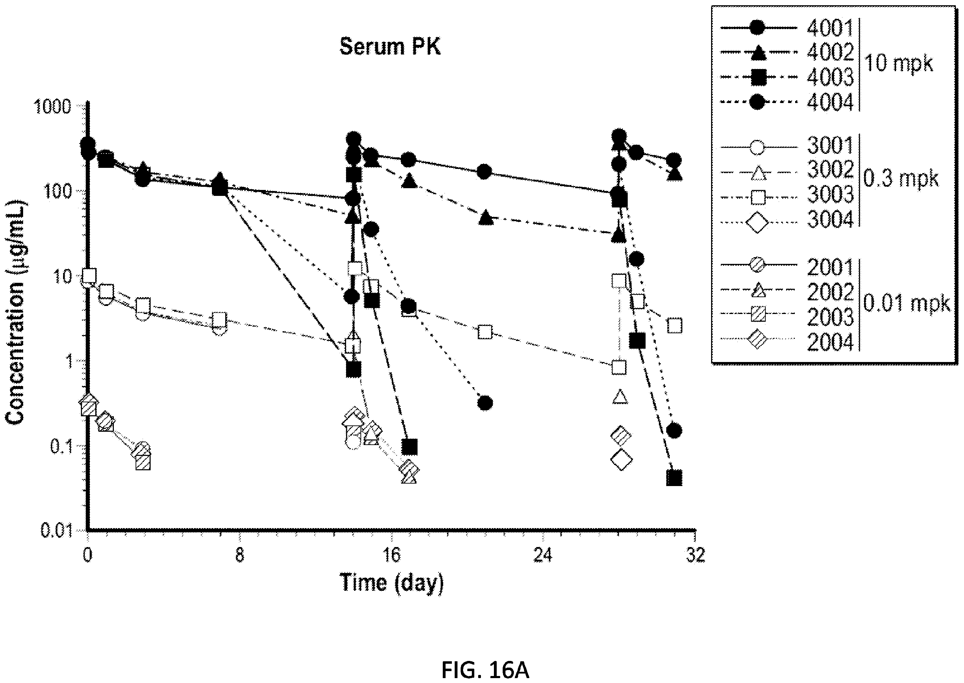

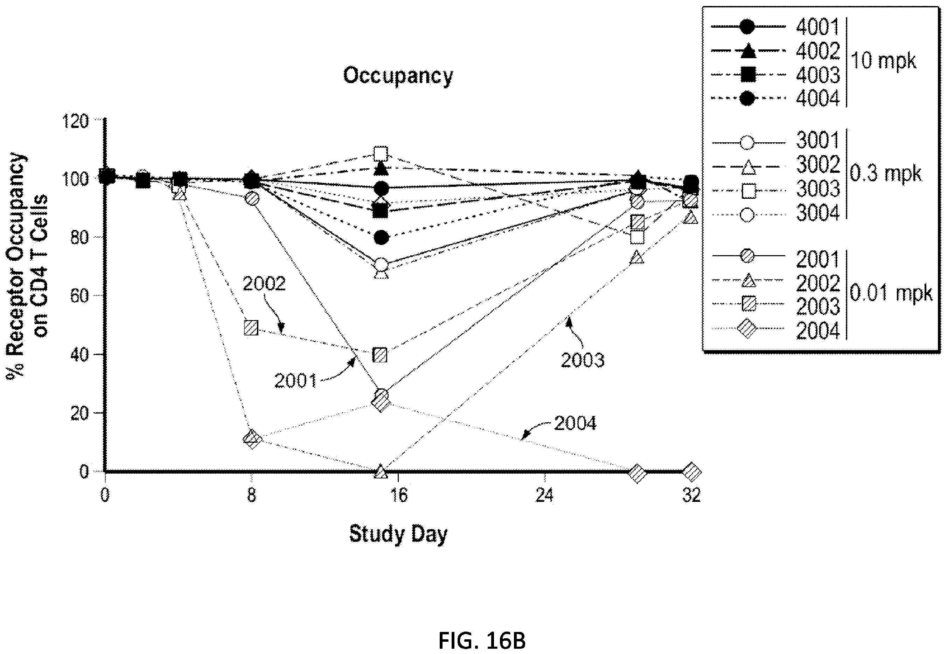

Exposure was confirmed in all animals by measurements of serum PK (**FIG. 16A**) and peripheral receptor occupancy (**FIG. 16B**)**.** The first cycle Cₘₐₓ dose was proportional for all three groups. PK was as expected for an IgG1 antibody based on AUC₀₋₁₄ at 10mg/kg. A high frequency of ATAs was observed: 3 out of 4 animals in 0.01 and 0.3mg/kg groups; and 2 out of 4 animals in the 10mg/kg group. The impact was decreased serum concentration and receptor occupancy. These results demonstrate that IV administration of 1A7.gr1 was well-tolerated in cynomolgus monkeys at the indicated dosing schedule.

### GLP toxicity study

The objective of the GLP toxicity study was to determine the toxicity of 1A7.gr1 in cynomolgus monkeys dosed intravenously at 0, 0.5, 5, and 30mg/kg/dose on study days 1, 15, 29, and 43, followed by 42 days without dosing (recovery period).

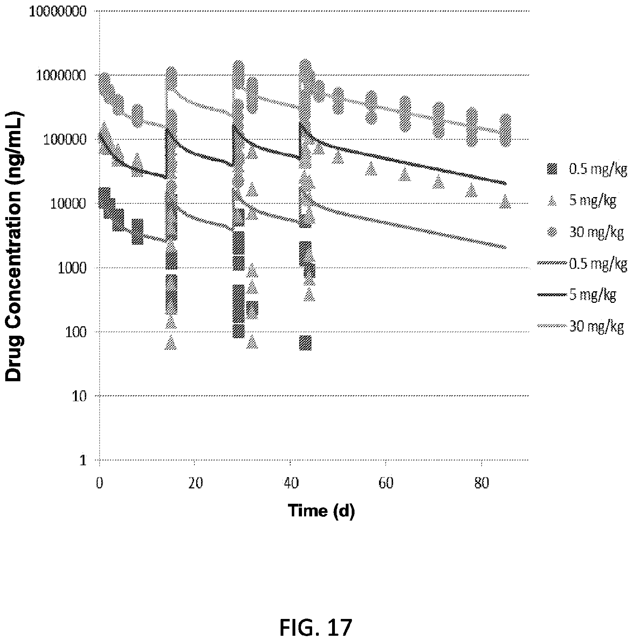

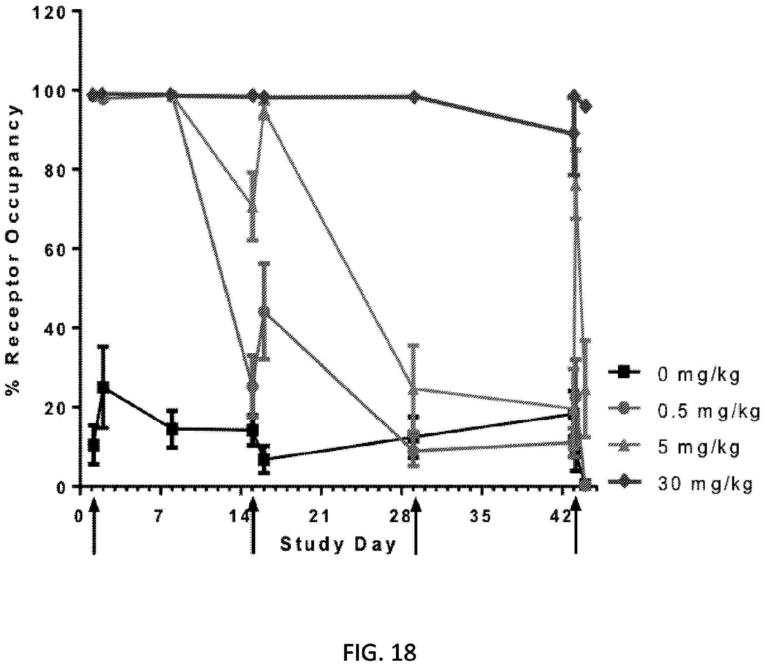

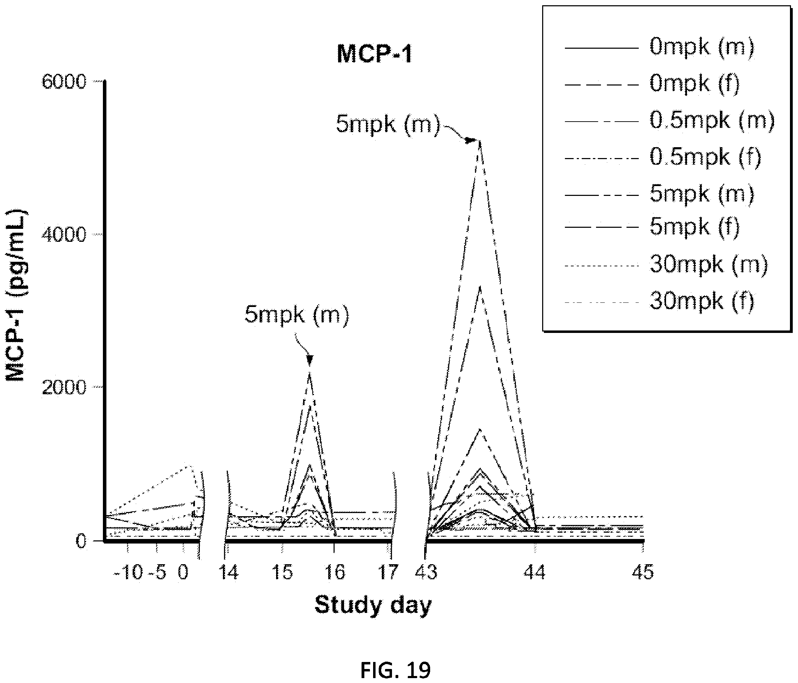

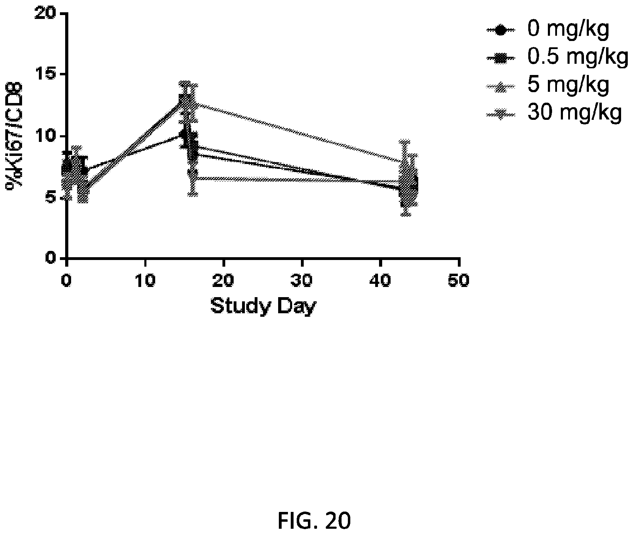

Intravenous administration of 1A7.gr1 IgG1 was well tolerated in cynomolgus monkeys up to 30mg/kg once every 2 weeks for 6 weeks, and all animals survived until their scheduled necropsy timepoints on Study Days 45 and 85. The expected, dose-proportional PK for an IgG1 antibody was observed (**FIG. 17**)**.** Exposure and receptor occupancy maintained at the 30mg/kg dose level (**FIG. 18**)**.** ATAs were detected in all animals in the 0.5 and 5mg/kg dose levels, but not the 30mg/kg dose level, with loss of exposure and receptor occupancy. Transient elevation of MCP-1 was observed in mid-dose animals (**FIG. 19**)**.** No significant activation or proliferation of peripheral T cells was observed (**FIG. 20**)**,** nor was any significant reduction in absolute peripheral T counts observed.

In summary, no clinical observations or changes in clinical pathology parameters after the first dose were observed. Acute post dose reactions, and transient increases in acute phase proteins and cytokines were observed beginning after 2^{nd} dose, potentially due to ATA (consistent in both studies). No drug-related organ weight changes, macroscopic, or microscopic findings were noted. ATAs were detected in all animals in the 0.5mg/kg and 5mg/kg (but not 30mg/kg) dose groups, with loss of exposure and receptor occupancy. 1A7.gr1 was found to bind human OX40 with high affinity (∼0.45nM), and PK was as expected for a typical IgG1 and dose-proportional. These project to a human CL=2.5mL/d/kg and t_{1/2}∼3 weeks. No significant PD effects were observed on T cell number or activation status in tissues or the periphery. Lack of significant activation or proliferation of peripheral T cells and lack of significant changes in absolute peripheral T cell counts was not surprising since OX40 is transiently expressed only on activated T cells and healthy cynos will have negligible activated T cells.

## Claims

1. An anti-human OX40 agonist antibody, wherein the antibody comprises a heavy chain variable domain (VH) comprising the amino acid sequence of SEQ ID NO:56 and a light chain variable domain (VL) comprising the amino acid sequence of SEQ ID NO:57.

2. The anti-human OX40 agonist antibody of claim 1, wherein the antibody depletes cells that express human OX40 *in vitro* and binds human OX40 with an affinity of equal to about 0.45 nM as determined using radioimmunoassay.

3. The anti-human OX40 agonist antibody of any one of claims 1 or 2, wherein the antibody depletes CD4+ effector T cells or regulatory T cells (Treg).

4. The anti-human OX40 agonist antibody of any one of claims 1 to 3, wherein the depleting is by ADCC and/or phagocytosis.

5. The anti-human OX40 agonist antibody of any one of claims 1 to 4, wherein the antibody binds human OX40 and cynomolgus OX40.

6. The anti-human OX40 agonist antibody of claim 5, wherein binding to cell surface-expressed OX40 on human and cynomolgus monkey T cells of labeled OX40 antibody is determined using a FACS assay and binding to human OX40 has an EC50 of less than or equal to 0.3 µg/ml and binding to cynomolgus OX40 has an EC50 of less than or equal to 1.5 µg/ml.

7. The anti-human OX40 agonist antibody of any one of claims 1 to 6, wherein antibody crosslinking is required for anti-human OX40 agonist antibody function.

8. The anti-human OX40 agonist antibody of any one of claims 1 to 7, wherein the antibody is a full length human IgG1 antibody.

9. An isolated nucleic acid encoding the antibody of any one of claims 1 to 8.

10. A host cell comprising the nucleic acid of claim 9.

11. A method of producing an antibody comprising culturing the host cell of claim 10 so that the antibody is produced.

12. The method of claim 11, further comprising recovering the antibody from the host cell.

13. An immunoconjugate comprising the antibody of any one of claims 1 to 8 and a cytotoxic agent.

14. A pharmaceutical formulation comprising the antibody of any one of claims 1 to 8 and a pharmaceutically acceptable carrier.

15. The pharmaceutical formulation of claim 14, comprising (a) the antibody of any one of claims 1 to 8 at a concentration between about 10 mg/mL and about 100 mg/mL, (b) a polysorbate, wherein the polysorbate concentration is about 0.02% w/v to about 0.06% w/v; (c) a histidine buffer at pH 5.0 to 6.0; and (d) a saccharide, wherein the saccharide concentration is about 120 mM to about 320 mM.

16. The antibody of any one of claims 1 to 8 for use as a medicament.

17. The antibody of any one of claims 1 to 8 for use in treating cancer.

18. The antibody of any one of claims 1 to 8 for use in a method of treating an individual having cancer comprising administering to the individual an effective amount of the antibody of any one of claims 1 to 8 and an additional therapeutic agent.

19. The antibody of any one of claims 1 to 8 for use in a method of claim 18, wherein the additional therapeutic agent comprises a chemotherapeutic agent.

20. The antibody of any one of claims 1 to 8 for use in a method of claim 18, wherein the additional therapeutic agent comprises a PD-1 axis binding antagonist.

## Patentansprüche

1. Antihuman-OX40-Agonist-Antikörper, wobei der Antikörper eine variable Domäne der schweren Kette (VH), umfassend die Aminosäuresequenz von SEQ ID NO:56, und eine variable Domäne der leichten Kette (VL), umfassend die Aminosäuresequenz von SEQ ID NO:57, umfasst.

2. Antihuman-OX40-Agonist-Antikörper nach Anspruch 1, wobei der Antikörper Zellen, die humanes OX40 exprimieren, *in vitro* depletiert und humanes OX40 mit einer Affinität gleich bis etwa 0,45 nM, bestimmt unter Verwendung eines Radioimmunassays, bindet.

3. Antihuman-OX40-Agonist-Antikörper nach einem der Ansprüche 1 oder 2, wobei der Antikörper CD4+ Effektor-T-Zellen oder regulatorische T-Zellen (Treg) depletiert.

4. Antihuman-OX40-Agonist-Antikörper nach einem der Ansprüche 1 bis 3, wobei die Depletion durch ADCC und/oder Phagozytose erfolgt.

5. Antihuman-OX40-Agonist-Antikörper nach einem der Ansprüche 1 bis 4, wobei der Antikörper humanes OX40 und Makaken-OX40 bindet.

6. Antihuman-OX40-Agonist-Antikörper nach Anspruch 5, wobei die Bindung an Zelloberflächen-exprimiertem OX40 an humanen und Makakenaffen-T-Zellen eines markierten OX40-Antikörpers unter Verwendung eines FACS-Assays bestimmt wird und die Bindung an humanes OX40 einen EC50 von weniger als oder gleich 0,3 µg/ml aufweist und die Bindung an Makaken-OX40 einen EC50 von weniger als oder gleich 1,5 µg/ml aufweist.

7. Antihuman-OX40-Agonist-Antikörper nach einem der Ansprüche 1 bis 6, wobei für die Antihuman-OX40-Agonist-Antikörperfunktion Antikörpervemetzung erforderlich ist.

8. Antihuman-OX40-Agonist-Antikörper nach einem der Ansprüche 1 bis 7, wobei der Antikörper ein humaner IgGl-Antikörper voller Länge ist.

9. Isolierte Nukleinsäure, die den Antikörper nach einem der Ansprüche 1 bis 8 codiert.

10. Wirtszelle, umfassend die Nukleinsäure nach Anspruch 9.

11. Verfahren zur Produktion eines Antikörpers, umfassend Kultivieren der Wirtszelle nach Anspruch 10, sodass der Antikörper produziert wird.

12. Verfahren nach Anspruch 11, ferner umfassend das Gewinnen des Antikörpers aus der Wirtszelle.

13. Immunkonjugat, umfassend den Antikörper nach einem der Ansprüche 1 bis 8 und ein zytotoxisches Mittel.

14. Pharmazeutische Formulierung, umfassend den Antikörper nach einem der Ansprüche 1 bis 8 und einen pharmazeutisch unbedenklichen Träger.

15. Pharmazeutische Formulierung nach Anspruch 14, umfassend (a) den Antikörper nach einem der Ansprüche 1 bis 8 in einer Konzentration zwischen etwa 10 mg/ml und etwa 100 mg/ml, (b) ein Polysorbat, wobei die Polysorbatkonzentration etwa 0,02 % (Gew./Vol.) bis etwa 0,06 % (Gew./Vol.) beträgt; (c) einen Histidinpuffer mit einem pH von 5,0 bis 6,0; und (d) ein Saccharid, wobei die Saccharidkonzentration etwa 120 mM bis etwa 320 mM beträgt.

16. Antikörper nach einem der Ansprüche 1 bis 8 zur Verwendung als ein Medikament.

17. Antikörper nach einem der Ansprüche 1 bis 8 zur Verwendung bei der Behandlung von Krebs.

18. Antikörper nach einem der Ansprüche 1 bis 8 zur Verwendung in einem Verfahren zur Behandlung eines Individuums mit Krebs, umfassend die Verabreichung einer wirksamen Menge des Antikörpers nach einem der Ansprüche 1 bis 8 und eines zusätzlichen Therapeutikums an das Individuum.

19. Antikörper nach einem der Ansprüche 1 bis 8 zur Verwendung in einem Verfahren nach Anspruch 18, wobei das zusätzliche Therapeutikum ein Chemotherapeutikum umfasst.

20. Antikörper nach einem der Ansprüche 1 bis 8 zur Verwendung in einem Verfahren nach Anspruch 18, wobei das zusätzliche Therapeutikum einen PD-1-Achsen-bindenden Antagonisten umfasst.

## Revendications

1. Anticorps agoniste anti-OX40 humain, dans lequel l'anticorps comprend un domaine variable de chaîne lourde (VH) comprenant la séquence d'acides aminés de SEQ ID NO : 56 et un domaine variable de chaîne légère (VL) comprenant la séquence d'acides aminés de SEQ ID NO : 57.

2. Anticorps agoniste anti-OX40 humain selon la revendication 1, dans lequel l'anticorps appauvrit les cellules qui expriment l'OX40 humain *in vitro* et se lie à l'OX40 humain avec une affinité égale à environ 0,45 nM comme déterminé par un dosage radio-immunologique.

3. Anticorps agoniste anti-OX40 humain selon l'une quelconque des revendications 1 ou 2, dans lequel l'anticorps appauvrit les cellules T effectrices CD4+ ou les cellules T régulatrices (Treg).

4. Anticorps agoniste anti-OX40 humain selon l'une quelconque des revendications 1 à 3, dans lequel l'appauvrissement est par ADCC et/ou phagocytose.

5. Anticorps agoniste anti-OX40 humain selon l'une quelconque des revendications 1 à 4, dans lequel l'anticorps se lie à l'OX40 humain et à l'OX40 de cynomolgus.

6. Anticorps agoniste anti-OX40 humain selon la revendication 5, dans lequel la liaison à l'OX40 exprimé sur la surface cellulaire sur les cellules T humaines et de singes cynomolgus de l'anticorps OX40 marqué est déterminée en utilisant un dosage FACS et la liaison à l'OX40 humain a une CE50 inférieure ou égale à 0,3 µg/ml et la liaison à l'OX40 de cynomolgus a une CE50 inférieure ou égale à 1,5 µg/ml.

7. Anticorps agoniste anti-OX40 humain selon l'une quelconque des revendications 1 à 6, dans lequel une réticulation de l'anticorps est nécessaire pour la fonction anticorps agoniste anti-OX40 humain.

8. Anticorps agoniste anti-OX40 humain selon l'une quelconque des revendications 1 à 7, dans lequel l'anticorps est un anticorps IgG1 humain pleine longueur.

9. Acide nucléique isolé codant pour l'anticorps selon l'une quelconque des revendications 1 à 8.

10. Cellule hôte comprenant l'acide nucléique selon la revendication 9.

11. Procédé de production d'un anticorps comprenant la culture de la cellule hôte selon la revendication 10 de sorte que l'anticorps est produit.

12. Procédé selon la revendication 11, comprenant en outre la récupération de l'anticorps de la cellule hôte.

13. Immunoconjugué comprenant l'anticorps selon l'une quelconque des revendications 1 à 8 et un agent cytotoxique.

14. Formulation pharmaceutique comprenant l'anticorps selon l'une quelconque des revendications 1 à 8 et un véhicule pharmaceutiquement acceptable.

15. Formulation pharmaceutique selon la revendication 14, comprenant (a) l'anticorps selon l'une quelconque des revendications 1 à 8 à une concentration comprise entre environ 10 mg/mL et environ 100 mg/mL, (b) un polysorbate, dans laquelle la concentration en polysorbate est d'environ 0,02 % p/v à environ 0,06 % p/v ; (c) un tampon d'histidine à un pH de 5,0 à 6,0 ; et (d) un saccharide, dans laquelle la concentration en saccharide est d'environ 120 mM à environ 320 mM.

16. Anticorps selon l'une quelconque des revendications 1 à 8 pour une utilisation comme médicament.

17. Anticorps selon l'une quelconque des revendications 1 à 8 pour une utilisation dans le traitement d'un cancer.

18. Anticorps selon l'une quelconque des revendications 1 à 8 pour une utilisation dans une méthode de traitement d'un individu atteint d'un cancer comprenant l'administration à l'individu d'une quantité efficace de l'anticorps selon l'une quelconque des revendications 1 à 8 et d'un agent thérapeutique supplémentaire.

19. Anticorps selon l'une quelconque des revendications 1 à 8 pour une utilisation dans une méthode selon la revendication 18, dans lequel l'agent thérapeutique supplémentaire comprend un agent chimiothérapeutique.

20. Anticorps selon l'une quelconque des revendications 1 à 8 pour utilisation dans une méthode selon la revendication 18, dans lequel l'agent thérapeutique supplémentaire comprend un antagoniste se liant à l'axe PD-1.
